# EUROPEAN PATENT APPLICATION

(11) **EP 3 978 611 A1**
(43) Date of publication of application: **06.04.2022**
(21) Application number: 20813406.4
(22) Date of filing: 29.05.2020
(51) Int. Cl.: C12N 15/62

(54) **CHIMERIC ANTIGEN RECEPTOR-EXPRESSING CELLS TARGETING ALK**

(30) Priority: 31.05.2019 JP 2019103074
(71) Applicant: Shinshu University, Matsumoto-shi, Nagano 390-8621 (JP)
(72) Inventor: NAKAZAWA, Yozo, Matsumoto-shi, Nagano 390-8621 (JP); SAITO, Shoji, Matsumoto-shi, Nagano 390-8621 (JP); YAGYU, Shigeki, Kyoto-shi, Kyoto 602-8566 (JP); NAKANO, Shigeru, Azumino-shi, Nagano 399-8304 (JP); MOMOSE, Takaki, Azumino-shi, Nagano 399-8304 (JP); HITOMI, Kenta, Azumino-shi, Nagano 399-8304 (JP)
(74) Representative: Vossius & Partner Patentanwälte Rechtsanwälte mbB
(86) International application number: PCT/JP2020/021348
(87) International publication number: WO 2020/241827

(57) **Abstract**

The present invention is intended to develop a chimeric antigen receptor (CAR) that is effective against solid tumor expressing anaplastic lymphoma kinase (ALK). The present invention provides a polynucleotide encoding a CAR protein comprising a target binding domain binding to an extracellular ligand binding region of ALK, a transmembrane domain, and an intracellular signaling domain. The target binding domain of the polynucleotide is selected from among FAM150A, FAM150B, and fragments thereof binding to the extracellular ligand binding region of ALK. The present invention also provides a genetically modified cell comprising the polynucleotide introduced thereinto.

SEQUENCE LISTING

<110>  KISSEI PHARMACEUTICAL CO., LTD
       SHINSHU UNIVERSITY
       Kyoto Prefectural Public University Corporation

<120>  Chimeric Antigen Receptor Expressing Cells Targeting ALK

<130>  PH-8340-PCT

<150>  2019-103074
<151>  2019-05-31

<160>  195

<170>  PatentIn version 3.5

<210>  1
<211>  387
<212>  DNA
<213>  Homo sapiens

<400>  1
atgcggcccc ttaagcccgg cgcccctttg cccgcactct tcctgctggc gctggctttg      60

tccccgcacg gagcccacgg gaggccccgg gggcgcaggg gagcgcgcgt cacggataag     120

gagcccaagc cgttgctttt cctccccgcg gccggggccg gccggactcc cagcggctcc     180

cggagcgcag aaatattccc aagagactct aacttaaaag acaaattcat aaagcatttc     240

acagggccgg tcacattttc accagaatgc agcaaacatt tccaccgact ctattacaat     300

accagggagt gctcaacgcc agcttattac aaaagatgtg ctagattgtt aacaagatta     360

gcagtgagtc cactgtgctc ccagacc                                         387


<210>  2
<211>  515
<212>  DNA
<213>  Artificial

<220>
<223>  XhoI-leader-FAM150A-Hinge-DraIII

<400>  2
ctcgagctca agcttcgaat tcgccaccat ggagttcgga ctgtcctggc tgtttctggt      60

ggccatactg aaaggggttc agtgcatgag gccactgaaa ccgggagcac ctcttcctgc     120

cctgtttctg ctcgccttgg cactgtctcc tcatggcgct catggcagac cacgcgggag     180

aaggggtgct agagtcacgg acaaagaacc caaacccctg ctgtttcttc cagctgcagg     240

ggctggacga actccctcag gcagtcggtc agccgagatt ttccctcggg atagcaatct     300

caaggacaag ttcatcaagc acttcaccgg tcctgtgacc ttctctcccg agtgtagcaa     360

gcactttcat cgcctgtatt acaacacacg tgaatgctcc acaccagcct actataagcg     420

gtgtgcgagg ctcctcactc gcttggccgt aagtccgctt tgcagccaaa ccgaacccaa     480

gtcctgcgat aaaacccaca catgtccacc gtgtg 515

<210> 3
<211> 456
<212> DNA
<213> Homo sapiens

<400> 3
atgcgcggac ccgggcaccc cctcctcctg gggctgctgc tggtgctggg ggcggcgggg 60

cgcggccggg ggggcgcgga gccccgggag ccggcggacg gacaggcgct gctgcggctg 120

gtggtggaac tcgtccagga gctgcggaag caccactcgg cggagcacaa gggcctgcag 180

ctcctcgggc gggactgcgc cctgggccgc gcggaggcgg cggggctggg gccttcgccg 240

gagcagcgag tggaaattgt tcctcgagat ctgaggatga aggacaagtt tctaaaacac 300

cttacaggcc ctctttattt tagtccaaag tgcagcaaac acttccatag actttatcac 360

aacaccagag actgcaccat tcctgcatac tataaaagat gcgccaggct tcttacccgg 420

ctggctgtca gtccagtgtg catggaggat aagcag 456

<210> 4
<211> 584
<212> DNA
<213> Artificial

<220>
<223> XhoI-leader-FAM150B-Hinge-DraIII

<400> 4
ctcgagctca agcttcgaat tcgccaccat ggagtttggg ctttcctggt tgttcctcgt 60

agccatcctc aaaggggttc agtgcatgag agggccagga catcctctgc ttctgggcct 120

gctgctggtg ttgggcgctg ctggcagagg tcgtggcgga gcagagccta gagagccagc 180

cgatgggcaa gcccttctgc ggcttgtggt cgaactcgtg caggaactgc ggaaacacca 240

ttccgctgaa cacaaaggcc tgcaactgct cggaagagac tgtgccttgg gaagggcaga 300

agcggctggt ctgggtccct caccagaaca gcgagtcgag attgtgcctc gcgatctccg 360

catgaaggac aagttcctga agcatctgac aggcccgctg tacttcagtc ccaagtgtag 420

caaacacttt catcgcctgt atcacaacac acgggattgc accatacccg cctattacaa 480

gaggtgtgca aggctgctga ctcgattggc cgtttctcct gtgtgcatgg aggacaaaca 540

ggagcccaag agctgcgaca agacgcacac ctgtccaccg tgtg 584

<210> 5
<211> 271
<212> PRT
<213> Artificial

<220>
<223> Humanized ALK48 scFv

<400>  5

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Val Lys Lys Pro Gly Ser Ser Val Lys Val Ser Cys Lys Ala
        35                  40                  45

Ser Gly Tyr Ala Phe Ser Ser Tyr Ile Ser Trp Val Arg Gln Ala Pro
    50                  55                  60

Gly Gln Gly Leu Glu Trp Met Gly Gly Gln Ile Tyr Pro Gly Asp Gly
65                  70                  75                  80

Asp Thr Asn Tyr Ala Gln Lys Phe Gln Gly Arg Val Thr Ile Thr Ala
                85                  90                  95

Asp Glu Ser Thr Ser Thr Ala Tyr Met Glu Leu Ser Ser Leu Arg Ser
            100                 105                 110

Glu Asp Thr Ala Val Tyr Tyr Cys Val Arg Tyr Tyr Tyr Gly Ser Ser
        115                 120                 125

Gly Tyr Phe Asp Tyr Trp Trp Gly Gln Gly Thr Met Val Thr Val Ser
    130                 135                 140

Ser Gly Gly Gly Gly Ser Gly Gly Gly Gly Ser Gly Gly Gly Gly Ser
145                 150                 155                 160

Glu Ile Val Leu Thr Gln Ser Pro Ala Thr Leu Ser Leu Ser Pro Gly
                165                 170                 175

Glu Arg Ala Thr Leu Ser Cys Arg Ala Ser Glu Ser Val Asp Asn Tyr
            180                 185                 190

Gly Ile Ser Phe Ala Trp Tyr Gln Gln Lys Pro Gly Gln Ala Pro Arg
        195                 200                 205

Leu Leu Ile Tyr Arg Ala Ser Arg Ala Thr Gly Ile Pro Ala Arg Phe
    210                 215                 220

Ser Gly Ser Gly Ser Gly Thr Asp Phe Thr Leu Thr Ile Ser Ser Leu
225                 230                 235                 240

Glu Pro Glu Asp Phe Ala Val Tyr Tyr Cys Gln Gln Asn Asn Lys Asp
                245                 250                 255

Pro Pro Thr Phe Phe Gly Gln Gly Thr Lys Leu Glu Ile Lys Arg
            260                 265                 270

<210>  6
<211>  270
<212>  PRT
<213>  Artificial

<220>
<223>  Murine ALK48 scFv

<400>  6

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Leu Val Lys Pro Gly Ala Ser Val Lys Ile Ser Cys Lys Ala
        35                  40                  45

Ser Gly Tyr Ala Phe Ser Ser Tyr Trp Met Asn Trp Val Lys Gln Arg
    50                  55                  60

Pro Gly Lys Gly Leu Glu Trp Ile Gly Gln Ile Tyr Pro Gly Asp Gly
65                  70                  75                  80

Asp Thr Thr Tyr Asn Gly Lys Phe Lys Gly Lys Ala Thr Leu Thr Ala
                85                  90                  95

Asp Lys Ser Ser Ser Thr Val Tyr Met Gln Leu Asn Ser Leu Thr Ser
            100                 105                 110

Glu Asp Ser Ala Val Tyr Phe Cys Val Arg Tyr Tyr Tyr Gly Ser Ser
        115                 120                 125

Gly Tyr Phe Asp Tyr Trp Gly Gln Gly Thr Thr Leu Thr Val Ser Ser
    130                 135                 140

Gly Gly Gly Gly Ser Gly Gly Gly Gly Ser Gly Gly Gly Gly Ser Asp
145                 150                 155                 160

Val Gln Met Ile Gln Thr Pro Asp Ser Leu Ala Val Ser Leu Gly Gln
                165                 170                 175

Arg Ala Thr Ile Ser Cys Arg Ala Ser Glu Ser Val Asp Asn Tyr Gly

180                 185                 190

Ile Ser Phe Met His Trp Tyr Gln Gln Lys Pro Gly Gln Ser Pro Lys
        195                 200                 205

Leu Leu Ile Tyr Arg Ala Ser Asn Leu Glu Ser Gly Ile Pro Ala Arg
    210                 215                 220

Phe Ser Gly Ser Gly Ser Arg Thr Asp Phe Thr Leu Thr Ile Asn Pro
225                 230                 235                 240

Val Glu Thr Asp Asp Val Ala Thr Tyr Tyr Cys Gln Gln Asn Asn Lys
                245                 250                 255

Asp Pro Pro Thr Phe Gly Gly Gly Thr Lys Leu Glu Ile Lys
            260                 265                 270

<210>  7
<211>  884
<212>  DNA
<213>  Artificial

<220>
<223>  XhoI-leader-humanized ALK48 scFv-Hinge-DraIII

<400>  7
ctcgagctca agcttcgaat tcgccaccat ggtgctgctg gttacctccc tccttctctg      60

cgagttgccg catcccgctt ttctcctgat tcccgacaca caggtccagc tccagcaatc     120

tggggcagaa gtgaagaaac ccgggtcaag tgtgaaagtc tcctgtaaag caagtggcta     180

tgcctttagc agctacattt cctgggttcg acaggcacct ggccaaggat tggaatggat     240

gggtggacag atctatcctg gggatggaga caccaattac gcccagaagt ttcagggtcg     300

cgtcacaata acggcagatg agtccactag cactgcctac atggagctga gctctcttcg     360

ctcagaggat acagccgtgt actattgcgt acggtactac tacggctctt ccggctattt     420

cgactattgg tggggacagg gaaccatggt aaccgtgagt tccggcggtg gaggaagcgg     480

aggcggcggg tcaggaggcg gtgggtctga gatcgttctg actcaatctc cagctaccct     540

gtctctgagc ccaggcgaac gtgctacctt gtcatgcaga gctagcgagt ctgtggataa     600

ctacggtata tcctttgcgt ggtatcagca gaaacctggg caagccccaa ggctgctgat     660

ctatcgggcc agtagggcta ctgggattcc ggccagattt agcgggagcg gcagtggtac     720

agacttcacg ctgaccatct caagccttga acccgaagat ttcgcggtgt attactgcca     780

gcagaacaat aaggacccac ctaccttctt cggccaaggc actaaactgg agatcaagag     840

agagcccaag tcctgtgaca agacacacac atgtccaccg tgtg                      884

<210>  8
<211>  881
<212>  DNA
<213>  Artificial

<220>
<223>  XhoI-leader-murine ALK48 scFv-Hinge-DraIII

<400>  8
ctcgagctca agcttcgaat tcgccaccat ggtcctgctc gtcacgtcac tgcttctgtg      60

cgagttgcct catcctgcct tcctgctgat tcccgataca caggtgcaac ttcagcagtc     120

aggcgcagaa ctggtcaaac caggtgctag cgtgaagatc tcctgcaagg ctagtgggta     180

cgcgttttcc tcctactgga tgaattgggt gaagcaacga ccaggcaagg ggttggagtg     240

gataggtcag atttatccag gcgatgggga tacaacatac aatgggaagt tcaagggaaa     300

ggccacactt actgccgaca aatcctctag caccgtgtac atgcagctga actccctgac     360

gtccgaagat agcgccgttt acttctgcgt gagatactac tacggcagct cagggtattt     420

cgactattgg ggtcaaggaa ccactttgac cgtatcttct ggcggaggcg gcagtggtgg     480

aggtggctca ggaggcggag ggtcagacgt tcagatgata cagacacctg actctctggc     540

cgtttccctc gggcagagag ccaccatcag ttgtcgcgct tctgagtctg tggataacta     600

tgggatcagc ttcatgcact ggtatcagca gaaacctggc caaagtccca aactcctcat     660

ctatcgggct agcaatctgg aaagcggcat tccggcacgt tttagtggca gcggaagcag     720

gaccgacttt accctgacca ttaatccggt ggaaactgac gatgtagcaa cctactattg     780

ccagcagaac aacaaggatc cacccacttt tggaggtgga acaaagctgg agatcaaaga     840

gcccaaaagc tgtgacaaaa cgcacacttg tccaccgtgt g                         881

<210>  9
<211>  1746
<212>  DNA
<213>  Artificial

<220>
<223>  Car 002 translation region

<400>  9
atggagttcg gactgtcctg gctgtttctg gtggccatac tgaaaggggt tcagtgcatg      60

aggccactga aaccgggagc acctcttcct gccctgtttc tgctcgcctt ggcactgtct     120

cctcatggcg ctcatggcag accacgcggg agaaggggtg ctagagtcac ggacaaagaa     180

cccaaacccc tgctgtttct tccagctgca ggggctggac gaactccctc aggcagtcgg     240

tcagccgaga ttttccctcg ggatagcaat ctcaaggaca agttcatcaa gcacttcacc     300

ggtcctgtga ccttctctcc cgagtgtagc aagcactttc atcgcctgta ttacaacaca     360

cgtgaatgct ccacaccagc ctactataag cggtgtgcga ggctcctcac tcgcttggcc     420

gtaagtccgc tttgcagcca aaccgaaccc aagtcctgcg ataaaaccca cacatgtcca 480

ccgtgtgatc ccgccgagcc caaatctcct gacaaaactc acacatgccc accgtgccca 540

gcacctgaac tcctgggggg accgtcagtc ttcctcttcc ccccaaaacc caaggacacc 600

ctcatgatct cccggacccc tgaggtcaca tgcgtggtgg tggacgtgag ccacgaagac 660

cctgaggtca agttcaactg gtacgtggac ggcgtggagg tgcataatgc caagacaaag 720

ccgcgggagg agcagtacaa cagcacgtac cgtgtggtca gcgtcctcac cgtcctgcac 780

caggactggc tgaatggcaa ggagtacaag tgcaaggtct ccaacaaagc cctcccagcc 840

cccatcgaga aaaccatctc caaagccaaa gggcagcccc gagaaccaca ggtgtacacc 900

ctgcccccat cccgggatga gctgaccaag aaccaggtca gcctgacctg cctggtcaaa 960

ggcttctatc ccagcgacat cgccgtggag tgggagagca atgggcaacc ggagaacaac 1020

tacaagacca cgcctcccgt gctggactcc gacggctcct tcttcctcta cagcaagctc 1080

accgtggaca agagcaggtg gcagcagggg aacgtcttct catgctccgt gatgcatgag 1140

gctctgcaca accactacac gcagaagagc ctctccctgt ctccgggtaa aaaagatccc 1200

aaattttggg tgctggtggt ggttggtgga gtcctggctt gctatagctt gctagtaaca 1260

gtggccttta ttattttctg ggtgaggagt aagaggagca ggctcctgca cagtgactac 1320

atgaacatga ctccccgccg ccccgggccc acccgcaagc attaccagcc ctatgcccca 1380

ccacgcgact tcgcagccta tcgctccaga gtgaagttca gcaggagcgc agacgccccc 1440

gcgtaccagc agggccagaa ccagctctat aacgagctca atctaggacg aagagaggag 1500

tacgatgttt tggacaagag acgtggccgg gaccctgaga tggggggaaa gccgagaagg 1560

aagaaccctc aggaaggcct gtacaatgaa ctgcagaaag ataagatggc ggaggcctac 1620

agtgagattg ggatgaaagg cgagcgccgg aggggcaagg ggcacgatgg cctttaccag 1680

ggtctcagta cagccaccaa ggacacctac gacgcccttc acatgcaggc cctgcctcct 1740

cgctaa 1746

<210> 10
<211> 1815
<212> DNA
<213> Artificial

<220>
<223> Car 003 translation region

<400> 10
atggagtttg ggctttcctg gttgttcctc gtagccatcc tcaaaggggt tcagtgcatg 60

agagggccag gacatcctct gcttctgggc ctgctgctgg tgttgggcgc tgctggcaga 120

ggtcgtggcg gagcagagcc tagagagcca gccgatgggc aagcccttct gcggcttgtg 180

gtcgaactcg tgcaggaact gcggaaacac cattccgctg aacacaaagg cctgcaactg 240

ctcggaagag actgtgcctt gggaagggca gaagcggctg gtctgggtcc ctcaccagaa 300

cagcgagtcg agattgtgcc tcgcgatctc cgcatgaagg acaagttcct gaagcatctg 360

acaggcccgc tgtacttcag tcccaagtgt agcaaacact ttcatcgcct gtatcacaac 420

acacgggatt gcaccatacc cgcctattac aagaggtgtg caaggctgct gactcgattg 480

gccgtttctc ctgtgtgcat ggaggacaaa caggagccca agagctgcga caagacgcac 540

acctgtccac cgtgtgatcc cgccgagccc aaatctcctg acaaaactca cacatgccca 600

ccgtgcccag cacctgaact cctgggggga ccgtcagtct tcctcttccc cccaaaaccc 660

aaggacaccc tcatgatctc ccggacccct gaggtcacat gcgtggtggt ggacgtgagc 720

cacgaagacc ctgaggtcaa gttcaactgg tacgtggacg gcgtggaggt gcataatgcc 780

aagacaaagc cgcgggagga gcagtacaac agcacgtacc gtgtggtcag cgtcctcacc 840

gtcctgcacc aggactggct gaatggcaag gagtacaagt gcaaggtctc caacaaagcc 900

ctcccagccc ccatcgagaa aaccatctcc aaagccaaag ggcagccccg agaaccacag 960

gtgtacaccc tgcccccatc ccgggatgag ctgaccaaga accaggtcag cctgacctgc 1020

ctggtcaaag gcttctatcc cagcgacatc gccgtggagt gggagagcaa tgggcaaccg 1080

gagaacaact acaagaccac gcctcccgtg ctggactccg acggctcctt cttcctctac 1140

agcaagctca ccgtggacaa gagcaggtgg cagcagggga acgtcttctc atgctccgtg 1200

atgcatgagg ctctgcacaa ccactacacg cagaagagcc tctccctgtc tccgggtaaa 1260

aaagatccca aattttgggt gctggtggtg gttggtggag tcctggcttg ctatagcttg 1320

ctagtaacag tggcctttat tattttctgg gtgaggagta agaggagcag gctcctgcac 1380

agtgactaca tgaacatgac tccccgccgc cccgggccca cccgcaagca ttaccagccc 1440

tatgccccac cacgcgactt cgcagcctat cgctccagag tgaagttcag caggagcgca 1500

gacgcccccg cgtaccagca gggccagaac cagctctata acgagctcaa tctaggacga 1560

agagaggagt acgatgtttt ggacaagaga cgtggccggg accctgagat ggggggaaag 1620

ccgagaagga agaaccctca ggaaggcctg tacaatgaac tgcagaaaga taagatggcg 1680

gaggcctaca gtgagattgg gatgaaaggc gagcgccgga ggggcaaggg gcacgatggc 1740

ctttaccagg gtctcagtac agccaccaag gacacctacg acgcccttca catgcaggcc 1800

ctgcctcctc gctaa 1815

<210> 11
<211> 2115
<212> DNA
<213> Artificial

<220>
<223> Car 004 translation region

<400> 11
atggtgctgc tggttacctc cctccttctc tgcgagttgc cgcatcccgc ttttctcctg 60
attcccgaca cacaggtcca gctccagcaa tctggggcag aagtgaagaa acccgggtca 120
agtgtgaaag tctcctgtaa agcaagtggc tatgccttta gcagctacat ttcctgggtt 180
cgacaggcac ctggccaagg attggaatgg atgggtggac agatctatcc tggggatgga 240
gacaccaatt acgcccagaa gtttcagggt cgcgtcacaa taacggcaga tgagtccact 300
agcactgcct acatggagct gagctctctt cgctcagagg atacagccgt gtactattgc 360
gtacggtact actacggctc ttccggctat ttcgactatt ggtggggaca gggaaccatg 420
gtaaccgtga gttccggcgg tggaggaagc ggaggcggcg ggtcaggagg cggtgggtct 480
gagatcgttc tgactcaatc tccagctacc ctgtctctga gcccaggcga acgtgctacc 540
ttgtcatgca gagctagcga gtctgtggat aactacggta tatcctttgc gtggtatcag 600
cagaaacctg ggcaagcccc aaggctgctg atctatcggg ccagtagggc tactgggatt 660
ccggccagat ttagcgggag cggcagtggt acagacttca cgctgaccat ctcaagcctt 720
gaacccgaag atttcgcggt gtattactgc cagcagaaca ataaggaccc acctaccttc 780
ttcggccaag gcactaaact ggagatcaag agagagccca agtcctgtga caagacacac 840
acatgtccac cgtgtgatcc cgccgagccc aaatctcctg acaaaactca cacatgccca 900
ccgtgcccag cacctgaact cctgggggga ccgtcagtct tcctcttccc cccaaaaccc 960
aaggacaccc tcatgatctc ccggacccct gaggtcacat gcgtggtggt ggacgtgagc 1020
cacgaagacc ctgaggtcaa gttcaactgg tacgtggacg gcgtggaggt gcataatgcc 1080
aagacaaagc cgcgggagga gcagtacaac agcacgtacc gtgtggtcag cgtcctcacc 1140
gtcctgcacc aggactggct gaatggcaag gagtacaagt gcaaggtctc caacaaagcc 1200
ctcccagccc ccatcgagaa aaccatctcc aaagccaaag ggcagccccg agaaccacag 1260
gtgtacaccc tgcccccatc ccgggatgag ctgaccaaga accaggtcag cctgacctgc 1320
ctggtcaaag gcttctatcc cagcgacatc gccgtggagt gggagagcaa tgggcaaccg 1380
gagaacaact acaagaccac gcctcccgtg ctggactccg acggctcctt cttcctctac 1440
agcaagctca ccgtggacaa gagcaggtgg cagcagggga acgtcttctc atgctccgtg 1500
atgcatgagg ctctgcacaa ccactacacg cagaagagcc tctccctgtc tccgggtaaa 1560
aaagatccca aattttgggt gctggtggtg gttggtggag tcctggcttg ctatagcttg 1620
ctagtaacag tggcctttat tattttctgg gtgaggagta agaggagcag gctcctgcac 1680
agtgactaca tgaacatgac tccccgccgc cccgggccca cccgcaagca ttaccagccc 1740
tatgccccac cacgcgactt cgcagcctat cgctccagag tgaagttcag caggagcgca 1800
gacgcccccg cgtaccagca gggccagaac cagctctata acgagctcaa tctaggacga 1860

agagaggagt acgatgtttt ggacaagaga cgtggccggg accctgagat ggggggaaag 1920

ccgagaagga agaaccctca ggaaggcctg tacaatgaac tgcagaaaga taagatggcg 1980

gaggcctaca gtgagattgg gatgaaaggc gagcgccgga ggggcaaggg gcacgatggc 2040

ctttaccagg gtctcagtac agccaccaag gacacctacg acgcccttca catgcaggcc 2100

ctgcctcctc gctaa 2115

<210> 12
<211> 2112
<212> DNA
<213> Artificial

<220>
<223> Car 005 translation region

<400> 12
atggtcctgc tcgtcacgtc actgcttctg tgcgagttgc ctcatcctgc cttcctgctg 60

attcccgata cacaggtgca acttcagcag tcaggcgcag aactggtcaa accaggtgct 120

agcgtgaaga tctcctgcaa ggctagtggg tacgcgtttt cctcctactg gatgaattgg 180

gtgaagcaac gaccaggcaa ggggttggag tggataggtc agatttatcc aggcgatggg 240

gatacaacat acaatgggaa gttcaaggga aaggccacac ttactgccga caaatcctct 300

agcaccgtgt acatgcagct gaactccctg acgtccgaag atagcgccgt ttacttctgc 360

gtgagatact actacggcag ctcagggtat ttcgactatt ggggtcaagg aaccactttg 420

accgtatctt ctggcggagg cggcagtggt ggaggtggct caggaggcgg agggtcagac 480

gttcagatga tacagacacc tgactctctg gccgtttccc tcgggcagag agccaccatc 540

agttgtcgcg cttctgagtc tgtggataac tatgggatca gcttcatgca ctggtatcag 600

cagaaacctg gccaaagtcc caaactcctc atctatcggg ctagcaatct ggaaagcggc 660

attccggcac gttttagtgg cagcggaagc aggaccgact ttaccctgac cattaatccg 720

gtggaaactg acgatgtagc aacctactat tgccagcaga acaacaagga tccacccact 780

tttggaggtg gaacaaagct ggagatcaaa gagcccaaaa gctgtgacaa aacgcacact 840

tgtccaccgt gtgatcccgc cgagcccaaa tctcctgaca aaactcacac atgcccaccg 900

tgcccagcac ctgaactcct ggggggaccg tcagtcttcc tcttcccccc aaaacccaag 960

gacaccctca tgatctcccg gacccctgag gtcacatgcg tggtggtgga cgtgagccac 1020

gaagaccctg aggtcaagtt caactggtac gtggacggcg tggaggtgca taatgccaag 1080

acaaagccgc gggaggagca gtacaacagc acgtaccgtg tggtcagcgt cctcaccgtc 1140

ctgcaccagg actggctgaa tggcaaggag tacaagtgca aggtctccaa caaagccctc 1200

ccagccccca tcgagaaaac catctccaaa gccaaagggc agccccgaga accacaggtg 1260

tacaccctgc ccccatcccg ggatgagctg accaagaacc aggtcagcct gacctgcctg 1320

gtcaaaggct tctatcccag cgacatcgcc gtggagtggg agagcaatgg gcaaccggag 1380

aacaactaca agaccacgcc tcccgtgctg gactccgacg gctccttctt cctctacagc 1440

aagctcaccg tggacaagag caggtggcag caggggaacg tcttctcatg ctccgtgatg 1500

catgaggctc tgcacaacca ctacacgcag aagagcctct ccctgtctcc gggtaaaaaa 1560

gatcccaaat tttgggtgct ggtggtggtt ggtggagtcc tggcttgcta tagcttgcta 1620

gtaacagtgg cctttattat tttctgggtg aggagtaaga ggagcaggct cctgcacagt 1680

gactacatga acatgactcc ccgccgcccc gggcccaccc gcaagcatta ccagccctat 1740

gccccaccac gcgacttcgc agcctatcgc tccagagtga agttcagcag gagcgcagac 1800

gcccccgcgt accagcaggg ccagaaccag ctctataacg agctcaatct aggacgaaga 1860

gaggagtacg atgttttgga caagagacgt ggccgggacc ctgagatggg gggaaagccg 1920

agaaggaaga accctcagga aggcctgtac aatgaactgc agaaagataa gatggcggag 1980

gcctacagtg agattgggat gaaaggcgag cgccggaggg gcaaggggca cgatggcctt 2040

taccagggtc tcagtacagc caccaaggac acctacgacg cccttcacat gcaggccctg 2100

cctcctcgct aa 2112

<210> 13
<211> 581
<212> PRT
<213> Artificial

<220>
<223> Car 002 translation region

<400> 13

Met Glu Phe Gly Leu Ser Trp Leu Phe Leu Val Ala Ile Leu Lys Gly
1               5                   10                  15

Val Gln Cys Met Arg Pro Leu Lys Pro Gly Ala Pro Leu Pro Ala Leu
            20                  25                  30

Phe Leu Leu Ala Leu Ala Leu Ser Pro His Gly Ala His Gly Arg Pro
        35                  40                  45

Arg Gly Arg Arg Gly Ala Arg Val Thr Asp Lys Glu Pro Lys Pro Leu
    50                  55                  60

Leu Phe Leu Pro Ala Ala Gly Ala Gly Arg Thr Pro Ser Gly Ser Arg
65                  70                  75                  80

Ser Ala Glu Ile Phe Pro Arg Asp Ser Asn Leu Lys Asp Lys Phe Ile
                85                  90                  95

Lys His Phe Thr Gly Pro Val Thr Phe Ser Pro Glu Cys Ser Lys His
        100                 105                 110

Phe His Arg Leu Tyr Tyr Asn Thr Arg Glu Cys Ser Thr Pro Ala Tyr
    115                 120                 125

Tyr Lys Arg Cys Ala Arg Leu Leu Thr Arg Leu Ala Val Ser Pro Leu
    130                 135                 140

Cys Ser Gln Thr Glu Pro Lys Ser Cys Asp Lys Thr His Thr Cys Pro
145                 150                 155                 160

Pro Cys Asp Pro Ala Glu Pro Lys Ser Pro Asp Lys Thr His Thr Cys
                165                 170                 175

Pro Pro Cys Pro Ala Pro Glu Leu Leu Gly Gly Pro Ser Val Phe Leu
        180                 185                 190

Phe Pro Pro Lys Pro Lys Asp Thr Leu Met Ile Ser Arg Thr Pro Glu
    195                 200                 205

Val Thr Cys Val Val Val Asp Val Ser His Glu Asp Pro Glu Val Lys
    210                 215                 220

Phe Asn Trp Tyr Val Asp Gly Val Glu Val His Asn Ala Lys Thr Lys
225                 230                 235                 240

Pro Arg Glu Glu Gln Tyr Asn Ser Thr Tyr Arg Val Val Ser Val Leu
                245                 250                 255

Thr Val Leu His Gln Asp Trp Leu Asn Gly Lys Glu Tyr Lys Cys Lys
        260                 265                 270

Val Ser Asn Lys Ala Leu Pro Ala Pro Ile Glu Lys Thr Ile Ser Lys
    275                 280                 285

Ala Lys Gly Gln Pro Arg Glu Pro Gln Val Tyr Thr Leu Pro Pro Ser
    290                 295                 300

Arg Asp Glu Leu Thr Lys Asn Gln Val Ser Leu Thr Cys Leu Val Lys
305                 310                 315                 320

Gly Phe Tyr Pro Ser Asp Ile Ala Val Glu Trp Glu Ser Asn Gly Gln
                325                 330                 335

Pro Glu Asn Asn Tyr Lys Thr Thr Pro Pro Val Leu Asp Ser Asp Gly

            340                 345                 350

Ser Phe Phe Leu Tyr Ser Lys Leu Thr Val Asp Lys Ser Arg Trp Gln
        355                 360                 365

Gln Gly Asn Val Phe Ser Cys Ser Val Met His Glu Ala Leu His Asn
    370                 375                 380

His Tyr Thr Gln Lys Ser Leu Ser Leu Ser Pro Gly Lys Lys Asp Pro
385                 390                 395                 400

Lys Phe Trp Val Leu Val Val Val Gly Gly Val Leu Ala Cys Tyr Ser
                405                 410                 415

Leu Leu Val Thr Val Ala Phe Ile Ile Phe Trp Val Arg Ser Lys Arg
            420                 425                 430

Ser Arg Leu Leu His Ser Asp Tyr Met Asn Met Thr Pro Arg Arg Pro
        435                 440                 445

Gly Pro Thr Arg Lys His Tyr Gln Pro Tyr Ala Pro Pro Arg Asp Phe
    450                 455                 460

Ala Ala Tyr Arg Ser Arg Val Lys Phe Ser Arg Ser Ala Asp Ala Pro
465                 470                 475                 480

Ala Tyr Gln Gln Gly Gln Asn Gln Leu Tyr Asn Glu Leu Asn Leu Gly
                485                 490                 495

Arg Arg Glu Glu Tyr Asp Val Leu Asp Lys Arg Arg Gly Arg Asp Pro
            500                 505                 510

Glu Met Gly Gly Lys Pro Arg Arg Lys Asn Pro Gln Glu Gly Leu Tyr
        515                 520                 525

Asn Glu Leu Gln Lys Asp Lys Met Ala Glu Ala Tyr Ser Glu Ile Gly
    530                 535                 540

Met Lys Gly Glu Arg Arg Arg Gly Lys Gly His Asp Gly Leu Tyr Gln
545                 550                 555                 560

Gly Leu Ser Thr Ala Thr Lys Asp Thr Tyr Asp Ala Leu His Met Gln
                565                 570                 575

Ala Leu Pro Pro Arg
            580

<210>  14
<211>  604
<212>  PRT
<213>  Artificial

<220>
<223>  Car 003 translation region

<400>  14

Met Glu Phe Gly Leu Ser Trp Leu Phe Leu Val Ala Ile Leu Lys Gly
1               5                   10                  15

Val Gln Cys Met Arg Gly Pro Gly His Pro Leu Leu Leu Gly Leu Leu
            20                  25                  30

Leu Val Leu Gly Ala Ala Gly Arg Gly Arg Gly Gly Ala Glu Pro Arg
        35                  40                  45

Glu Pro Ala Asp Gly Gln Ala Leu Leu Arg Leu Val Val Glu Leu Val
    50                  55                  60

Gln Glu Leu Arg Lys His His Ser Ala Glu His Lys Gly Leu Gln Leu
65                  70                  75                  80

Leu Gly Arg Asp Cys Ala Leu Gly Arg Ala Glu Ala Ala Gly Leu Gly
                85                  90                  95

Pro Ser Pro Glu Gln Arg Val Glu Ile Val Pro Arg Asp Leu Arg Met
            100                 105                 110

Lys Asp Lys Phe Leu Lys His Leu Thr Gly Pro Leu Tyr Phe Ser Pro
        115                 120                 125

Lys Cys Ser Lys His Phe His Arg Leu Tyr His Asn Thr Arg Asp Cys
    130                 135                 140

Thr Ile Pro Ala Tyr Tyr Lys Arg Cys Ala Arg Leu Leu Thr Arg Leu
145                 150                 155                 160

Ala Val Ser Pro Val Cys Met Glu Asp Lys Gln Glu Pro Lys Ser Cys
                165                 170                 175

Asp Lys Thr His Thr Cys Pro Pro Cys Asp Pro Ala Glu Pro Lys Ser
            180                 185                 190

Pro Asp Lys Thr His Thr Cys Pro Pro Cys Pro Ala Pro Glu Leu Leu
        195                 200                 205

Gly Gly Pro Ser Val Phe Leu Phe Pro Pro Lys Pro Lys Asp Thr Leu

210                 215                 220

Met Ile Ser Arg Thr Pro Glu Val Thr Cys Val Val Val Asp Val Ser
225                 230                 235                 240

His Glu Asp Pro Glu Val Lys Phe Asn Trp Tyr Val Asp Gly Val Glu
                245                 250                 255

Val His Asn Ala Lys Thr Lys Pro Arg Glu Glu Gln Tyr Asn Ser Thr
            260                 265                 270

Tyr Arg Val Val Ser Val Leu Thr Val Leu His Gln Asp Trp Leu Asn
        275                 280                 285

Gly Lys Glu Tyr Lys Cys Lys Val Ser Asn Lys Ala Leu Pro Ala Pro
    290                 295                 300

Ile Glu Lys Thr Ile Ser Lys Ala Lys Gly Gln Pro Arg Glu Pro Gln
305                 310                 315                 320

Val Tyr Thr Leu Pro Pro Ser Arg Asp Glu Leu Thr Lys Asn Gln Val
                325                 330                 335

Ser Leu Thr Cys Leu Val Lys Gly Phe Tyr Pro Ser Asp Ile Ala Val
            340                 345                 350

Glu Trp Glu Ser Asn Gly Gln Pro Glu Asn Asn Tyr Lys Thr Thr Pro
        355                 360                 365

Pro Val Leu Asp Ser Asp Gly Ser Phe Phe Leu Tyr Ser Lys Leu Thr
    370                 375                 380

Val Asp Lys Ser Arg Trp Gln Gln Gly Asn Val Phe Ser Cys Ser Val
385                 390                 395                 400

Met His Glu Ala Leu His Asn His Tyr Thr Gln Lys Ser Leu Ser Leu
                405                 410                 415

Ser Pro Gly Lys Lys Asp Pro Lys Phe Trp Val Leu Val Val Val Gly
            420                 425                 430

Gly Val Leu Ala Cys Tyr Ser Leu Leu Val Thr Val Ala Phe Ile Ile
        435                 440                 445

Phe Trp Val Arg Ser Lys Arg Ser Arg Leu Leu His Ser Asp Tyr Met
    450                 455                 460

Asn Met Thr Pro Arg Arg Pro Gly Pro Thr Arg Lys His Tyr Gln Pro
465                 470                 475                 480

Tyr Ala Pro Pro Arg Asp Phe Ala Ala Tyr Arg Ser Arg Val Lys Phe
                485                 490                 495

Ser Arg Ser Ala Asp Ala Pro Ala Tyr Gln Gln Gly Gln Asn Gln Leu
            500                 505                 510

Tyr Asn Glu Leu Asn Leu Gly Arg Arg Glu Glu Tyr Asp Val Leu Asp
        515                 520                 525

Lys Arg Arg Gly Arg Asp Pro Glu Met Gly Gly Lys Pro Arg Arg Lys
    530                 535                 540

Asn Pro Gln Glu Gly Leu Tyr Asn Glu Leu Gln Lys Asp Lys Met Ala
545                 550                 555                 560

Glu Ala Tyr Ser Glu Ile Gly Met Lys Gly Glu Arg Arg Arg Gly Lys
                565                 570                 575

Gly His Asp Gly Leu Tyr Gln Gly Leu Ser Thr Ala Thr Lys Asp Thr
            580                 585                 590

Tyr Asp Ala Leu His Met Gln Ala Leu Pro Pro Arg
        595                 600

<210>  15
<211>  746
<212>  PRT
<213>  Artificial

<220>
<223>  Car 004 translation region

<400>  15

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Val Lys Lys Pro Gly Ser Ser Val Lys Val Ser Cys Lys Ala
        35                  40                  45

Ser Gly Tyr Ala Phe Ser Ser Tyr Ile Ser Trp Val Arg Gln Ala Pro
    50                  55                  60

Gly Gln Gly Leu Glu Trp Met Gly Gly Gln Ile Tyr Pro Gly Asp Gly

65                  70                  75                  80

Asp Thr Asn Tyr Ala Gln Lys Phe Gln Gly Arg Val Thr Ile Thr Ala
                85                  90                  95

Asp Glu Ser Thr Ser Thr Ala Tyr Met Glu Leu Ser Ser Leu Arg Ser
            100                 105                 110

Glu Asp Thr Ala Val Tyr Tyr Cys Val Arg Tyr Tyr Tyr Gly Ser Ser
        115                 120                 125

Gly Tyr Phe Asp Tyr Trp Trp Gly Gln Gly Thr Met Val Thr Val Ser
    130                 135                 140

Ser Gly Gly Gly Gly Ser Gly Gly Gly Gly Ser Gly Gly Gly Gly Ser
145                 150                 155                 160

Glu Ile Val Leu Thr Gln Ser Pro Ala Thr Leu Ser Leu Ser Pro Gly
                165                 170                 175

Glu Arg Ala Thr Leu Ser Cys Arg Ala Ser Glu Ser Val Asp Asn Tyr
            180                 185                 190

Gly Ile Ser Phe Ala Trp Tyr Gln Gln Lys Pro Gly Gln Ala Pro Arg
        195                 200                 205

Leu Leu Ile Tyr Arg Ala Ser Arg Ala Thr Gly Ile Pro Ala Arg Phe
    210                 215                 220

Ser Gly Ser Gly Ser Gly Thr Asp Phe Thr Leu Thr Ile Ser Ser Leu
225                 230                 235                 240

Glu Pro Glu Asp Phe Ala Val Tyr Tyr Cys Gln Gln Asn Asn Lys Asp
                245                 250                 255

Pro Pro Thr Phe Phe Gly Gln Gly Thr Lys Leu Glu Ile Lys Arg Glu
            260                 265                 270

Pro Lys Ser Cys Asp Lys Thr His Thr Cys Pro Pro Cys Asp Pro Ala
        275                 280                 285

Glu Pro Lys Ser Pro Asp Lys Thr His Thr Cys Pro Pro Cys Pro Ala
    290                 295                 300

Pro Glu Leu Leu Gly Gly Pro Ser Val Phe Leu Phe Pro Pro Lys Pro
305                 310                 315                 320

Lys Asp Thr Leu Met Ile Ser Arg Thr Pro Glu Val Thr Cys Val Val
                325                 330                 335

Val Asp Val Ser His Glu Asp Pro Glu Val Lys Phe Asn Trp Tyr Val
            340                 345                 350

Asp Gly Val Glu Val His Asn Ala Lys Thr Lys Pro Arg Glu Glu Gln
        355                 360                 365

Tyr Asn Ser Thr Tyr Arg Val Val Ser Val Leu Thr Val Leu His Gln
    370                 375                 380

Asp Trp Leu Asn Gly Lys Glu Tyr Lys Cys Lys Val Ser Asn Lys Ala
385                 390                 395                 400

Leu Pro Ala Pro Ile Glu Lys Thr Ile Ser Lys Ala Lys Gly Gln Pro
                405                 410                 415

Arg Glu Pro Gln Val Tyr Thr Leu Pro Pro Ser Arg Asp Glu Leu Thr
            420                 425                 430

Lys Asn Gln Val Ser Leu Thr Cys Leu Val Lys Gly Phe Tyr Pro Ser
        435                 440                 445

Asp Ile Ala Val Glu Trp Glu Ser Asn Gly Gln Pro Glu Asn Asn Tyr
    450                 455                 460

Lys Thr Thr Pro Pro Val Leu Asp Ser Asp Gly Ser Phe Phe Leu Tyr
465                 470                 475                 480

Ser Lys Leu Thr Val Asp Lys Ser Arg Trp Gln Gln Gly Asn Val Phe
                485                 490                 495

Ser Cys Ser Val Met His Glu Ala Leu His Asn His Tyr Thr Gln Lys
            500                 505                 510

Ser Leu Ser Leu Ser Pro Gly Lys Ala Ala Ala Thr Thr Thr Pro Ala
        515                 520                 525

Pro Arg Pro Pro Thr Pro Ala Pro Thr Ile Ala Ser Gln Pro Leu Ser
    530                 535                 540

Leu Arg Pro Glu Ala Cys Arg Pro Ala Ala Gly Gly Ala Val His Thr
545                 550                 555                 560

Arg Gly Leu Asp Phe Ala Cys Asp Ile Tyr Ile Trp Ala Pro Leu Ala
                565                 570                 575

Gly Thr Cys Gly Val Leu Leu Leu Ser Leu Val Ile Thr Leu Tyr Cys
                580                 585                 590

Lys Arg Gly Arg Lys Lys Leu Leu Tyr Ile Phe Lys Gln Pro Phe Met
        595                 600                 605

Arg Pro Val Gln Thr Thr Gln Glu Glu Asp Gly Cys Ser Cys Arg Phe
    610                 615                 620

Pro Glu Glu Glu Glu Gly Gly Cys Glu Leu Arg Val Lys Phe Ser Arg
625                 630                 635                 640

Ser Ala Asp Ala Pro Ala Tyr Gln Gln Gly Gln Asn Gln Leu Tyr Asn
                645                 650                 655

Glu Leu Asn Leu Gly Arg Arg Glu Glu Tyr Asp Val Leu Asp Lys Arg
                660                 665                 670

Arg Gly Arg Asp Pro Glu Met Gly Gly Lys Pro Arg Arg Lys Asn Pro
        675                 680                 685

Gln Glu Gly Leu Tyr Asn Glu Leu Gln Lys Asp Lys Met Ala Glu Ala
    690                 695                 700

Tyr Ser Glu Ile Gly Met Lys Gly Glu Arg Arg Arg Gly Lys Gly His
705                 710                 715                 720

Asp Gly Leu Tyr Gln Gly Leu Ser Thr Ala Thr Lys Asp Thr Tyr Asp
                725                 730                 735

Ala Leu His Met Gln Ala Leu Pro Pro Arg
                740                 745

<210>  16
<211>  703
<212>  PRT
<213>  Artificial

<220>
<223>  Car 005 translation region

<400>  16

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
                20                  25                  30

Ala Glu Leu Val Lys Pro Gly Ala Ser Val Lys Ile Ser Cys Lys Ala
        35                  40                  45

Ser Gly Tyr Ala Phe Ser Ser Tyr Trp Met Asn Trp Val Lys Gln Arg
    50                  55                  60

Pro Gly Lys Gly Leu Glu Trp Ile Gly Gln Ile Tyr Pro Gly Asp Gly
65                  70                  75                  80

Asp Thr Thr Tyr Asn Gly Lys Phe Lys Gly Lys Ala Thr Leu Thr Ala
                85                  90                  95

Asp Lys Ser Ser Ser Thr Val Tyr Met Gln Leu Asn Ser Leu Thr Ser
            100                 105                 110

Glu Asp Ser Ala Val Tyr Phe Cys Val Arg Tyr Tyr Tyr Gly Ser Ser
        115                 120                 125

Gly Tyr Phe Asp Tyr Trp Gly Gln Gly Thr Thr Leu Thr Val Ser Ser
    130                 135                 140

Gly Gly Gly Gly Ser Gly Gly Gly Gly Ser Gly Gly Gly Gly Ser Asp
145                 150                 155                 160

Val Gln Met Ile Gln Thr Pro Asp Ser Leu Ala Val Ser Leu Gly Gln
                165                 170                 175

Arg Ala Thr Ile Ser Cys Arg Ala Ser Glu Ser Val Asp Asn Tyr Gly
            180                 185                 190

Ile Ser Phe Met His Trp Tyr Gln Gln Lys Pro Gly Gln Ser Pro Lys
        195                 200                 205

Leu Leu Ile Tyr Arg Ala Ser Asn Leu Glu Ser Gly Ile Pro Ala Arg
    210                 215                 220

Phe Ser Gly Ser Gly Ser Arg Thr Asp Phe Thr Leu Thr Ile Asn Pro
225                 230                 235                 240

Val Glu Thr Asp Asp Val Ala Thr Tyr Tyr Cys Gln Gln Asn Asn Lys
                245                 250                 255

Asp Pro Pro Thr Phe Gly Gly Gly Thr Lys Leu Glu Ile Lys Glu Pro
            260                 265                 270

Lys Ser Cys Asp Lys Thr His Thr Cys Pro Pro Cys Asp Pro Ala Glu
        275                 280                 285

Pro Lys Ser Pro Asp Lys Thr His Thr Cys Pro Pro Cys Pro Ala Pro
    290                 295                 300

Glu Leu Leu Gly Gly Pro Ser Val Phe Leu Phe Pro Pro Lys Pro Lys
305                 310                 315                 320

Asp Thr Leu Met Ile Ser Arg Thr Pro Glu Val Thr Cys Val Val Val
                325                 330                 335

Asp Val Ser His Glu Asp Pro Glu Val Lys Phe Asn Trp Tyr Val Asp
            340                 345                 350

Gly Val Glu Val His Asn Ala Lys Thr Lys Pro Arg Glu Glu Gln Tyr
        355                 360                 365

Asn Ser Thr Tyr Arg Val Val Ser Val Leu Thr Val Leu His Gln Asp
    370                 375                 380

Trp Leu Asn Gly Lys Glu Tyr Lys Cys Lys Val Ser Asn Lys Ala Leu
385                 390                 395                 400

Pro Ala Pro Ile Glu Lys Thr Ile Ser Lys Ala Lys Gly Gln Pro Arg
                405                 410                 415

Glu Pro Gln Val Tyr Thr Leu Pro Pro Ser Arg Asp Glu Leu Thr Lys
            420                 425                 430

Asn Gln Val Ser Leu Thr Cys Leu Val Lys Gly Phe Tyr Pro Ser Asp
        435                 440                 445

Ile Ala Val Glu Trp Glu Ser Asn Gly Gln Pro Glu Asn Asn Tyr Lys
    450                 455                 460

Thr Thr Pro Pro Val Leu Asp Ser Asp Gly Ser Phe Phe Leu Tyr Ser
465                 470                 475                 480

Lys Leu Thr Val Asp Lys Ser Arg Trp Gln Gln Gly Asn Val Phe Ser
                485                 490                 495

Cys Ser Val Met His Glu Ala Leu His Asn His Tyr Thr Gln Lys Ser
            500                 505                 510

Leu Ser Leu Ser Pro Gly Lys Lys Asp Pro Lys Phe Trp Val Leu Val
        515                 520                 525

Val Val Gly Gly Val Leu Ala Cys Tyr Ser Leu Leu Val Thr Val Ala
    530                 535                 540

Phe Ile Ile Phe Trp Val Arg Ser Lys Arg Ser Arg Leu Leu His Ser
545                 550                 555                 560

Asp Tyr Met Asn Met Thr Pro Arg Arg Pro Gly Pro Thr Arg Lys His
                565                 570                 575

Tyr Gln Pro Tyr Ala Pro Pro Arg Asp Phe Ala Ala Tyr Arg Ser Arg
            580                 585                 590

Val Lys Phe Ser Arg Ser Ala Asp Ala Pro Ala Tyr Gln Gln Gly Gln
        595                 600                 605

Asn Gln Leu Tyr Asn Glu Leu Asn Leu Gly Arg Arg Glu Glu Tyr Asp
    610                 615                 620

Val Leu Asp Lys Arg Arg Gly Arg Asp Pro Glu Met Gly Gly Lys Pro
625                 630                 635                 640

Arg Arg Lys Asn Pro Gln Glu Gly Leu Tyr Asn Glu Leu Gln Lys Asp
                645                 650                 655

Lys Met Ala Glu Ala Tyr Ser Glu Ile Gly Met Lys Gly Glu Arg Arg
            660                 665                 670

Arg Gly Lys Gly His Asp Gly Leu Tyr Gln Gly Leu Ser Thr Ala Thr
        675                 680                 685

Lys Asp Thr Tyr Asp Ala Leu His Met Gln Ala Leu Pro Pro Arg
    690                 695                 700

<210>  17
<211>  57
<212>  DNA
<213>  Homo sapiens

<400>  17
atgggagcca tcgggctcct gtggctcctg ccgctgctgc tttccacggc agctgtg          57

<210>  18
<211>  1149
<212>  DNA
<213>  Homo sapiens

<400>  18
acagcaccca aatcaagaaa cctgtttgag agaaacccaa acaaggagct gaaacccggg      60

gaaaattcac caagacagac ccccatcttt gaccctacag ttcattggct gttcaccaca     120

tgtggggcca gcgggcccca tggccccacc caggcacagt gcaacaacgc ctaccagaac     180

tccaacctga gcgtggaggt ggggagcgag ggccccctga aaggcatcca gatctggaag 240

gtgccagcca ccgacaccta cagcatctcg ggctacggag ctgctggcgg gaaaggcggg 300

aagaacacca tgatgcggtc ccacggcgtg tctgtgctgg gcatcttcaa cctggagaag 360

gatgacatgc tgtacatcct ggttgggcag cagggagagg acgcctgccc cagtacaaac 420

cagttaatcc agaaagtctg cattggagag aacaatgtga tagaagaaga aatccgtgtg 480

aacagaagcg tgcatgagtg ggcaggaggc ggaggaggag ggggtggagc cacctacgta 540

tttaagatga aggatggagt gccggtgccc ctgatcattg cagccggagg tggtggcagg 600

gcctacgggg ccaagacaga cacgttccac ccagagagac tggagaataa ctcctcggtt 660

ctagggctaa acggcaattc cggagccgca ggtggtggag gtggctggaa tgataacact 720

tccttgctct gggccggaaa atctttgcag gagggtgcca ccggaggaca ttcctgcccc 780

caggccatga agaagtgggg gtgggagaca agagggggtt tcggaggggg tggagggggg 840

tgctcctcag gtggaggagg cggaggatat ataggcggca atgcagcctc aaacaatgac 900

cccgaaatgg atggggaaga tggggtttcc ttcatcagtc cactgggcat cctgtacacc 960

ccagctttaa aagtgatgga aggccacggg gaagtgaata ttaagcatta tctaaactgc 1020

agtcactgtg aggtagacga atgtcacatg gaccctgaaa gccacaaggt catctgcttc 1080

tgtgaccacg ggacggtgct ggctgaggat ggcgtctcct gcattgtgtc acccaccccg 1140

gagccacac 1149

<210> 19
<211> 8
<212> PRT
<213> Artificial

<220>
<223> Histidine tag

<400> 19

His His His His His His His His
1               5

<210> 20
<211> 12
<212> PRT
<213> Artificial

<220>
<223> PA tag

<400> 20

Gly Val Ala Met Pro Gly Ala Glu Asp Asp Val Val
1               5                   10

<210>  21
<211>  6
<212>  DNA
<213>  Artificial

<220>
<223>  Kozak sequence

<400>  21
gccacc                                                                  6


<210>  22
<211>  1278
<212>  DNA
<213>  Artificial

<220>
<223>  Kozak-ALK-His-PA

<400>  22
gccaccatgg gtgccatcgg gttgctgtgg cttctccccc tcttgctttc tactgctgca      60

gttactgccc cgaaatcccg caatctgttt gaacggaacc ccaacaagga gctaaagcct     120

ggagagaatt caccgagaca gactcccata ttcgatccaa cggtccattg gctgttcacc     180

acttgtgggg ctagtgggcc tcatggccct acccaagcac agtgcaacaa cgcctatcag     240

aacagcaatc tctctgtgga agtaggcagc gaagggccac ttaaggggat ccagatttgg     300

aaggtgcctg ctacagacac ctactccatt agtggctatg gtgcggcagg tgggaaagga     360

ggcaagaaca ccatgatgcg atcccatggc gtcagtgtac tcggtatctt caacctggag     420

aaagatgaca tgctgtacat tctcgttgga caacagggtg aagacgcgtg cccatcgacc     480

aaccagctga tccagaaagt gtgcataggc gagaataacg tcatcgagga ggagattcgg     540

gtgaatagat cggtgcatga gtgggcgggt gggggtggag ggggcggagg ggccacatac     600

gttttcaaga tgaaggatgg cgtacccgtt cccctgatca ttgctgcagg tggcggtgga     660

agggcctatg gtgccaaaac cgacaccttt catcccgaaa ggttagagaa caatagtagc     720

gtgctaggcc tgaatgggaa ttcaggcgct gcaggagggg gaggcggatg gaacgataat     780

acgtccctcc tgtgggccgg caaatccctg caagagggcg ctacaggagg acacagctgt     840

cctcaggcca tgaagaagtg gggctgggaa acacgtggag ggtttggggg gggcggcgga     900

gggtgttcat ctggaggcgg cggcggtggg tatattggcg gaaatgctgc atctaacaat     960

gaccctgaga tggatggcga agatggtgtg tctttcatct cacccttggg catactttac    1020

actcctgccc tgaaagtgat ggagggacac ggagaggtga atatcaagca ctacttgaac    1080

tgttcccact gcgaggtgga cgaatgccac atggacccag aaagccacaa agtgatctgc    1140

ttttgtgacc acggcacagt cttagccgaa gatggagtca gctgcattgt gagccccaca    1200

ccagagccgc accaccatca tcaccatcac caccatgggg ttgccatgcc aggggctgaa    1260

gacgatgtgg tctgataa                                                    1278

<210>  23
<211>  1272
<212>  DNA
<213>  Homo sapiens

<400>  23
atgggctgct ggggacagct gctggtgtgg ttcggagccg cgggcgccat tctctgctct      60

agcccggggt cccaggagac ttttctgcgg tcctcgcccc tgccgctggc aagtcccagc     120

cccgggaccc cgaaagtcag cgccccgcct agtatcttgg agccagcctc cccgctgaat     180

tctccgggca ccgaggggtc ttggctgttt tctacctgcg gggccagcgg ccggcatggg     240

cccacacaga cacaatgtga cggggcgtac gcggggacca gcgtggtggt gaccgtgggg     300

gccgccgggc agctgagagg cgtgcagctg tggcgcgtgc cgggccctgg ccagtatctg     360

atctcagcct acggagccgc gggcggcaaa ggcgccaaga accacctgtc gcgggcgcat     420

ggcgtcttcg tctcagcaat cttctccctc ggtctcgggg agtcgctgta catcctggtg     480

gggcagcagg gagaggacgc ctgtcccgga ggtagcccgg agagccagct cgtctgcctc     540

ggggagtctc gagccgttga agagcacgcg gcgatggatg ggagcgaagg ggtcccgggg     600

tcgcggcgct gggcgggagg tggcgggggt ggcgggggcg ccacctacgt tttccgggtg     660

cgcgctggcg agctggaacc gttgctggtg gcggccggag gcggcggtcg ggcctacctg     720

aggccgcggg accgaggccg gactcaggcc tcccccgaga aactggagaa ccgctcggag     780

gcgcccggga gcggcgggag aggcggggcg gcaggtggtg ggggcggctg gacgtcgcgg     840

gctccctctc cgcaggccgg ccgctcactg caggaggggg cggagggcgg ccagggctgc     900

tccgaggctt gggcgaccct tggctgggcc gcggccggcg gcttcggggg cggcggcggg     960

gcctgcactg cgggcggagg cggcggcggc tacagggggg gcgacgcttc agagactgac    1020

aacctctggg ctgatgggga agatggagta tccttcatac accccagcag cgagctcttc    1080

ctgcagcctc tggcagtcac cgagaaccac ggagaggtag agatccgaag gcacctcaac    1140

tgcagtcact gccctttgag agactgccaa tggcaggcag agctccagct ggctgaatgc    1200

ctgtgcccag aaggcatgga gctagctgtg gataacgtca cctgcatgga cctgcacaag    1260

cccccaggcc ct                                                         1272

<210>  24
<211>  1344
<212>  DNA
<213>  Artificial

<220>
<223>  Kozak-LTK-His-PA

<400>  24

gccaccatgg gctgttgggg tcagttgctg gtgtggtttg gagcagcagg ggcaatcctc 60

tgcagctctc cgggcagcca ggaaacgttc ctgaggtcct cccccttgcc tttggccagc 120

ccttccccaa gggatccgaa agtgtcagcg ccaccctcga tcctcgagcc tgcttcaccc 180

ctgaatagcc ccggaactga ggggtcatgg ctattctcta cttgcggcgc ctctggtcgg 240

catggcccca cacagactca gtgcgatggt gcctatgcgg gtaccagcgt agtggtgacc 300

gttggggctg ccggccagct tcggggggta cagctgtgga gagtcccagg accaggccag 360

tacctgatat ccgcatatgg tgcagctgga ggcaaaggcg cgaagaacca cttaagccga 420

gcccatggcg tgttcgtctc tgcgatcttt tccctcggac tcggggagtc tttgtacatt 480

ctggtagggc aacagggaga ggatgcctgt cccggggggt caccggaatc gcagcttgtg 540

tgcttagggg aaagcagagc cgtggaagag cacgcagcca tggacggttc tgaaggagtc 600

cctgggagtc gtcggtgggc aggtggaggt ggaggaggcg gaggggccac ctacgtgttc 660

cgcgtgagag ctggagaact cgaaccactc ctggtggctg ccgggggcgg agggagggca 720

tacttacgcc cacgcgacag aggccggaca caagcctcac cagaaaagct cgagaacagg 780

tccgaggccc ccggaagtgg cggcagagga ggagccgcag gcggtggcgg tgggtggaca 840

agtcgagcac cctcccctca agctggaagg agtcttcagg aaggggctga gggcggacag 900

ggatgctcag aggcttgggc cacccttggt tgggctgccg caggaggctt tggggggggt 960

ggcggggctt gtactgccgg cggaggtggc ggcgggtatc gaggtggcga cgcttctgag 1020

acggacaatc tgtgggccga tggcgaggac ggcgttagct tcattcaccc cagtagcgaa 1080

ctgtttctgc agccacttgc tgtcacagag aaccatggcg aggttgagat tcgtcggcac 1140

ctaaactgct cccactgtcc cctgcgcgat tgtcagtggc aagccgaact gcaactggcc 1200

gagtgtctgt gccctgaagg gatggagcta gcggtggaca atgtgacctg catggacttg 1260

cacaagccac ctggccctca ccatcatcac caccatcacc atggggtcgc tatgcctggc 1320

gccgaggatg atgtcgtttg ataa 1344

<210> 25
<211> 8
<212> PRT
<213> Artificial

<220>
<223> HRV3C protease cleavage sequence

<400> 25

Leu Glu Val Leu Phe Gln Gly Pro
1               5

<210> 26
<211> 227

<212>   PRT
<213>   Homo sapiens

<400>   26

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  27
<211>  5
<212>  PRT
<213>  Artificial

<220>
<223>  G4S linker

<400>  27
Gly Gly Gly Gly Ser
1               5


<210>  28
<211>  792
<212>  DNA
<213>  Artificial

<220>
<223>  Kozak-HRV3C-Fc-His-PA

<400>  28
gccaccctgg aagtgctgtt tcagggaccc gataaaaccc atacctgtcc accatgtcct      60

gctcccgaac ttctgggagg accaagtgtg ttcctgtttc cacctaaacc caaagacacg     120

ttgatgatat cccgtactcc agaggtgact tgtgtggtgg tggatgtgtc tcatgaggat     180

cctgaggtga agttcaactg gtatgtcgac ggtgtggaag tccacaatgc caagaccaag     240

cctagggagg agcagtacaa ctccacgtat cgggtagtct ccgtactgac agtgctgcat     300

caggactggc tcaatgggaa ggagtacaaa tgcaaggtta gcaacaaggc tcttcccgcg     360

cccatcgaaa agaccatctc taaggccaaa ggtcagccca gagaacccca ggtttataca     420

ctcccgcctt cacgagatga gctgaccaag aaccaagtca gcctcacatg cttggtcaaa     480

ggcttttatc cgagtgacat tgcagtcgag tgggaatcca atggccaacc agagaacaac     540

tacaaaacaa ctccgcccgt tctcgacagc gatgggagct tctttctgta ctccaaactg     600

accgtagaca agtctcgctg gcaacagggc aatgtgttca gttgcagcgt gatgcacgaa     660

gcccttcaca atcactacac tcagaagtca ctgtctctga gccctgggaa aggcggcggt     720

ggctcacatc accatcacca ccaccatcac ggagtggcca tgcctggggc agaggatgac     780

gttgtttgat aa                                                         792


<210>  29
<211>  447
<212>  DNA
<213>  Artificial

<220>
<223>  Kozak-FAM150A-HRV3C-His

<400>  29
gccaccatgc gcccactgaa accaggtgct cctcttcctg ccctgtttct gctggctctg      60

gcactgtctc cccatggcgc tcatggcaga cctcgcggtc gtagaggagc aagggtgact 120

gacaaagagc ccaaaccgtt gctcttcctt ccagccgcag gagctgggag gacaccctct 180

ggcagtaggt cagccgagat tttccctcgg gatagcaatc tgaaggacaa gttcatcaag 240

cacttcacag gacccgtgac ctttagccca gagtgctcca agcactttca ccgcctctac 300

tacaacaccc gggaatgctc cactcccgcc tattataagc ggtgtgccag actgctgacc 360

cgattggcgg tttcaccgct ctgtagccaa acgctcgaag tcctgtttca ggggcctcat 420

caccaccatc accatcacca ttgataa 447

<210> 30
<211> 516
<212> DNA
<213> Artificial

<220>
<223> Kozak-FAM150B-HRV3C-His

<400> 30
gccaccatga gagggcctgg acatcccctg cttctcggcc ttctcctggt tctgggagct 60

gctgggagag gcagaggagg ggcagagcct cgggaaccag ccgatggcca agcgcttctg 120

aggctggtcg ttgagctggt gcaggaactc cggaaacacc acagtgccga gcacaaaggc 180

ctgcagctgc ttggacggga ctgtgcattg ggtcgagccg aagctgcagg gttgggtccc 240

tcaccggagc aaagggtgga gatcgtgcct agagatctcc gcatgaagga caagttcctg 300

aagcacctga caggcccact gtacttcagc ccaaaatgct ccaagcactt tcatcgcctg 360

tatcacaaca ctcgcgattg caccattccc gcctactata agcgttgtgc caggctgctg 420

acgcgattgg ctgtgtctcc cgtatgcatg gaggacaaac agctggaagt cctctttcag 480

ggtcctcatc accatcatca ccatcaccat tgataa 516

<210> 31
<211> 35
<212> DNA
<213> Artificial

<220>
<223> forward primer

<400> 31
gatccagcct ccggactgcc accatgggtg ccatc 35

<210> 32
<211> 35
<212> DNA
<213> Artificial

<220>
<223> reverse primer

<400>  32
tgaaacagca cttccaggtg cggctctggt gtggg                                35

<210>  33
<211>  35
<212>  DNA
<213>  Artificial

<220>
<223>  forward primer

<400>  33
gatccagcct ccggactgcc accatgggct gttgg                                35

<210>  34
<211>  35
<212>  DNA
<213>  Artificial

<220>
<223>  reverse primer

<400>  34
tgaaacagca cttccagagg gccaggtggc ttgtg                                35

<210>  35
<211>  24
<212>  DNA
<213>  Artificial

<220>
<223>  forward primer

<400>  35
ctggaagtgc tgtttcaggg accc                                            24

<210>  36
<211>  35
<212>  DNA
<213>  Artificial

<220>
<223>  reverse primer

<400>  36
atcaaactca ttactaatta tcaaacaacg tcatc                                35

<210>  37
<211>  662
<212>  PRT
<213>  Artificial

<220>
<223>  ALK 001P

<400>  37

Met Gly Ala Ile Gly Leu Leu Trp Leu Leu Pro Leu Leu Leu Ser Thr
1               5                   10                  15

Ala Ala Val Thr Ala Pro Lys Ser Arg Asn Leu Phe Glu Arg Asn Pro
            20                  25                  30

Asn Lys Glu Leu Lys Pro Gly Glu Asn Ser Pro Arg Gln Thr Pro Ile
        35                  40                  45

Phe Asp Pro Thr Val His Trp Leu Phe Thr Thr Cys Gly Ala Ser Gly
    50                  55                  60

Pro His Gly Pro Thr Gln Ala Gln Cys Asn Asn Ala Tyr Gln Asn Ser
65                  70                  75                  80

Asn Leu Ser Val Glu Val Gly Ser Glu Gly Pro Leu Lys Gly Ile Gln
                85                  90                  95

Ile Trp Lys Val Pro Ala Thr Asp Thr Tyr Ser Ile Ser Gly Tyr Gly
            100                 105                 110

Ala Ala Gly Gly Lys Gly Gly Lys Asn Thr Met Met Arg Ser His Gly
        115                 120                 125

Val Ser Val Leu Gly Ile Phe Asn Leu Glu Lys Asp Asp Met Leu Tyr
    130                 135                 140

Ile Leu Val Gly Gln Gln Gly Glu Asp Ala Cys Pro Ser Thr Asn Gln
145                 150                 155                 160

Leu Ile Gln Lys Val Cys Ile Gly Glu Asn Asn Val Ile Glu Glu Glu
                165                 170                 175

Ile Arg Val Asn Arg Ser Val His Glu Trp Ala Gly Gly Gly Gly Gly
            180                 185                 190

Gly Gly Gly Ala Thr Tyr Val Phe Lys Met Lys Asp Gly Val Pro Val
        195                 200                 205

Pro Leu Ile Ile Ala Ala Gly Gly Gly Gly Arg Ala Tyr Gly Ala Lys
    210                 215                 220

Thr Asp Thr Phe His Pro Glu Arg Leu Glu Asn Asn Ser Ser Val Leu
225                 230                 235                 240

Gly Leu Asn Gly Asn Ser Gly Ala Ala Gly Gly Gly Gly Gly Trp Asn
                245                 250                 255

Asp Asn Thr Ser Leu Leu Trp Ala Gly Lys Ser Leu Gln Glu Gly Ala
            260                 265                 270

Thr Gly Gly His Ser Cys Pro Gln Ala Met Lys Lys Trp Gly Trp Glu
        275                 280                 285

Thr Arg Gly Gly Phe Gly Gly Gly Gly Gly Gly Cys Ser Ser Gly Gly
    290                 295                 300

Gly Gly Gly Gly Tyr Ile Gly Gly Asn Ala Ala Ser Asn Asn Asp Pro
305                 310                 315                 320

Glu Met Asp Gly Glu Asp Gly Val Ser Phe Ile Ser Pro Leu Gly Ile
                325                 330                 335

Leu Tyr Thr Pro Ala Leu Lys Val Met Glu Gly His Gly Glu Val Asn
            340                 345                 350

Ile Lys His Tyr Leu Asn Cys Ser His Cys Glu Val Asp Glu Cys His
        355                 360                 365

Met Asp Pro Glu Ser His Lys Val Ile Cys Phe Cys Asp His Gly Thr
    370                 375                 380

Val Leu Ala Glu Asp Gly Val Ser Cys Ile Val Ser Pro Thr Pro Glu
385                 390                 395                 400

Pro His Leu Glu Val Leu Phe Gln Gly Pro Asp Lys Thr His Thr Cys
                405                 410                 415

Pro Pro Cys Pro Ala Pro Glu Leu Leu Gly Gly Pro Ser Val Phe Leu
            420                 425                 430

Phe Pro Pro Lys Pro Lys Asp Thr Leu Met Ile Ser Arg Thr Pro Glu
        435                 440                 445

Val Thr Cys Val Val Val Asp Val Ser His Glu Asp Pro Glu Val Lys
    450                 455                 460

Phe Asn Trp Tyr Val Asp Gly Val Glu Val His Asn Ala Lys Thr Lys
465                 470                 475                 480

Pro Arg Glu Glu Gln Tyr Asn Ser Thr Tyr Arg Val Val Ser Val Leu
                485                 490                 495

Thr Val Leu His Gln Asp Trp Leu Asn Gly Lys Glu Tyr Lys Cys Lys
            500                 505                 510

Val Ser Asn Lys Ala Leu Pro Ala Pro Ile Glu Lys Thr Ile Ser Lys
        515                 520                 525

Ala Lys Gly Gln Pro Arg Glu Pro Gln Val Tyr Thr Leu Pro Pro Ser
    530                 535                 540

Arg Asp Glu Leu Thr Lys Asn Gln Val Ser Leu Thr Cys Leu Val Lys
545                 550                 555                 560

Gly Phe Tyr Pro Ser Asp Ile Ala Val Glu Trp Glu Ser Asn Gly Gln
                565                 570                 575

Pro Glu Asn Asn Tyr Lys Thr Thr Pro Pro Val Leu Asp Ser Asp Gly
            580                 585                 590

Ser Phe Phe Leu Tyr Ser Lys Leu Thr Val Asp Lys Ser Arg Trp Gln
        595                 600                 605

Gln Gly Asn Val Phe Ser Cys Ser Val Met His Glu Ala Leu His Asn
    610                 615                 620

His Tyr Thr Gln Lys Ser Leu Ser Leu Ser Pro Gly Lys Gly Gly Gly
625                 630                 635                 640

Gly Ser His His His His His His His His Gly Val Ala Met Pro Gly
                645                 650                 655

Ala Glu Asp Asp Val Val
            660

<210>  38
<211>  1986
<212>  DNA
<213>  Artificial

<220>
<223>  ALK 001P coding sequence

<400>  38
atgggtgcca tcgggttgct gtggcttctc cccctcttgc tttctactgc tgcagttact      60

gccccgaaat cccgcaatct gtttgaacgg aaccccaaca aggagctaaa gcctggagag     120

aattcaccga gacagactcc catattcgat ccaacggtcc attggctgtt caccacttgt     180

ggggctagtg ggcctcatgg ccctacccaa gcacagtgca acaacgccta tcagaacagc     240

aatctctctg tggaagtagg cagcgaaggg ccacttaagg ggatccagat ttggaaggtg     300

cctgctacag acacctactc cattagtggc tatggtgcgg caggtgggaa aggaggcaag     360

aacaccatga tgcgatccca tggcgtcagt gtactcggta tcttcaacct ggagaaagat 420

gacatgctgt acattctcgt tggacaacag ggtgaagacg cgtgcccatc gaccaaccag 480

ctgatccaga aagtgtgcat aggcgagaat aacgtcatcg aggaggagat tcgggtgaat 540

agatcggtgc atgagtgggc gggtgggggt ggagggggcg gaggggccac atacgttttc 600

aagatgaagg atggcgtacc cgttcccctg atcattgctg caggtggcgg tggaagggcc 660

tatggtgcca aaaccgacac ctttcatccc gaaaggttag agaacaatag tagcgtgcta 720

ggcctgaatg ggaattcagg cgctgcagga gggggaggcg gatggaacga taatacgtcc 780

ctcctgtggg ccggcaaatc cctgcaagag ggcgctacag gaggacacag ctgtcctcag 840

gccatgaaga agtggggctg ggaaacacgt ggagggtttg gggggggcgg cggagggtgt 900

tcatctggag gcggcggcgg tgggtatatt ggcggaaatg ctgcatctaa caatgaccct 960

gagatggatg gcgaagatgg tgtgtctttc atctcaccct tgggcatact ttacactcct 1020

gccctgaaag tgatggaggg acacggagag gtgaatatca agcactactt gaactgttcc 1080

cactgcgagg tggacgaatg ccacatggac ccagaaagcc acaaagtgat ctgcttttgt 1140

gaccacggca cagtcttagc cgaagatgga gtcagctgca ttgtgagccc cacaccagag 1200

ccgcacctgg aagtgctgtt tcagggaccc gataaaaccc atacctgtcc accatgtcct 1260

gctcccgaac ttctgggagg accaagtgtg ttcctgtttc cacctaaacc caaagacacg 1320

ttgatgatat cccgtactcc agaggtgact tgtgtggtgg tggatgtgtc tcatgaggat 1380

cctgaggtga agttcaactg gtatgtcgac ggtgtggaag tccacaatgc caagaccaag 1440

cctagggagg agcagtacaa ctccacgtat cgggtagtct ccgtactgac agtgctgcat 1500

caggactggc tcaatgggaa ggagtacaaa tgcaaggtta gcaacaaggc tcttcccgcg 1560

cccatcgaaa agaccatctc taaggccaaa ggtcagccca gagaacccca ggtttataca 1620

ctcccgcctt cacgagatga gctgaccaag aaccaagtca gcctcacatg cttggtcaaa 1680

ggcttttatc cgagtgacat tgcagtcgag tgggaatcca atggccaacc agagaacaac 1740

tacaaaacaa ctccgcccgt tctcgacagc gatgggagct tctttctgta ctccaaactg 1800

accgtagaca agtctcgctg gcaacagggc aatgtgttca gttgcagcgt gatgcacgaa 1860

gcccttcaca atcactacac tcagaagtca ctgtctctga gccctgggaa aggcggcggt 1920

ggctcacatc accatcacca ccaccatcac ggagtggcca tgcctggggc agaggatgac 1980

gttgtt 1986

<210> 39
<211> 684
<212> PRT
<213> Artificial

<220>

<223>  LTK 001P

<400>  39

Met Gly Cys Trp Gly Gln Leu Leu Val Trp Phe Gly Ala Ala Gly Ala
1               5                   10                  15

Ile Leu Cys Ser Ser Pro Gly Ser Gln Glu Thr Phe Leu Arg Ser Ser
            20                  25                  30

Pro Leu Pro Leu Ala Ser Pro Ser Pro Arg Asp Pro Lys Val Ser Ala
        35                  40                  45

Pro Pro Ser Ile Leu Glu Pro Ala Ser Pro Leu Asn Ser Pro Gly Thr
    50                  55                  60

Glu Gly Ser Trp Leu Phe Ser Thr Cys Gly Ala Ser Gly Arg His Gly
65                  70                  75                  80

Pro Thr Gln Thr Gln Cys Asp Gly Ala Tyr Ala Gly Thr Ser Val Val
                85                  90                  95

Val Thr Val Gly Ala Ala Gly Gln Leu Arg Gly Val Gln Leu Trp Arg
            100                 105                 110

Val Pro Gly Pro Gly Gln Tyr Leu Ile Ser Ala Tyr Gly Ala Ala Gly
        115                 120                 125

Gly Lys Gly Ala Lys Asn His Leu Ser Arg Ala His Gly Val Phe Val
    130                 135                 140

Ser Ala Ile Phe Ser Leu Gly Leu Gly Glu Ser Leu Tyr Ile Leu Val
145                 150                 155                 160

Gly Gln Gln Gly Glu Asp Ala Cys Pro Gly Gly Ser Pro Glu Ser Gln
                165                 170                 175

Leu Val Cys Leu Gly Glu Ser Arg Ala Val Glu Glu His Ala Ala Met
            180                 185                 190

Asp Gly Ser Glu Gly Val Pro Gly Ser Arg Arg Trp Ala Gly Gly Gly
        195                 200                 205

Gly Gly Gly Gly Gly Ala Thr Tyr Val Phe Arg Val Arg Ala Gly Glu
    210                 215                 220

Leu Glu Pro Leu Leu Val Ala Ala Gly Gly Gly Gly Arg Ala Tyr Leu
225                 230                 235                 240

Arg Pro Arg Asp Arg Gly Arg Thr Gln Ala Ser Pro Glu Lys Leu Glu
                245                 250                 255

Asn Arg Ser Glu Ala Pro Gly Ser Gly Gly Arg Gly Gly Ala Ala Gly
            260                 265                 270

Gly Gly Gly Gly Trp Thr Ser Arg Ala Pro Ser Pro Gln Ala Gly Arg
        275                 280                 285

Ser Leu Gln Glu Gly Ala Glu Gly Gly Gln Gly Cys Ser Glu Ala Trp
    290                 295                 300

Ala Thr Leu Gly Trp Ala Ala Ala Gly Gly Phe Gly Gly Gly Gly Gly
305                 310                 315                 320

Ala Cys Thr Ala Gly Gly Gly Gly Gly Gly Tyr Arg Gly Gly Asp Ala
                325                 330                 335

Ser Glu Thr Asp Asn Leu Trp Ala Asp Gly Glu Asp Gly Val Ser Phe
            340                 345                 350

Ile His Pro Ser Ser Glu Leu Phe Leu Gln Pro Leu Ala Val Thr Glu
        355                 360                 365

Asn His Gly Glu Val Glu Ile Arg Arg His Leu Asn Cys Ser His Cys
    370                 375                 380

Pro Leu Arg Asp Cys Gln Trp Gln Ala Glu Leu Gln Leu Ala Glu Cys
385                 390                 395                 400

Leu Cys Pro Glu Gly Met Glu Leu Ala Val Asp Asn Val Thr Cys Met
                405                 410                 415

Asp Leu His Lys Pro Pro Gly Pro Leu Glu Val Leu Phe Gln Gly Pro
            420                 425                 430

Asp Lys Thr His Thr Cys Pro Pro Cys Pro Ala Pro Glu Leu Leu Gly
        435                 440                 445

Gly Pro Ser Val Phe Leu Phe Pro Pro Lys Pro Lys Asp Thr Leu Met
    450                 455                 460

Ile Ser Arg Thr Pro Glu Val Thr Cys Val Val Val Asp Val Ser His
465                 470                 475                 480

Glu Asp Pro Glu Val Lys Phe Asn Trp Tyr Val Asp Gly Val Glu Val
                485                 490                 495

His Asn Ala Lys Thr Lys Pro Arg Glu Glu Gln Tyr Asn Ser Thr Tyr
            500                 505                 510

Arg Val Val Ser Val Leu Thr Val Leu His Gln Asp Trp Leu Asn Gly
        515                 520                 525

Lys Glu Tyr Lys Cys Lys Val Ser Asn Lys Ala Leu Pro Ala Pro Ile
    530                 535                 540

Glu Lys Thr Ile Ser Lys Ala Lys Gly Gln Pro Arg Glu Pro Gln Val
545                 550                 555                 560

Tyr Thr Leu Pro Pro Ser Arg Asp Glu Leu Thr Lys Asn Gln Val Ser
                565                 570                 575

Leu Thr Cys Leu Val Lys Gly Phe Tyr Pro Ser Asp Ile Ala Val Glu
            580                 585                 590

Trp Glu Ser Asn Gly Gln Pro Glu Asn Asn Tyr Lys Thr Thr Pro Pro
        595                 600                 605

Val Leu Asp Ser Asp Gly Ser Phe Phe Leu Tyr Ser Lys Leu Thr Val
    610                 615                 620

Asp Lys Ser Arg Trp Gln Gln Gly Asn Val Phe Ser Cys Ser Val Met
625                 630                 635                 640

His Glu Ala Leu His Asn His Tyr Thr Gln Lys Ser Leu Ser Leu Ser
                645                 650                 655

Pro Gly Lys Gly Gly Gly Gly Ser His His His His His His His His
            660                 665                 670

Gly Val Ala Met Pro Gly Ala Glu Asp Asp Val Val
        675                 680

<210> 40
<211> 2052
<212> DNA
<213> Artificial

<220>
<223> LTK 001P coding sequence

<400> 40
atgggctgtt ggggtcagtt gctggtgtgg tttggagcag caggggcaat cctctgcagc 60

tctccgggca gccaggaaac gttcctgagg tcctccccct tgcctttggc cagcccttcc 120

ccaagggatc cgaaagtgtc agcgccaccc tcgatcctcg agcctgcttc acccctgaat 180

agccccggaa ctgaggggtc atggctattc tctacttgcg gcgcctctgg tcggcatggc 240

cccacacaga ctcagtgcga tggtgcctat gcgggtacca gcgtagtggt gaccgttggg 300

gctgccggcc agcttcgggg ggtacagctg tggagagtcc caggaccagg ccagtacctg 360

atatccgcat atggtgcagc tggaggcaaa ggcgcgaaga accacttaag ccgagcccat 420

ggcgtgttcg tctctgcgat cttttccctc ggactcgggg agtctttgta cattctggta 480

gggcaacagg gagaggatgc ctgtcccggg gggtcaccgg aatcgcagct tgtgtgctta 540

ggggaaagca gagccgtgga agagcacgca gccatggacg gttctgaagg agtccctggg 600

agtcgtcggt gggcaggtgg aggtggagga ggcggagggg ccacctacgt gttccgcgtg 660

agagctggag aactcgaacc actcctggtg gctgccgggg gcggagggag ggcatactta 720

cgcccacgcg acagaggccg gacacaagcc tcaccagaaa agctcgagaa caggtccgag 780

gcccccggaa gtggcggcag aggaggagcc gcaggcggtg gcggtgggtg gacaagtcga 840

gcaccctccc ctcaagctgg aaggagtctt caggaagggg ctgagggcgg acagggatgc 900

tcagaggctt gggccaccct tggttgggct gccgcaggag gctttggggg gggtggcggg 960

gcttgtactg ccggcggagg tggcggcggg tatcgaggtg gcgacgcttc tgagacggac 1020

aatctgtggg ccgatggcga ggacggcgtt agcttcattc accccagtag cgaactgttt 1080

ctgcagccac ttgctgtcac agagaaccat ggcgaggttg agattcgtcg gcacctaaac 1140

tgctcccact gtcccctgcg cgattgtcag tggcaagccg aactgcaact ggccgagtgt 1200

ctgtgccctg aagggatgga gctagcggtg gacaatgtga cctgcatgga cttgcacaag 1260

ccacctggcc ctctggaagt gctgtttcag ggacccgata aaacccatac ctgtccacca 1320

tgtcctgctc ccgaacttct gggaggacca agtgtgttcc tgtttccacc taaacccaaa 1380

gacacgttga tgatatcccg tactccagag gtgacttgtg tggtggtgga tgtgtctcat 1440

gaggatcctg aggtgaagtt caactggtat gtcgacggtg tggaagtcca caatgccaag 1500

accaagccta gggaggagca gtacaactcc acgtatcggg tagtctccgt actgacagtg 1560

ctgcatcagg actggctcaa tgggaaggag tacaaatgca aggttagcaa caaggctctt 1620

cccgcgccca tcgaaaagac catctctaag gccaaaggtc agcccagaga accccaggtt 1680

tatacactcc cgccttcacg agatgagctg accaagaacc aagtcagcct cacatgcttg 1740

gtcaaaggct tttatccgag tgacattgca gtcgagtggg aatccaatgg ccaaccagag 1800

aacaactaca aaacaactcc gcccgttctc gacagcgatg ggagcttctt tctgtactcc 1860

aaactgaccg tagacaagtc tcgctggcaa cagggcaatg tgttcagttg cagcgtgatg 1920

cacgaagccc ttcacaatca ctacactcag aagtcactgt ctctgagccc tgggaaaggc 1980

ggcggtggct cacatcacca tcaccaccac catcacggag tggccatgcc tggggcagag 2040

gatgacgttg tt                                                          2052

<210>  41
<211>  35
<212>  DNA
<213>  Artificial

<220>
<223>  forward primer for 1st PCR

<400>  41
gtgctcttcc aaggaccagc cgcaggagct gggag                                  35

<210>  42
<211>  39
<212>  DNA
<213>  Artificial

<220>
<223>  reverse primer for 1st and 2nd PCRs

<400>  42
ggtaccgcgg ccgcttcatc acgtttggct acagagcgg                              39

<210>  43
<211>  43
<212>  DNA
<213>  Artificial

<220>
<223>  forward primer for 2nd PCR

<400>  43
cagagaacag attggaggtc tggaagtgct cttccaagga cca                         43

<210>  44
<211>  384
<212>  DNA
<213>  Artificial

<220>
<223>  synthetic polynucleotide for fusion protein

<400>  44
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggt                                            384

<210>  45
<211>  128
<212>  PRT
<213>  Artificial

<220>
<223>  fusion protein

<400>  45

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

<210>  46
<211>  216
<212>  PRT
<213>  Artificial

<220>
<223>  Ligand 001P

<400>  46

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Ala Gly Ala Gly Arg Thr Pro
    130                 135                 140

Ser Gly Ser Arg Ser Ala Glu Ile Phe Pro Arg Asp Ser Asn Leu Lys
145                 150                 155                 160

Asp Lys Phe Ile Lys His Phe Thr Gly Pro Val Thr Phe Ser Pro Glu
                165                 170                 175

Cys Ser Lys His Phe His Arg Leu Tyr Tyr Asn Thr Arg Glu Cys Ser
            180                 185                 190

Thr Pro Ala Tyr Tyr Lys Arg Cys Ala Arg Leu Leu Thr Arg Leu Ala
        195                 200                 205

Val Ser Pro Leu Cys Ser Gln Thr
    210                 215

<210>  47
<211>  648
<212>  DNA
<213>  Artificial

<220>
<223>  Ligand 001P coding sequence

<400>  47
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt 300

gaaattcaag ctgatcagac ccctgaagat ttggacatgg aggataacga tattattgag 360

gctcacagag aacagattgg aggtctggaa gtgctcttcc aaggaccagc cgcaggagct 420

gggaggacac cctctggcag taggtcagcc gagattttcc ctcgggatag caatctgaag 480

gacaagttca tcaagcactt cacaggaccc gtgaccttta gcccagagtg ctccaagcac 540

tttcaccgcc tctactacaa cacccgggaa tgctccactc ccgcctatta taagcggtgt 600

gccagactgc tgacccgatt ggcggtttca ccgctctgta gccaaacg 648

<210> 48
<211> 35
<212> DNA
<213> Artificial

<220>
<223> forward primer for 1st PCR

<400> 48
gtgctcttcc aaggaccagc cgaagctgca gggtt 35

<210> 49
<211> 39
<212> DNA
<213> Artificial

<220>
<223> reverse primer for 1st and 2nd PCRs

<400> 49
ggtaccgcgg ccgcttcatc actgtttgtc ctccatgca 39

<210> 50
<211> 218
<212> PRT
<213> Artificial

<220>
<223> Ligand 002P

<400> 50

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Glu Ala Ala Gly Leu Gly Pro
    130                 135                 140

Ser Pro Glu Gln Arg Val Glu Ile Val Pro Arg Asp Leu Arg Met Lys
145                 150                 155                 160

Asp Lys Phe Leu Lys His Leu Thr Gly Pro Leu Tyr Phe Ser Pro Lys
                165                 170                 175

Cys Ser Lys His Phe His Arg Leu Tyr His Asn Thr Arg Asp Cys Thr
            180                 185                 190

Ile Pro Ala Tyr Tyr Lys Arg Cys Ala Arg Leu Leu Thr Arg Leu Ala
        195                 200                 205

Val Ser Pro Val Cys Met Glu Asp Lys Gln
    210                 215

<210>  51
<211>  654
<212>  DNA
<213>  Artificial

<220>
<223>  Ligand 002P coding sequence

<400>  51
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagc cgaagctgca 420

gggttgggtc cctcaccgga gcaaagggtg gagatcgtgc ctagagatct ccgcatgaag 480

gacaagttcc tgaagcacct gacaggccca ctgtacttca gcccaaaatg ctccaagcac 540

tttcatcgcc tgtatcacaa cactcgcgat tgcaccattc ccgcctacta taagcgttgt 600

gccaggctgc tgacgcgatt ggctgtgtct cccgtatgca tggaggacaa acag 654

<210> 52
<211> 33
<212> DNA
<213> Artificial

<220>
<223> forward primer

<400> 52
gctgcagggg ctggacgaac tccctcaggc agt 33

<210> 53
<211> 34
<212> DNA
<213> Artificial

<220>
<223> reverse primer

<400> 53
gcactgaacc cctttcagta tggccaccag aaac 34

<210> 54
<211> 36
<212> DNA
<213> Artificial

<220>
<223> forward primer

<400> 54
gcagaagcgg ctggtctggg tccctcacca gaacag 36

<210> 55
<211> 34
<212> DNA
<213> Artificial

<220>
<223> reverse primer

<400> 55
gcactgaacc cctttgagga tggctacgag gaac 34

<210> 56
<211> 1599
<212> DNA
<213> Artificial

<220>
<223>  CAR 007 translation region

<400>  56
atggagttcg gactgtcctg gctgtttctg gtggccatac tgaaaggggt tcagtgcgct      60

gcaggggctg gacgaactcc ctcaggcagt cggtcagccg agattttccc tcgggatagc     120

aatctcaagg acaagttcat caagcacttc accggtcctg tgaccttctc tcccgagtgt     180

agcaagcact ttcatcgcct gtattacaac acacgtgaat gctccacacc agcctactat     240

aagcggtgtg cgaggctcct cactcgcttg gccgtaagtc cgctttgcag ccaaaccgaa     300

cccaagtcct gcgataaaac ccacacatgt ccaccgtgtg atcccgccga gcccaaatct     360

cctgacaaaa ctcacacatg cccaccgtgc ccagcacctg aactcctggg gggaccgtca     420

gtcttcctct tccccccaaa acccaaggac accctcatga tctcccggac ccctgaggtc     480

acatgcgtgg tggtggacgt gagccacgaa gaccctgagg tcaagttcaa ctggtacgtg     540

gacggcgtgg aggtgcataa tgccaagaca aagccgcggg aggagcagta caacagcacg     600

taccgtgtgg tcagcgtcct caccgtcctg caccaggact ggctgaatgg caaggagtac     660

aagtgcaagg tctccaacaa agccctccca gcccccatcg agaaaaccat ctccaaagcc     720

aaagggcagc cccgagaacc acaggtgtac accctgcccc catcccggga tgagctgacc     780

aagaaccagg tcagcctgac ctgcctggtc aaaggcttct atcccagcga catcgccgtg     840

gagtgggaga gcaatgggca accggagaac aactacaaga ccacgcctcc cgtgctggac     900

tccgacggct ccttcttcct ctacagcaag ctcaccgtgg acaagagcag gtggcagcag     960

gggaacgtct tctcatgctc cgtgatgcat gaggctctgc acaaccacta cacgcagaag    1020

agcctctccc tgtctccggg taaaaaagat cccaaatttt gggtgctggt ggtggttggt    1080

ggagtcctgg cttgctatag cttgctagta acagtggcct ttattatttt ctgggtgagg    1140

agtaagagga gcaggctcct gcacagtgac tacatgaaca tgactccccg ccgccccggg    1200

cccacccgca agcattacca gccctatgcc ccaccacgcg acttcgcagc ctatcgctcc    1260

agagtgaagt tcagcaggag cgcagacgcc cccgcgtacc agcagggcca gaaccagctc    1320

tataacgagc tcaatctagg acgaagagag gagtacgatg ttttggacaa gagacgtggc    1380

cgggaccctg agatgggggg aaagccgaga aggaagaacc ctcaggaagg cctgtacaat    1440

gaactgcaga aagataagat ggcggaggcc tacagtgaga ttgggatgaa aggcgagcgc    1500

cggaggggca aggggcacga tggcctttac cagggtctca gtacagccac caaggacacc    1560

tacgacgccc ttcacatgca ggccctgcct cctcgctaa                           1599

<210>  57
<211>  1605
<212>  DNA

<213>  Artificial

<220>
<223>  CAR 008 translation region

<400>  57
atggagtttg ggctttcctg gttgttcctc gtagccatcc tcaaaggggt tcagtgcgca      60

gaagcggctg gtctgggtcc ctcaccagaa cagcgagtcg agattgtgcc tcgcgatctc     120

cgcatgaagg acaagttcct gaagcatctg acaggcccgc tgtacttcag tcccaagtgt     180

agcaaacact ttcatcgcct gtatcacaac acacgggatt gcaccatacc cgcctattac     240

aagaggtgtg caaggctgct gactcgattg gccgtttctc ctgtgtgcat ggaggacaaa     300

caggagccca agagctgcga caagacgcac acctgtccac cgtgtgatcc cgccgagccc     360

aaatctcctg acaaaactca cacatgccca ccgtgcccag cacctgaact cctgggggga     420

ccgtcagtct tcctcttccc cccaaaaccc aaggacaccc tcatgatctc ccggacccct     480

gaggtcacat gcgtggtggt ggacgtgagc cacgaagacc ctgaggtcaa gttcaactgg     540

tacgtggacg gcgtggaggt gcataatgcc aagacaaagc cgcgggagga gcagtacaac     600

agcacgtacc gtgtggtcag cgtcctcacc gtcctgcacc aggactggct gaatggcaag     660

gagtacaagt gcaaggtctc caacaaagcc ctcccagccc ccatcgagaa aaccatctcc     720

aaagccaaag ggcagccccg agaaccacag gtgtacaccc tgcccccatc ccgggatgag     780

ctgaccaaga accaggtcag cctgacctgc ctggtcaaag gcttctatcc cagcgacatc     840

gccgtggagt gggagagcaa tgggcaaccg gagaacaact acaagaccac gcctcccgtg     900

ctggactccg acggctcctt cttcctctac agcaagctca ccgtggacaa gagcaggtgg     960

cagcagggga acgtcttctc atgctccgtg atgcatgagg ctctgcacaa ccactacacg    1020

cagaagagcc tctccctgtc tccgggtaaa aaagatccca aattttgggt gctggtggtg    1080

gttggtggag tcctggcttg ctatagcttg ctagtaacag tggcctttat tattttctgg    1140

gtgaggagta agaggagcag gctcctgcac agtgactaca tgaacatgac tccccgccgc    1200

cccgggccca cccgcaagca ttaccagccc tatgccccac cacgcgactt cgcagcctat    1260

cgctccagag tgaagttcag caggagcgca gacgcccccg cgtaccagca gggccagaac    1320

cagctctata acgagctcaa tctaggacga agagaggagt acgatgtttt ggacaagaga    1380

cgtggccggg accctgagat ggggggaaag ccgagaagga agaaccctca ggaaggcctg    1440

tacaatgaac tgcagaaaga taagatggcg gaggcctaca gtgagattgg gatgaaaggc    1500

gagcgccgga ggggcaaggg gcacgatggc ctttaccagg gtctcagtac agccaccaag    1560

gacacctacg acgcccttca catgcaggcc ctgccccctc gctaa                    1605

<210>  58
<211>  534

<212>  PRT
<213>  Artificial

<220>
<223>  Car 008 translation region

<400>  58

Met Glu Phe Gly Leu Ser Trp Leu Phe Leu Val Ala Ile Leu Lys Gly
1               5                   10                  15

Val Gln Cys Ala Glu Ala Ala Gly Leu Gly Pro Ser Pro Glu Gln Arg
            20                  25                  30

Val Glu Ile Val Pro Arg Asp Leu Arg Met Lys Asp Lys Phe Leu Lys
        35                  40                  45

His Leu Thr Gly Pro Leu Tyr Phe Ser Pro Lys Cys Ser Lys His Phe
    50                  55                  60

His Arg Leu Tyr His Asn Thr Arg Asp Cys Thr Ile Pro Ala Tyr Tyr
65                  70                  75                  80

Lys Arg Cys Ala Arg Leu Leu Thr Arg Leu Ala Val Ser Pro Val Cys
                85                  90                  95

Met Glu Asp Lys Gln Glu Pro Lys Ser Cys Asp Lys Thr His Thr Cys
            100                 105                 110

Pro Pro Cys Asp Pro Ala Glu Pro Lys Ser Pro Asp Lys Thr His Thr
        115                 120                 125

Cys Pro Pro Cys Pro Ala Pro Glu Leu Leu Gly Gly Pro Ser Val Phe
    130                 135                 140

Leu Phe Pro Pro Lys Pro Lys Asp Thr Leu Met Ile Ser Arg Thr Pro
145                 150                 155                 160

Glu Val Thr Cys Val Val Val Asp Val Ser His Glu Asp Pro Glu Val
                165                 170                 175

Lys Phe Asn Trp Tyr Val Asp Gly Val Glu Val His Asn Ala Lys Thr
            180                 185                 190

Lys Pro Arg Glu Glu Gln Tyr Asn Ser Thr Tyr Arg Val Val Ser Val
        195                 200                 205

Leu Thr Val Leu His Gln Asp Trp Leu Asn Gly Lys Glu Tyr Lys Cys
    210                 215                 220

Lys Val Ser Asn Lys Ala Leu Pro Ala Pro Ile Glu Lys Thr Ile Ser
225                 230                 235                 240

Lys Ala Lys Gly Gln Pro Arg Glu Pro Gln Val Tyr Thr Leu Pro Pro
                245                 250                 255

Ser Arg Asp Glu Leu Thr Lys Asn Gln Val Ser Leu Thr Cys Leu Val
            260                 265                 270

Lys Gly Phe Tyr Pro Ser Asp Ile Ala Val Glu Trp Glu Ser Asn Gly
        275                 280                 285

Gln Pro Glu Asn Asn Tyr Lys Thr Thr Pro Pro Val Leu Asp Ser Asp
    290                 295                 300

Gly Ser Phe Phe Leu Tyr Ser Lys Leu Thr Val Asp Lys Ser Arg Trp
305                 310                 315                 320

Gln Gln Gly Asn Val Phe Ser Cys Ser Val Met His Glu Ala Leu His
                325                 330                 335

Asn His Tyr Thr Gln Lys Ser Leu Ser Leu Ser Pro Gly Lys Lys Asp
            340                 345                 350

Pro Lys Phe Trp Val Leu Val Val Val Gly Gly Val Leu Ala Cys Tyr
        355                 360                 365

Ser Leu Leu Val Thr Val Ala Phe Ile Ile Phe Trp Val Arg Ser Lys
    370                 375                 380

Arg Ser Arg Leu Leu His Ser Asp Tyr Met Asn Met Thr Pro Arg Arg
385                 390                 395                 400

Pro Gly Pro Thr Arg Lys His Tyr Gln Pro Tyr Ala Pro Pro Arg Asp
                405                 410                 415

Phe Ala Ala Tyr Arg Ser Arg Val Lys Phe Ser Arg Ser Ala Asp Ala
            420                 425                 430

Pro Ala Tyr Gln Gln Gly Gln Asn Gln Leu Tyr Asn Glu Leu Asn Leu
        435                 440                 445

Gly Arg Arg Glu Glu Tyr Asp Val Leu Asp Lys Arg Arg Gly Arg Asp
    450                 455                 460

Pro Glu Met Gly Gly Lys Pro Arg Arg Lys Asn Pro Gln Glu Gly Leu
465                 470                 475                 480

Tyr Asn Glu Leu Gln Lys Asp Lys Met Ala Glu Ala Tyr Ser Glu Ile
                485                 490                 495

Gly Met Lys Gly Glu Arg Arg Arg Gly Lys Gly His Asp Gly Leu Tyr
            500                 505                 510

Gln Gly Leu Ser Thr Ala Thr Lys Asp Thr Tyr Asp Ala Leu His Met
        515                 520                 525

Gln Ala Leu Pro Pro Arg
    530

<210>  59
<211>  41
<212>  DNA
<213>  Artificial

<220>
<223>  forward primer

<400>  59
gatccagcct ccggactgcc accatggaga cagacacact c                          41

<210>  60
<211>  41
<212>  DNA
<213>  Artificial

<220>
<223>  reverse primer

<400>  60
atcaaactca ttactaatca tcactgtttg tcctccatgc a                          41

<210>  61
<211>  35
<212>  DNA
<213>  Artificial

<220>
<223>  forward primer

<400>  61
gtgctcttcc aaggaccagg ggcagagcct cggga                                 35

<210>  62
<211>  264
<212>  PRT
<213>  Artificial

<220>
<223>  Ligand 004P

<400>  62

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Gly Ala Glu Pro Arg Glu Pro Ala
    130                 135                 140

Asp Gly Gln Ala Leu Leu Arg Leu Val Val Glu Leu Val Gln Glu Leu
145                 150                 155                 160

Arg Lys His His Ser Ala Glu His Lys Gly Leu Gln Leu Leu Gly Arg
                165                 170                 175

Asp Cys Ala Leu Gly Arg Ala Glu Ala Ala Gly Leu Gly Pro Ser Pro
            180                 185                 190

Glu Gln Arg Val Glu Ile Val Pro Arg Asp Leu Arg Met Lys Asp Lys
        195                 200                 205

Phe Leu Lys His Leu Thr Gly Pro Leu Tyr Phe Ser Pro Lys Cys Ser
    210                 215                 220

Lys His Phe His Arg Leu Tyr His Asn Thr Arg Asp Cys Thr Ile Pro
225                 230                 235                 240

Ala Tyr Tyr Lys Arg Cys Ala Arg Leu Leu Thr Arg Leu Ala Val Ser
                245                 250                 255

Pro Val Cys Met Glu Asp Lys Gln
            260

<210>  63
<211>  792
<212>  DNA
<213>  Artificial

<220>
<223>  Ligand 004P coding sequence

<400>  63
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagg ggcagagcct     420

cgggaaccag ccgatggcca agcgcttctg aggctggtcg ttgagctggt gcaggaactc     480

cggaaacacc acagtgccga gcacaaaggc ctgcagctgc ttggacggga ctgtgcattg     540

ggtcgagccg aagctgcagg gttgggtccc tcaccggagc aaagggtgga gatcgtgcct     600

agagatctcc gcatgaagga caagttcctg aagcacctga caggcccact gtacttcagc     660

ccaaaatgct ccaagcactt tcatcgcctg tatcacaaca ctcgcgattg caccattccc     720

gcctactata agcgttgtgc caggctgctg acgcgattgg ctgtgtctcc cgtatgcatg     780

gaggacaaac ag                                                         792


<210>  64
<211>  35
<212>  DNA
<213>  Artificial

<220>
<223>  forward primer

<400>  64
ctcttccaag gaccagcatt gggtcgagcc gaagc                                 35


<210>  65
<211>  35
<212>  DNA
<213>  Artificial

<220>
<223>  forward primer

<400>  65
ctcttccaag gaccaggtcg agccgaagct gcagg                                35

<210>  66
<211>  35
<212>  DNA
<213>  Artificial

<220>
<223>  forward primer

<400>  66
ctcttccaag gaccagctgc agggttgggt ccctc                                35

<210>  67
<211>  35
<212>  DNA
<213>  Artificial

<220>
<223>  forward primer

<400>  67
ctcttccaag gaccagggtt gggtccctca ccgga                                35

<210>  68
<211>  35
<212>  DNA
<213>  Artificial

<220>
<223>  forward primer

<400>  68
ctcttccaag gaccaggtcc ctcaccggag caaag                                35

<210>  69
<211>  35
<212>  DNA
<213>  Artificial

<220>
<223>  forward primer

<400>  69
ctcttccaag gaccatcacc ggagcaaagg gtgga                                35

<210>  70
<211>  35
<212>  DNA
<213>  Artificial

<220>
<223>  forward primer

<400>  70
ctcttccaag gaccagagca aagggtggag atcgt                                35

<210>  71
<211>  35
<212>  DNA
<213>  Artificial

<220>
<223>  forward primer

<400>  71
ctcttccaag gaccaagggt ggagatcgtg cctag                                35


<210>  72
<211>  35
<212>  DNA
<213>  Artificial

<220>
<223>  forward primer

<400>  72
ctcttccaag gaccagagat cgtgcctaga gatct                                35


<210>  73
<211>  35
<212>  DNA
<213>  Artificial

<220>
<223>  forward primer

<400>  73
ctcttccaag gaccagtgcc tagagatctc cgcat                                35


<210>  74
<211>  35
<212>  DNA
<213>  Artificial

<220>
<223>  forward primer

<400>  74
ctcttccaag gaccaagaga tctccgcatg aagga                                35


<210>  75
<211>  35
<212>  DNA
<213>  Artificial

<220>
<223>  forward primer

<400>  75
ctcttccaag gaccactccg catgaaggac aagtt                                35


<210>  76

<211>  35
<212>  DNA
<213>  Artificial

<220>
<223>  forward primer

<400>  76
ctcttccaag gaccaatgaa ggacaagttc ctgaa                                35


<210>  77
<211>  35
<212>  DNA
<213>  Artificial

<220>
<223>  forward primer

<400>  77
ctcttccaag gaccagacaa gttcctgaag cacct                                35


<210>  78
<211>  35
<212>  DNA
<213>  Artificial

<220>
<223>  forward primer

<400>  78
ctcttccaag gaccattcct gaagcacctg acagg                                35


<210>  79
<211>  35
<212>  DNA
<213>  Artificial

<220>
<223>  forward primer

<400>  79
ctcttccaag gaccaaagca cctgacaggc ccact                                35


<210>  80
<211>  35
<212>  DNA
<213>  Artificial

<220>
<223>  forward primer

<400>  80
ctcttccaag gaccactgac aggcccactg tactt                                35


<210>  81
<211>  35
<212>  DNA
<213>  Artificial

<220>
<223>  forward primer

<400>  81
ctcttccaag gaccaggccc actgtacttc agccc                                35


<210>  82
<211>  39
<212>  DNA
<213>  Artificial

<220>
<223>  reverse primer

<400>  82
caaactcatt actaatcatc agtcctccat gcatacggg                            39


<210>  83
<211>  39
<212>  DNA
<213>  Artificial

<220>
<223>  reverse primer

<400>  83
caaactcatt actaatcatc acatgcatac gggagacac                            39


<210>  84
<211>  24
<212>  DNA
<213>  Artificial

<220>
<223>  reverse primer

<400>  84
tggtccttgg aagagcactt ccag                                            24


<210>  85
<211>  222
<212>  PRT
<213>  Artificial

<220>
<223>  Ligand 005P

<400>  85

Met Glu Thr Asp Thr Leu Leu Leu Trp Val Leu Leu Leu Trp Val Pro
1               5                   10                  15


Gly Ser Thr Gly Asp Gly His His His His His His Gly Ser Leu Gln
            20                  25                  30


Asp Ser Glu Val Asn Gln Glu Ala Lys Pro Glu Val Lys Pro Glu Val

35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Leu Gly Arg Ala Glu Ala Ala
    130                 135                 140

Gly Leu Gly Pro Ser Pro Glu Gln Arg Val Glu Ile Val Pro Arg Asp
145                 150                 155                 160

Leu Arg Met Lys Asp Lys Phe Leu Lys His Leu Thr Gly Pro Leu Tyr
                165                 170                 175

Phe Ser Pro Lys Cys Ser Lys His Phe His Arg Leu Tyr His Asn Thr
            180                 185                 190

Arg Asp Cys Thr Ile Pro Ala Tyr Tyr Lys Arg Cys Ala Arg Leu Leu
        195                 200                 205

Thr Arg Leu Ala Val Ser Pro Val Cys Met Glu Asp Lys Gln
    210                 215                 220

<210>  86
<211>  666
<212>  DNA
<213>  Artificial

<220>
<223>  Ligand 005P coding sequence

<400>  86
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt 300

gaaattcaag ctgatcagac ccctgaagat ttggacatgg aggataacga tattattgag 360

gctcacagag aacagattgg aggtctggaa gtgctcttcc aaggaccagc attgggtcga 420

gccgaagctg cagggttggg tccctcaccg gagcaaaggg tggagatcgt gcctagagat 480

ctccgcatga aggacaagtt cctgaagcac ctgacaggcc cactgtactt cagcccaaaa 540

tgctccaagc actttcatcg cctgtatcac aacactcgcg attgcaccat tcccgcctac 600

tataagcgtt gtgccaggct gctgacgcga ttggctgtgt ctcccgtatg catggaggac 660

aaacag 666

<210> 87
<211> 220
<212> PRT
<213> Artificial

<220>
<223> Ligand 006P

<400> 87

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Gly Arg Ala Glu Ala Ala Gly Leu
    130                 135                 140

Gly Pro Ser Pro Glu Gln Arg Val Glu Ile Val Pro Arg Asp Leu Arg
145                 150                 155                 160

Met Lys Asp Lys Phe Leu Lys His Leu Thr Gly Pro Leu Tyr Phe Ser
                165                 170                 175

Pro Lys Cys Ser Lys His Phe His Arg Leu Tyr His Asn Thr Arg Asp
            180                 185                 190

Cys Thr Ile Pro Ala Tyr Tyr Lys Arg Cys Ala Arg Leu Leu Thr Arg
        195                 200                 205

Leu Ala Val Ser Pro Val Cys Met Glu Asp Lys Gln
    210                 215                 220

<210>  88
<211>  660
<212>  DNA
<213>  Artificial

<220>
<223>  Ligand 006P coding sequence

<400>  88
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagg tcgagccgaa     420

gctgcagggt tgggtccctc accggagcaa agggtggaga tcgtgcctag agatctccgc     480

atgaaggaca agttcctgaa gcacctgaca ggcccactgt acttcagccc aaaatgctcc     540

aagcactttc atcgcctgta tcacaacact cgcgattgca ccattcccgc ctactataag     600

cgttgtgcca ggctgctgac gcgattggct gtgtctcccg tatgcatgga ggacaaacag     660

<210>  89
<211>  216
<212>  PRT
<213>  Artificial

<220>
<223>  Ligand 007P

<400>  89

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Ala Gly Leu Gly Pro Ser Pro
    130                 135                 140

Glu Gln Arg Val Glu Ile Val Pro Arg Asp Leu Arg Met Lys Asp Lys
145                 150                 155                 160

Phe Leu Lys His Leu Thr Gly Pro Leu Tyr Phe Ser Pro Lys Cys Ser
                165                 170                 175

Lys His Phe His Arg Leu Tyr His Asn Thr Arg Asp Cys Thr Ile Pro
            180                 185                 190

Ala Tyr Tyr Lys Arg Cys Ala Arg Leu Leu Thr Arg Leu Ala Val Ser
        195                 200                 205

Pro Val Cys Met Glu Asp Lys Gln
    210                 215

<210>  90
<211>  648
<212>  DNA
<213>  Artificial

<220>
<223>  Ligand 007P coding sequence

<400>  90
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagc tgcagggttg     420

ggtccctcac cggagcaaag ggtggagatc gtgcctagag atctccgcat gaaggacaag     480

ttcctgaagc acctgacagg cccactgtac ttcagcccaa aatgctccaa gcactttcat     540

cgcctgtatc acaacactcg cgattgcacc attcccgcct actataagcg ttgtgccagg     600

ctgctgacgc gattggctgt gtctcccgta tgcatggagg acaaacag                  648

<210>  91
<211>  214
<212>  PRT
<213>  Artificial

<220>
<223>  Ligand 008P

<400>  91

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Gly Leu Gly Pro Ser Pro Glu Gln
    130                 135                 140

Arg Val Glu Ile Val Pro Arg Asp Leu Arg Met Lys Asp Lys Phe Leu
145                 150                 155                 160

Lys His Leu Thr Gly Pro Leu Tyr Phe Ser Pro Lys Cys Ser Lys His
                165                 170                 175

Phe His Arg Leu Tyr His Asn Thr Arg Asp Cys Thr Ile Pro Ala Tyr
            180                 185                 190

Tyr Lys Arg Cys Ala Arg Leu Leu Thr Arg Leu Ala Val Ser Pro Val
        195                 200                 205

Cys Met Glu Asp Lys Gln
    210

<210>  92
<211>  642
<212>  DNA
<213>  Artificial

<220>
<223>  Ligand 008P coding sequence

<400>  92
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagg gttgggtccc     420

tcaccggagc aaagggtgga gatcgtgcct agagatctcc gcatgaagga caagttcctg     480

aagcacctga caggcccact gtacttcagc ccaaaatgct ccaagcactt tcatcgcctg     540

tatcacaaca ctcgcgattg caccattccc gcctactata agcgttgtgc caggctgctg     600

acgcgattgg ctgtgtctcc cgtatgcatg gaggacaaac ag                        642

<210>  93
<211>  212
<212>  PRT

<213>  Artificial

<220>
<223>  Ligand 009P

<400>  93

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Gly Pro Ser Pro Glu Gln Arg Val
    130                 135                 140

Glu Ile Val Pro Arg Asp Leu Arg Met Lys Asp Lys Phe Leu Lys His
145                 150                 155                 160

Leu Thr Gly Pro Leu Tyr Phe Ser Pro Lys Cys Ser Lys His Phe His
                165                 170                 175

Arg Leu Tyr His Asn Thr Arg Asp Cys Thr Ile Pro Ala Tyr Tyr Lys
            180                 185                 190

Arg Cys Ala Arg Leu Leu Thr Arg Leu Ala Val Ser Pro Val Cys Met
        195                 200                 205

Glu Asp Lys Gln
    210

<210>  94
<211>  636
<212>  DNA
<213>  Artificial

<220>
<223>  Ligand 009P coding sequence

<400>  94
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagg tccctcaccg     420

gagcaaaggg tggagatcgt gcctagagat ctccgcatga aggacaagtt cctgaagcac     480

ctgacaggcc cactgtactt cagcccaaaa tgctccaagc actttcatcg cctgtatcac     540

aacactcgcg attgcaccat tcccgcctac tataagcgtt gtgccaggct gctgacgcga     600

ttggctgtgt ctcccgtatg catggaggac aaacag                               636

<210>  95
<211>  210
<212>  PRT
<213>  Artificial

<220>
<223>  Ligand 010P

<400>  95

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu

85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ser Pro Glu Gln Arg Val Glu Ile
    130                 135                 140

Val Pro Arg Asp Leu Arg Met Lys Asp Lys Phe Leu Lys His Leu Thr
145                 150                 155                 160

Gly Pro Leu Tyr Phe Ser Pro Lys Cys Ser Lys His Phe His Arg Leu
                165                 170                 175

Tyr His Asn Thr Arg Asp Cys Thr Ile Pro Ala Tyr Tyr Lys Arg Cys
            180                 185                 190

Ala Arg Leu Leu Thr Arg Leu Ala Val Ser Pro Val Cys Met Glu Asp
        195                 200                 205

Lys Gln
    210

<210>  96
<211>  630
<212>  DNA
<213>  Artificial

<220>
<223>  Ligand 010P coding sequence

<400>  96
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccatc accggagcaa     420

agggtggaga tcgtgcctag agatctccgc atgaaggaca agttcctgaa gcacctgaca     480

ggcccactgt acttcagccc aaaatgctcc aagcactttc atcgcctgta tcacaacact     540

cgcgattgca ccattcccgc ctactataag cgttgtgcca ggctgctgac gcgattggct     600

gtgtctcccg tatgcatgga ggacaaacag                                    630

<210>  97
<211>  208
<212>  PRT
<213>  Artificial

<220>
<223>  Ligand 011P

<400>  97

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Glu Gln Arg Val Glu Ile Val Pro
    130                 135                 140

Arg Asp Leu Arg Met Lys Asp Lys Phe Leu Lys His Leu Thr Gly Pro
145                 150                 155                 160

Leu Tyr Phe Ser Pro Lys Cys Ser Lys His Phe His Arg Leu Tyr His
                165                 170                 175

Asn Thr Arg Asp Cys Thr Ile Pro Ala Tyr Tyr Lys Arg Cys Ala Arg
            180                 185                 190

Leu Leu Thr Arg Leu Ala Val Ser Pro Val Cys Met Glu Asp Lys Gln

195                 200                 205

<210>  98
<211>  624
<212>  DNA
<213>  Artificial

<220>
<223>  Ligand 011P coding sequence

<400>  98
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccaga gcaaagggtg     420

gagatcgtgc ctagagatct ccgcatgaag gacaagttcc tgaagcacct gacaggccca     480

ctgtacttca gcccaaaatg ctccaagcac tttcatcgcc tgtatcacaa cactcgcgat     540

tgcaccattc ccgcctacta taagcgttgt gccaggctgc tgacgcgatt ggctgtgtct     600

cccgtatgca tggaggacaa acag                                            624

<210>  99
<211>  206
<212>  PRT
<213>  Artificial

<220>
<223>  Ligand 012P

<400>  99

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Arg Val Glu Ile Val Pro Arg Asp
    130                 135                 140

Leu Arg Met Lys Asp Lys Phe Leu Lys His Leu Thr Gly Pro Leu Tyr
145                 150                 155                 160

Phe Ser Pro Lys Cys Ser Lys His Phe His Arg Leu Tyr His Asn Thr
                165                 170                 175

Arg Asp Cys Thr Ile Pro Ala Tyr Tyr Lys Arg Cys Ala Arg Leu Leu
            180                 185                 190

Thr Arg Leu Ala Val Ser Pro Val Cys Met Glu Asp Lys Gln
        195                 200                 205

<210>  100
<211>  618
<212>  DNA
<213>  Artificial

<220>
<223>  Ligand 012P coding sequence

<400>  100
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccaag ggtggagatc     420

gtgcctagag atctccgcat gaaggacaag ttcctgaagc acctgacagg cccactgtac     480

ttcagcccaa aatgctccaa gcactttcat cgcctgtatc acaacactcg cgattgcacc     540

attcccgcct actataagcg ttgtgccagg ctgctgacgc gattggctgt gtctcccgta     600

tgcatggagg acaaacag                                                    618

<210>  101
<211>  204
<212>  PRT
<213>  Artificial

<220>
<223>  Ligand 013P

<400>  101

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Glu Ile Val Pro Arg Asp Leu Arg
    130                 135                 140

Met Lys Asp Lys Phe Leu Lys His Leu Thr Gly Pro Leu Tyr Phe Ser
145                 150                 155                 160

Pro Lys Cys Ser Lys His Phe His Arg Leu Tyr His Asn Thr Arg Asp
                165                 170                 175

Cys Thr Ile Pro Ala Tyr Tyr Lys Arg Cys Ala Arg Leu Leu Thr Arg
            180                 185                 190

Leu Ala Val Ser Pro Val Cys Met Glu Asp Lys Gln
        195                 200

<210>  102
<211>  612
<212>  DNA
<213>  Artificial

<220>
<223>  Ligand 013P coding sequence

<400>  102
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccaga gatcgtgcct     420

agagatctcc gcatgaagga caagttcctg aagcacctga caggcccact gtacttcagc     480

ccaaaatgct ccaagcactt tcatcgcctg tatcacaaca ctcgcgattg caccattccc     540

gcctactata agcgttgtgc caggctgctg acgcgattgg ctgtgtctcc cgtatgcatg     600

gaggacaaac ag                                                         612


<210>  103
<211>  202
<212>  PRT
<213>  Artificial

<220>
<223>  Ligand 014P

<400>  103

Met Glu Thr Asp Thr Leu Leu Leu Trp Val Leu Leu Leu Trp Val Pro
1               5                   10                  15


Gly Ser Thr Gly Asp Gly His His His His His His Gly Ser Leu Gln
            20                  25                  30


Asp Ser Glu Val Asn Gln Glu Ala Lys Pro Glu Val Lys Pro Glu Val
        35                  40                  45


Lys Pro Glu Thr His Ile Asn Leu Lys Val Ser Asp Gly Ser Ser Glu
    50                  55                  60


Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Val Pro Arg Asp Leu Arg Met Lys
    130                 135                 140

Asp Lys Phe Leu Lys His Leu Thr Gly Pro Leu Tyr Phe Ser Pro Lys
145                 150                 155                 160

Cys Ser Lys His Phe His Arg Leu Tyr His Asn Thr Arg Asp Cys Thr
                165                 170                 175

Ile Pro Ala Tyr Tyr Lys Arg Cys Ala Arg Leu Leu Thr Arg Leu Ala
            180                 185                 190

Val Ser Pro Val Cys Met Glu Asp Lys Gln
        195                 200

<210>  104
<211>  606
<212>  DNA
<213>  Artificial

<220>
<223>  Ligand 014P coding sequence

<400>  104
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagt gcctagagat     420

ctccgcatga aggacaagtt cctgaagcac ctgacaggcc cactgtactt cagcccaaaa     480

tgctccaagc actttcatcg cctgtatcac aacactcgcg attgcaccat tcccgcctac     540

tataagcgtt gtgccaggct gctgacgcga ttggctgtgt ctcccgtatg catggaggac     600

aaacag                                                                606

<210>  105
<211>  200
<212>  PRT
<213>  Artificial

<220>
<223>  Ligand 015P

<400>  105

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Arg Asp Leu Arg Met Lys Asp Lys
    130                 135                 140

Phe Leu Lys His Leu Thr Gly Pro Leu Tyr Phe Ser Pro Lys Cys Ser
145                 150                 155                 160

Lys His Phe His Arg Leu Tyr His Asn Thr Arg Asp Cys Thr Ile Pro
                165                 170                 175

Ala Tyr Tyr Lys Arg Cys Ala Arg Leu Leu Thr Arg Leu Ala Val Ser
            180                 185                 190

Pro Val Cys Met Glu Asp Lys Gln
        195                 200

<210>  106
<211>  600
<212>  DNA
<213>  Artificial

<220>
<223>  Ligand 015P coding sequence

<400>  106
atggagacag acacactcct gctatgggta ctgctgctct gggttccagg ttccactggt        60

gacggtcatc accatcatca tcacgggtcc ctgcaggact cagaagtcaa tcaagaagct       120

aagccagagg tcaagccaga agtcaagcct gagactcaca tcaatttaaa ggtgtccgat       180

ggatcttcag agatcttctt caagatcaaa aagaccactc ctttaagaag gctgatggaa       240

gcgttcgcta aaagacaggg taaggaaatg gactccttaa cgttcttgta cgacggtatt       300

gaaattcaag ctgatcagac ccctgaagat ttggacatgg aggataacga tattattgag       360

gctcacagag aacagattgg aggtctggaa gtgctcttcc aaggaccaag agatctccgc       420

atgaaggaca agttcctgaa gcacctgaca ggcccactgt acttcagccc aaaatgctcc       480

aagcactttc atcgcctgta tcacaacact cgcgattgca ccattcccgc ctactataag       540

cgttgtgcca ggctgctgac gcgattggct gtgtctcccg tatgcatgga ggacaaacag       600


<210>  107
<211>  198
<212>  PRT
<213>  Artificial

<220>
<223>  Ligand 016P

<400>  107

Met Glu Thr Asp Thr Leu Leu Leu Trp Val Leu Leu Leu Trp Val Pro
1               5                   10                  15


Gly Ser Thr Gly Asp Gly His His His His His His Gly Ser Leu Gln
            20                  25                  30


Asp Ser Glu Val Asn Gln Glu Ala Lys Pro Glu Val Lys Pro Glu Val
        35                  40                  45


Lys Pro Glu Thr His Ile Asn Leu Lys Val Ser Asp Gly Ser Ser Glu
    50                  55                  60


Ile Phe Phe Lys Ile Lys Lys Thr Thr Pro Leu Arg Arg Leu Met Glu
65                  70                  75                  80


Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Leu Arg Met Lys Asp Lys Phe Leu
    130                 135                 140

Lys His Leu Thr Gly Pro Leu Tyr Phe Ser Pro Lys Cys Ser Lys His
145                 150                 155                 160

Phe His Arg Leu Tyr His Asn Thr Arg Asp Cys Thr Ile Pro Ala Tyr
                165                 170                 175

Tyr Lys Arg Cys Ala Arg Leu Leu Thr Arg Leu Ala Val Ser Pro Val
            180                 185                 190

Cys Met Glu Asp Lys Gln
        195

<210>  108
<211>  594
<212>  DNA
<213>  Artificial

<220>
<223>  Ligand 016P coding sequence

<400>  108
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccact ccgcatgaag     420

gacaagttcc tgaagcacct gacaggccca ctgtacttca gcccaaaatg ctccaagcac     480

tttcatcgcc tgtatcacaa cactcgcgat tgcaccattc ccgcctacta taagcgttgt     540

gccaggctgc tgacgcgatt ggctgtgtct cccgtatgca tggaggacaa acag           594

<210>  109
<211>  196
<212>  PRT

<213>  Artificial

<220>
<223>  Ligand 017P

<400>  109

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Met Lys Asp Lys Phe Leu Lys His
    130                 135                 140

Leu Thr Gly Pro Leu Tyr Phe Ser Pro Lys Cys Ser Lys His Phe His
145                 150                 155                 160

Arg Leu Tyr His Asn Thr Arg Asp Cys Thr Ile Pro Ala Tyr Tyr Lys
                165                 170                 175

Arg Cys Ala Arg Leu Leu Thr Arg Leu Ala Val Ser Pro Val Cys Met
            180                 185                 190

Glu Asp Lys Gln
        195

<210>  110
<211>  588
<212>  DNA
<213>  Artificial

<220>
<223>  Ligand 017P coding sequence

<400>  110
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccaat gaaggacaag     420

ttcctgaagc acctgacagg cccactgtac ttcagcccaa aatgctccaa gcactttcat     480

cgcctgtatc acaacactcg cgattgcacc attcccgcct actataagcg ttgtgccagg     540

ctgctgacgc gattggctgt gtctcccgta tgcatggagg acaaacag                  588

<210>  111
<211>  194
<212>  PRT
<213>  Artificial

<220>
<223>  Ligand 018P

<400>  111

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
      115                 120                 125

Leu Glu Val Leu Phe Gln Gly Pro Asp Lys Phe Leu Lys His Leu Thr
    130                 135                 140

Gly Pro Leu Tyr Phe Ser Pro Lys Cys Ser Lys His Phe His Arg Leu
145                 150                 155                 160

Tyr His Asn Thr Arg Asp Cys Thr Ile Pro Ala Tyr Tyr Lys Arg Cys
                165                 170                 175

Ala Arg Leu Leu Thr Arg Leu Ala Val Ser Pro Val Cys Met Glu Asp
            180                 185                 190

Lys Gln

<210>  112
<211>  582
<212>  DNA
<213>  Artificial

<220>
<223>  Ligand 018P coding sequence

<400>  112
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccaga caagttcctg     420

aagcacctga caggcccact gtacttcagc ccaaaatgct ccaagcactt tcatcgcctg     480

tatcacaaca ctcgcgattg caccattccc gcctactata agcgttgtgc caggctgctg     540

acgcgattgg ctgtgtctcc cgtatgcatg gaggacaaac ag                        582

<210>  113
<211>  192
<212>  PRT
<213>  Artificial

<220>
<223>  Ligand 019P

<400>  113

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Phe Leu Lys His Leu Thr Gly Pro
    130                 135                 140

Leu Tyr Phe Ser Pro Lys Cys Ser Lys His Phe His Arg Leu Tyr His
145                 150                 155                 160

Asn Thr Arg Asp Cys Thr Ile Pro Ala Tyr Tyr Lys Arg Cys Ala Arg
                165                 170                 175

Leu Leu Thr Arg Leu Ala Val Ser Pro Val Cys Met Glu Asp Lys Gln
            180                 185                 190


<210>  114
<211>  576
<212>  DNA
<213>  Artificial

<220>
<223>  Ligand 019P coding sequence

<400>  114
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat 180

ggatcttcag agatcttctt caagatcaaa aagaccactc ctttaagaag gctgatggaa 240

gcgttcgcta aaagacaggg taaggaaatg gactccttaa cgttcttgta cgacggtatt 300

gaaattcaag ctgatcagac ccctgaagat ttggacatgg aggataacga tattattgag 360

gctcacagag aacagattgg aggtctggaa gtgctcttcc aaggaccatt cctgaagcac 420

ctgacaggcc cactgtactt cagcccaaaa tgctccaagc actttcatcg cctgtatcac 480

aacactcgcg attgcaccat tcccgcctac tataagcgtt gtgccaggct gctgacgcga 540

ttggctgtgt ctcccgtatg catggaggac aaacag 576

<210> 115
<211> 190
<212> PRT
<213> Artificial

<220>
<223> Ligand 020P

<400> 115

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Lys His Leu Thr Gly Pro Leu Tyr
    130                 135                 140

Phe Ser Pro Lys Cys Ser Lys His Phe His Arg Leu Tyr His Asn Thr
145                 150                 155                 160

Arg Asp Cys Thr Ile Pro Ala Tyr Tyr Lys Arg Cys Ala Arg Leu Leu
                165                 170                 175

Thr Arg Leu Ala Val Ser Pro Val Cys Met Glu Asp Lys Gln
            180                 185                 190

<210>  116
<211>  570
<212>  DNA
<213>  Artificial

<220>
<223>  Ligand 020P coding sequence

<400>  116
atggagacag acacactcct gctatgggta ctgctgctct gggttccagg ttccactggt        60

gacggtcatc accatcatca tcacggttcc ctgcaggact cagaagtcaa tcaagaagct        120

aagccagagg tcaagccaga agtcaagcct gagactcaca tcaatttaaa ggtgtccgat        180

ggatcttcag agatcttctt caagatcaaa aagaccactc ctttaagaag gctgatggaa        240

gcgttcgcta aaagacaggg taaggaaatg gactccttaa cgttcttgta cgacggtatt        300

gaaattcaag ctgatcagac ccctgaagat ttggacatgg aggataacga tattattgag        360

gctcacagag aacagattgg aggtctggaa gtgctcttcc aaggaccaaa gcacctgaca        420

ggcccactgt acttcagccc aaaatgctcc aagcactttc atcgcctgta tcacaacact        480

cgcgattgca ccattcccgc ctactataag cgttgtgcca ggctgctgac gcgattggct        540

gtgtctcccg tatgcatgga ggacaaacag                                          570

<210>  117
<211>  188
<212>  PRT
<213>  Artificial

<220>
<223>  Ligand 021P

<400>  117

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Leu Thr Gly Pro Leu Tyr Phe Ser
    130                 135                 140

Pro Lys Cys Ser Lys His Phe His Arg Leu Tyr His Asn Thr Arg Asp
145                 150                 155                 160

Cys Thr Ile Pro Ala Tyr Tyr Lys Arg Cys Ala Arg Leu Leu Thr Arg
                165                 170                 175

Leu Ala Val Ser Pro Val Cys Met Glu Asp Lys Gln
            180                 185

<210>  118
<211>  564
<212>  DNA
<213>  Artificial

<220>
<223>  Ligand 021P coding sequence

<400>  118
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccact gacaggccca     420

ctgtacttca gcccaaaatg ctccaagcac tttcatcgcc tgtatcacaa cactcgcgat     480

tgcaccattc ccgcctacta taagcgttgt gccaggctgc tgacgcgatt ggctgtgtct     540

cccgtatgca tggaggacaa acag                                                564

<210>  119
<211>  186
<212>  PRT
<213>  Artificial

<220>
<223>  Ligand 022P

<400>  119

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Gly Pro Leu Tyr Phe Ser Pro Lys
    130                 135                 140

Cys Ser Lys His Phe His Arg Leu Tyr His Asn Thr Arg Asp Cys Thr
145                 150                 155                 160

Ile Pro Ala Tyr Tyr Lys Arg Cys Ala Arg Leu Leu Thr Arg Leu Ala
                165                 170                 175

Val Ser Pro Val Cys Met Glu Asp Lys Gln
            180                 185

<210>  120

<211>  558
<212>  DNA
<213>  Artificial

<220>
<223>  Ligand 022P coding sequence

<400>  120
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagg cccactgtac     420

ttcagcccaa aatgctccaa gcactttcat cgcctgtatc acaacactcg cgattgcacc     480

attcccgcct actataagcg ttgtgccagg ctgctgacgc gattggctgt gtctcccgta     540

tgcatggagg acaaacag                                                   558

<210>  121
<211>  216
<212>  PRT
<213>  Artificial

<220>
<223>  Ligand 023P

<400>  121

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Glu Ala Ala Gly Leu Gly Pro
    130                 135                 140

Ser Pro Glu Gln Arg Val Glu Ile Val Pro Arg Asp Leu Arg Met Lys
145                 150                 155                 160

Asp Lys Phe Leu Lys His Leu Thr Gly Pro Leu Tyr Phe Ser Pro Lys
                165                 170                 175

Cys Ser Lys His Phe His Arg Leu Tyr His Asn Thr Arg Asp Cys Thr
            180                 185                 190

Ile Pro Ala Tyr Tyr Lys Arg Cys Ala Arg Leu Leu Thr Arg Leu Ala
        195                 200                 205

Val Ser Pro Val Cys Met Glu Asp
    210                 215

<210>  122
<211>  648
<212>  DNA
<213>  Artificial

<220>
<223>  Ligand 023P coding sequence

<400>  122
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagc cgaagctgca     420

gggttgggtc cctcaccgga gcaaagggtg gagatcgtgc ctagagatct ccgcatgaag     480

gacaagttcc tgaagcacct gacaggccca ctgtacttca gcccaaaatg ctccaagcac     540

tttcatcgcc tgtatcacaa cactcgcgat tgcaccattc ccgcctacta taagcgttgt     600

gccaggctgc tgacgcgatt ggctgtgtct cccgtatgca tggaggac                  648

<210>  123
<211>  214
<212>  PRT
<213>  Artificial

<220>
<223>  Ligand 024P

<400>  123

Met Glu Thr Asp Thr Leu Leu Leu Trp Val Leu Leu Leu Trp Val Pro
1               5                   10                  15

Gly Ser Thr Gly Asp Gly His His His His His His Gly Ser Leu Gln
            20                  25                  30

Asp Ser Glu Val Asn Gln Glu Ala Lys Pro Glu Val Lys Pro Glu Val
        35                  40                  45

Lys Pro Glu Thr His Ile Asn Leu Lys Val Ser Asp Gly Ser Ser Glu
    50                  55                  60

Ile Phe Phe Lys Ile Lys Lys Thr Thr Pro Leu Arg Arg Leu Met Glu
65                  70                  75                  80

Ala Phe Ala Lys Arg Gln Gly Lys Glu Met Asp Ser Leu Thr Phe Leu
                85                  90                  95

Tyr Asp Gly Ile Glu Ile Gln Ala Asp Gln Thr Pro Glu Asp Leu Asp
            100                 105                 110

Met Glu Asp Asn Asp Ile Ile Glu Ala His Arg Glu Gln Ile Gly Gly
        115                 120                 125

Leu Glu Val Leu Phe Gln Gly Pro Ala Glu Ala Ala Gly Leu Gly Pro
    130                 135                 140

Ser Pro Glu Gln Arg Val Glu Ile Val Pro Arg Asp Leu Arg Met Lys
145                 150                 155                 160

Asp Lys Phe Leu Lys His Leu Thr Gly Pro Leu Tyr Phe Ser Pro Lys
                165                 170                 175

Cys Ser Lys His Phe His Arg Leu Tyr His Asn Thr Arg Asp Cys Thr
            180                 185                 190

Ile Pro Ala Tyr Tyr Lys Arg Cys Ala Arg Leu Leu Thr Arg Leu Ala
        195                 200                 205

Val Ser Pro Val Cys Met
    210

<210>  124
<211>  642
<212>  DNA
<213>  Artificial

<220>
<223>  Ligand 024P coding sequence

<400>  124
atggagacag acacactcct gctatgggta ctgctgctct gggttccagg ttccactggt      60

gacggtcatc accatcatca tcacgggtcc ctgcaggact cagaagtcaa tcaagaagct     120

aagccagagg tcaagccaga agtcaagcct gagactcaca tcaatttaaa ggtgtccgat     180

ggatcttcag agatcttctt caagatcaaa aagaccactc ctttaagaag gctgatggaa     240

gcgttcgcta aaagacaggg taaggaaatg gactccttaa cgttcttgta cgacggtatt     300

gaaattcaag ctgatcagac ccctgaagat ttggacatgg aggataacga tattattgag     360

gctcacagag aacagattgg aggtctggaa gtgctcttcc aaggaccagc cgaagctgca     420

gggttgggtc cctcaccgga gcaaagggtg gagatcgtgc ctagagatct ccgcatgaag     480

gacaagttcc tgaagcacct gacaggccca ctgtacttca gcccaaaatg ctccaagcac     540

tttcatcgcc tgtatcacaa cactcgcgat tgcaccattc ccgcctacta taagcgttgt     600

gccaggctgc tgacgcgatt ggctgtgtct cccgtatgca tg                        642

<210>  125
<211>  114
<212>  PRT
<213>  Homo sapiens

<400>  125

Ala Ala Ala Thr Thr Thr Pro Ala Pro Arg Pro Pro Thr Pro Ala Pro
1               5                   10                  15

Thr Ile Ala Ser Gln Pro Leu Ser Leu Arg Pro Glu Ala Cys Arg Pro
            20                  25                  30

Ala Ala Gly Gly Ala Val His Thr Arg Gly Leu Asp Phe Ala Cys Asp
        35                  40                  45

Ile Tyr Ile Trp Ala Pro Leu Ala Gly Thr Cys Gly Val Leu Leu Leu
    50                  55                  60

Ser Leu Val Ile Thr Leu Tyr Cys Lys Arg Gly Arg Lys Lys Leu Leu
65                  70                  75                  80

Tyr Ile Phe Lys Gln Pro Phe Met Arg Pro Val Gln Thr Thr Gln Glu
                85                  90                  95

Glu Asp Gly Cys Ser Cys Arg Phe Pro Glu Glu Glu Glu Gly Gly Cys
            100                 105                 110

Glu Leu

<210>  126
<211>  372
<212>  DNA
<213>  Artificial

<220>
<223>  15bp+8a-41BB

<400>  126
ctgtctccgg gtaaagccgc ggcaaccacc actcctgctc ccagaccacc tactcccgct      60

cccaccatag cctctcaacc tctgagcctt aggccagaag cctgtcgtcc tgctgctggt     120

ggagccgttc acacaagggg attggacttt gcctgcgaca tctacatttg ggcaccattg     180

gcaggcacat gtggcgtgct tctcctgtca ctcgtcatta ccctgtattg caaacgaggg     240

cgcaagaagc tgctgtacat cttcaaacag ccctttatgc ggccagtgca gacgacacag     300

gaggaggatg ggtgctcctg cagattcccg gaagaggagg aaggtggctg tgagctgaga     360

gtgaagttca gc                                                         372

<210>  127
<211>  33
<212>  DNA
<213>  Artificial

<220>
<223>  forward primer

<400>  127
ctgtctccgg gtaaagccgc ggcaaccacc act                                   33

<210>  128
<211>  33
<212>  DNA
<213>  Artificial

<220>
<223>  reverse primer

<400>  128
gctgaacttc actctcagct cacagccacc ttc                                   33

<210>  129
<211>  28
<212>  DNA

<213>  Artificial

<220>
<223>  forward primer

<400>  129
agagtgaagt tcagcaggag cgcagacg                                          28


<210>  130
<211>  28
<212>  DNA
<213>  Artificial

<220>
<223>  reverse primer

<400>  130
tttacccgga gacagggaga ggctcttc                                          28


<210>  131
<211>  1872
<212>  DNA
<213>  Artificial

<220>
<223>  CAR 009 translation region

<400>  131
atggagttcg gactgtcctg gctgtttctg gtggccatac tgaaaggggt tcagtgcatg        60

aggccactga aaccgggagc acctcttcct gccctgtttc tgctcgcctt ggcactgtct       120

cctcatggcg ctcatggcag accacgcggg agaaggggtg ctagagtcac ggacaaagaa       180

cccaaacccc tgctgtttct tccagctgca ggggctggac gaactccctc aggcagtcgg       240

tcagccgaga ttttccctcg ggatagcaat ctcaaggaca agttcatcaa gcacttcacc       300

ggtcctgtga ccttctctcc cgagtgtagc aagcactttc atcgcctgta ttacaacaca       360

cgtgaatgct ccacaccagc ctactataag cggtgtgcga ggctcctcac tcgcttggcc       420

gtaagtccgc tttgcagcca aaccgaaccc aagtcctgcg ataaaaccca cacatgtcca       480

ccgtgtgatc ccgccgagcc caaatctcct gacaaaactc acacatgccc accgtgccca       540

gcacctgaac tcctgggggg accgtcagtc ttcctcttcc ccccaaaacc caaggacacc       600

ctcatgatct cccggacccc tgaggtcaca tgcgtggtgg tggacgtgag ccacgaagac       660

cctgaggtca agttcaactg gtacgtggac ggcgtggagg tgcataatgc caagacaaag       720

ccgcgggagg agcagtacaa cagcacgtac cgtgtggtca gcgtcctcac cgtcctgcac       780

caggactggc tgaatggcaa ggagtacaag tgcaaggtct ccaacaaagc cctcccagcc       840

cccatcgaga aaaccatctc caaagccaaa gggcagcccc gagaaccaca ggtgtacacc       900

ctgcccccat cccgggatga gctgaccaag aaccaggtca gcctgacctg cctggtcaaa       960

ggcttctatc ccagcgacat cgccgtggag tgggagagca atgggcaacc ggagaacaac      1020

tacaagacca cgcctcccgt gctggactcc gacggctcct tcttcctcta cagcaagctc 1080

accgtggaca agagcaggtg gcagcagggg aacgtcttct catgctccgt gatgcatgag 1140

gctctgcaca accactacac gcagaagagc ctctccctgt ctccgggtaa agccgcggca 1200

accaccactc ctgctcccag accacctact cccgctccca ccatagcctc tcaacctctg 1260

agccttaggc cagaagcctg tcgtcctgct gctggtggag ccgttcacac aaggggattg 1320

gactttgcct gcgacatcta catttgggca ccattggcag gcacatgtgg cgtgcttctc 1380

ctgtcactcg tcattaccct gtattgcaaa cgagggcgca agaagctgct gtacatcttc 1440

aaacagccct ttatgcggcc agtgcagacg acacaggagg aggatgggtg ctcctgcaga 1500

ttcccggaag aggaggaagg tggctgtgag ctgagagtga agttcagcag gagcgcagac 1560

gcccccgcgt accagcaggg ccagaaccag ctctataacg agctcaatct aggacgaaga 1620

gaggagtacg atgttttgga caagagacgt ggccgggacc ctgagatggg gggaaagccg 1680

agaaggaaga accctcagga aggcctgtac aatgaactgc agaaagataa gatggcggag 1740

gcctacagtg agattgggat gaaaggcgag cgccggaggg gcaaggggca cgatggcctt 1800

taccagggtc tcagtacagc caccaaggac acctacgacg cccttcacat gcaggccctg 1860

cctcctcgct aa 1872

<210> 132
<211> 1941
<212> DNA
<213> Artificial

<220>
<223> CAR 010 translation region

<400> 132
   atggagtttg ggctttcctg gttgttcctc gtagccatcc tcaaaggggt tcagtgcatg 60
   agagggccag gacatcctct gcttctgggc ctgctgctgg tgttgggcgc tgctggcaga 120
   ggtcgtggcg gagcagagcc tagagagcca gccgatgggc aagcccttct gcggcttgtg 180
   gtcgaactcg tgcaggaact gcggaaacac cattccgctg aacacaaagg cctgcaactg 240
   ctcggaagag actgtgcctt gggaagggca gaagcggctg gtctgggtcc ctcaccagaa 300
   cagcgagtcg agattgtgcc tcgcgatctc cgcatgaagg acaagttcct gaagcatctg 360
   acaggcccgc tgtacttcag tcccaagtgt agcaaacact ttcatcgcct gtatcacaac 420
   acacggattg gcaccatacc cgcctattac aagaggtgtg caaggctgct gactcgattg 480
   gccgtttctc ctgtgtgcat ggaggacaaa caggagccca agagctgcga caagacgcac 540
   acctgtccac cgtgtgatcc cgccgagccc aaatctcctg acaaaactca cacatgccca 600
   ccgtgcccag cacctgaact cctgggggga ccgtcagtct tcctcttccc cccaaaaccc 660
   aaggacaccc tcatgatctc ccggacccct gaggtcacat gcgtggtggt ggacgtgagc 720
   cacgaagacc ctgaggtcaa gttcaactgg tacgtggacg gcgtggaggt gcataatgcc 780
   aagacaaagc cgcgggagga gcagtacaac agcacgtacc gtgtggtcag cgtcctcacc 840
   gtcctgcacc aggactggct gaatggcaag gagtacaagt gcaaggtctc caacaaagcc 900
   ctcccagccc ccatcgagaa aaccatctcc aaagccaaag ggcagccccg agaaccacag 960
   gtgtacaccc tgcccccatc ccgggatgag ctgaccaaga accaggtcag cctgacctgc 1020
   ctggtcaaag gcttctatcc cagcgacatc gccgtggagt gggagagcaa tgggcaaccg 1080
   gagaacaact acaagaccac gcctcccgtg ctggactccg acggctcctt cttcctctac 1140
   agcaagctca ccgtggacaa gagcaggtgg cagcagggga acgtcttctc atgctccgtg 1200
   atgcatgagg ctctgcacaa ccactacacg cagaagagcc tctccctgtc tccgggtaaa 1260
   gccgcggcaa ccaccactcc tgctcccaga ccacctactc ccgctcccac catagcctct 1320
   caacctctga gccttaggcc agaagcctgt cgtcctgctg ctggtggagc cgttcacaca 1380
   aggggattgg actttgcctg cgacatctac atttgggcac cattggcagg cacatgtggc 1440
   gtgcttctcc tgtcactcgt cattaccctg tattgcaaac gagggcgcaa gaagctgctg 1500
   tacatcttca aacagccctt tatgcggcca gtgcagacga cacaggagga ggatgggtgc 1560
   tcctgcagat tcccggaaga ggaggaaggt ggctgtgagc tgagagtgaa gttcagcagg 1620
   agcgcagacg cccccgcgta ccagcagggc cagaaccagc tctataacga gctcaatcta 1680
   ggacgaagag aggagtacga tgttttggac aagagacgtg gccgggaccc tgagatgggg 1740
   ggaaagccga gaaggaagaa ccctcaggaa ggcctgtaca atgaactgca gaaagataag 1800
   atggcggagg cctacagtga gattgggatg aaaggcgagc gccggagggg caaggggcac 1860
   gatggccttt accagggtct cagtacagcc accaaggaca cctacgacgc ccttcacatg 1920
   caggccctgc ctcctcgcta a 1941

<210> 133
<211> 1725
<212> DNA
<213> Artificial

<220>
<223> CAR 011 translation region

<400> 133
atggagttcg gactgtcctg gctgtttctg gtggccatac tgaaaggggt tcagtgcgct 60

gcaggggctg gacgaactcc ctcaggcagt cggtcagccg agattttccc tcgggatagc 120

aatctcaagg acaagttcat caagcacttc accggtcctg tgaccttctc tcccgagtgt 180

agcaagcact ttcatcgcct gtattacaac acacgtgaat gctccacacc agcctactat 240

aagcggtgtg cgaggctcct cactcgcttg gccgtaagtc cgctttgcag ccaaaccgaa 300

cccaagtcct gcgataaaac ccacacatgt ccaccgtgtg atcccgccga gcccaaatct 360

cctgacaaaa ctcacacatg cccaccgtgc ccagcacctg aactcctggg gggaccgtca 420

gtcttcctct tccccccaaa acccaaggac accctcatga tctcccggac ccctgaggtc 480

acatgcgtgg tggtggacgt gagccacgaa gaccctgagg tcaagttcaa ctggtacgtg 540

gacggcgtgg aggtgcataa tgccaagaca aagccgcggg aggagcagta caacagcacg 600

taccgtgtgg tcagcgtcct caccgtcctg caccaggact ggctgaatgg caaggagtac 660

aagtgcaagg tctccaacaa agccctccca gcccccatcg agaaaaccat ctccaaagcc 720

aaagggcagc cccgagaacc acaggtgtac accctgcccc catcccggga tgagctgacc 780

aagaaccagg tcagcctgac ctgcctggtc aaaggcttct atcccagcga catcgccgtg 840

gagtgggaga gcaatgggca accggagaac aactacaaga ccacgcctcc cgtgctggac 900

tccgacggct ccttcttcct ctacagcaag ctcaccgtgg acaagagcag gtggcagcag 960

gggaacgtct tctcatgctc cgtgatgcat gaggctctgc acaaccacta cacgcagaag 1020

agcctctccc tgtctccggg taaagccgcg gcaaccacca ctcctgctcc cagaccacct 1080

actcccgctc ccaccatagc ctctcaacct ctgagcctta ggccagaagc ctgtcgtcct 1140

gctgctggtg gagccgttca cacaagggga ttggactttg cctgcgacat ctacatttgg 1200

gcaccattgg caggcacatg tggcgtgctt ctcctgtcac tcgtcattac cctgtattgc 1260

aaacgagggc gcaagaagct gctgtacatc ttcaaacagc cctttatgcg gccagtgcag 1320

acgacacagg aggaggatgg gtgctcctgc agattcccgg aagaggagga aggtggctgt 1380

gagctgagag tgaagttcag caggagcgca gacgcccccg cgtaccagca gggccagaac 1440

cagctctata acgagctcaa tctaggacga agagaggagt acgatgtttt ggacaagaga 1500

cgtggccggg accctgagat ggggggaaag ccgagaagga agaaccctca ggaaggcctg 1560

tacaatgaac tgcagaaaga taagatggcg gaggcctaca gtgagattgg gatgaaaggc 1620

gagcgccgga ggggcaaggg gcacgatggc ctttaccagg gtctcagtac agccaccaag 1680

gacacctacg acgcccttca catgcaggcc ctgcctcctc gctaa 1725

<210> 134
<211> 1731
<212> DNA
<213> Artificial

<220>
<223> CAR 012 translation region

<400> 134
atggagtttg ggctttcctg gttgttcctc gtagccatcc tcaaaggggt tcagtgcgca 60

gaagcggctg gtctgggtcc ctcaccagaa cagcgagtcg agattgtgcc tcgcgatctc 120

cgcatgaagg acaagttcct gaagcatctg acaggcccgc tgtacttcag tcccaagtgt 180

agcaaacact ttcatcgcct gtatcacaac acacgggatt gcaccatacc cgcctattac 240

aagaggtgtg caaggctgct gactcgattg gccgtttctc ctgtgtgcat ggaggacaaa 300

caggagccca agagctgcga caagacgcac acctgtccac cgtgtgatcc cgccgagccc 360

aaatctcctg acaaaactca cacatgccca ccgtgcccag cacctgaact cctgggggga 420

ccgtcagtct tcctcttccc cccaaaaccc aaggacaccc tcatgatctc ccggacccct 480

gaggtcacat gcgtggtggt ggacgtgagc cacgaagacc ctgaggtcaa gttcaactgg 540

tacgtggacg gcgtggaggt gcataatgcc aagacaaagc cgcgggagga gcagtacaac 600

agcacgtacc gtgtggtcag cgtcctcacc gtcctgcacc aggactggct gaatggcaag 660

gagtacaagt gcaaggtctc caacaaagcc ctcccagccc ccatcgagaa aaccatctcc 720

aaagccaaag ggcagccccg agaaccacag gtgtacaccc tgcccccatc ccgggatgag 780

ctgaccaaga accaggtcag cctgacctgc ctggtcaaag gcttctatcc cagcgacatc 840

gccgtggagt gggagagcaa tgggcaaccg gagaacaact acaagaccac gcctcccgtg 900

ctggactccg acggctcctt cttcctctac agcaagctca ccgtggacaa gagcaggtgg 960

cagcagggga acgtcttctc atgctccgtg atgcatgagg ctctgcacaa ccactacacg 1020

cagaagagcc tctccctgtc tccgggtaaa gccgcggcaa ccaccactcc tgctcccaga 1080

ccacctactc ccgctcccac catagcctct caacctctga gccttaggcc agaagcctgt 1140

cgtcctgctg ctggtggagc cgttcacaca aggggattgg actttgcctg cgacatctac 1200

atttgggcac cattggcagg cacatgtggc gtgcttctcc tgtcactcgt cattaccctg 1260

tattgcaaac gagggcgcaa gaagctgctg tacatcttca aacagccctt tatgcggcca 1320

gtgcagacga cacaggagga ggatgggtgc tcctgcagat tcccggaaga ggaggaaggt 1380

ggctgtgagc tgagagtgaa gttcagcagg agcgcagacg cccccgcgta ccagcagggc 1440

cagaaccagc tctataacga gctcaatcta ggacgaagag aggagtacga tgttttggac 1500

aagagacgtg gccgggaccc tgagatgggg ggaaagccga gaaggaagaa ccctcaggaa 1560

ggcctgtaca atgaactgca gaaagataag atggcggagg cctacagtga gattgggatg 1620

aaaggcgagc gccggagggg caaggggcac gatggccttt accagggtct cagtacagcc 1680

accaaggaca cctacgacgc ccttcacatg caggccctgc ctcctcgcta a 1731

<210> 135
<211> 2241
<212> DNA
<213> Artificial

<220>
<223> CAR 013 translation region

<400>  135
   atggtgctgc tggttacctc cctccttctc tgcgagttgc cgcatcccgc ttttctcctg      60
   attcccgaca cacaggtcca gctccagcaa tctggggcag aagtgaagaa acccgggtca     120
   agtgtgaaag tctcctgtaa agcaagtggc tatgccttta gcagctacat ttcctgggtt     180
   cgacaggcac ctggccaagg attggaatgg atgggtggac agatctatcc tggggatgga     240
   gacaccaatt acgcccagaa gtttcagggt cgcgtcacaa taacggcaga tgagtccact     300
   agcactgcct acatggagct gagctctctt cgctcagagg atacagccgt gtactattgc     360
   gtacggtact actacggctc ttccggctat ttcgactatt ggtggggaca gggaaccatg     420
   gtaaccgtga gttccggcgg tggaggaagc ggaggcggcg ggtcaggagg cggtgggtct     480
   gagatcgttc tgactcaatc tccagctacc ctgtctctga gcccaggcga acgtgctacc     540
   ttgtcatgca gagctagcga gtctgtggat aactacggta tatcctttgc gtggtatcag     600
   cagaaacctg ggcaagcccc aaggctgctg atctatcggg ccagtagggc tactgggatt     660
   ccggccagat ttagcgggag cggcagtggt acagacttca cgctgaccat ctcaagcctt     720
   gaacccgaag atttcgcggt gtattactgc cagcagaaca ataaggaccc acctaccttc     780
   ttcggccaag gcactaaact ggagatcaag agagagccca agtcctgtga caagacacac     840
   acatgtccac cgtgtgatcc cgccgagccc aaatctcctg acaaaactca cacatgccca     900
   ccgtgcccag cacctgaact cctgggggga ccgtcagtct tcctcttccc cccaaaaccc     960
   aaggacaccc tcatgatctc ccggacccct gaggtcacat gcgtggtggt ggacgtgagc    1020
   cacgaagacc ctgaggtcaa gttcaactgg tacgtggacg gcgtggaggt gcataatgcc    1080
   aagacaaagc cgcgggagga gcagtacaac agcacgtacc gtgtggtcag cgtcctcacc    1140
   gtcctgcacc aggactggct gaatggcaag gagtacaagt gcaaggtctc caacaaagcc    1200
   ctcccagccc ccatcgagaa aaccatctcc aaagccaaag ggcagccccg agaaccacag    1260
   gtgtacaccc tgcccccatc ccgggatgag ctgaccaaga accaggtcag cctgacctgc    1320
   ctggtcaaag gcttctatcc cagcgacatc gccgtggagt gggagagcaa tgggcaaccg    1380
   gagaacaact acaagaccac gcctcccgtg ctggactccg acggctcctt cttcctctac    1440
   agcaagctca ccgtggacaa gagcaggtgg cagcagggga acgtcttctc atgctccgtg    1500
   atgcatgagg ctctgcacaa ccactacacg cagaagagcc tctccctgtc tccgggtaaa    1560
   gccgcggcaa ccaccactcc tgctcccaga ccacctactc ccgctcccac catagcctct    1620
   caacctctga gccttaggcc agaagcctgt cgtcctgctg ctggtggagc cgttcacaca    1680
   aggggattgg actttgcctg cgacatctac atttgggcac cattggcagg cacatgtggc    1740
   gtgcttctcc tgtcactcgt cattaccctg tattgcaaac gagggcgcaa gaagctgctg    1800
   tacatcttca aacagccctt tatgcggcca gtgcagacga cacaggagga ggatgggtgc    1860
   tcctgcagat tcccggaaga ggaggaaggt ggctgtgagc tgagagtgaa gttcagcagg 1920
   agcgcagacg cccccgcgta ccagcagggc cagaaccagc tctataacga gctcaatcta 1980
   ggacgaagag aggagtacga tgttttggac aagagacgtg gccgggaccc tgagatgggg 2040
   ggaaagccga gaaggaagaa ccctcaggaa ggcctgtaca atgaactgca gaaagataag 2100
   atggcggagg cctacagtga gattgggatg aaaggcgagc gccggagggg caaggggcac 2160
   gatggccttt accagggtct cagtacagcc accaaggaca cctacgacgc ccttcacatg 2220
   caggccctgc ctcctcgcta a 2241

<210> 136
<211> 2238
<212> DNA
<213> Artificial

<220>
<223> CAR 014 translation region

<400> 136
atggtcctgc tcgtcacgtc actgcttctg tgcgagttgc ctcatcctgc cttcctgctg 60

attcccgata cacaggtgca acttcagcag tcaggcgcag aactggtcaa accaggtgct 120

agcgtgaaga tctcctgcaa ggctagtggg tacgcgtttt cctcctactg gatgaattgg 180

gtgaagcaac gaccaggcaa ggggttggag tggataggtc agatttatcc aggcgatggg 240

gatacaacat acaatgggaa gttcaaggga aaggccacac ttactgccga caaatcctct 300

agcaccgtgt acatgcagct gaactccctg acgtccgaag atagcgccgt ttacttctgc 360

gtgagatact actacggcag ctcagggtat ttcgactatt ggggtcaagg aaccactttg 420

accgtatctt ctggcggagg cggcagtggt ggaggtggct caggaggcgg agggtcagac 480

gttcagatga tacagacacc tgactctctg gccgtttccc tcgggcagag agccaccatc 540

agttgtcgcg cttctgagtc tgtggataac tatgggatca gcttcatgca ctggtatcag 600

cagaaacctg gccaaagtcc caaactcctc atctatcggg ctagcaatct ggaaagcggc 660

attccggcac gttttagtgg cagcggaagc aggaccgact ttaccctgac cattaatccg 720

gtggaaactg acgatgtagc aacctactat tgccagcaga acaacaagga tccacccact 780

tttggaggtg gaacaaagct ggagatcaaa gagcccaaaa gctgtgacaa aacgcacact 840

tgtccaccgt gtgatcccgc cgagcccaaa tctcctgaca aaactcacac atgcccaccg 900

tgcccagcac ctgaactcct ggggggaccg tcagtcttcc tcttcccccc aaaacccaag 960

gacaccctca tgatctcccg gacccctgag gtcacatgcg tggtggtgga cgtgagccac 1020

gaagaccctg aggtcaagtt caactggtac gtggacggcg tggaggtgca taatgccaag 1080

acaaagccgc gggaggagca gtacaacagc acgtaccgtg tggtcagcgt cctcaccgtc 1140

ctgcaccagg actggctgaa tggcaaggag tacaagtgca aggtctccaa caaagccctc 1200

ccagccccca tcgagaaaac catctccaaa gccaaagggc agccccgaga accacaggtg 1260

tacaccctgc ccccatcccg ggatgagctg accaagaacc aggtcagcct gacctgcctg 1320

gtcaaaggct tctatcccag cgacatcgcc gtggagtggg agagcaatgg gcaaccggag 1380

aacaactaca agaccacgcc tcccgtgctg gactccgacg gctccttctt cctctacagc 1440

aagctcaccg tggacaagag caggtggcag caggggaacg tcttctcatg ctccgtgatg 1500

catgaggctc tgcacaacca ctacacgcag aagagcctct ccctgtctcc gggtaaagcc 1560

gcggcaacca ccactcctgc tcccagacca cctactcccg ctcccaccat agcctctcaa 1620

cctctgagcc ttaggccaga agcctgtcgt cctgctgctg gtggagccgt tcacacaagg 1680

ggattggact ttgcctgcga catctacatt tgggcaccat tggcaggcac atgtggcgtg 1740

cttctcctgt cactcgtcat taccctgtat tgcaaacgag ggcgcaagaa gctgctgtac 1800

atcttcaaac agccctttat gcggccagtg cagacgacac aggaggagga tgggtgctcc 1860

tgcagattcc cggaagagga ggaaggtggc tgtgagctga gagtgaagtt cagcaggagc 1920

gcagacgccc ccgcgtacca gcagggccag aaccagctct ataacgagct caatctagga 1980

cgaagagagg agtacgatgt tttggacaag agacgtggcc gggaccctga gatgggggga 2040

aagccgagaa ggaagaaccc tcaggaaggc ctgtacaatg aactgcagaa agataagatg 2100

gcggaggcct acagtgagat tgggatgaaa ggcgagcgcc ggaggggcaa ggggcacgat 2160

ggcctttacc agggtctcag tacagccacc aaggacacct acgacgccct tcacatgcag 2220

gccctgcctc ctcgctaa 2238

<210> 137
<211> 623
<212> PRT
<213> Artificial

<220>
<223> CAR 009 translation region

<400> 137

Met Glu Phe Gly Leu Ser Trp Leu Phe Leu Val Ala Ile Leu Lys Gly
1               5                   10                  15

Val Gln Cys Met Arg Pro Leu Lys Pro Gly Ala Pro Leu Pro Ala Leu
            20                  25                  30

Phe Leu Leu Ala Leu Ala Leu Ser Pro His Gly Ala His Gly Arg Pro
        35                  40                  45

Arg Gly Arg Arg Gly Ala Arg Val Thr Asp Lys Glu Pro Lys Pro Leu
    50                  55                  60

Leu Phe Leu Pro Ala Ala Gly Ala Gly Arg Thr Pro Ser Gly Ser Arg
65                  70                  75                  80

Ser Ala Glu Ile Phe Pro Arg Asp Ser Asn Leu Lys Asp Lys Phe Ile
                85                  90                  95

Lys His Phe Thr Gly Pro Val Thr Phe Ser Pro Glu Cys Ser Lys His
            100                 105                 110

Phe His Arg Leu Tyr Tyr Asn Thr Arg Glu Cys Ser Thr Pro Ala Tyr
        115                 120                 125

Tyr Lys Arg Cys Ala Arg Leu Leu Thr Arg Leu Ala Val Ser Pro Leu
    130                 135                 140

Cys Ser Gln Thr Glu Pro Lys Ser Cys Asp Lys Thr His Thr Cys Pro
145                 150                 155                 160

Pro Cys Asp Pro Ala Glu Pro Lys Ser Pro Asp Lys Thr His Thr Cys
                165                 170                 175

Pro Pro Cys Pro Ala Pro Glu Leu Leu Gly Gly Pro Ser Val Phe Leu
            180                 185                 190

Phe Pro Pro Lys Pro Lys Asp Thr Leu Met Ile Ser Arg Thr Pro Glu
        195                 200                 205

Val Thr Cys Val Val Val Asp Val Ser His Glu Asp Pro Glu Val Lys
    210                 215                 220

Phe Asn Trp Tyr Val Asp Gly Val Glu Val His Asn Ala Lys Thr Lys
225                 230                 235                 240

Pro Arg Glu Glu Gln Tyr Asn Ser Thr Tyr Arg Val Val Ser Val Leu
                245                 250                 255

Thr Val Leu His Gln Asp Trp Leu Asn Gly Lys Glu Tyr Lys Cys Lys
            260                 265                 270

Val Ser Asn Lys Ala Leu Pro Ala Pro Ile Glu Lys Thr Ile Ser Lys
        275                 280                 285

Ala Lys Gly Gln Pro Arg Glu Pro Gln Val Tyr Thr Leu Pro Pro Ser
    290                 295                 300

Arg Asp Glu Leu Thr Lys Asn Gln Val Ser Leu Thr Cys Leu Val Lys
305                 310                 315                 320

Gly Phe Tyr Pro Ser Asp Ile Ala Val Glu Trp Glu Ser Asn Gly Gln
                325                 330                 335

Pro Glu Asn Asn Tyr Lys Thr Thr Pro Pro Val Leu Asp Ser Asp Gly
            340                 345                 350

Ser Phe Phe Leu Tyr Ser Lys Leu Thr Val Asp Lys Ser Arg Trp Gln
        355                 360                 365

Gln Gly Asn Val Phe Ser Cys Ser Val Met His Glu Ala Leu His Asn
    370                 375                 380

His Tyr Thr Gln Lys Ser Leu Ser Leu Ser Pro Gly Lys Ala Ala Ala
385                 390                 395                 400

Thr Thr Thr Pro Ala Pro Arg Pro Pro Thr Pro Ala Pro Thr Ile Ala
                405                 410                 415

Ser Gln Pro Leu Ser Leu Arg Pro Glu Ala Cys Arg Pro Ala Ala Gly
            420                 425                 430

Gly Ala Val His Thr Arg Gly Leu Asp Phe Ala Cys Asp Ile Tyr Ile
        435                 440                 445

Trp Ala Pro Leu Ala Gly Thr Cys Gly Val Leu Leu Leu Ser Leu Val
    450                 455                 460

Ile Thr Leu Tyr Cys Lys Arg Gly Arg Lys Lys Leu Leu Tyr Ile Phe
465                 470                 475                 480

Lys Gln Pro Phe Met Arg Pro Val Gln Thr Thr Gln Glu Glu Asp Gly
                485                 490                 495

Cys Ser Cys Arg Phe Pro Glu Glu Glu Glu Gly Gly Cys Glu Leu Arg
            500                 505                 510

Val Lys Phe Ser Arg Ser Ala Asp Ala Pro Ala Tyr Gln Gln Gly Gln
        515                 520                 525

Asn Gln Leu Tyr Asn Glu Leu Asn Leu Gly Arg Arg Glu Glu Tyr Asp
    530                 535                 540

Val Leu Asp Lys Arg Arg Gly Arg Asp Pro Glu Met Gly Gly Lys Pro
545                 550                 555                 560

Arg Arg Lys Asn Pro Gln Glu Gly Leu Tyr Asn Glu Leu Gln Lys Asp

565                 570                 575

Lys Met Ala Glu Ala Tyr Ser Glu Ile Gly Met Lys Gly Glu Arg Arg
            580                 585                 590

Arg Gly Lys Gly His Asp Gly Leu Tyr Gln Gly Leu Ser Thr Ala Thr
        595                 600                 605

Lys Asp Thr Tyr Asp Ala Leu His Met Gln Ala Leu Pro Pro Arg
    610                 615                 620

<210>  138
<211>  646
<212>  PRT
<213>  Artificial

<220>
<223>  CAR 010 translation region

<400>  138

Met Glu Phe Gly Leu Ser Trp Leu Phe Leu Val Ala Ile Leu Lys Gly
1               5                   10                  15

Val Gln Cys Met Arg Gly Pro Gly His Pro Leu Leu Leu Gly Leu Leu
            20                  25                  30

Leu Val Leu Gly Ala Ala Gly Arg Gly Arg Gly Gly Ala Glu Pro Arg
        35                  40                  45

Glu Pro Ala Asp Gly Gln Ala Leu Leu Arg Leu Val Val Glu Leu Val
    50                  55                  60

Gln Glu Leu Arg Lys His His Ser Ala Glu His Lys Gly Leu Gln Leu
65                  70                  75                  80

Leu Gly Arg Asp Cys Ala Leu Gly Arg Ala Glu Ala Ala Gly Leu Gly
                85                  90                  95

Pro Ser Pro Glu Gln Arg Val Glu Ile Val Pro Arg Asp Leu Arg Met
            100                 105                 110

Lys Asp Lys Phe Leu Lys His Leu Thr Gly Pro Leu Tyr Phe Ser Pro
        115                 120                 125

Lys Cys Ser Lys His Phe His Arg Leu Tyr His Asn Thr Arg Asp Cys
    130                 135                 140

Thr Ile Pro Ala Tyr Tyr Lys Arg Cys Ala Arg Leu Leu Thr Arg Leu
145                 150                 155                 160

Ala Val Ser Pro Val Cys Met Glu Asp Lys Gln Glu Pro Lys Ser Cys
                165                 170                 175

Asp Lys Thr His Thr Cys Pro Pro Cys Asp Pro Ala Glu Pro Lys Ser
            180                 185                 190

Pro Asp Lys Thr His Thr Cys Pro Pro Cys Pro Ala Pro Glu Leu Leu
        195                 200                 205

Gly Gly Pro Ser Val Phe Leu Phe Pro Pro Lys Pro Lys Asp Thr Leu
    210                 215                 220

Met Ile Ser Arg Thr Pro Glu Val Thr Cys Val Val Val Asp Val Ser
225                 230                 235                 240

His Glu Asp Pro Glu Val Lys Phe Asn Trp Tyr Val Asp Gly Val Glu
                245                 250                 255

Val His Asn Ala Lys Thr Lys Pro Arg Glu Glu Gln Tyr Asn Ser Thr
            260                 265                 270

Tyr Arg Val Val Ser Val Leu Thr Val Leu His Gln Asp Trp Leu Asn
        275                 280                 285

Gly Lys Glu Tyr Lys Cys Lys Val Ser Asn Lys Ala Leu Pro Ala Pro
    290                 295                 300

Ile Glu Lys Thr Ile Ser Lys Ala Lys Gly Gln Pro Arg Glu Pro Gln
305                 310                 315                 320

Val Tyr Thr Leu Pro Pro Ser Arg Asp Glu Leu Thr Lys Asn Gln Val
                325                 330                 335

Ser Leu Thr Cys Leu Val Lys Gly Phe Tyr Pro Ser Asp Ile Ala Val
            340                 345                 350

Glu Trp Glu Ser Asn Gly Gln Pro Glu Asn Asn Tyr Lys Thr Thr Pro
        355                 360                 365

Pro Val Leu Asp Ser Asp Gly Ser Phe Phe Leu Tyr Ser Lys Leu Thr
    370                 375                 380

Val Asp Lys Ser Arg Trp Gln Gln Gly Asn Val Phe Ser Cys Ser Val
385                 390                 395                 400

Met His Glu Ala Leu His Asn His Tyr Thr Gln Lys Ser Leu Ser Leu

405                 410                 415

Ser Pro Gly Lys Ala Ala Ala Thr Thr Thr Pro Ala Pro Arg Pro Pro
            420                 425                 430

Thr Pro Ala Pro Thr Ile Ala Ser Gln Pro Leu Ser Leu Arg Pro Glu
        435                 440                 445

Ala Cys Arg Pro Ala Ala Gly Gly Ala Val His Thr Arg Gly Leu Asp
    450                 455                 460

Phe Ala Cys Asp Ile Tyr Ile Trp Ala Pro Leu Ala Gly Thr Cys Gly
465                 470                 475                 480

Val Leu Leu Leu Ser Leu Val Ile Thr Leu Tyr Cys Lys Arg Gly Arg
                485                 490                 495

Lys Lys Leu Leu Tyr Ile Phe Lys Gln Pro Phe Met Arg Pro Val Gln
            500                 505                 510

Thr Thr Gln Glu Glu Asp Gly Cys Ser Cys Arg Phe Pro Glu Glu Glu
        515                 520                 525

Glu Gly Gly Cys Glu Leu Arg Val Lys Phe Ser Arg Ser Ala Asp Ala
    530                 535                 540

Pro Ala Tyr Gln Gln Gly Gln Asn Gln Leu Tyr Asn Glu Leu Asn Leu
545                 550                 555                 560

Gly Arg Arg Glu Glu Tyr Asp Val Leu Asp Lys Arg Arg Gly Arg Asp
                565                 570                 575

Pro Glu Met Gly Gly Lys Pro Arg Arg Lys Asn Pro Gln Glu Gly Leu
            580                 585                 590

Tyr Asn Glu Leu Gln Lys Asp Lys Met Ala Glu Ala Tyr Ser Glu Ile
        595                 600                 605

Gly Met Lys Gly Glu Arg Arg Arg Gly Lys Gly His Asp Gly Leu Tyr
    610                 615                 620

Gln Gly Leu Ser Thr Ala Thr Lys Asp Thr Tyr Asp Ala Leu His Met
625                 630                 635                 640

Gln Ala Leu Pro Pro Arg
                645

<210>  139
<211>  574
<212>  PRT
<213>  Artificial

<220>
<223>  CAR 011 translation region

<400>  139

Met Glu Phe Gly Leu Ser Trp Leu Phe Leu Val Ala Ile Leu Lys Gly
1               5                   10                  15

Val Gln Cys Ala Ala Gly Ala Gly Arg Thr Pro Ser Gly Ser Arg Ser
            20                  25                  30

Ala Glu Ile Phe Pro Arg Asp Ser Asn Leu Lys Asp Lys Phe Ile Lys
        35                  40                  45

His Phe Thr Gly Pro Val Thr Phe Ser Pro Glu Cys Ser Lys His Phe
    50                  55                  60

His Arg Leu Tyr Tyr Asn Thr Arg Glu Cys Ser Thr Pro Ala Tyr Tyr
65                  70                  75                  80

Lys Arg Cys Ala Arg Leu Leu Thr Arg Leu Ala Val Ser Pro Leu Cys
                85                  90                  95

Ser Gln Thr Glu Pro Lys Ser Cys Asp Lys Thr His Thr Cys Pro Pro
            100                 105                 110

Cys Asp Pro Ala Glu Pro Lys Ser Pro Asp Lys Thr His Thr Cys Pro
        115                 120                 125

Pro Cys Pro Ala Pro Glu Leu Leu Gly Gly Pro Ser Val Phe Leu Phe
    130                 135                 140

Pro Pro Lys Pro Lys Asp Thr Leu Met Ile Ser Arg Thr Pro Glu Val
145                 150                 155                 160

Thr Cys Val Val Val Asp Val Ser His Glu Asp Pro Glu Val Lys Phe
                165                 170                 175

Asn Trp Tyr Val Asp Gly Val Glu Val His Asn Ala Lys Thr Lys Pro
            180                 185                 190

Arg Glu Glu Gln Tyr Asn Ser Thr Tyr Arg Val Val Ser Val Leu Thr
        195                 200                 205

Val Leu His Gln Asp Trp Leu Asn Gly Lys Glu Tyr Lys Cys Lys Val

210                 215                 220

Ser Asn Lys Ala Leu Pro Ala Pro Ile Glu Lys Thr Ile Ser Lys Ala
225                 230                 235                 240

Lys Gly Gln Pro Arg Glu Pro Gln Val Tyr Thr Leu Pro Pro Ser Arg
                245                 250                 255

Asp Glu Leu Thr Lys Asn Gln Val Ser Leu Thr Cys Leu Val Lys Gly
            260                 265                 270

Phe Tyr Pro Ser Asp Ile Ala Val Glu Trp Glu Ser Asn Gly Gln Pro
        275                 280                 285

Glu Asn Asn Tyr Lys Thr Thr Pro Pro Val Leu Asp Ser Asp Gly Ser
    290                 295                 300

Phe Phe Leu Tyr Ser Lys Leu Thr Val Asp Lys Ser Arg Trp Gln Gln
305                 310                 315                 320

Gly Asn Val Phe Ser Cys Ser Val Met His Glu Ala Leu His Asn His
                325                 330                 335

Tyr Thr Gln Lys Ser Leu Ser Leu Ser Pro Gly Lys Ala Ala Ala Thr
            340                 345                 350

Thr Thr Pro Ala Pro Arg Pro Pro Thr Pro Ala Pro Thr Ile Ala Ser
        355                 360                 365

Gln Pro Leu Ser Leu Arg Pro Glu Ala Cys Arg Pro Ala Ala Gly Gly
    370                 375                 380

Ala Val His Thr Arg Gly Leu Asp Phe Ala Cys Asp Ile Tyr Ile Trp
385                 390                 395                 400

Ala Pro Leu Ala Gly Thr Cys Gly Val Leu Leu Leu Ser Leu Val Ile
                405                 410                 415

Thr Leu Tyr Cys Lys Arg Gly Arg Lys Lys Leu Leu Tyr Ile Phe Lys
            420                 425                 430

Gln Pro Phe Met Arg Pro Val Gln Thr Thr Gln Glu Glu Asp Gly Cys
        435                 440                 445

Ser Cys Arg Phe Pro Glu Glu Glu Glu Gly Gly Cys Glu Leu Arg Val
    450                 455                 460

Lys Phe Ser Arg Ser Ala Asp Ala Pro Ala Tyr Gln Gln Gly Gln Asn
465                 470                 475                 480

Gln Leu Tyr Asn Glu Leu Asn Leu Gly Arg Arg Glu Glu Tyr Asp Val
                485                 490                 495

Leu Asp Lys Arg Arg Gly Arg Asp Pro Glu Met Gly Gly Lys Pro Arg
            500                 505                 510

Arg Lys Asn Pro Gln Glu Gly Leu Tyr Asn Glu Leu Gln Lys Asp Lys
        515                 520                 525

Met Ala Glu Ala Tyr Ser Glu Ile Gly Met Lys Gly Glu Arg Arg Arg
    530                 535                 540

Gly Lys Gly His Asp Gly Leu Tyr Gln Gly Leu Ser Thr Ala Thr Lys
545                 550                 555                 560

Asp Thr Tyr Asp Ala Leu His Met Gln Ala Leu Pro Pro Arg
                565                 570

<210>  140
<211>  576
<212>  PRT
<213>  Artificial

<220>
<223>  CAR 012 translation region

<400>  140

Met Glu Phe Gly Leu Ser Trp Leu Phe Leu Val Ala Ile Leu Lys Gly
1               5                   10                  15

Val Gln Cys Ala Glu Ala Ala Gly Leu Gly Pro Ser Pro Glu Gln Arg
            20                  25                  30

Val Glu Ile Val Pro Arg Asp Leu Arg Met Lys Asp Lys Phe Leu Lys
        35                  40                  45

His Leu Thr Gly Pro Leu Tyr Phe Ser Pro Lys Cys Ser Lys His Phe
    50                  55                  60

His Arg Leu Tyr His Asn Thr Arg Asp Cys Thr Ile Pro Ala Tyr Tyr
65                  70                  75                  80

Lys Arg Cys Ala Arg Leu Leu Thr Arg Leu Ala Val Ser Pro Val Cys
                85                  90                  95

Met Glu Asp Lys Gln Glu Pro Lys Ser Cys Asp Lys Thr His Thr Cys

100                 105                 110

Pro Pro Cys Asp Pro Ala Glu Pro Lys Ser Pro Asp Lys Thr His Thr
        115                 120                 125

Cys Pro Pro Cys Pro Ala Pro Glu Leu Leu Gly Gly Pro Ser Val Phe
    130                 135                 140

Leu Phe Pro Pro Lys Pro Lys Asp Thr Leu Met Ile Ser Arg Thr Pro
145                 150                 155                 160

Glu Val Thr Cys Val Val Val Asp Val Ser His Glu Asp Pro Glu Val
                165                 170                 175

Lys Phe Asn Trp Tyr Val Asp Gly Val Glu Val His Asn Ala Lys Thr
            180                 185                 190

Lys Pro Arg Glu Glu Gln Tyr Asn Ser Thr Tyr Arg Val Val Ser Val
        195                 200                 205

Leu Thr Val Leu His Gln Asp Trp Leu Asn Gly Lys Glu Tyr Lys Cys
    210                 215                 220

Lys Val Ser Asn Lys Ala Leu Pro Ala Pro Ile Glu Lys Thr Ile Ser
225                 230                 235                 240

Lys Ala Lys Gly Gln Pro Arg Glu Pro Gln Val Tyr Thr Leu Pro Pro
                245                 250                 255

Ser Arg Asp Glu Leu Thr Lys Asn Gln Val Ser Leu Thr Cys Leu Val
            260                 265                 270

Lys Gly Phe Tyr Pro Ser Asp Ile Ala Val Glu Trp Glu Ser Asn Gly
        275                 280                 285

Gln Pro Glu Asn Asn Tyr Lys Thr Thr Pro Pro Val Leu Asp Ser Asp
    290                 295                 300

Gly Ser Phe Phe Leu Tyr Ser Lys Leu Thr Val Asp Lys Ser Arg Trp
305                 310                 315                 320

Gln Gln Gly Asn Val Phe Ser Cys Ser Val Met His Glu Ala Leu His
                325                 330                 335

Asn His Tyr Thr Gln Lys Ser Leu Ser Leu Ser Pro Gly Lys Ala Ala
            340                 345                 350

Ala Thr Thr Thr Pro Ala Pro Arg Pro Pro Thr Pro Ala Pro Thr Ile
        355                 360                 365

Ala Ser Gln Pro Leu Ser Leu Arg Pro Glu Ala Cys Arg Pro Ala Ala
    370                 375                 380

Gly Gly Ala Val His Thr Arg Gly Leu Asp Phe Ala Cys Asp Ile Tyr
385                 390                 395                 400

Ile Trp Ala Pro Leu Ala Gly Thr Cys Gly Val Leu Leu Leu Ser Leu
                405                 410                 415

Val Ile Thr Leu Tyr Cys Lys Arg Gly Arg Lys Lys Leu Leu Tyr Ile
            420                 425                 430

Phe Lys Gln Pro Phe Met Arg Pro Val Gln Thr Thr Gln Glu Glu Asp
        435                 440                 445

Gly Cys Ser Cys Arg Phe Pro Glu Glu Glu Glu Gly Gly Cys Glu Leu
    450                 455                 460

Arg Val Lys Phe Ser Arg Ser Ala Asp Ala Pro Ala Tyr Gln Gln Gly
465                 470                 475                 480

Gln Asn Gln Leu Tyr Asn Glu Leu Asn Leu Gly Arg Arg Glu Glu Tyr
                485                 490                 495

Asp Val Leu Asp Lys Arg Arg Gly Arg Asp Pro Glu Met Gly Gly Lys
            500                 505                 510

Pro Arg Arg Lys Asn Pro Gln Glu Gly Leu Tyr Asn Glu Leu Gln Lys
        515                 520                 525

Asp Lys Met Ala Glu Ala Tyr Ser Glu Ile Gly Met Lys Gly Glu Arg
    530                 535                 540

Arg Arg Gly Lys Gly His Asp Gly Leu Tyr Gln Gly Leu Ser Thr Ala
545                 550                 555                 560

Thr Lys Asp Thr Tyr Asp Ala Leu His Met Gln Ala Leu Pro Pro Arg
                565                 570                 575

<210> 141
<211> 746
<212> PRT
<213> Artificial

<220>
<223> CAR 013 translation region

<400>  141

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Val Lys Lys Pro Gly Ser Ser Val Lys Val Ser Cys Lys Ala
        35                  40                  45

Ser Gly Tyr Ala Phe Ser Ser Tyr Ile Ser Trp Val Arg Gln Ala Pro
    50                  55                  60

Gly Gln Gly Leu Glu Trp Met Gly Gly Gln Ile Tyr Pro Gly Asp Gly
65                  70                  75                  80

Asp Thr Asn Tyr Ala Gln Lys Phe Gln Gly Arg Val Thr Ile Thr Ala
                85                  90                  95

Asp Glu Ser Thr Ser Thr Ala Tyr Met Glu Leu Ser Ser Leu Arg Ser
            100                 105                 110

Glu Asp Thr Ala Val Tyr Tyr Cys Val Arg Tyr Tyr Tyr Gly Ser Ser
        115                 120                 125

Gly Tyr Phe Asp Tyr Trp Trp Gly Gln Gly Thr Met Val Thr Val Ser
    130                 135                 140

Ser Gly Gly Gly Gly Ser Gly Gly Gly Gly Ser Gly Gly Gly Gly Ser
145                 150                 155                 160

Glu Ile Val Leu Thr Gln Ser Pro Ala Thr Leu Ser Leu Ser Pro Gly
                165                 170                 175

Glu Arg Ala Thr Leu Ser Cys Arg Ala Ser Glu Ser Val Asp Asn Tyr
            180                 185                 190

Gly Ile Ser Phe Ala Trp Tyr Gln Gln Lys Pro Gly Gln Ala Pro Arg
        195                 200                 205

Leu Leu Ile Tyr Arg Ala Ser Arg Ala Thr Gly Ile Pro Ala Arg Phe
    210                 215                 220

Ser Gly Ser Gly Ser Gly Thr Asp Phe Thr Leu Thr Ile Ser Ser Leu
225                 230                 235                 240

Glu Pro Glu Asp Phe Ala Val Tyr Tyr Cys Gln Gln Asn Asn Lys Asp
                245                 250                 255

Pro Pro Thr Phe Phe Gly Gln Gly Thr Lys Leu Glu Ile Lys Arg Glu
            260                 265                 270

Pro Lys Ser Cys Asp Lys Thr His Thr Cys Pro Pro Cys Asp Pro Ala
        275                 280                 285

Glu Pro Lys Ser Pro Asp Lys Thr His Thr Cys Pro Pro Cys Pro Ala
    290                 295                 300

Pro Glu Leu Leu Gly Gly Pro Ser Val Phe Leu Phe Pro Pro Lys Pro
305                 310                 315                 320

Lys Asp Thr Leu Met Ile Ser Arg Thr Pro Glu Val Thr Cys Val Val
                325                 330                 335

Val Asp Val Ser His Glu Asp Pro Glu Val Lys Phe Asn Trp Tyr Val
            340                 345                 350

Asp Gly Val Glu Val His Asn Ala Lys Thr Lys Pro Arg Glu Glu Gln
        355                 360                 365

Tyr Asn Ser Thr Tyr Arg Val Val Ser Val Leu Thr Val Leu His Gln
    370                 375                 380

Asp Trp Leu Asn Gly Lys Glu Tyr Lys Cys Lys Val Ser Asn Lys Ala
385                 390                 395                 400

Leu Pro Ala Pro Ile Glu Lys Thr Ile Ser Lys Ala Lys Gly Gln Pro
                405                 410                 415

Arg Glu Pro Gln Val Tyr Thr Leu Pro Pro Ser Arg Asp Glu Leu Thr
            420                 425                 430

Lys Asn Gln Val Ser Leu Thr Cys Leu Val Lys Gly Phe Tyr Pro Ser
        435                 440                 445

Asp Ile Ala Val Glu Trp Glu Ser Asn Gly Gln Pro Glu Asn Asn Tyr
    450                 455                 460

Lys Thr Thr Pro Pro Val Leu Asp Ser Asp Gly Ser Phe Phe Leu Tyr
465                 470                 475                 480

Ser Lys Leu Thr Val Asp Lys Ser Arg Trp Gln Gln Gly Asn Val Phe
                485                 490                 495

Ser Cys Ser Val Met His Glu Ala Leu His Asn His Tyr Thr Gln Lys
            500                 505                 510

Ser Leu Ser Leu Ser Pro Gly Lys Ala Ala Ala Thr Thr Thr Pro Ala
        515                 520                 525

Pro Arg Pro Pro Thr Pro Ala Pro Thr Ile Ala Ser Gln Pro Leu Ser
    530                 535                 540

Leu Arg Pro Glu Ala Cys Arg Pro Ala Ala Gly Gly Ala Val His Thr
545                 550                 555                 560

Arg Gly Leu Asp Phe Ala Cys Asp Ile Tyr Ile Trp Ala Pro Leu Ala
                565                 570                 575

Gly Thr Cys Gly Val Leu Leu Leu Ser Leu Val Ile Thr Leu Tyr Cys
            580                 585                 590

Lys Arg Gly Arg Lys Lys Leu Leu Tyr Ile Phe Lys Gln Pro Phe Met
        595                 600                 605

Arg Pro Val Gln Thr Thr Gln Glu Glu Asp Gly Cys Ser Cys Arg Phe
    610                 615                 620

Pro Glu Glu Glu Glu Gly Gly Cys Glu Leu Arg Val Lys Phe Ser Arg
625                 630                 635                 640

Ser Ala Asp Ala Pro Ala Tyr Gln Gln Gly Gln Asn Gln Leu Tyr Asn
                645                 650                 655

Glu Leu Asn Leu Gly Arg Arg Glu Glu Tyr Asp Val Leu Asp Lys Arg
            660                 665                 670

Arg Gly Arg Asp Pro Glu Met Gly Gly Lys Pro Arg Arg Lys Asn Pro
        675                 680                 685

Gln Glu Gly Leu Tyr Asn Glu Leu Gln Lys Asp Lys Met Ala Glu Ala
    690                 695                 700

Tyr Ser Glu Ile Gly Met Lys Gly Glu Arg Arg Arg Gly Lys Gly His
705                 710                 715                 720

Asp Gly Leu Tyr Gln Gly Leu Ser Thr Ala Thr Lys Asp Thr Tyr Asp
                725                 730                 735

Ala Leu His Met Gln Ala Leu Pro Pro Arg
            740                 745

<210> 142
<211> 745
<212> PRT
<213> Artificial

<220>
<223> CAR 014 translation region

<400> 142

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Leu Val Lys Pro Gly Ala Ser Val Lys Ile Ser Cys Lys Ala
        35                  40                  45

Ser Gly Tyr Ala Phe Ser Ser Tyr Trp Met Asn Trp Val Lys Gln Arg
    50                  55                  60

Pro Gly Lys Gly Leu Glu Trp Ile Gly Gln Ile Tyr Pro Gly Asp Gly
65                  70                  75                  80

Asp Thr Thr Tyr Asn Gly Lys Phe Lys Gly Lys Ala Thr Leu Thr Ala
                85                  90                  95

Asp Lys Ser Ser Ser Thr Val Tyr Met Gln Leu Asn Ser Leu Thr Ser
            100                 105                 110

Glu Asp Ser Ala Val Tyr Phe Cys Val Arg Tyr Tyr Tyr Gly Ser Ser
        115                 120                 125

Gly Tyr Phe Asp Tyr Trp Gly Gln Gly Thr Thr Leu Thr Val Ser Ser
    130                 135                 140

Gly Gly Gly Gly Ser Gly Gly Gly Gly Ser Gly Gly Gly Gly Ser Asp
145                 150                 155                 160

Val Gln Met Ile Gln Thr Pro Asp Ser Leu Ala Val Ser Leu Gly Gln
                165                 170                 175

Arg Ala Thr Ile Ser Cys Arg Ala Ser Glu Ser Val Asp Asn Tyr Gly
            180                 185                 190

Ile Ser Phe Met His Trp Tyr Gln Gln Lys Pro Gly Gln Ser Pro Lys
        195                 200                 205

Leu Leu Ile Tyr Arg Ala Ser Asn Leu Glu Ser Gly Ile Pro Ala Arg
    210                 215                 220

Phe Ser Gly Ser Gly Ser Arg Thr Asp Phe Thr Leu Thr Ile Asn Pro
225                 230                 235                 240

Val Glu Thr Asp Asp Val Ala Thr Tyr Tyr Cys Gln Gln Asn Asn Lys
                245                 250                 255

Asp Pro Pro Thr Phe Gly Gly Gly Thr Lys Leu Glu Ile Lys Glu Pro
            260                 265                 270

Lys Ser Cys Asp Lys Thr His Thr Cys Pro Pro Cys Asp Pro Ala Glu
        275                 280                 285

Pro Lys Ser Pro Asp Lys Thr His Thr Cys Pro Pro Cys Pro Ala Pro
    290                 295                 300

Glu Leu Leu Gly Gly Pro Ser Val Phe Leu Phe Pro Pro Lys Pro Lys
305                 310                 315                 320

Asp Thr Leu Met Ile Ser Arg Thr Pro Glu Val Thr Cys Val Val Val
                325                 330                 335

Asp Val Ser His Glu Asp Pro Glu Val Lys Phe Asn Trp Tyr Val Asp
            340                 345                 350

Gly Val Glu Val His Asn Ala Lys Thr Lys Pro Arg Glu Glu Gln Tyr
        355                 360                 365

Asn Ser Thr Tyr Arg Val Val Ser Val Leu Thr Val Leu His Gln Asp
    370                 375                 380

Trp Leu Asn Gly Lys Glu Tyr Lys Cys Lys Val Ser Asn Lys Ala Leu
385                 390                 395                 400

Pro Ala Pro Ile Glu Lys Thr Ile Ser Lys Ala Lys Gly Gln Pro Arg
                405                 410                 415

Glu Pro Gln Val Tyr Thr Leu Pro Pro Ser Arg Asp Glu Leu Thr Lys
            420                 425                 430

Asn Gln Val Ser Leu Thr Cys Leu Val Lys Gly Phe Tyr Pro Ser Asp
        435                 440                 445

Ile Ala Val Glu Trp Glu Ser Asn Gly Gln Pro Glu Asn Asn Tyr Lys
    450                 455                 460

Thr Thr Pro Pro Val Leu Asp Ser Asp Gly Ser Phe Phe Leu Tyr Ser
465                 470                 475                 480

Lys Leu Thr Val Asp Lys Ser Arg Trp Gln Gln Gly Asn Val Phe Ser
                485                 490                 495

Cys Ser Val Met His Glu Ala Leu His Asn His Tyr Thr Gln Lys Ser
            500                 505                 510

Leu Ser Leu Ser Pro Gly Lys Ala Ala Ala Thr Thr Thr Pro Ala Pro
        515                 520                 525

Arg Pro Pro Thr Pro Ala Pro Thr Ile Ala Ser Gln Pro Leu Ser Leu
    530                 535                 540

Arg Pro Glu Ala Cys Arg Pro Ala Ala Gly Gly Ala Val His Thr Arg
545                 550                 555                 560

Gly Leu Asp Phe Ala Cys Asp Ile Tyr Ile Trp Ala Pro Leu Ala Gly
                565                 570                 575

Thr Cys Gly Val Leu Leu Leu Ser Leu Val Ile Thr Leu Tyr Cys Lys
            580                 585                 590

Arg Gly Arg Lys Lys Leu Leu Tyr Ile Phe Lys Gln Pro Phe Met Arg
        595                 600                 605

Pro Val Gln Thr Thr Gln Glu Glu Asp Gly Cys Ser Cys Arg Phe Pro
    610                 615                 620

Glu Glu Glu Glu Gly Gly Cys Glu Leu Arg Val Lys Phe Ser Arg Ser
625                 630                 635                 640

Ala Asp Ala Pro Ala Tyr Gln Gln Gly Gln Asn Gln Leu Tyr Asn Glu
                645                 650                 655

Leu Asn Leu Gly Arg Arg Glu Glu Tyr Asp Val Leu Asp Lys Arg Arg
            660                 665                 670

Gly Arg Asp Pro Glu Met Gly Gly Lys Pro Arg Arg Lys Asn Pro Gln
        675                 680                 685

Glu Gly Leu Tyr Asn Glu Leu Gln Lys Asp Lys Met Ala Glu Ala Tyr
    690                 695                 700

Ser Glu Ile Gly Met Lys Gly Glu Arg Arg Arg Gly Lys Gly His Asp

705                 710                 715                 720

Gly Leu Tyr Gln Gly Leu Ser Thr Ala Thr Lys Asp Thr Tyr Asp Ala
                725                 730                 735

Leu His Met Gln Ala Leu Pro Pro Arg
            740                 745

<210>  143
<211>  19
<212>  PRT
<213>  Homo sapiens

<400>  143

Met Glu Phe Gly Leu Ser Trp Leu Phe Leu Val Ala Ile Leu Lys Gly
1               5                   10                  15

Val Gln Cys

<210>  144
<211>  24
<212>  PRT
<213>  Homo sapiens

<400>  144

Met Val Leu Leu Val Thr Ser Leu Leu Leu Cys Glu Leu Pro His Pro
1               5                   10                  15

Ala Phe Leu Leu Ile Pro Asp Thr
            20

<210>  145
<211>  129
<212>  PRT
<213>  Homo sapiens

<400>  145

Met Arg Pro Leu Lys Pro Gly Ala Pro Leu Pro Ala Leu Phe Leu Leu
1               5                   10                  15

Ala Leu Ala Leu Ser Pro His Gly Ala His Gly Arg Pro Arg Gly Arg
            20                  25                  30

Arg Gly Ala Arg Val Thr Asp Lys Glu Pro Lys Pro Leu Leu Phe Leu
        35                  40                  45

Pro Ala Ala Gly Ala Gly Arg Thr Pro Ser Gly Ser Arg Ser Ala Glu
    50                  55                  60

Ile Phe Pro Arg Asp Ser Asn Leu Lys Asp Lys Phe Ile Lys His Phe
65                  70                  75                  80

Thr Gly Pro Val Thr Phe Ser Pro Glu Cys Ser Lys His Phe His Arg
                85                  90                  95

Leu Tyr Tyr Asn Thr Arg Glu Cys Ser Thr Pro Ala Tyr Tyr Lys Arg
            100                 105                 110

Cys Ala Arg Leu Leu Thr Arg Leu Ala Val Ser Pro Leu Cys Ser Gln
        115                 120                 125

Thr

<210>  146
<211>  80
<212>  PRT
<213>  Homo sapiens

<400>  146

Ala Ala Gly Ala Gly Arg Thr Pro Ser Gly Ser Arg Ser Ala Glu Ile
1               5                   10                  15

Phe Pro Arg Asp Ser Asn Leu Lys Asp Lys Phe Ile Lys His Phe Thr
            20                  25                  30

Gly Pro Val Thr Phe Ser Pro Glu Cys Ser Lys His Phe His Arg Leu
        35                  40                  45

Tyr Tyr Asn Thr Arg Glu Cys Ser Thr Pro Ala Tyr Tyr Lys Arg Cys
    50                  55                  60

Ala Arg Leu Leu Thr Arg Leu Ala Val Ser Pro Leu Cys Ser Gln Thr
65                  70                  75                  80

<210>  147
<211>  152
<212>  PRT
<213>  Homo sapiens

<400>  147

Met Arg Gly Pro Gly His Pro Leu Leu Leu Gly Leu Leu Leu Val Leu
1               5                   10                  15

Gly Ala Ala Gly Arg Gly Arg Gly Gly Ala Glu Pro Arg Glu Pro Ala
            20                  25                  30

Asp Gly Gln Ala Leu Leu Arg Leu Val Val Glu Leu Val Gln Glu Leu
        35                  40                  45

Arg Lys His His Ser Ala Glu His Lys Gly Leu Gln Leu Leu Gly Arg
    50                  55                  60

Asp Cys Ala Leu Gly Arg Ala Glu Ala Ala Gly Leu Gly Pro Ser Pro
65                  70                  75                  80

Glu Gln Arg Val Glu Ile Val Pro Arg Asp Leu Arg Met Lys Asp Lys
                85                  90                  95

Phe Leu Lys His Leu Thr Gly Pro Leu Tyr Phe Ser Pro Lys Cys Ser
            100                 105                 110

Lys His Phe His Arg Leu Tyr His Asn Thr Arg Asp Cys Thr Ile Pro
        115                 120                 125

Ala Tyr Tyr Lys Arg Cys Ala Arg Leu Leu Thr Arg Leu Ala Val Ser
    130                 135                 140

Pro Val Cys Met Glu Asp Lys Gln
145                 150

<210>  148
<211>  82
<212>  PRT
<213>  Homo sapiens

<400>  148

Ala Glu Ala Ala Gly Leu Gly Pro Ser Pro Glu Gln Arg Val Glu Ile
1               5                   10                  15

Val Pro Arg Asp Leu Arg Met Lys Asp Lys Phe Leu Lys His Leu Thr
            20                  25                  30

Gly Pro Leu Tyr Phe Ser Pro Lys Cys Ser Lys His Phe His Arg Leu
        35                  40                  45

Tyr His Asn Thr Arg Asp Cys Thr Ile Pro Ala Tyr Tyr Lys Arg Cys
    50                  55                  60

Ala Arg Leu Leu Thr Arg Leu Ala Val Ser Pro Val Cys Met Glu Asp
65                  70                  75                  80

Lys Gln

<210>  149
<211>  29
<212>  PRT
<213>  Artificial

<220>
<223>  Hinge-Spacer-Hinge

<400>  149

Glu Pro Lys Ser Cys Asp Lys Thr His Thr Cys Pro Pro Cys Asp Pro
1               5                   10                  15

Ala Glu Pro Lys Ser Pro Asp Lys Thr His Thr Cys Pro
            20                  25

<210>  150
<211>  113
<212>  PRT
<213>  Homo sapiens

<400>  150

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Glu Gln Tyr Asn Ser Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys

<210>  151
<211>  107
<212>  PRT
<213>  Homo sapiens

<400>  151

Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  152
<211>  68
<212>  PRT
<213>  Homo sapiens

<400>  152

Phe Trp Val Leu Val Val Val Gly Gly Val Leu Ala Cys Tyr Ser Leu
1               5                   10                  15

Leu Val Thr Val Ala Phe Ile Ile Phe Trp Val Arg Ser Lys Arg Ser
            20                  25                  30

Arg Leu Leu His Ser Asp Tyr Met Asn Met Thr Pro Arg Arg Pro Gly
        35                  40                  45

Pro Thr Arg Lys His Tyr Gln Pro Tyr Ala Pro Pro Arg Asp Phe Ala
    50                  55                  60

Ala Tyr Arg Ser
65

<210>  153
<211>  112
<212>  PRT
<213>  Homo sapiens

<400>  153

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  154
<211>  102
<212>  PRT
<213>  Homo sapiens

<400>  154

Arg Pro Arg Gly Arg Arg Gly Ala Arg Val Thr Asp Lys Glu Pro Lys
 1               5                   10                  15

Pro Leu Leu Phe Leu Pro Ala Ala Gly Ala Gly Arg Thr Pro Ser Gly
            20                  25                  30

Ser Arg Ser Ala Glu Ile Phe Pro Arg Asp Ser Asn Leu Lys Asp Lys
        35                  40                  45

Phe Ile Lys His Phe Thr Gly Pro Val Thr Phe Ser Pro Glu Cys Ser
    50                  55                  60

Lys His Phe His Arg Leu Tyr Tyr Asn Thr Arg Glu Cys Ser Thr Pro
65                  70                  75                  80

Ala Tyr Tyr Lys Arg Cys Ala Arg Leu Leu Thr Arg Leu Ala Val Ser
                85                  90                  95

Pro Leu Cys Ser Gln Thr
            100

<210>  155
<211>  128
<212>  PRT
<213>  Homo sapiens

<400>  155

Gly Ala Glu Pro Arg Glu Pro Ala Asp Gly Gln Ala Leu Leu Arg Leu
1               5                   10                  15

Val Val Glu Leu Val Gln Glu Leu Arg Lys His His Ser Ala Glu His
            20                  25                  30

Lys Gly Leu Gln Leu Leu Gly Arg Asp Cys Ala Leu Gly Arg Ala Glu
        35                  40                  45

Ala Ala Gly Leu Gly Pro Ser Pro Glu Gln Arg Val Glu Ile Val Pro
    50                  55                  60

Arg Asp Leu Arg Met Lys Asp Lys Phe Leu Lys His Leu Thr Gly Pro
65                  70                  75                  80

Leu Tyr Phe Ser Pro Lys Cys Ser Lys His Phe His Arg Leu Tyr His
                85                  90                  95

Asn Thr Arg Asp Cys Thr Ile Pro Ala Tyr Tyr Lys Arg Cys Ala Arg
            100                 105                 110

Leu Leu Thr Arg Leu Ala Val Ser Pro Val Cys Met Glu Asp Lys Gln
        115                 120                 125


<210>  156
<211>  30
<212>  DNA
<213>  Artificial

<220>
<223>  forward primer for CAR 015 amplification

<400>  156
atgaaggaca agttcctgaa gcatctgaca                                        30


<210>  157
<211>  30
<212>  DNA
<213>  Artificial

<220>
<223>  forward primer for CAR 016 amplification

<400>  157
gacaagttcc tgaagcatct gacaggcccg                                        30

<210> 158
<211> 30
<212> DNA
<213> Artificial

<220>
<223> forward primer for CAR 017 amplification

<400> 158
aagcatctga caggcccgct gtacttcagt 30


<210> 159
<211> 30
<212> DNA
<213> Artificial

<220>
<223> forward primer for CAR 018 amplification

<400> 159
ctgacaggcc cgctgtactt cagtcccaag 30


<210> 160
<211> 30
<212> DNA
<213> Artificial

<220>
<223> forward primer for CAR 019 amplification

<400> 160
ggcccgctgt acttcagtcc caagtgtagc 30


<210> 161
<211> 1539
<212> DNA
<213> Artificial

<220>
<223> CAR 015 translation region

<400> 161
atggagtttg ggctttcctg gttgttcctc gtagccatcc tcaaaggggt tcagtgcatg 60

aaggacaagt tcctgaagca tctgacaggc ccgctgtact tcagtcccaa gtgtagcaaa 120

cactttcatc gcctgtatca caacacacgg gattgcacca tacccgccta ttacaagagg 180

tgtgcaaggc tgctgactcg attggccgtt tctcctgtgt gcatggagga caaacaggag 240

cccaagagct gcgacaagac gcacacctgt ccaccgtgtg atcccgccga gcccaaatct 300

cctgacaaaa ctcacacatg cccaccgtgc ccagcacctg aactcctggg gggaccgtca 360

gtcttcctct tccccccaaa acccaaggac accctcatga tctcccggac ccctgaggtc 420

acatgcgtgg tggtggacgt gagccacgaa gaccctgagg tcaagttcaa ctggtacgtg 480

gacggcgtgg aggtgcataa tgccaagaca aagccgcggg aggagcagta caacagcacg 540

taccgtgtgg tcagcgtcct caccgtcctg caccaggact ggctgaatgg caaggagtac 600

aagtgcaagg tctccaacaa agccctccca gcccccatcg agaaaaccat ctccaaagcc 660

aaagggcagc cccgagaacc acaggtgtac accctgcccc catcccggga tgagctgacc 720

aagaaccagg tcagcctgac ctgcctggtc aaaggcttct atcccagcga catcgccgtg 780

gagtgggaga gcaatgggca accggagaac aactacaaga ccacgcctcc cgtgctggac 840

tccgacggct ccttcttcct ctacagcaag ctcaccgtgg acaagagcag gtggcagcag 900

gggaacgtct tctcatgctc cgtgatgcat gaggctctgc acaaccacta cacgcagaag 960

agcctctccc tgtctccggg taaaaaagat cccaaatttt gggtgctggt ggtggttggt 1020

ggagtcctgg cttgctatag cttgctagta acagtggcct ttattatttt ctgggtgagg 1080

agtaagagga gcaggctcct gcacagtgac tacatgaaca tgactccccg ccgccccggg 1140

cccacccgca agcattacca gccctatgcc ccaccacgcg acttcgcagc ctatcgctcc 1200

agagtgaagt tcagcaggag cgcagacgcc cccgcgtacc agcagggcca gaaccagctc 1260

tataacgagc tcaatctagg acgaagagag gagtacgatg ttttggacaa gagacgtggc 1320

cgggaccctg agatgggggg aaagccgaga aggaagaacc ctcaggaagg cctgtacaat 1380

gaactgcaga aagataagat ggcggaggcc tacagtgaga ttgggatgaa aggcgagcgc 1440

cggaggggca aggggcacga tggcctttac cagggtctca gtacagccac caaggacacc 1500

tacgacgccc ttcacatgca ggccctgcct cctcgctaa 1539

<210> 162
<211> 1533
<212> DNA
<213> Artificial

<220>
<223> CAR 016 translation region

<400> 162
atggagtttg ggctttcctg gttgttcctc gtagccatcc tcaaaggggt tcagtgcgac 60

aagttcctga agcatctgac aggcccgctg tacttcagtc ccaagtgtag caaacacttt 120

catcgcctgt atcacaacac acgggattgc accatacccg cctattacaa gaggtgtgca 180

aggctgctga ctcgattggc cgtttctcct gtgtgcatgg aggacaaaca ggagcccaag 240

agctgcgaca agacgcacac ctgtccaccg tgtgatcccg ccgagcccaa atctcctgac 300

aaaactcaca catgcccacc gtgcccagca cctgaactcc tggggggacc gtcagtcttc 360

ctcttccccc caaaacccaa ggacaccctc atgatctccc ggacccctga ggtcacatgc 420

gtggtggtgg acgtgagcca cgaagaccct gaggtcaagt tcaactggta cgtggacggc 480

gtggaggtgc ataatgccaa gacaaagccg cgggaggagc agtacaacag cacgtaccgt 540

gtggtcagcg tcctcaccgt cctgcaccag gactggctga atggcaagga gtacaagtgc 600

aaggtctcca acaaagccct cccagccccc atcgagaaaa ccatctccaa agccaaaggg 660

cagccccgag aaccacaggt gtacaccctg cccccatccc gggatgagct gaccaagaac 720

caggtcagcc tgacctgcct ggtcaaaggc ttctatccca gcgacatcgc cgtggagtgg 780

gagagcaatg ggcaaccgga gaacaactac aagaccacgc ctcccgtgct ggactccgac 840

ggctccttct tcctctacag caagctcacc gtggacaaga gcaggtggca gcaggggaac 900

gtcttctcat gctccgtgat gcatgaggct ctgcacaacc actacacgca gaagagcctc 960

tccctgtctc cgggtaaaaa agatcccaaa ttttgggtgc tggtggtggt tggtggagtc 1020

ctggcttgct atagcttgct agtaacagtg gcctttatta ttttctgggt gaggagtaag 1080

aggagcaggc tcctgcacag tgactacatg aacatgactc cccgccgccc cgggcccacc 1140

cgcaagcatt accagcccta tgccccacca cgcgacttcg cagcctatcg ctccagagtg 1200

aagttcagca ggagcgcaga cgcccccgcg taccagcagg gccagaacca gctctataac 1260

gagctcaatc taggacgaag agaggagtac gatgttttgg acaagagacg tggccgggac 1320

cctgagatgg ggggaaagcc gagaaggaag aaccctcagg aaggcctgta caatgaactg 1380

cagaaagata agatggcgga ggcctacagt gagattggga tgaaaggcga gcgccggagg 1440

ggcaaggggc acgatggcct ttaccagggt ctcagtacag ccaccaagga cacctacgac 1500

gcccttcaca tgcaggccct gcctcctcgc taa 1533

<210> 163
<211> 1522
<212> DNA
<213> Artificial

<220>
<223> CAR 017 translation region

<400> 163
atggagtttg ggctttcctg gttgttcctc gtagccatcc tcaaaggggt tcagtgcaag 60

catctgacag gcccgctgta cttcagtccc aagtgtagca aacactttca tcgcctgtat 120

cacaacacac gggattgcac catacccgcc tattacaaga ggtgtgcaag gctgctgact 180

cgattggccg tttctcctgt gtgcatggag gacaaacagg agcccaagag ctgcgacaag 240

acgcacacct gtccaccgtg tgatcccgcc gagcccaaat ctcctgacaa aactcacaca 300

tgcccaccgt gcccagcacc tgaactcctg gggggaccgt cagtcttcct cttcccccca 360

aaacccaagg acaccctcat gatctcccgg acccctgagg tcacatgcgt ggtggtggac 420

gtgagccacg aagaccctga ggtcaagttc aactggtacg tggacggcgt ggaggtgcat 480

aatgccaaga caaagccgcg ggaggagcag tacaacagca cgtaccgtgt ggtcagcgtc 540

ctcaccgtcc tgcaccagga ctggctgaat ggcaaggagt acaagtgcaa ggtctccaac 600

aaagccctcc cagcccccat cgagaaaacc atctccaaag ccaaagggca gccccgagaa 660

ccacaggtgt acaccctgcc cccatcccgg gatgagctga ccaagaacca ggtcagcctg 720

acctgcctgg tcaaaggctt ctatcccagc gacatcgccg tggagtggga gagcaatggg 780

caaccggaga acaactacaa gaccacgcct cccgtgctgg actccgacgg ctccttcttc 840

ctctacagca agctcaccgt ggacaagagc aggtggcagc aggggaacgt cttctcatgc 900

tccgtgatgc atgaggctct gcacaaccac tacacgcaga agagcctctc cctgtctccg 960

ggtaaaaaag atcccaaatt ttgggtgctg gtggtggttg gtggagtcct ggcttgctat 1020

agcttgctag taacagtggc ctttattatt ttctgggtga ggagtaagag gagcaggctc 1080

ctgcacagtg actacatgaa catgactccc cgccgccccg ggcccacccg caagcattac 1140

cagccctatg ccccaccacg cgacttcgca gcctatcgct ccagagtgaa gttcagcagg 1200

agcgcagacg cccccgcgta ccagcagggc cagaaccagc tctataacga gctcaatcta 1260

ggacgaagag aggagtacga tgttttggac aagagacgtg gccgggaccc tgagatgggg 1320

ggaaagccga gaaggaagaa ccctcaggaa ggcctgtaca atgaactgca gaaagataag 1380

atggcggagg cctacagtga gattgggatg aaaggcgagc gccggagggg caaggggcac 1440

gatggccttt accagggtct cagtacagcc accaaggaca cctacgacgc ccttcacatg 1500

caggccctgc ctcctcgcta ag 1522

<210> 164
<211> 1515
<212> DNA
<213> Artificial

<220>
<223> CAR 018 translation region

<400> 164
atggagtttg ggctttcctg gttgttcctc gtagccatcc tcaaaggggt tcagtgcctg 60

acaggcccgc tgtacttcag tcccaagtgt agcaaacact ttcatcgcct gtatcacaac 120

acacgggatt gcaccatacc cgcctattac aagaggtgtg caaggctgct gactcgattg 180

gccgtttctc ctgtgtgcat ggaggacaaa caggagccca agagctgcga caagacgcac 240

acctgtccac cgtgtgatcc cgccgagccc aaatctcctg acaaaactca cacatgccca 300

ccgtgcccag cacctgaact cctgggggga ccgtcagtct tcctcttccc cccaaaaccc 360

aaggacaccc tcatgatctc ccggacccct gaggtcacat gcgtggtggt ggacgtgagc 420

cacgaagacc ctgaggtcaa gttcaactgg tacgtggacg gcgtggaggt gcataatgcc 480

aagacaaagc cgcgggagga gcagtacaac agcacgtacc gtgtggtcag cgtcctcacc 540

gtcctgcacc aggactggct gaatggcaag gagtacaagt gcaaggtctc caacaaagcc 600

ctcccagccc ccatcgagaa aaccatctcc aaagccaaag ggcagccccg agaaccacag 660

gtgtacaccc tgcccccatc ccgggatgag ctgaccaaga accaggtcag cctgacctgc 720

ctggtcaaag gcttctatcc cagcgacatc gccgtggagt gggagagcaa tgggcaaccg 780

gagaacaact acaagaccac gcctcccgtg ctggactccg acggctcctt cttcctctac 840

agcaagctca ccgtggacaa gagcaggtgg cagcagggga acgtcttctc atgctccgtg 900

atgcatgagg ctctgcacaa ccactacacg cagaagagcc tctccctgtc tccgggtaaa 960

aaagatccca aattttgggt gctggtggtg gttggtggag tcctggcttg ctatagcttg 1020

ctagtaacag tggcctttat tattttctgg gtgaggagta agaggagcag gctcctgcac 1080

agtgactaca tgaacatgac tccccgccgc cccgggccca cccgcaagca ttaccagccc 1140

tatgccccac cacgcgactt cgcagcctat cgctccagag tgaagttcag caggagcgca 1200

gacgcccccg cgtaccagca gggccagaac cagctctata acgagctcaa tctaggacga 1260

agagaggagt acgatgtttt ggacaagaga cgtggccggg accctgagat ggggggaaag 1320

ccgagaagga agaaccctca ggaaggcctg tacaatgaac tgcagaaaga taagatggcg 1380

gaggcctaca gtgagattgg gatgaaaggc gagcgccgga ggggcaaggg gcacgatggc 1440

ctttaccagg gtctcagtac agccaccaag gacacctacg acgcccttca catgcaggcc 1500

ctgcctcctc gctaa 1515

<210> 165
<211> 1509
<212> DNA
<213> Artificial

<220>
<223> CAR 019 translation region

<400> 165
atggagtttg ggctttcctg gttgttcctc gtagccatcc tcaaaggggt tcagtgcggc 60

ccgctgtact tcagtcccaa gtgtagcaaa cactttcatc gcctgtatca caacacacgg 120

gattgcacca tacccgccta ttacaagagg tgtgcaaggc tgctgactcg attggccgtt 180

tctcctgtgt gcatggagga caaacaggag cccaagagct gcgacaagac gcacacctgt 240

ccaccgtgtg atcccgccga gcccaaatct cctgacaaaa ctcacacatg cccaccgtgc 300

ccagcacctg aactcctggg gggaccgtca gtcttcctct tccccccaaa acccaaggac 360

accctcatga tctcccggac ccctgaggtc acatgcgtgg tggtggacgt gagccacgaa 420

gaccctgagg tcaagttcaa ctggtacgtg gacggcgtgg aggtgcataa tgccaagaca 480

aagccgcggg aggagcagta caacagcacg taccgtgtgg tcagcgtcct caccgtcctg 540

caccaggact ggctgaatgg caaggagtac aagtgcaagg tctccaacaa agccctccca 600

gcccccatcg agaaaaccat ctccaaagcc aaagggcagc cccgagaacc acaggtgtac 660

accctgcccc catcccggga tgagctgacc aagaaccagg tcagcctgac ctgcctggtc 720

aaaggcttct atcccagcga catcgccgtg gagtgggaga gcaatgggca accggagaac 780

aactacaaga ccacgcctcc cgtgctggac tccgacggct ccttcttcct ctacagcaag 840

ctcaccgtgg acaagagcag gtggcagcag gggaacgtct tctcatgctc cgtgatgcat 900

gaggctctgc acaaccacta cacgcagaag agcctctccc tgtctccggg taaaaaagat 960

cccaaatttt gggtgctggt ggtggttggt ggagtcctgg cttgctatag cttgctagta 1020

acagtggcct ttattatttt ctgggtgagg agtaagagga gcaggctcct gcacagtgac 1080

tacatgaaca tgactccccg ccgccccggg cccacccgca agcattacca gccctatgcc 1140

ccaccacgcg acttcgcagc ctatcgctcc agagtgaagt tcagcaggag cgcagacgcc 1200

cccgcgtacc agcagggcca gaaccagctc tataacgagc tcaatctagg acgaagagag 1260

gagtacgatg ttttggacaa gagacgtggc cgggaccctg agatgggggg aaagccgaga 1320

aggaagaacc ctcaggaagg cctgtacaat gaactgcaga aagataagat ggcggaggcc 1380

tacagtgaga ttgggatgaa aggcgagcgc cggaggggca aggggcacga tggcctttac 1440

cagggtctca gtacagccac caaggacacc tacgacgccc ttcacatgca ggccctgcct 1500

cctcgctaa 1509

<210> 166
<211> 512
<212> PRT
<213> Artificial

<220>
<223> CAR 015

<400> 166

Met Glu Phe Gly Leu Ser Trp Leu Phe Leu Val Ala Ile Leu Lys Gly
1               5                   10                  15

Val Gln Cys Met Lys Asp Lys Phe Leu Lys His Leu Thr Gly Pro Leu
            20                  25                  30

Tyr Phe Ser Pro Lys Cys Ser Lys His Phe His Arg Leu Tyr His Asn
        35                  40                  45

Thr Arg Asp Cys Thr Ile Pro Ala Tyr Tyr Lys Arg Cys Ala Arg Leu
    50                  55                  60

Leu Thr Arg Leu Ala Val Ser Pro Val Cys Met Glu Asp Lys Gln Glu
65                  70                  75                  80

Pro Lys Ser Cys Asp Lys Thr His Thr Cys Pro Pro Cys Asp Pro Ala
                85                  90                  95

Glu Pro Lys Ser Pro Asp Lys Thr His Thr Cys Pro Pro Cys Pro Ala
        100                 105                 110

Pro Glu Leu Leu Gly Gly Pro Ser Val Phe Leu Phe Pro Pro Lys Pro
    115                 120                 125

Lys Asp Thr Leu Met Ile Ser Arg Thr Pro Glu Val Thr Cys Val Val
    130                 135                 140

Val Asp Val Ser His Glu Asp Pro Glu Val Lys Phe Asn Trp Tyr Val
145                 150                 155                 160

Asp Gly Val Glu Val His Asn Ala Lys Thr Lys Pro Arg Glu Glu Gln
                165                 170                 175

Tyr Asn Ser Thr Tyr Arg Val Val Ser Val Leu Thr Val Leu His Gln
        180                 185                 190

Asp Trp Leu Asn Gly Lys Glu Tyr Lys Cys Lys Val Ser Asn Lys Ala
    195                 200                 205

Leu Pro Ala Pro Ile Glu Lys Thr Ile Ser Lys Ala Lys Gly Gln Pro
    210                 215                 220

Arg Glu Pro Gln Val Tyr Thr Leu Pro Pro Ser Arg Asp Glu Leu Thr
225                 230                 235                 240

Lys Asn Gln Val Ser Leu Thr Cys Leu Val Lys Gly Phe Tyr Pro Ser
                245                 250                 255

Asp Ile Ala Val Glu Trp Glu Ser Asn Gly Gln Pro Glu Asn Asn Tyr
        260                 265                 270

Lys Thr Thr Pro Pro Val Leu Asp Ser Asp Gly Ser Phe Phe Leu Tyr
    275                 280                 285

Ser Lys Leu Thr Val Asp Lys Ser Arg Trp Gln Gln Gly Asn Val Phe
    290                 295                 300

Ser Cys Ser Val Met His Glu Ala Leu His Asn His Tyr Thr Gln Lys
305                 310                 315                 320

Ser Leu Ser Leu Ser Pro Gly Lys Lys Asp Pro Lys Phe Trp Val Leu
                325                 330                 335

Val Val Val Gly Gly Val Leu Ala Cys Tyr Ser Leu Leu Val Thr Val

340                 345                 350

Ala Phe Ile Ile Phe Trp Val Arg Ser Lys Arg Ser Arg Leu Leu His
        355                 360                 365

Ser Asp Tyr Met Asn Met Thr Pro Arg Arg Pro Gly Pro Thr Arg Lys
    370                 375                 380

His Tyr Gln Pro Tyr Ala Pro Pro Arg Asp Phe Ala Ala Tyr Arg Ser
385                 390                 395                 400

Arg Val Lys Phe Ser Arg Ser Ala Asp Ala Pro Ala Tyr Gln Gln Gly
                405                 410                 415

Gln Asn Gln Leu Tyr Asn Glu Leu Asn Leu Gly Arg Arg Glu Glu Tyr
            420                 425                 430

Asp Val Leu Asp Lys Arg Arg Gly Arg Asp Pro Glu Met Gly Gly Lys
        435                 440                 445

Pro Arg Arg Lys Asn Pro Gln Glu Gly Leu Tyr Asn Glu Leu Gln Lys
    450                 455                 460

Asp Lys Met Ala Glu Ala Tyr Ser Glu Ile Gly Met Lys Gly Glu Arg
465                 470                 475                 480

Arg Arg Gly Lys Gly His Asp Gly Leu Tyr Gln Gly Leu Ser Thr Ala
                485                 490                 495

Thr Lys Asp Thr Tyr Asp Ala Leu His Met Gln Ala Leu Pro Pro Arg
            500                 505                 510

<210>  167
<211>  510
<212>  PRT
<213>  Artificial

<220>
<223>  CAR 016

<400>  167

Met Glu Phe Gly Leu Ser Trp Leu Phe Leu Val Ala Ile Leu Lys Gly
1               5                   10                  15

Val Gln Cys Asp Lys Phe Leu Lys His Leu Thr Gly Pro Leu Tyr Phe
            20                  25                  30

Ser Pro Lys Cys Ser Lys His Phe His Arg Leu Tyr His Asn Thr Arg
        35                  40                  45

Asp Cys Thr Ile Pro Ala Tyr Tyr Lys Arg Cys Ala Arg Leu Leu Thr
    50                  55                  60

Arg Leu Ala Val Ser Pro Val Cys Met Glu Asp Lys Gln Glu Pro Lys
65                  70                  75                  80

Ser Cys Asp Lys Thr His Thr Cys Pro Pro Cys Asp Pro Ala Glu Pro
                85                  90                  95

Lys Ser Pro Asp Lys Thr His Thr Cys Pro Pro Cys Pro Ala Pro Glu
            100                 105                 110

Leu Leu Gly Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Lys Phe Asn Trp Tyr Val Asp Gly
145                 150                 155                 160

Val Glu Val His Asn Ala Lys Thr Lys Pro Arg Glu Glu Gln Tyr Asn
                165                 170                 175

Ser Thr Tyr Arg Val Val Ser Val Leu Thr Val Leu His Gln Asp Trp
            180                 185                 190

Leu Asn Gly Lys Glu Tyr Lys Cys Lys Val Ser Asn Lys Ala Leu Pro
        195                 200                 205

Ala Pro Ile Glu Lys Thr Ile Ser Lys Ala Lys Gly Gln Pro Arg Glu
    210                 215                 220

Pro Gln Val Tyr Thr Leu Pro Pro Ser Arg Asp Glu Leu Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Val Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys

290                 295                 300

Ser Val Met His Glu Ala Leu His Asn His Tyr Thr Gln Lys Ser Leu
305                 310                 315                 320

Ser Leu Ser Pro Gly Lys Lys Asp Pro Lys Phe Trp Val Leu Val Val
                325                 330                 335

Val Gly Gly Val Leu Ala Cys Tyr Ser Leu Leu Val Thr Val Ala Phe
            340                 345                 350

Ile Ile Phe Trp Val Arg Ser Lys Arg Ser Arg Leu Leu His Ser Asp
        355                 360                 365

Tyr Met Asn Met Thr Pro Arg Arg Pro Gly Pro Thr Arg Lys His Tyr
    370                 375                 380

Gln Pro Tyr Ala Pro Pro Arg Asp Phe Ala Ala Tyr Arg Ser Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Arg
        435                 440                 445

Arg Lys Asn Pro Gln Glu Gly Leu Tyr Asn Glu Leu Gln Lys Asp Lys
    450                 455                 460

Met Ala Glu Ala Tyr Ser Glu Ile Gly Met Lys Gly Glu Arg Arg Arg
465                 470                 475                 480

Gly Lys Gly His Asp Gly Leu Tyr Gln Gly Leu Ser Thr Ala Thr Lys
                485                 490                 495

Asp Thr Tyr Asp Ala Leu His Met Gln Ala Leu Pro Pro Arg
            500                 505                 510

<210>  168
<211>  506
<212>  PRT
<213>  Artificial

<220>
<223>  CAR 017

<400>  168

Met Glu Phe Gly Leu Ser Trp Leu Phe Leu Val Ala Ile Leu Lys Gly
1               5                   10                  15

Val Gln Cys Lys His Leu Thr Gly Pro Leu Tyr Phe Ser Pro Lys Cys
            20                  25                  30

Ser Lys His Phe His Arg Leu Tyr His Asn Thr Arg Asp Cys Thr Ile
        35                  40                  45

Pro Ala Tyr Tyr Lys Arg Cys Ala Arg Leu Leu Thr Arg Leu Ala Val
    50                  55                  60

Ser Pro Val Cys Met Glu Asp Lys Gln Glu Pro Lys Ser Cys Asp Lys
65                  70                  75                  80

Thr His Thr Cys Pro Pro Cys Asp Pro Ala Glu Pro Lys Ser Pro Asp
                85                  90                  95

Lys Thr His Thr Cys Pro Pro Cys Pro Ala Pro Glu Leu Leu Gly Gly
            100                 105                 110

Pro Ser Val Phe Leu Phe Pro Pro Lys Pro Lys Asp Thr Leu Met Ile
        115                 120                 125

Ser Arg Thr Pro Glu Val Thr Cys Val Val Val Asp Val Ser His Glu
    130                 135                 140

Asp Pro Glu Val Lys Phe Asn Trp Tyr Val Asp Gly Val Glu Val His
145                 150                 155                 160

Asn Ala Lys Thr Lys Pro Arg Glu Glu Gln Tyr Asn Ser Thr Tyr Arg
                165                 170                 175

Val Val Ser Val Leu Thr Val Leu His Gln Asp Trp Leu Asn Gly Lys
            180                 185                 190

Glu Tyr Lys Cys Lys Val Ser Asn Lys Ala Leu Pro Ala Pro Ile Glu
        195                 200                 205

Lys Thr Ile Ser Lys Ala Lys Gly Gln Pro Arg Glu Pro Gln Val Tyr
    210                 215                 220

Thr Leu Pro Pro Ser Arg Asp Glu Leu Thr Lys Asn Gln Val Ser Leu
225                 230                 235                 240

Thr Cys Leu Val Lys Gly Phe Tyr Pro Ser Asp Ile Ala Val Glu Trp

245                 250                 255

Glu Ser Asn Gly Gln Pro Glu Asn Asn Tyr Lys Thr Thr Pro Pro Val
            260                 265                 270

Leu Asp Ser Asp Gly Ser Phe Phe Leu Tyr Ser Lys Leu Thr Val Asp
        275                 280                 285

Lys Ser Arg Trp Gln Gln Gly Asn Val Phe Ser Cys Ser Val Met His
    290                 295                 300

Glu Ala Leu His Asn His Tyr Thr Gln Lys Ser Leu Ser Leu Ser Pro
305                 310                 315                 320

Gly Lys Lys Asp Pro Lys Phe Trp Val Leu Val Val Val Gly Gly Val
                325                 330                 335

Leu Ala Cys Tyr Ser Leu Leu Val Thr Val Ala Phe Ile Ile Phe Trp
            340                 345                 350

Val Arg Ser Lys Arg Ser Arg Leu Leu His Ser Asp Tyr Met Asn Met
        355                 360                 365

Thr Pro Arg Arg Pro Gly Pro Thr Arg Lys His Tyr Gln Pro Tyr Ala
    370                 375                 380

Pro Pro Arg Asp Phe Ala Ala Tyr Arg Ser Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Arg Arg Lys Asn Pro
        435                 440                 445

Gln Glu Gly Leu Tyr Asn Glu Leu Gln Lys Asp Lys Met Ala Glu Ala
    450                 455                 460

Tyr Ser Glu Ile Gly Met Lys Gly Glu Arg Arg Arg Gly Lys Gly His
465                 470                 475                 480

Asp Gly Leu Tyr Gln Gly Leu Ser Thr Ala Thr Lys Asp Thr Tyr Asp
                485                 490                 495

Ala Leu His Met Gln Ala Leu Pro Pro Arg
            500                 505

<210>  169
<211>  504
<212>  PRT
<213>  Artificial

<220>
<223>  CAR 018

<400>  169

Met Glu Phe Gly Leu Ser Trp Leu Phe Leu Val Ala Ile Leu Lys Gly
1               5                   10                  15

Val Gln Cys Leu Thr Gly Pro Leu Tyr Phe Ser Pro Lys Cys Ser Lys
            20                  25                  30

His Phe His Arg Leu Tyr His Asn Thr Arg Asp Cys Thr Ile Pro Ala
        35                  40                  45

Tyr Tyr Lys Arg Cys Ala Arg Leu Leu Thr Arg Leu Ala Val Ser Pro
    50                  55                  60

Val Cys Met Glu Asp Lys Gln Glu Pro Lys Ser Cys Asp Lys Thr His
65                  70                  75                  80

Thr Cys Pro Pro Cys Asp Pro Ala Glu Pro Lys Ser Pro Asp Lys Thr
                85                  90                  95

His Thr Cys Pro Pro Cys Pro Ala Pro Glu Leu Leu Gly Gly Pro Ser
            100                 105                 110

Val Phe Leu Phe Pro Pro Lys Pro Lys Asp Thr Leu Met Ile Ser Arg
        115                 120                 125

Thr Pro Glu Val Thr Cys Val Val Val Asp Val Ser His Glu Asp Pro
    130                 135                 140

Glu Val Lys Phe Asn Trp Tyr Val Asp Gly Val Glu Val His Asn Ala
145                 150                 155                 160

Lys Thr Lys Pro Arg Glu Glu Gln Tyr Asn Ser Thr Tyr Arg Val Val
                165                 170                 175

Ser Val Leu Thr Val Leu His Gln Asp Trp Leu Asn Gly Lys Glu Tyr
            180                 185                 190

Lys Cys Lys Val Ser Asn Lys Ala Leu Pro Ala Pro Ile Glu Lys Thr

195                 200                 205

Ile Ser Lys Ala Lys Gly Gln Pro Arg Glu Pro Gln Val Tyr Thr Leu
    210                 215                 220

Pro Pro Ser Arg Asp Glu Leu Thr Lys Asn Gln Val Ser Leu Thr Cys
225                 230                 235                 240

Leu Val Lys Gly Phe Tyr Pro Ser Asp Ile Ala Val Glu Trp Glu Ser
                245                 250                 255

Asn Gly Gln Pro Glu Asn Asn Tyr Lys Thr Thr Pro Pro Val Leu Asp
            260                 265                 270

Ser Asp Gly Ser Phe Phe Leu Tyr Ser Lys Leu Thr Val Asp Lys Ser
        275                 280                 285

Arg Trp Gln Gln Gly Asn Val Phe Ser Cys Ser Val Met His Glu Ala
    290                 295                 300

Leu His Asn His Tyr Thr Gln Lys Ser Leu Ser Leu Ser Pro Gly Lys
305                 310                 315                 320

Lys Asp Pro Lys Phe Trp Val Leu Val Val Val Gly Gly Val Leu Ala
                325                 330                 335

Cys Tyr Ser Leu Leu Val Thr Val Ala Phe Ile Ile Phe Trp Val Arg
            340                 345                 350

Ser Lys Arg Ser Arg Leu Leu His Ser Asp Tyr Met Asn Met Thr Pro
        355                 360                 365

Arg Arg Pro Gly Pro Thr Arg Lys His Tyr Gln Pro Tyr Ala Pro Pro
    370                 375                 380

Arg Asp Phe Ala Ala Tyr Arg Ser Arg Val Lys Phe Ser Arg Ser Ala
385                 390                 395                 400

Asp Ala Pro Ala Tyr Gln Gln Gly Gln Asn Gln Leu Tyr Asn Glu Leu
                405                 410                 415

Asn Leu Gly Arg Arg Glu Glu Tyr Asp Val Leu Asp Lys Arg Arg Gly
            420                 425                 430

Arg Asp Pro Glu Met Gly Gly Lys Pro Arg Arg Lys Asn Pro Gln Glu
        435                 440                 445

Gly Leu Tyr Asn Glu Leu Gln Lys Asp Lys Met Ala Glu Ala Tyr Ser
    450                 455                 460

Glu Ile Gly Met Lys Gly Glu Arg Arg Arg Gly Lys Gly His Asp Gly
465                 470                 475                 480

Leu Tyr Gln Gly Leu Ser Thr Ala Thr Lys Asp Thr Tyr Asp Ala Leu
                485                 490                 495

His Met Gln Ala Leu Pro Pro Arg
            500

<210>  170
<211>  502
<212>  PRT
<213>  Artificial

<220>
<223>  CAR 019

<400>  170

Met Glu Phe Gly Leu Ser Trp Leu Phe Leu Val Ala Ile Leu Lys Gly
1               5                   10                  15

Val Gln Cys Gly Pro Leu Tyr Phe Ser Pro Lys Cys Ser Lys His Phe
            20                  25                  30

His Arg Leu Tyr His Asn Thr Arg Asp Cys Thr Ile Pro Ala Tyr Tyr
        35                  40                  45

Lys Arg Cys Ala Arg Leu Leu Thr Arg Leu Ala Val Ser Pro Val Cys
    50                  55                  60

Met Glu Asp Lys Gln Glu Pro Lys Ser Cys Asp Lys Thr His Thr Cys
65                  70                  75                  80

Pro Pro Cys Asp Pro Ala Glu Pro Lys Ser Pro Asp Lys Thr His Thr
                85                  90                  95

Cys Pro Pro Cys Pro Ala Pro Glu Leu Leu Gly Gly Pro Ser Val Phe
            100                 105                 110

Leu Phe Pro Pro Lys Pro Lys Asp Thr Leu Met Ile Ser Arg Thr Pro
        115                 120                 125

Glu Val Thr Cys Val Val Val Asp Val Ser His Glu Asp Pro Glu Val
    130                 135                 140

Lys Phe Asn Trp Tyr Val Asp Gly Val Glu Val His Asn Ala Lys Thr

145                 150                 155                 160

Lys Pro Arg Glu Glu Gln Tyr Asn Ser Thr Tyr Arg Val Val Ser Val
                165                 170                 175

Leu Thr Val Leu His Gln Asp Trp Leu Asn Gly Lys Glu Tyr Lys Cys
            180                 185                 190

Lys Val Ser Asn Lys Ala Leu Pro Ala Pro Ile Glu Lys Thr Ile Ser
        195                 200                 205

Lys Ala Lys Gly Gln Pro Arg Glu Pro Gln Val Tyr Thr Leu Pro Pro
    210                 215                 220

Ser Arg Asp Glu Leu Thr Lys Asn Gln Val Ser Leu Thr Cys Leu Val
225                 230                 235                 240

Lys Gly Phe Tyr Pro Ser Asp Ile Ala Val Glu Trp Glu Ser Asn Gly
                245                 250                 255

Gln Pro Glu Asn Asn Tyr Lys Thr Thr Pro Pro Val Leu Asp Ser Asp
            260                 265                 270

Gly Ser Phe Phe Leu Tyr Ser Lys Leu Thr Val Asp Lys Ser Arg Trp
        275                 280                 285

Gln Gln Gly Asn Val Phe Ser Cys Ser Val Met His Glu Ala Leu His
    290                 295                 300

Asn His Tyr Thr Gln Lys Ser Leu Ser Leu Ser Pro Gly Lys Lys Asp
305                 310                 315                 320

Pro Lys Phe Trp Val Leu Val Val Val Gly Gly Val Leu Ala Cys Tyr
                325                 330                 335

Ser Leu Leu Val Thr Val Ala Phe Ile Ile Phe Trp Val Arg Ser Lys
            340                 345                 350

Arg Ser Arg Leu Leu His Ser Asp Tyr Met Asn Met Thr Pro Arg Arg
        355                 360                 365

Pro Gly Pro Thr Arg Lys His Tyr Gln Pro Tyr Ala Pro Pro Arg Asp
    370                 375                 380

Phe Ala Ala Tyr Arg Ser Arg Val Lys Phe Ser Arg Ser Ala Asp Ala
385                 390                 395                 400

Pro Ala Tyr Gln Gln Gly Gln Asn Gln Leu Tyr Asn Glu Leu Asn Leu
                405                 410                 415

Gly Arg Arg Glu Glu Tyr Asp Val Leu Asp Lys Arg Arg Gly Arg Asp
            420                 425                 430

Pro Glu Met Gly Gly Lys Pro Arg Arg Lys Asn Pro Gln Glu Gly Leu
        435                 440                 445

Tyr Asn Glu Leu Gln Lys Asp Lys Met Ala Glu Ala Tyr Ser Glu Ile
    450                 455                 460

Gly Met Lys Gly Glu Arg Arg Arg Gly Lys Gly His Asp Gly Leu Tyr
465                 470                 475                 480

Gln Gly Leu Ser Thr Ala Thr Lys Asp Thr Tyr Asp Ala Leu His Met
                485                 490                 495

Gln Ala Leu Pro Pro Arg
            500

<210>  171
<211>  39
<212>  DNA
<213>  Artificial

<220>
<223>  forward primer

<400>  171
gccgcggcaa ccaccactcc tgctcccaga ccacctact                              39

<210>  172
<211>  37
<212>  DNA
<213>  Artificial

<220>
<223>  reverse primer

<400>  172
tgggcatgtg tgagttttgt caggagattt gggctcg                                37

<210>  173
<211>  1071
<212>  DNA
<213>  Artificial

<220>
<223>  CAR 020 translation region

<400>  173
atggagtttg ggctttcctg gttgttcctc gtagccatcc tcaaaggggt tcagtgcgca       60

gaagcggctg gtctgggtcc ctcaccagaa cagcgagtcg agattgtgcc tcgcgatctc 120

cgcatgaagg acaagttcct gaagcatctg acaggcccgc tgtacttcag tcccaagtgt 180

agcaaacact ttcatcgcct gtatcacaac acacgggatt gcaccatacc cgcctattac 240

aagaggtgtg caaggctgct gactcgattg gccgtttctc ctgtgtgcat ggaggacaaa 300

caggagccca agagctgcga caagacgcac acctgtccac cgtgtgatcc cgccgagccc 360

aaatctcctg acaaaactca cacatgccca gccgcggcaa ccaccactcc tgctcccaga 420

ccacctactc ccgctcccac catagcctct caacctctga gccttaggcc agaagcctgt 480

cgtcctgctg ctggtggagc cgttcacaca aggggattgg actttgcctg cgacatctac 540

atttgggcac cattggcagg cacatgtggc gtgcttctcc tgtcactcgt cattaccctg 600

tattgcaaac gagggcgcaa gaagctgctg tacatcttca aacagccctt tatgcggcca 660

gtgcagacga cacaggagga ggatgggtgc tcctgcagat tcccggaaga ggaggaaggt 720

ggctgtgagc tgagagtgaa gttcagcagg agcgcagacg cccccgcgta ccagcagggc 780

cagaaccagc tctataacga gctcaatcta ggacgaagag aggagtacga tgttttggac 840

aagagacgtg gccgggaccc tgagatgggg ggaaagccga gaaggaagaa ccctcaggaa 900

ggcctgtaca atgaactgca gaaagataag atggcggagg cctacagtga gattgggatg 960

aaaggcgagc gccggagggg caaggggcac gatggccttt accagggtct cagtacagcc 1020

accaaggaca cctacgacgc ccttcacatg caggccctgc ctcctcgcta a 1071

<210> 174
<211> 356
<212> PRT
<213> Artificial

<220>
<223> CAR 020

<400> 174

Met Glu Phe Gly Leu Ser Trp Leu Phe Leu Val Ala Ile Leu Lys Gly
1               5                   10                  15

Val Gln Cys Ala Glu Ala Ala Gly Leu Gly Pro Ser Pro Glu Gln Arg
            20                  25                  30

Val Glu Ile Val Pro Arg Asp Leu Arg Met Lys Asp Lys Phe Leu Lys
        35                  40                  45

His Leu Thr Gly Pro Leu Tyr Phe Ser Pro Lys Cys Ser Lys His Phe
    50                  55                  60

His Arg Leu Tyr His Asn Thr Arg Asp Cys Thr Ile Pro Ala Tyr Tyr
65                  70                  75                  80

Lys Arg Cys Ala Arg Leu Leu Thr Arg Leu Ala Val Ser Pro Val Cys
                85                  90                  95

Met Glu Asp Lys Gln Glu Pro Lys Ser Cys Asp Lys Thr His Thr Cys
            100                 105                 110

Pro Pro Cys Asp Pro Ala Glu Pro Lys Ser Pro Asp Lys Thr His Thr
        115                 120                 125

Cys Pro Ala Ala Ala Thr Thr Thr Pro Ala Pro Arg Pro Pro Thr Pro
    130                 135                 140

Ala Pro Thr Ile Ala Ser Gln Pro Leu Ser Leu Arg Pro Glu Ala Cys
145                 150                 155                 160

Arg Pro Ala Ala Gly Gly Ala Val His Thr Arg Gly Leu Asp Phe Ala
                165                 170                 175

Cys Asp Ile Tyr Ile Trp Ala Pro Leu Ala Gly Thr Cys Gly Val Leu
            180                 185                 190

Leu Leu Ser Leu Val Ile Thr Leu Tyr Cys Lys Arg Gly Arg Lys Lys
        195                 200                 205

Leu Leu Tyr Ile Phe Lys Gln Pro Phe Met Arg Pro Val Gln Thr Thr
    210                 215                 220

Gln Glu Glu Asp Gly Cys Ser Cys Arg Phe Pro Glu Glu Glu Glu Gly
225                 230                 235                 240

Gly Cys Glu Leu Arg Val Lys Phe Ser Arg Ser Ala Asp Ala Pro Ala
                245                 250                 255

Tyr Gln Gln Gly Gln Asn Gln Leu Tyr Asn Glu Leu Asn Leu Gly Arg
            260                 265                 270

Arg Glu Glu Tyr Asp Val Leu Asp Lys Arg Arg Gly Arg Asp Pro Glu
        275                 280                 285

Met Gly Gly Lys Pro Arg Arg Lys Asn Pro Gln Glu Gly Leu Tyr Asn
    290                 295                 300

Glu Leu Gln Lys Asp Lys Met Ala Glu Ala Tyr Ser Glu Ile Gly Met
305                 310                 315                 320

Lys Gly Glu Arg Arg Arg Gly Lys Gly His Asp Gly Leu Tyr Gln Gly

325                 330                 335

Leu Ser Thr Ala Thr Lys Asp Thr Tyr Asp Ala Leu His Met Gln Ala
            340                 345                 350

Leu Pro Pro Arg
        355

<210>  175
<211>  2271
<212>  DNA
<213>  Homo sapiens

<400>  175
   atgggagcca tcgggctcct gtggctcctg ccgctgctgc tttccacggc agctgtgggc      60
   tccgggatgg ggaccggcca gcgcgcgggc tccccagctg cggggccgcc gctgcagccc     120
   cgggagccac tcagctactc gcgcctgcag aggaagagtc tggcagttga cttcgtggtg     180
   ccctcgctct tccgtgtcta cgcccgggac ctactgctgc caccatcctc ctcggagctg     240
   aaggctggca ggcccgaggc ccgcggctcg ctagctctgg actgcgcccc gctgctcagg     300
   ttgctggggc cggcgccggg ggtctcctgg accgccggtt caccagcccc ggcagaggcc     360
   cggacgctgt ccagggtgct gaagggcggc tccgtgcgca agctccggcg tgccaagcag     420
   ttggtgctgg agctgggcga ggaggcgatc ttggagggtt gcgtcgggcc ccccggggag     480
   gcggctgtgg ggctgctcca gttcaatctc agcgagctgt tcagttggtg gattcgccaa     540
   ggcgaagggc gactgaggat ccgcctgatg cccgagaaga aggcgtcgga agtgggcaga     600
   gagggaaggc tgtccgcggc aattcgcgcc tcccagcccc gccttctctt ccagatcttc     660
   gggactggtc atagctcctt ggaatcacca acaaacatgc cttctccttc tcctgattat     720
   tttacatgga atctcacctg gataatgaaa gactccttcc ctttcctgtc tcatcgcagc     780
   cgatatggtc tggagtgcag ctttgacttc ccctgtgagc tggagtattc ccctccactg     840
   catgacctca ggaaccagag ctggtcctgg cgccgcatcc cctccgagga ggcctcccag     900
   atggacttgc tggatgggcc tggggcagag cgttctaagg agatgcccag aggctccttt     960
   ctccttctca acacctcagc tgactccaag cacaccatcc tgagtccgtg gatgaggagc    1020
   agcagtgagc actgcacact ggccgtctcg gtgcacaggc acctgcagcc ctctggaagg    1080
   tacattgccc agctgctgcc ccacaacgag gctgcaagag agatcctcct gatgcccact    1140
   ccagggaagc atggttggac agtgctccag ggaagaatcg ggcgtccaga caacccattt    1200
   cgagtggccc tggaatacat ctccagtgga aaccgcagct tgtctgcagt ggacttcttt    1260
   gccctgaaga actgcagtga aggaacatcc ccaggctcca agatggccct gcagagctcc    1320
   ttcacttgtt ggaatgggac agtcctccag cttgggcagg cctgtgactt ccaccaggac    1380
   tgtgcccagg gagaagatga gagccagatg tgccggaaac tgcctgtggg tttttactgc 1440
   aactttgaag atggcttctg tggctggacc caaggcacac tgtcacccca cactcctcaa 1500
   tggcaggtca ggaccctaaa ggatgcccgg ttccaggacc accaagacca tgctctattg 1560
   ctcagtacca ctgatgtccc cgcttctgaa agtgctacag tgaccagtgc tacgtttcct 1620
   gcaccgatca agagctctcc atgtgagctc cgaatgtcct ggctcattcg tggagtcttg 1680
   aggggaaacg tgtccttggt gctagtggag aacaaaaccg ggaaggagca aggcaggatg 1740
   gtctggcatg tcgccgccta tgaaggcttg agcctgtggc agtggatggt gttgcctctc 1800
   ctcgatgtgt ctgacaggtt ctggctgcag atggtcgcat ggtggggaca aggatccaga 1860
   gccatcgtgg cttttgacaa tatctccatc agcctggact gctacctcac cattagcgga 1920
   gaggacaaga tcctgcagaa tacagcaccc aaatcaagaa acctgtttga gagaaaccca 1980
   aacaaggagc tgaaacccgg ggaaaattca ccaagacaga cccccatctt tgaccctaca 2040
   gttcattggc tgttcaccac atgtggggcc agcggccccc atggccccac ccaggcacag 2100
   tgcaacaacg cctaccagaa ctccaacctg agcgtggagg tggggagcga gggccccctg 2160
   aaaggcatcc agatctggaa ggtgccagcc accgacacct acagcatctc gggctacgga 2220
   gctgctggcg ggaaaggcgg gaagaacacc atgatgcggt cccacggcgt g 2271

<210> 176
<211> 2283
<212> DNA
<213> Artificial

<220>
<223> HindIII-Kozak-ALK-DraIII

<400> 176
   aagcttgcca ccatgggagc catcgggctc ctgtggctcc tgccgctgct gctttccacg 60
   gcagctgtgg gctccgggat ggggaccggc cagcgcgcgg gctccccagc tgcggggccg 120
   ccgctgcagc cccgggagcc actcagctac tcgcgcctgc agaggaagag tctggcagtt 180
   gacttcgtgg tgccctcgct cttccgtgtc tacgcccggg acctactgct gccaccatcc 240
   tcctcggagc tgaaggctgg caggcccgag gcccgcggct cgctagctct ggactgcgcc 300
   ccgctgctca ggttgctggg gccggcgccg ggggtctcct ggaccgccgg ttcaccagcc 360
   ccggcagagg cccggacgct gtccagggtg ctgaaggcgg gctccgtgcg caagctccgg 420
   cgtgccaagc agttggtgct ggagctgggc gaggaggcga tcttggaggg ttgcgtcggg 480
   ccccccgggg aggcggctgt ggggctgctc cagttcaatc tcagcgagct gttcagttgg 540
   tggattcgcc aaggcgaagg gcgactgagg atccgcctga tgcccgagaa gaaggcgtcg 600
   gaagtgggca gagagggaag gctgtccgcg gcaattcgcg cctcccagcc ccgccttctc 660
   ttccagatct tcgggactgg tcatagctcc ttggaatcac caacaaacat gccttctcct 720
   tctcctgatt attttacatg gaatctcacc tggataatga aagactcctt ccctttcctg 780
   tctcatcgca gccgatatgg tctggagtgc agctttgact tcccctgtga gctggagtat 840
   tcccctccac tgcatgacct caggaaccag agctggtcct ggcgccgcat cccctccgag 900
   gaggcctccc agatggactt gctggatggg cctggggcag agcgttctaa ggagatgccc 960
   agaggctcct ttctccttct caacacctca gctgactcca agcacaccat cctgagtccg 1020
   tggatgagga gcagcagtga gcactgcaca ctggccgtct cggtgcacag gcacctgcag 1080
   ccctctggaa ggtacattgc ccagctgctg ccccacaacg aggctgcaag agagatcctc 1140
   ctgatgccca ctccagggaa gcatggttgg acagtgctcc agggaagaat cgggcgtcca 1200
   gacaacccat ttcgagtggc cctggaatac atctccagtg gaaaccgcag cttgtctgca 1260
   gtggacttct ttgccctgaa gaactgcagt gaaggaacat ccccaggctc caagatggcc 1320
   ctgcagagct ccttcacttg ttggaatggg acagtcctcc agcttgggca ggcctgtgac 1380
   ttccaccagg actgtgccca gggagaagat gagagccaga tgtgccggaa actgcctgtg 1440
   ggtttttact gcaactttga agatggcttc tgtggctgga cccaaggcac actgtcaccc 1500
   cacactcctc aatggcaggt caggacccta aaggatgccc ggttccagga ccaccaagac 1560
   catgctctat tgctcagtac cactgatgtc cccgcttctg aaagtgctac agtgaccagt 1620
   gctacgtttc ctgcaccgat caagagctct ccatgtgagc tccgaatgtc ctggctcatt 1680
   cgtggagtct tgaggggaaa cgtgtccttg gtgctagtgg agaacaaaac cgggaaggag 1740
   caaggcagga tggtctggca tgtcgccgcc tatgaaggct tgagcctgtg gcagtggatg 1800
   gtgttgcctc tcctcgatgt gtctgacagg ttctggctgc agatggtcgc atggtgggga 1860
   caaggatcca gagccatcgt ggcttttgac aatatctcca tcagcctgga ctgctacctc 1920
   accattagcg gagaggacaa gatcctgcag aatacagcac ccaaatcaag aaacctgttt 1980
   gagagaaacc caaacaagga gctgaaaccc ggggaaaatt caccaagaca gacccccatc 2040
   tttgacccta cagttcattg gctgttcacc acatgtgggg ccagcgggcc ccatggcccc 2100
   acccaggcac agtgcaacaa cgcctaccag aactccaacc tgagcgtgga ggtggggagc 2160
   gagggccccc tgaaaggcat ccagatctgg aaggtgccag ccaccgacac ctacagcatc 2220
   tcgggctacg gagctgctgg cgggaaaggc gggaagaaca ccatgatgcg gtcccacggc 2280
   gtg 2283

<210> 177
<211> 2601
<212> DNA
<213> Homo sapiens

<400> 177
cacggcgtgt ctgtgctggg catcttcaac ctggagaagg atgacatgct gtacatcctg 60

gttgggcagc agggagagga cgcctgcccc agtacaaacc agttaatcca gaaagtctgc 120

attggagaga acaatgtgat agaagaagaa atccgtgtga acagaagcgt gcatgagtgg 180

gcaggaggcg gaggaggagg gggtggagcc acctacgtat ttaagatgaa ggatggagtg 240

ccggtgcccc tgatcattgc agccggaggt ggtggcaggg cctacggggc caagacagac 300

acgttccacc cagagagact ggagaataac tcctcggttc tagggctaaa cggcaattcc 360

ggagccgcag gtggtggagg tggctggaat gataacactt ccttgctctg ggccggaaaa 420

tctttgcagg agggtgccac cggaggacat tcctgccccc aggccatgaa gaagtggggg 480

tgggagacaa gagggggttt cggagggggt ggaggggggt gctcctcagg tggaggaggc 540

ggaggatata taggcggcaa tgcagcctca aacaatgacc ccgaaatgga tggggaagat 600

ggggtttcct tcatcagtcc actgggcatc ctgtacaccc cagctttaaa agtgatggaa 660

ggccacgggg aagtgaatat taagcattat ctaaactgca gtcactgtga ggtagacgaa 720

tgtcacatgg accctgaaag ccacaaggtc atctgcttct gtgaccacgg gacggtgctg 780

gctgaggatg gcgtctcctg cattgtgtca cccaccccgg agccacacct gccactctcg 840

ctgatcctct ctgtggtgac ctctgccctc gtggccgccc tggtcctggc tttctccggc 900

atcatgattg tgtaccgccg gaagcaccag gagctgcaag ccatgcagat ggagctgcag 960

agccctgagt acaagctgag caagctccgc acctcgacca tcatgaccga ctacaacccc 1020

aactactgct ttgctggcaa gacctcctcc atcagtgacc tgaaggaggt gccgcggaaa 1080

aacatcaccc tcattcgggg tctgggccat ggcgcctttg gggaggtgta tgaaggccag 1140

gtgtccggaa tgcccaacga cccaagcccc ctgcaagtgg ctgtgaagac gctgcctgaa 1200

gtgtgctctg aacaggacga actggatttc ctcatggaag ccctgatcat cagcaaattc 1260

aaccaccaga acattgttcg ctgcattggg gtgagcctgc aatccctgcc ccggttcatc 1320

ctgctggagc tcatggcggg gggagacctc aagtccttcc tccgagagac ccgccctcgc 1380

ccgagccagc cctcctccct ggccatgctg gaccttctgc acgtggctcg ggacattgcc 1440

tgtggctgtc agtatttgga ggaaaaccac ttcatccacc gagacattgc tgccagaaac 1500

tgcctcttga cctgtccagg ccctggaaga gtggccaaga ttggagactt cgggatggcc 1560

cgagacatct acagggcgag ctactataga aagggaggct gtgccatgct gccagttaag 1620

tggatgcccc cagaggcctt catggaagga atattcactt ctaaaacaga cacatggtcc 1680

tttggagtgc tgctatggga aatcttttct cttggatata tgccataccc cagcaaaagc 1740

aaccaggaag ttctggagtt tgtcaccagt ggaggccgga tggacccacc caagaactgc 1800

cctgggcctg tataccggat aatgactcag tgctggcaac atcagcctga agacaggccc 1860

aactttgcca tcattttgga gaggattgaa tactgcaccc aggacccgga tgtaatcaac 1920

accgctttgc cgatagaata tggtccactt gtggaagagg aagagaaagt gcctgtgagg 1980

cccaaggacc ctgagggggt tcctcctctc ctggtctctc aacaggcaaa acgggaggag 2040

gagcgcagcc cagctgcccc accacctctg cctaccacct cctctggcaa ggctgcaaag 2100

aaacccacag ctgcagagat ctctgttcga gtccctagag ggccggccgt ggaaggggga 2160

cacgtgaata tggcattctc tcagtccaac cctccttcgg agttgcacaa ggtccacgga 2220

tccagaaaca agcccaccag cttgtggaac ccaacgtacg gctcctggtt tacagagaaa 2280

cccaccaaaa agaataatcc tatagcaaag aaggagccac acgacagggg taacctgggg 2340

ctggagggaa gctgtactgt cccacctaac gttgcaactg ggagacttcc gggggcctca 2400

ctgctcctag agccctcttc gctgactgcc aatatgaagg aggtacctct gttcaggcta 2460

cgtcacttcc cttgtgggaa tgtcaattac ggctaccagc aacagggctt gcccttagaa 2520

gccgctactg cccctggagc tggtcattac gaggatacca ttctgaaaag caagaatagc 2580

atgaaccagc ctgggccctg a 2601

<210> 178
<211> 2612
<212> DNA
<213> Artificial

<220>
<223> DraIII-ALK-TGA-NotI

<400> 178
cacggcgtgt ctgtgctggg catcttcaac ctggagaagg atgacatgct gtacatcctg 60

gttgggcagc agggagagga cgcctgcccc agtacaaacc agttaatcca gaaagtctgc 120

attggagaga acaatgtgat agaagaagaa atccgtgtga acagaagcgt gcatgagtgg 180

gcaggaggcg gaggaggagg gggtggagcc acctacgtat ttaagatgaa ggatggagtg 240

ccggtgcccc tgatcattgc agccggaggt ggtggcaggg cctacggggc caagacagac 300

acgttccacc cagagagact ggagaataac tcctcggttc tagggctaaa cggcaattcc 360

ggagccgcag gtggtggagg tggctggaat gataacactt ccttgctctg ggccggaaaa 420

tctttgcagg agggtgccac cggaggacat tcctgccccc aggccatgaa gaagtggggg 480

tgggagacaa gagggggttt cggagggggt ggaggggggt gctcctcagg tggaggaggc 540

ggaggatata taggcggcaa tgcagcctca aacaatgacc ccgaaatgga tggggaagat 600

ggggtttcct tcatcagtcc actgggcatc ctgtacaccc cagctttaaa agtgatggaa 660

ggccacgggg aagtgaatat taagcattat ctaaactgca gtcactgtga ggtagacgaa 720

tgtcacatgg accctgaaag ccacaaggtc atctgcttct gtgaccacgg gacggtgctg 780

gctgaggatg gcgtctcctg cattgtgtca cccaccccgg agccacacct gccactctcg 840

ctgatcctct ctgtggtgac ctctgccctc gtggccgccc tggtcctggc tttctccggc 900

atcatgattg tgtaccgccg gaagcaccag gagctgcaag ccatgcagat ggagctgcag 960

agccctgagt acaagctgag caagctccgc acctcgacca tcatgaccga ctacaacccc 1020

aactactgct ttgctggcaa gacctcctcc atcagtgacc tgaaggaggt gccgcggaaa 1080

aacatcaccc tcattcgggg tctgggccat ggcgcctttg gggaggtgta tgaaggccag 1140

gtgtccggaa tgcccaacga cccaagcccc ctgcaagtgg ctgtgaagac gctgcctgaa 1200

gtgtgctctg aacaggacga actggatttc ctcatggaag ccctgatcat cagcaaattc 1260

aaccaccaga acattgttcg ctgcattggg gtgagcctgc aatccctgcc ccggttcatc 1320

ctgctggagc tcatggcggg gggagacctc aagtccttcc tccgagagac ccgccctcgc 1380

ccgagccagc cctcctccct ggccatgctg gaccttctgc acgtggctcg ggacattgcc 1440

tgtggctgtc agtatttgga ggaaaaccac ttcatccacc gagacattgc tgccagaaac 1500

tgcctcttga cctgtccagg ccctggaaga gtggccaaga ttggagactt cgggatggcc 1560

cgagacatct acagggcgag ctactataga aagggaggct gtgccatgct gccagttaag 1620

tggatgcccc cagaggcctt catggaagga atattcactt ctaaaacaga cacatggtcc 1680

tttggagtgc tgctatggga aatcttttct cttggatata tgccataccc cagcaaaagc 1740

aaccaggaag ttctggagtt tgtcaccagt ggaggccgga tggacccacc caagaactgc 1800

cctgggcctg tataccggat aatgactcag tgctggcaac atcagcctga agacaggccc 1860

aactttgcca tcattttgga gaggattgaa tactgcaccc aggacccgga tgtaatcaac 1920

accgctttgc cgatagaata tggtccactt gtggaagagg aagagaaagt gcctgtgagg 1980

cccaaggacc ctgagggggt tcctcctctc ctggtctctc aacaggcaaa acgggaggag 2040

gagcgcagcc cagctgcccc accacctctg cctaccacct cctctggcaa ggctgcaaag 2100

aaacccacag ctgcagagat ctctgttcga gtccctagag ggccggccgt ggaaggggga 2160

cacgtgaata tggcattctc tcagtccaac cctccttcgg agttgcacaa ggtccacgga 2220

tccagaaaca agcccaccag cttgtggaac ccaacgtacg gctcctggtt tacagagaaa 2280

cccaccaaaa agaataatcc tatagcaaag aaggagccac acgacagggg taacctgggg 2340

ctggagggaa gctgtactgt cccacctaac gttgcaactg ggagacttcc gggggcctca 2400

ctgctcctag agccctcttc gctgactgcc aatatgaagg aggtacctct gttcaggcta 2460

cgtcacttcc cttgtgggaa tgtcaattac ggctaccagc aacagggctt gcccttagaa 2520

gccgctactg cccctggagc tggtcattac gaggatacca ttctgaaaag caagaatagc 2580

atgaaccagc ctgggccctg atgagcggcc gc 2612

<210> 179
<211> 4860
<212> DNA

<213>  Artificial

<220>
<223>  pEHX-ALK translation region

<400>  179
atgggagcca tcgggctcct gtggctcctg ccgctgctgc tttccacggc agctgtgggc      60

tccgggatgg ggaccggcca gcgcgcgggc tccccagctg cggggccgcc gctgcagccc     120

cgggagccac tcagctactc gcgcctgcag aggaagagtc tggcagttga cttcgtggtg     180

ccctcgctct tccgtgtcta cgcccgggac ctactgctgc caccatcctc ctcggagctg     240

aaggctggca ggcccgaggc ccgcggctcg ctagctctgg actgcgcccc gctgctcagg     300

ttgctggggc cggcgccggg ggtctcctgg accgccggtt caccagcccc ggcagaggcc     360

cggacgctgt ccagggtgct gaagggcggc tccgtgcgca agctccggcg tgccaagcag     420

ttggtgctgg agctgggcga ggaggcgatc ttggagggtt gcgtcgggcc ccccggggag     480

gcggctgtgg ggctgctcca gttcaatctc agcgagctgt tcagttggtg gattcgccaa     540

ggcgaagggc gactgaggat ccgcctgatg cccgagaaga aggcgtcgga agtgggcaga     600

gagggaaggc tgtccgcggc aattcgcgcc tcccagcccc gccttctctt ccagatcttc     660

gggactggtc atagctcctt ggaatcacca acaaacatgc cttctccttc tcctgattat     720

tttacatgga atctcacctg gataatgaaa gactccttcc ctttcctgtc tcatcgcagc     780

cgatatggtc tggagtgcag ctttgacttc ccctgtgagc tggagtattc ccctccactg     840

catgacctca ggaaccagag ctggtcctgg cgccgcatcc cctccgagga ggcctcccag     900

atggacttgc tggatgggcc tggggcagag cgttctaagg agatgcccag aggctccttt     960

ctccttctca acacctcagc tgactccaag cacaccatcc tgagtccgtg gatgaggagc    1020

agcagtgagc actgcacact ggccgtctcg gtgcacaggc acctgcagcc ctctggaagg    1080

tacattgccc agctgctgcc ccacaacgag gctgcaagag agatcctcct gatgcccact    1140

ccagggaagc atggttggac agtgctccag ggaagaatcg ggcgtccaga caacccattt    1200

cgagtggccc tggaatacat ctccagtgga aaccgcagct tgtctgcagt ggacttcttt    1260

gccctgaaga actgcagtga aggaacatcc ccaggctcca agatggccct gcagagctcc    1320

ttcacttgtt ggaatgggac agtcctccag cttgggcagg cctgtgactt ccaccaggac    1380

tgtgcccagg gagaagatga gagccagatg tgccggaaac tgcctgtggg tttttactgc    1440

aactttgaag atggcttctg tggctggacc caaggcacac tgtcacccca cactcctcaa    1500

tggcaggtca ggaccctaaa ggatgcccgg ttccaggacc accaagacca tgctctattg    1560

ctcagtacca ctgatgtccc cgcttctgaa agtgctacag tgaccagtgc tacgtttcct    1620

gcaccgatca agagctctcc atgtgagctc cgaatgtcct ggctcattcg tggagtcttg    1680

aggggaaacg tgtccttggt gctagtggag aacaaaaccg ggaaggagca aggcaggatg    1740

gtctggcatg tcgccgccta tgaaggcttg agcctgtggc agtggatggt gttgcctctc 1800

ctcgatgtgt ctgacaggtt ctggctgcag atggtcgcat ggtggggaca aggatccaga 1860

gccatcgtgg cttttgacaa tatctccatc agcctggact gctacctcac cattagcgga 1920

gaggacaaga tcctgcagaa tacagcaccc aaatcaagaa acctgtttga gagaaaccca 1980

aacaaggagc tgaaacccgg ggaaaattca ccaagacaga cccccatctt tgaccctaca 2040

gttcattggc tgttcaccac atgtggggcc agcgggcccc atggccccac ccaggcacag 2100

tgcaacaacg cctaccagaa ctccaacctg agcgtggagg tggggagcga gggccccctg 2160

aaaggcatcc agatctggaa ggtgccagcc accgacacct acagcatctc gggctacgga 2220

gctgctggcg ggaaaggcgg gaagaacacc atgatgcggt cccacggcgt gtctgtgctg 2280

ggcatcttca acctggagaa ggatgacatg ctgtacatcc tggttgggca gcagggagag 2340

gacgcctgcc ccagtacaaa ccagttaatc cagaaagtct gcattggaga gaacaatgtg 2400

atagaagaag aaatccgtgt gaacagaagc gtgcatgagt gggcaggagg cggaggagga 2460

gggggtggag ccacctacgt atttaagatg aaggatggag tgccggtgcc cctgatcatt 2520

gcagccggag gtggtggcag ggcctacggg gccaagacag acacgttcca cccagagaga 2580

ctggagaata actcctcggt tctagggcta aacggcaatt ccggagccgc aggtggtgga 2640

ggtggctgga atgataacac ttccttgctc tgggccggaa aatctttgca ggagggtgcc 2700

accggaggac attcctgccc ccaggccatg aagaagtggg ggtgggagac aagagggggt 2760

ttcggagggg gtggaggggg gtgctcctca ggtggaggag gcggaggata tataggcggc 2820

aatgcagcct caaacaatga ccccgaaatg gatggggaag atggggtttc cttcatcagt 2880

ccactgggca tcctgtacac cccagcttta aaagtgatgg aaggccacgg ggaagtgaat 2940

attaagcatt atctaaactg cagtcactgt gaggtagacg aatgtcacat ggaccctgaa 3000

agccacaagg tcatctgctt ctgtgaccac gggacggtgc tggctgagga tggcgtctcc 3060

tgcattgtgt cacccacccc ggagccacac ctgccactct cgctgatcct ctctgtggtg 3120

acctctgccc tcgtggccgc cctggtcctg gctttctccg gcatcatgat tgtgtaccgc 3180

cggaagcacc aggagctgca agccatgcag atggagctgc agagccctga gtacaagctg 3240

agcaagctcc gcacctcgac catcatgacc gactacaacc ccaactactg ctttgctggc 3300

aagacctcct ccatcagtga cctgaaggag gtgccgcgga aaaacatcac cctcattcgg 3360

ggtctgggcc atggcgcctt tggggaggtg tatgaaggcc aggtgtccgg aatgcccaac 3420

gacccaagcc ccctgcaagt ggctgtgaag acgctgcctg aagtgtgctc tgaacaggac 3480

gaactggatt tcctcatgga agccctgatc atcagcaaat tcaaccacca gaacattgtt 3540

cgctgcattg gggtgagcct gcaatccctg ccccggttca tcctgctgga gctcatggcg 3600

gggggagacc tcaagtcctt cctccgagag acccgccctc gcccgagcca gccctcctcc 3660

ctggccatgc tggaccttct gcacgtggct cgggacattg cctgtggctg tcagtatttg 3720

gaggaaaacc acttcatcca ccgagacatt gctgccagaa actgcctctt gacctgtcca 3780

ggccctggaa gagtggccaa gattggagac ttcgggatgg cccgagacat ctacagggcg 3840

agctactata gaaagggagg ctgtgccatg ctgccagtta agtggatgcc cccagaggcc 3900

ttcatggaag gaatattcac ttctaaaaca gacacatggt cctttggagt gctgctatgg 3960

gaaatctttt ctcttggata tatgccatac cccagcaaaa gcaaccagga agttctggag 4020

tttgtcacca gtggaggccg gatggaccca cccaagaact gccctgggcc tgtataccgg 4080

ataatgactc agtgctggca acatcagcct gaagacaggc ccaactttgc catcattttg 4140

gagaggattg aatactgcac ccaggacccg gatgtaatca acaccgcttt gccgatagaa 4200

tatggtccac ttgtggaaga ggaagagaaa gtgcctgtga ggcccaagga ccctgagggg 4260

gttcctcctc tcctggtctc tcaacaggca aaacgggagg aggagcgcag cccagctgcc 4320

ccaccacctc tgcctaccac ctcctctggc aaggctgcaa agaaacccac agctgcagag 4380

atctctgttc gagtccctag agggccggcc gtggaagggg gacacgtgaa tatggcattc 4440

tctcagtcca accctccttc ggagttgcac aaggtccacg gatccagaaa caagcccacc 4500

agcttgtgga acccaacgta cggctcctgg tttacagaga aacccaccaa aaagaataat 4560

cctatagcaa agaaggagcc acacgacagg ggtaacctgg ggctggaggg aagctgtact 4620

gtcccaccta acgttgcaac tgggagactt ccgggggcct cactgctcct agagccctct 4680

tcgctgactg ccaatatgaa ggaggtacct ctgttcaggc tacgtcactt cccttgtggg 4740

aatgtcaatt acggctacca gcaacagggc ttgcccttag aagccgctac tgcccctgga 4800

gctggtcatt acgaggatac cattctgaaa agcaagaata gcatgaacca gcctgggccc 4860

<210> 180
<211> 39
<212> DNA
<213> Artificial

<220>
<223> forward primer

<400> 180
taagcggccg ccaccatggg ctgttggggt cagttgctg 39

<210> 181
<211> 39
<212> DNA
<213> Artificial

<220>
<223> reverse primer

<400>  181
ttagctagct ccatcccttc agggcacaga cactcggcc                              39


<210>  182
<211>  1238
<212>  DNA
<213>  Artificial

<220>
<223>  NotI-Kozak-LTK-NheI

<400>  182
gcggccgcca ccatgggctg ttggggtcag ttgctggtgt ggtttggagc agcaggggca      60

atcctctgca gctctccggg cagccaggaa acgttcctga ggtcctcccc cttgcctttg     120

gccagccctt ccccaaggga tccgaaagtg tcagcgccac cctcgatcct cgagcctgct     180

tcacccctga atagccccgg aactgagggg tcatggctat tctctacttg cggcgcctct     240

ggtcggcatg gccccacaca gactcagtgc gatggtgcct atgcgggtac cagcgtagtg     300

gtgaccgttg gggctgccgg ccagcttcgg ggggtacagc tgtggagagt cccaggacca     360

ggccagtacc tgatatccgc atatggtgca gctggaggca aaggcgcgaa gaaccactta     420

agccgagccc atggcgtgtt cgtctctgcg atcttttccc tcggactcgg ggagtctttg     480

tacattctgg tagggcaaca gggagaggat gcctgtcccg gggggtcacc ggaatcgcag     540

cttgtgtgct taggggaaag cagagccgtg gaagagcacg cagccatgga cggttctgaa     600

ggagtccctg ggagtcgtcg gtgggcaggt ggaggtggag gaggcggagg ggccacctac     660

gtgttccgcg tgagagctgg agaactcgaa ccactcctgg tggctgccgg gggcggaggg     720

agggcatact tacgcccacg cgacagaggc cggacacaag cctcaccaga aaagctcgag     780

aacaggtccg aggcccccgg aagtggcggc agaggaggag ccgcaggcgg tggcggtggg     840

tggacaagtc gagcaccctc ccctcaagct ggaaggagtc ttcaggaagg ggctgagggc     900

ggacagggat gctcagaggc ttgggccacc cttggttggg ctgccgcagg aggctttggg     960

gggggtggcg gggcttgtac tgccggcgga ggtggcggcg ggtatcgagg tggcgacgct    1020

tctgagacgg acaatctgtg ggccgatggc gaggacggcg ttagcttcat tcaccccagt    1080

agcgaactgt ttctgcagcc acttgctgtc acagagaacc atggcgaggt tgagattcgt    1140

cggcacctaa actgctccca ctgtcccctg cgcgattgtc agtggcaagc cgaactgcaa    1200

ctggccgagt gtctgtgccc tgaagggatg gagctagc                            1238


<210>  183
<211>  1384
<212>  DNA
<213>  Artificial

<220>
<223>  NheI-LTK-TAA-XbaI

<400>  183
gctagcggtt gataacgtga catgcatgga tctgcacaaa cctcccgggc ctcttgtgct      60

catggtcgca gttgtcgcta cttccacatt gtccctgctg atggtttgcg gggtcctcat     120

cctggtcaag cagaagaagt ggcagggtct gcaggagatg aggctgccca gccccgaact     180

tgaactgtct aaactgagga ccagtgccat tcggacagca cccaatccct actattgcca     240

ggttggcctc ggccccgctc aaagctggcc actccctcct ggggtaacag aagtgagtcc     300

agccaatgtg accctcctcc gcgcacttgg ccatggagcc tttggtgagg tgtatgaggg     360

gcttgtcatt ggcttgccag gagactcatc tccactgcaa gtggccatca agaccctccc     420

tgagctgtgt agcccgcagg atgaactcga ctttctgatg gaagcgctga tcattagcaa     480

gtttcgccac cagaatatcg tgcgttgtgt ggggctttcc ctgcgcgcta ctcctcggtt     540

gattttgctg gagctcatga gtggcggaga catgaaatcc ttcctgagac attcccgacc     600

gcatctgggg caacctagtc ccctcgtaat gagggatctg ctgcaactcg cacaggacat     660

cgctcagggc tgccactatc tggaagagaa ccacttcatt caccgagaca ttgcggccag     720

aaattgcctg ctgtcttgtg ccgggccttc tagggtggct aaaatcgggg attttggcat      780

ggctcgggac atataccggg cctcatacta ccgcagagga gatcgggccc ttctcccggt     840

gaagtggatg ccacccgaag cctttctgga agggatcttc acctctaaga cggattcctg     900

gagctttggc gtgcttctgt gggagatatt ctccctggga tatatgcctt accctggtag     960

aacgaaccag gaggtgctgg acttcgtcgt aggcggaggc aggatggatc ctcccagggg    1020

atgtcctgga ccagtttacc gaatcatgac tcagtgctgg caacatgagc ccgagcttag    1080

accaagcttc gcatctatac tggaacgcct gcagtattgc acccaggacc cagacgtgtt    1140

gaacagcctg ctgcccatgg aactgggccc cactcccgag gaggagggca caagtggact    1200

cgggaacaga agcctggagt gtttgcggcc accgcaaccc caggaactta gccctgagaa    1260

gctgaaaagc tggggtggct caccattggg tccatggctg tcctcaggtc tgaaaccact    1320

gaaatctcgc ggactgcagc cgcagaactt gtggaatccc acctaccgtt catgataatc    1380

taga                                                                  1384

<210>  184
<211>  35
<212>  DNA
<213>  Artificial

<220>
<223>  forward primer

<400>  184
attgctagcg gttgataacg tgacatgcat ggatc                                 35

<210>  185
<211>  35
<212>  DNA
<213>  Artificial

<220>
<223>  reverse primer

<400>  185
taagcggccg cttatcatga acggtaggtg ggatt                                35


<210>  186
<211>  1386
<212>  DNA
<213>  Artificial

<220>
<223>  NheI-LTK-TGATAA-NotI

<400>  186
gctagcggtt gataacgtga catgcatgga tctgcacaaa cctcccgggc ctcttgtgct      60

catggtcgca gttgtcgcta cttccacatt gtccctgctg atggtttgcg gggtcctcat      120

cctggtcaag cagaagaagt ggcagggtct gcaggagatg aggctgccca gccccgaact     180

tgaactgtct aaactgagga ccagtgccat tcggacagca cccaatccct actattgcca     240

ggttggcctc ggccccgctc aaagctggcc actccctcct ggggtaacag aagtgagtcc     300

agccaatgtg accctcctcc gcgcacttgg ccatggagcc tttggtgagg tgtatgaggg     360

gcttgtcatt ggcttgccag gagactcatc tccactgcaa gtggccatca agaccctccc     420

tgagctgtgt agcccgcagg atgaactcga ctttctgatg gaagcgctga tcattagcaa     480

gtttcgccac cagaatatcg tgcgttgtgt ggggctttcc ctgcgcgcta ctcctcggtt     540

gattttgctg gagctcatga gtggcggaga catgaaatcc ttcctgagac attcccgacc     600

gcatctgggg caacctagtc ccctcgtaat gagggatctg ctgcaactcg cacaggacat     660

cgctcagggc tgccactatc tggaagagaa ccacttcatt caccgagaca ttgcggccag     720

aaattgcctg ctgtcttgtg ccgggccttc tagggtggct aaaatcgggg attttggcat     780

ggctcgggac atataccggg cctcatacta ccgcagagga gatcgggccc ttctcccggt     840

gaagtggatg ccacccgaag cctttctgga agggatcttc acctctaaga cggattcctg     900

gagctttggc gtgcttctgt gggagatatt ctccctggga tatatgcctt accctggtag     960

aacgaaccag gaggtgctgg acttcgtcgt aggcggaggc aggatggatc ctcccagggg    1020

atgtcctgga ccagtttacc gaatcatgac tcagtgctgg caacatgagc ccgagcttag    1080

accaagcttc gcatctatac tggaacgcct gcagtattgc acccaggacc cagacgtgtt    1140

gaacagcctg ctgcccatgg aactgggccc cactcccgag gaggagggca caagtggact    1200

cgggaacaga agcctggagt gtttgcggcc accgcaaccc caggaactta gccctgagaa    1260

gctgaaaagc tggggtggct caccattggg tccatggctg tcctcaggtc tgaaaccact      1320

gaaatctcgc ggactgcagc cgcagaactt gtggaatccc acctaccgtt catgataagc      1380

ggccgc                                                                  1386


<210>  187
<211>  2592
<212>  DNA
<213>  Artificial

<220>
<223>  pEHX-LTK translation region

<400>  187
   atgggctgtt ggggtcagtt gctggtgtgg tttggagcag caggggcaat cctctgcagc        60
   tctccgggca gccaggaaac gttcctgagg tcctccccct tgcctttggc cagcccttcc       120
   ccaagggatc cgaaagtgtc agcgccaccc tcgatcctcg agcctgcttc acccctgaat       180
   agccccggaa ctgaggggtc atggctattc tctacttgcg gcgcctctgg tcggcatggc       240
   cccacacaga ctcagtgcga tggtgcctat gcgggtacca gcgtagtggt gaccgttggg       300
   gctgccggcc agcttcgggg ggtacagctg tggagagtcc caggaccagg ccagtacctg       360
   atatccgcat atggtgcagc tggaggcaaa ggcgcgaaga accacttaag ccgagcccat       420
   ggcgtgttcg tctctgcgat cttttccctc ggactcgggg agtctttgta cattctggta       480
   gggcaacagg gagaggatgc ctgtcccggg gggtcaccgg aatcgcagct tgtgtgctta       540
   ggggaaagca gagccgtgga agagcacgca gccatggacg gttctgaagg agtccctggg       600
   agtcgtcggt gggcaggtgg aggtggagga ggcggagggg ccacctacgt gttccgcgtg       660
   agagctggag aactcgaacc actcctggtg gctgccgggg gcggagggag ggcatactta       720
   cgcccacgcg acagaggccg gacacaagcc tcaccagaaa agctcgagaa caggtccgag       780
   gcccccggaa gtggcggcag aggaggagcc gcaggcggtg gcggtgggtg gacaagtcga       840
   gcaccctccc ctcaagctgg aaggagtctt caggaagggg ctgagggcgg acagggatgc       900
   tcagaggctt gggccaccct tggttgggct gccgcaggag gctttggggg gggtggcggg       960
   gcttgtactg ccggcggagg tggcggcggg tatcgaggtg gcgacgcttc tgagacggac      1020
   aatctgtggg ccgatggcga ggacggcgtt agcttcattc accccagtag cgaactgttt      1080
   ctgcagccac ttgctgtcac agagaaccat ggcgaggttg agattcgtcg gcacctaaac      1140
   tgctcccact gtcccctgcg cgattgtcag tggcaagccg aactgcaact ggccgagtgt      1200
   ctgtgccctg aagggatgga gctagcggtt gataacgtga catgcatgga tctgcacaaa      1260
   cctcccgggc ctcttgtgct catggtcgca gttgtcgcta cttccacatt gtccctgctg      1320
   atggtttgcg gggtcctcat cctggtcaag cagaagaagt ggcagggtct gcaggagatg      1380
   aggctgccca gccccgaact tgaactgtct aaactgagga ccagtgccat tcggacagca      1440
   cccaatccct actattgcca ggttggcctc ggccccgctc aaagctggcc actccctcct 1500
   ggggtaacag aagtgagtcc agccaatgtg accctcctcc gcgcacttgg ccatggagcc 1560
   tttggtgagg tgtatgaggg gcttgtcatt ggcttgccag gagactcatc tccactgcaa 1620
   gtggccatca agaccctccc tgagctgtgt agcccgcagg atgaactcga ctttctgatg 1680
   gaagcgctga tcattagcaa gtttcgccac cagaatatcg tgcgttgtgt ggggctttcc 1740
   ctgcgcgcta ctcctcggtt gattttgctg gagctcatga gtggcggaga catgaaatcc 1800
   ttcctgagac attcccgacc gcatctgggg caacctagtc ccctcgtaat gagggatctg 1860
   ctgcaactcg cacaggacat cgctcagggc tgccactatc tggaagagaa ccacttcatt 1920
   caccgagaca ttgcggccag aaattgcctg ctgtcttgtg ccgggccttc tagggtggct 1980
   aaaatcgggg attttggcat ggctcgggac atataccggg cctcatacta ccgcagagga 2040
   gatcgggccc ttctcccggt gaagtggatg ccacccgaag cctttctgga agggatcttc 2100
   acctctaaga cggattcctg gagctttggc gtgcttctgt gggagatatt ctccctggga 2160
   tatatgcctt accctggtag aacgaaccag gaggtgctgg acttcgtcgt aggcggaggc 2220
   aggatggatc ctcccagggg atgtcctgga ccagtttacc gaatcatgac tcagtgctgg 2280
   caacatgagc ccgagcttag accaagcttc gcatctatac tggaacgcct gcagtattgc 2340
   acccaggacc cagacgtgtt gaacagcctg ctgcccatgg aactgggccc cactcccgag 2400
   gaggagggca caagtggact cgggaacaga agcctggagt gtttgcggcc accgcaaccc 2460
   caggaactta gccctgagaa gctgaaaagc tggggtggct caccattggg tccatggctg 2520
   tcctcaggtc tgaaaccact gaaatctcgc ggactgcagc cgcagaactt gtggaatccc 2580
   acctaccgtt ca 2592

<210> 188
<211> 23
<212> DNA
<213> Artificial

<220>
<223> forward primer for ALK amplification

<400> 188
ccatcgtggc ttttgacaat atc 23

<210> 189
<211> 23
<212> DNA
<213> Artificial

<220>
<223> reverse primer for ALK amplification

<400> 189

ggtgctgtat tctgcaggat ctt 23

<210> 190
<211> 20
<212> DNA
<213> Artificial

<220>
<223> forward primer for LTK amplification

<400> 190
gcggagggag ggcatactta 20

<210> 191
<211> 20
<212> DNA
<213> Artificial

<220>
<223> reverse primer for LTK amplification

<400> 191
tcggacctgt tctcgagctt 20

<210> 192
<211> 930
<212> DNA
<213> Artificial

<220>
<223> CAR 021 translation region

<400> 192
atggagtttg ggctttcctg gttgttcctc gtagccatcc tcaaaggggt tcagtgcgca 60

gaagcggctg gtctgggtcc ctcaccagaa cagcgagtcg agattgtgcc tcgcgatctc 120

cgcatgaagg acaagttcct gaagcatctg acaggcccgc tgtacttcag tcccaagtgt 180

agcaaacact ttcatcgcct gtatcacaac acacgggatt gcaccatacc cgcctattac 240

aagaggtgtg caaggctgct gactcgattg gccgtttctc ctgtgtgcat ggaggacaaa 300

caggagccca agagctgcga caagacgcac acctgtccac cgtgtgatcc cgccgagccc 360

aaatctcctg acaaaactca cacatgccca ttttgggtgc tggtggtggt tggtggagtc 420

ctggcttgct atagcttgct agtaacagtg gcctttatta ttttctgggt gaggagtaag 480

aggagcaggc tcctgcacag tgactacatg aacatgactc cccgccgccc cgggcccacc 540

cgcaagcatt accagcccta tgccccacca cgcgacttcg cagcctatcg ctccagagtg 600

aagttcagca ggagcgcaga cgcccccgcg taccagcagg gccagaacca gctctataac 660

gagctcaatc taggacgaag agaggagtac gatgttttgg acaagagacg tggccgggac 720

cctgagatgg ggggaaagcc gagaaggaag aaccctcagg aaggcctgta caatgaactg 780

cagaaagata agatggcgga ggcctacagt gagattggga tgaaaggcga gcgccggagg 840

ggcaaggggc acgatggcct ttaccagggt ctcagtacag ccaccaagga cacctacgac 900

gcccttcaca tgcaggccct gccccctcgc 930

<210> 193
<211> 1437
<212> DNA
<213> Artificial

<220>
<223> CAR 022 translation region

<400> 193
atggtcctgc tcgtcacgtc actgcttctg tgcgagttgc ctcatcctgc cttcctgctg 60

attcccgata cacaggtgca acttcagcag tcaggcgcag aactggtcaa accaggtgct 120

agcgtgaaga tctcctgcaa ggctagtggg tacgcgtttt cctcctactg gatgaattgg 180

gtgaagcaac gaccaggcaa ggggttggag tggataggtc agatttatcc aggcgatggg 240

gatacaacat acaatgggaa gttcaaggga aaggccacac ttactgccga caaatcctct 300

agcaccgtgt acatgcagct gaactccctg acgtccgaag atagcgccgt ttacttctgc 360

gtgagatact actacggcag ctcagggtat ttcgactatt ggggtcaagg aaccactttg 420

accgtatctt ctggcggagg cggcagtggt ggaggtggct caggaggcgg agggtcagac 480

gttcagatga tacagacacc tgactctctg gccgtttccc tcgggcagag agccaccatc 540

agttgtcgcg cttctgagtc tgtggataac tatgggatca gcttcatgca ctggtatcag 600

cagaaacctg gccaaagtcc caaactcctc atctatcggg ctagcaatct ggaaagcggc 660

attccggcac gttttagtgg cagcggaagc aggaccgact ttaccctgac cattaatccg 720

gtggaaactg acgatgtagc aacctactat tgccagcaga acaacaagga tccacccact 780

tttggaggtg gaacaaagct ggagatcaaa gagcccaaaa gctgtgacaa aacgcacact 840

tgtccaccgt gtgatcccgc cgagcccaaa tctcctgaca aaactcacac atgcccattt 900

tgggtgctgg tggtggttgg tggagtcctg gcttgctata gcttgctagt aacagtggcc 960

tttattattt tctgggtgag gagtaagagg agcaggctcc tgcacagtga ctacatgaac 1020

atgactcccc gccgccccgg gcccacccgc aagcattacc agccctatgc cccaccacgc 1080

gacttcgcag cctatcgctc cagagtgaag ttcagcagga gcgcagacgc ccccgcgtac 1140

cagcagggcc agaaccagct ctataacgag ctcaatctag gacgaagaga ggagtacgat 1200

gttttggaca agagacgtgg ccgggaccct gagatggggg gaaagccgag aaggaagaac 1260

cctcaggaag gcctgtacaa tgaactgcag aaagataaga tggcggaggc ctacagtgag 1320

attgggatga aaggcgagcg ccggaggggc aaggggcacg atggccttta ccagggtctc 1380

agtacagcca ccaaggacac ctacgacgcc cttcacatgc aggccctgcc ccctcgc 1437

<210>  194
<211>  310
<212>  PRT
<213>  Artificial

<220>
<223>  CAR 021 translation region

<400>  194

Met Glu Phe Gly Leu Ser Trp Leu Phe Leu Val Ala Ile Leu Lys Gly
1               5                   10                  15

Val Gln Cys Ala Glu Ala Ala Gly Leu Gly Pro Ser Pro Glu Gln Arg
            20                  25                  30

Val Glu Ile Val Pro Arg Asp Leu Arg Met Lys Asp Lys Phe Leu Lys
        35                  40                  45

His Leu Thr Gly Pro Leu Tyr Phe Ser Pro Lys Cys Ser Lys His Phe
    50                  55                  60

His Arg Leu Tyr His Asn Thr Arg Asp Cys Thr Ile Pro Ala Tyr Tyr
65                  70                  75                  80

Lys Arg Cys Ala Arg Leu Leu Thr Arg Leu Ala Val Ser Pro Val Cys
                85                  90                  95

Met Glu Asp Lys Gln Glu Pro Lys Ser Cys Asp Lys Thr His Thr Cys
            100                 105                 110

Pro Pro Cys Asp Pro Ala Glu Pro Lys Ser Pro Asp Lys Thr His Thr
        115                 120                 125

Cys Pro Phe Trp Val Leu Val Val Val Gly Gly Val Leu Ala Cys Tyr
    130                 135                 140

Ser Leu Leu Val Thr Val Ala Phe Ile Ile Phe Trp Val Arg Ser Lys
145                 150                 155                 160

Arg Ser Arg Leu Leu His Ser Asp Tyr Met Asn Met Thr Pro Arg Arg
                165                 170                 175

Pro Gly Pro Thr Arg Lys His Tyr Gln Pro Tyr Ala Pro Pro Arg Asp
            180                 185                 190

Phe Ala Ala Tyr Arg Ser Arg Val Lys Phe Ser Arg Ser Ala Asp Ala
        195                 200                 205

Pro Ala Tyr Gln Gln Gly Gln Asn Gln Leu Tyr Asn Glu Leu Asn Leu
    210                 215                 220

Gly Arg Arg Glu Glu Tyr Asp Val Leu Asp Lys Arg Arg Gly Arg Asp
225                 230                 235                 240

Pro Glu Met Gly Gly Lys Pro Arg Arg Lys Asn Pro Gln Glu Gly Leu
                245                 250                 255

Tyr Asn Glu Leu Gln Lys Asp Lys Met Ala Glu Ala Tyr Ser Glu Ile
            260                 265                 270

Gly Met Lys Gly Glu Arg Arg Arg Gly Lys Gly His Asp Gly Leu Tyr
        275                 280                 285

Gln Gly Leu Ser Thr Ala Thr Lys Asp Thr Tyr Asp Ala Leu His Met
    290                 295                 300

Gln Ala Leu Pro Pro Arg
305                 310

<210>  195
<211>  479
<212>  PRT
<213>  Artificial

<220>
<223>  CAR 022 translation region

<400>  195

Met Val Leu Leu Val Thr Ser Leu Leu Leu Cys Glu Leu Pro His Pro
1               5                   10                  15

Ala Phe Leu Leu Ile Pro Asp Thr Gln Val Gln Leu Gln Gln Ser Gly
            20                  25                  30

Ala Glu Leu Val Lys Pro Gly Ala Ser Val Lys Ile Ser Cys Lys Ala
        35                  40                  45

Ser Gly Tyr Ala Phe Ser Ser Tyr Trp Met Asn Trp Val Lys Gln Arg
    50                  55                  60

Pro Gly Lys Gly Leu Glu Trp Ile Gly Gln Ile Tyr Pro Gly Asp Gly
65                  70                  75                  80

Asp Thr Thr Tyr Asn Gly Lys Phe Lys Gly Lys Ala Thr Leu Thr Ala
                85                  90                  95

Asp Lys Ser Ser Ser Thr Val Tyr Met Gln Leu Asn Ser Leu Thr Ser

            100                 105                 110

Glu Asp Ser Ala Val Tyr Phe Cys Val Arg Tyr Tyr Tyr Gly Ser Ser
        115                 120                 125

Gly Tyr Phe Asp Tyr Trp Gly Gln Gly Thr Thr Leu Thr Val Ser Ser
    130                 135                 140

Gly Gly Gly Gly Ser Gly Gly Gly Gly Ser Gly Gly Gly Gly Ser Asp
145                 150                 155                 160

Val Gln Met Ile Gln Thr Pro Asp Ser Leu Ala Val Ser Leu Gly Gln
                165                 170                 175

Arg Ala Thr Ile Ser Cys Arg Ala Ser Glu Ser Val Asp Asn Tyr Gly
            180                 185                 190

Ile Ser Phe Met His Trp Tyr Gln Gln Lys Pro Gly Gln Ser Pro Lys
        195                 200                 205

Leu Leu Ile Tyr Arg Ala Ser Asn Leu Glu Ser Gly Ile Pro Ala Arg
    210                 215                 220

Phe Ser Gly Ser Gly Ser Arg Thr Asp Phe Thr Leu Thr Ile Asn Pro
225                 230                 235                 240

Val Glu Thr Asp Asp Val Ala Thr Tyr Tyr Cys Gln Gln Asn Asn Lys
                245                 250                 255

Asp Pro Pro Thr Phe Gly Gly Gly Thr Lys Leu Glu Ile Lys Glu Pro
            260                 265                 270

Lys Ser Cys Asp Lys Thr His Thr Cys Pro Pro Cys Asp Pro Ala Glu
        275                 280                 285

Pro Lys Ser Pro Asp Lys Thr His Thr Cys Pro Phe Trp Val Leu Val
    290                 295                 300

Val Val Gly Gly Val Leu Ala Cys Tyr Ser Leu Leu Val Thr Val Ala
305                 310                 315                 320

Phe Ile Ile Phe Trp Val Arg Ser Lys Arg Ser Arg Leu Leu His Ser
                325                 330                 335

Asp Tyr Met Asn Met Thr Pro Arg Arg Pro Gly Pro Thr Arg Lys His
            340                 345                 350

Tyr Gln Pro Tyr Ala Pro Pro Arg Asp Phe Ala Ala Tyr Arg Ser Arg
        355                 360                 365

Val Lys Phe Ser Arg Ser Ala Asp Ala Pro Ala Tyr Gln Gln Gly Gln
    370                 375                 380

Asn Gln Leu Tyr Asn Glu Leu Asn Leu Gly Arg Arg Glu Glu Tyr Asp
385                 390                 395                 400

Val Leu Asp Lys Arg Arg Gly Arg Asp Pro Glu Met Gly Gly Lys Pro
                405                 410                 415

Arg Arg Lys Asn Pro Gln Glu Gly Leu Tyr Asn Glu Leu Gln Lys Asp
            420                 425                 430

Lys Met Ala Glu Ala Tyr Ser Glu Ile Gly Met Lys Gly Glu Arg Arg
        435                 440                 445

Arg Gly Lys Gly His Asp Gly Leu Tyr Gln Gly Leu Ser Thr Ala Thr
    450                 455                 460

Lys Asp Thr Tyr Asp Ala Leu His Met Gln Ala Leu Pro Pro Arg
465                 470                 475

## Description

### Technical Field

The present invention relates to a genetically modified cell expressing a chimeric antigen receptor, which is useful in the field of adoptive immunotherapy, and a method for producing the same.

### Background Art

Adoptive immunotherapy using T cells expressing a chimeric antigen receptor (CAR) (CAR-T) targeting a tumor-related antigen has been reported to have a potent antitumor effect, and its development has advanced rapidly in recent years. Particularly, the development of CARs aimed at the treatment of B cell tumor has advanced and already reached clinical application. In the field of solid tumors, however, the development of CARs is still in progress and clinical application thereof is not yet realized.

The correlation between anaplastic lymphoma kinase (ALK), which is a receptor tyrosine kinase expressed at a high level in tumor tissue of neuroblastoma, and solid tumor has been reported for a long time (Non-Patent Literature 1). In large-scale cohort studies conducted in Japan, it has been reported that, in addition to abnormality of ALK gene, high ALK scores (quantification of pathological ALK expression) would affect the prognosis of neuroblastoma (Non-Patent Literature 2). Thus, development of a novel therapeutic agent targeting ALK is expected for the treatment of solid tumors including neuroblastoma.

In the past, ALK inhibitory agents using small molecule compounds had been developed, and therapeutic agents for ALK fusion-positive lung cancer have been applied in clinical settings (Non-Patent Literature 3). However, ALK that is expressed at a high level in the case of neuroblastoma is not of a gene fusion type, but is reported to be a wild-type or point mutation type having an extracellular ligand-binding domain, and small molecule inhibitory agents are considered ineffective. Accordingly, development of novel therapeutic agents has been desired.

As CAR-T therapeutic techniques targeting ALK, research on scFv-type CAR-T using, as an antigen binding domain, a single-chain antibody fragment (scFv) derived from an antibody against ALK has been reported (Patent Literature 1, Non-Patent Literature 4).

Meanwhile, ALK is a receptor tyrosine kinase (RTKs) as with the leukocyte tyrosine kinase (LTK), and it was known as an orphan receptor whose physiological ligand has not yet been found (Non-Patent Literature 5). In recent years, however, low-molecular-weight proteins, FAM150A (11.5 kDa) and FAM150B (14.5 kDa), were found to functionally bind to ALK and LTK (Non-Patent Literatures 6 and 7). Further, a fragment of the FAM150B referred to as an "AD domain (N terminal defect)" is reported to exert the binding ability and the phosphorylation ability equivalent to those exerted by the full-length sequence on ALK (Non-Patent Literature 8).

### Citation List

### Patent Literatures

Patent Literature 1: WO 2015/069922

### Non-Patent Literatures

Non-Patent Literature 1: Nat. Rev. Cancer, 2013; 13: 685-700
Non-Patent Literature 2: Oncotarget, 2017, vol. 8, No. 64, pp. 107513-107529
Non-Patent Literature 3: Cold Spring Harb. Mol. Case Stud., 2017; 3: a001115
Non-Patent Literature 4: Molecular Therapy, 2017, vol. 25, 9, 2189-2201
Non-Patent Literature 5: PNAS, 2014, vol. 111, 44, 15741-15745
Non-Patent Literature 6: eLife, 2015, 4, e09811
Non-Patent Literature 7: PNAS, 2015, vol. 112, 52, 15862-15867
Non-Patent Literature 8: PNAS, 2018, vol. 115, 33, 8340-8345

### Summary of Invention

### Technical Problem

Antitumor activity of scFv-type CAR-T is reported to be limited both *in vitro* and *in vivo.* A problem as a CAR-T therapeutic agent is also reported: antitumor activity of scFv-type CAR-T depends on the ALK expression level in a target tumor, and activity thereof on a tumor expressing low levels of ALK cannot be expected. Under the above circumstances, further development of CARs that can be effective on ALK-expressing solid tumors is desired. Solutions

The present inventors have conducted concentrated studies in consideration of applicability of ligand-type CAR using, as an antigen binding domain, FAM150A and FAM150B, which may be physiological ligands of ALK (hereinafter, referred to as "ALK.CAR"), as an adoptive immunotherapeutic agent against solid tumors. In addition, they have focused on regions highly homologous in FAM150A and FAM150B, and examined ALK.CAR using truncated fragments lacking the N terminus and/or the C terminus as an antigen binding domain (hereinafter, referred to as "TrALK.CAR").

As a result, they discovered that T cells into which ALK.CAR and TrALK.CAR had been introduced (hereinafter, referred to as "ALK.CAR-T" and "TrALK.CAR-T," respectively) exerts potent antitumor activity against ALK-expressing tumor cells, which has led to the completion of the present invention.

Specifically, the present invention provides the followings:
[1] A polynucleotide encoding a chimeric antigen receptor (CAR) protein comprising a target binding domain that binds to an extracellular ligand binding region of anaplastic lymphoma kinase (ALK), a transmembrane domain, and an intracellular signaling domain, wherein the target binding domain is selected from among FAM150A, FAM150B, and fragments thereof binding to the extracellular ligand binding region of ALK.
[2] The polynucleotide according to [1], wherein the target binding domain is a truncated fragment of FAM150A and/or FAM150B.
[3] The polynucleotide according to [1], wherein the target binding domain is a polypeptide consising of an amino acid sequence selected from the group consisting of SEQ ID NO: 154 (FAM150A), SEQ ID NO: 146 (TrFAM150A), SEQ ID NO: 155 (FAM150B), and SEQ ID NO: 148 (TrFAM150B).
[4] The polynucleotide according to [1], wherein the target binding domain is a polypeptide consisting of an amino acid sequence having 90% or higher sequence identity to the amino acid sequence represented by SEQ ID NO: 146 (TrFAM150A) or SEQ ID NO: 148 (TrFAM150B).
[5] A vector comprising the polynucleotide according to any of [1] to [4].
[6] A genetically modified cell comprising the polynucleotide according to any of [1] to [4] or the vector according to [5] introduced thereinto.
[7] The cell according to [6], which expresses a CAR protein binding to an ALK-expressing cell on a cell membrane.
[8] A method for preparing a CAR protein-expressing cell comprising introducing the polynucleotide according to any of [1] to [4] or the vector according to [5] into a cell.
[9] The method according to [8], wherein the polynucleotide or the vector is introduced into the cell by the transposon method.
[10] The method according to [9], wherein the transposon method is the piggyBac method.
[11] A kit comprising the vector according to [5] used for preparing a CAR protein-expressing cell targeting an ALK-expressing cell.
[12] A therapeutic agent for a disease associated with an ALK-expressing cell, comprising the cell according to [6] or [7].
[13] A pharmaceutical composition comprising the therapeutic agent according to [12] and a pharmaceutically acceptable carrier.
[14] The therapeutic agent according to [12] or the composition according to [13], wherein the disease associated with an ALK-expressing cell is a solid tumor selected from among neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer.
[15] A method for treatment of a solid tumor selected from among neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer, comprising administering a therapeutically effective amount of the therapeutic agent according to [12] or the composition according to [13] to a patient.

The present description encompasses the contents disclosed in Japanese Patent Application No. 2019-103074 on which the priority of the present application is based.

### Advantageous Effects of Invention

The present invention provides a genetically modified cell that binds to a target cell expressing ALK on a cell surface and exerts antitumor effects. Thus, the cell according to the present invention can be used as an adoptive immunotherapeutic agent for solid tumor diseases including neuroblastoma.

### Brief Description of Drawings

Fig. 1 shows a vector map of CAR001.
Fig. 2 shows a vector map of CAR 002 (FAM150A-28z).
Fig. 3 shows a vector map of CAR 003 (FAM150B-28z).
Fig. 4 shows a vector map of CAR 004 (hALK48-28z).
Fig. 5 shows a vector map of CAR 005 (ALK48-28z).
Fig. 6 shows antitumor activity of CAR-T 002 to CAR-T 005 or mock-T cells (Effector, E) obtained from Donor-1 against neuroblastoma cell line SH-SY5Y (Target, T). The tumor cell proliferation rates (%) are calculated relative to tumor cell proliferation rate (100%) in the group to which no CAR-T was administered (i.e., the CAR-T non-administered group; No CAR). A: E:T = 4:1; B: E:T = 2:1; C: E:T=1:1. The effects of CAR-T 002 to CAR-T 005 to kill SH-SY5Y were confirmed, and the effects of CAR-T 002 and CAR-T 003 were higher than those of CAR-T 004 and CAR-T 005.
Fig. 7 shows antitumor activity of CAR-T 002 to CAR-T 006 or mock-T cells (E) obtained from Donor-2 against neuroblastoma cell line SH-SY5Y (T). The tumor cell proliferation rates (%) are calculated relative to tumor cell proliferation rate (100%) in the CAR-T non-administered group (No CAR). A: E:T = 4:1; B: E:T = 2:1; C: E:T=1:1. The effects of CAR-T 002 to CAR-T 005 to kill SH-SY5Y were confirmed, and the effects of CAR-T 002 and CAR-T 003 were higher than those of CAR-T 004 and CAR-T 005.
Fig. 8 shows antitumor activity of CAR-T 002 to CAR-T 005 or mock-T cells (E) obtained from Donor-1 against neuroblastoma cell line NB-1 (T). A: E:T = 4:1; B: E:T = 2:1; C: E:T=1:1. The effects of CAR-T 002 to CAR-T 005 to kill NB-1 were confirmed, and the effects of CAR-T 002 and CAR-T 003 were higher than those of CAR-T 004 and CAR-T 005.
Fig. 9 shows antitumor activity of CAR-T 002 to CAR-T 006 or mock-T cells (E) obtained from Donor-2 against neuroblastoma cell line NB-1 (T). A: E:T = 4:1; B: E:T = 2:1; C: E:T=1:1). The effects of CAR-T 002 to CAR-T 005 to kill NB-1 were confirmed, and the effects of CAR-T 002 and CAR-T 003 were higher than those of CAR-T 004 and CAR-T 005.
Fig. 10 shows antitumor activity of CAR-T 002 to CAR-T 006 or mock-T cells (E) obtained from Donor-2 against neuroblastoma cell line IMR-32 (T). A: E:T = 4:1; B: E:T = 2:1; C: E:T = 1:1. The effects of CAR-T 002 to CAR-T 005 to kill IMR-32 were confirmed, and the effects of CAR-T 003 were particularly high.
Fig. 11 shows a domain map of the protein used in Example 4.
Fig. 12 shows the results of verification of binding of ALK 001P and LTK 001P to Ligand 001P and Ligand 002P via surface plasmon resonance analysis. Ligand 001P shows a higher binding ability to LTK 001P (B) than to ALK 001P (A). Ligand 002P is slowly detached from ALK 001P (C) and LTK 001P (D), which shows a high binding ability thereto.
Fig. 13 shows a vector map of CAR 007 (FAM150ATr-28z).
Fig. 14 shows a vector map of CAR 008 (FAM150BTr-28z).
Fig. 15 shows antitumor activity of CAR-T 003, CAR-T 008, and CAR-T 006 or mock-T cells (E) obtained from Donor-1 against SH-SY5Y (T). A: E:T = 4:1; B: E:T = 2:1; C: E:T=1:1. The effects of CAR-T 003 and CAR-T 008 to kill SH-SY5Y were confirmed. In particular, the effects of CAR-T 008 to kill SH-SY5Y were higher than those of CAR-T 003, and also high at E:T = 1:1.
Fig. 16 shows antitumor activity of CAR-T 003, CAR-T 008, and CAR-T 006 or mock-T cells (E) obtained from Donor-1 against IMR-32 (T). A: E:T = 4:1; B: E:T = 2:1; C: E:T = 1:1. The effects of CAR-T 003 and CAR-T 008 to kill IMR-32 were confirmed. In particular, the effects of CAR-T 008 to kill IMR-32 were higher than those of CAR-T 003, and also high at E:T = 1:1.
Fig. 17 shows antitumor activity of CAR-T 003, CAR-T 008, and CAR-T 006 or mock-T cells (E) obtained from Donor-2 against SH-SY5Y (T) (A: E:T = 4:1; B: E:T = 2:1; C: E:T = 1:1), and the proliferation curves plotting the relative tumor cell counts (D: E:T = 4:1; E: E:T = 2:1; F: E:T = 1:1). The effects of CAR-T 003 and CAR-T 008 to kill SH-SY5Y were confirmed. In particular, the effects of CAR-T 008 to kill SH-SY5Y were higher than those of CAR-T 003, and also high at E:T = 1:1.
Fig. 18 shows antitumor activity of CAR-T 003, CAR-T 008, and CAR-T 006 or mock-T cells (E) obtained from Donor-2 against IMR-32 (T) (A: E:T = 4:1; B: E:T = 2:1; C: E:T = 1:1), and the tumor cell proliferation curves (D: E:T = 4:1; E: E:T = 2:1; F: E:T = 1:1). The effects of CAR-T 003 and CAR-T 008 to kill IMR-32 were confirmed. In particular, the effects of CAR-T 008 were higher than those of CAR-T 003, and also high at E:T = 1:1.
Fig. 19 shows a domain map of the protein used in Examples 9 and 10.
Fig. 20-1 shows the results of verification of binding of ALK 001P and LTK 001P to Ligand 005P and Ligand 006P via surface plasmon resonance analysis.
Fig. 20-2 shows the results of verification of binding of ALK 001P and LTK 001P to Ligand 003P and Ligand 007P via surface plasmon resonance analysis.
Fig. 20-3 shows the results of verification of binding of ALK 001P and LTK 001P to Ligand 008P and Ligand 009P via surface plasmon resonance analysis.
Fig. 20-4 shows the results of verification of binding of ALK 001P and LTK 001P to Ligand 010P and Ligand 011P via surface plasmon resonance analysis.
Fig. 21-1 shows the results of verification of binding of ALK 001P and LTK 001P to Ligand 012P and Ligand 013P via surface plasmon resonance analysis.
Fig. 21-2 shows the results of verification of binding of ALK 001P and LTK 001P to Ligand 014P and Ligand 015P via surface plasmon resonance analysis.
Fig. 21-3 shows the results of verification of binding of ALK 001P and LTK 001P to Ligand 016P and Ligand 017P via surface plasmon resonance analysis.
Fig. 21-4 shows the results of verification of binding of ALK 001P and LTK 001P to Ligand 018P and Ligand 019P via surface plasmon resonance analysis.
Fig. 22-1 shows the results of verification of binding of ALK 001P and LTK 001P to Ligand 020P and Ligand 021P via surface plasmon resonance analysis.
Fig. 22-2 shows the results of verification of binding of ALK 001P and LTK 001P to Ligand 022P and Ligand 023P via surface plasmon resonance analysis.
Fig. 22-3 shows the results of verification of binding of ALK 001P and LTK 001P to Ligand 024P via surface plasmon resonance analysis.
Fig. 23 shows a vector map of CAR 009 (FAM150A-8αBBz).
Fig. 24 shows a vector map of CAR 010 (FAM150B-8αBBz).
Fig. 25 shows a vector map of CAR 011 (FAM150ATr-8αBBz).
Fig. 26 shows a vector map of CAR 012 (FAM150BTr-8αBBz).
Fig. 27 shows a vector map of CAR 013 (hALK48-8αBBz).
Fig. 28 shows a vector map of CAR 014 (ALK48-8αBBz).
Fig. 29 shows antitumor activity of CAR-T 009 to CAR-T 014 or mock-T cells (E) obtained from Donor-1 against SH-SY5Y (T) (A: E:T = 4:1; B: E:T = 2:1; C: E:T = 1:1), and the tumor cell proliferation curves (D: E:T = 4:1; E: E:T = 2:1; F: E:T = 1:1).
Fig. 30 shows antitumor activity of CAR-T 009 to CAR-T 014 or mock-T cells (E) obtained from Donor-1 against MDA-MB231 ffLuc (T) (A: E:T = 4:1; B :E:T = 2:1; C :E:T = 1:1), and the tumor cell proliferation curves (D: E:T = 4:1; E: E:T = 2:1; F: E:T = 1:1).
Fig. 31 shows a vector map of CAR 015 (FAM150BT14-28z).
Fig. 32 shows a vector map of CAR 016 (FAM150BT15-28z).
Fig. 33 shows a vector map of CAR 017 (FAM150BT17-28z).
Fig. 34 shows a vector map of CAR 018 (FAM150BT18-28z).
Fig. 35 shows a vector map of CAR 019 (FAM150BT19-28z).
Fig. 36 shows antitumor activity of CAR-T 015, CAR-T 016, CAR-T 017, CAR-T 018, CAR-T 019, CAR-T 008, and CAR-T 006 or mock-T cells (E) obtained from Donor-1 against SH-SY5Y (T), NB-1 (T), IMR32 (T), and MDA-MB231 ffLuc(T) (E:T = 1:1).
Fig. 37 shows a vector map of CAR 020 (FAM150BTr-BBz dCH2CH3).
Fig. 38 shows antitumor activity of CAR-T 006, CAR-T 012, and CAR-T 020 or mock-T cells (E) obtained from Donor-1 against SH-SY5Y (T), NB-1 (T), IMR32 (T), and MDA-MB231 ffLuc (T) (E:T = 1:1).
Fig. 39 shows a vector map of pEHX-ALK.
Fig. 40 shows a vector map of pEHX-LTK.
Fig. 41 shows the results of flow cytometric analysis of ALK-expressing clones and LTK-expressing clones.
Fig. 42 shows a vector map of CAR 021 (FAM150BTr-28z dCH2CH3).
Fig. 43 shows a vector map of CAR 022 (ALK48 scFv-28z dCH2CH3).
Fig. 44 shows transitions in the cell index over time when CAR-T 021, CAR-T 022, or mock-T cells are added to A24 cells highly expressing ALK (A: E:T = 40:1; B: E:T = 20:1; C: E:T = 10:1). "A24" indicates the results when cultured in the absence of effector cells.
Fig. 45 shows transitions in the cell index over time when CAR-T 021, CAR-T 022, or mock-T cells are added to L10 cells highly expressing LTK (A: E:T = 40:1; B: E:T = 20:1; C: E:T = 10:1). "L10" indicates the results when cultured in the absence of effector cells.

### Embodiments of the Invention

The embodiments of the present invention are described in detail below.

As described above, the present invention provides a polynucleotide encoding a chimeric antigen receptor (CAR) protein comprising a target binding domain that binds to an extracellular ligand binding region of anaplastic lymphoma kinase (ALK), a transmembrane domain, and an intracellular signaling domain, wherein the target binding domain is selected from among FAM150A, FAM150B, and fragments thereof binding to the extracellular ligand binding region of ALK.

In the case where a single antigen is targeted in immunotherapy, it is preferable that CAR be capable of recognizing a plurality of antigens, from the viewpoint of, for example, reduction of a risk of cancer recurrence caused by the growth of an antigen quenching escape mutant that can be generated during the process of various therapies. Accordingly, an aspect of the present invention provides a bispecific CAR. For example, an embodiment of the present invention provides a polynucleotide encoding a CAR protein having a target binding domain that binds to extracellular ligand binding regions of ALK and LTK.

The term "anaplastic lymphoma kinase (ALK)" used herein refers to a receptor tyrosine kinase that belongs to the insulin receptor superfamily identified in 1994 as a fusion molecule with nucleophosmin in case of anaplastic large cell lymphoma. ALK is a cell membrane-binding protein that is known to be expressed on the cell surface of solid tumor such as neuroblastoma, breast cancer, or lung cancer. The extracellular domain of ALK functions as a receptor including a region that is rich in MAM and glycine, and the intracellular kinase domain is associated with signal transduction after a ligand binds to the receptor. When a ligand binds to the receptor and ALK is then activated, tumor cells are proliferated by the action of the kinase.

Examples of ALK-expressing tumors include solid tumors, such as neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer. High-level expression of ALK in such tumor cells is reported. In addition, canceration caused by mutation in the kinase domain is known. It is also known that, when ALK forms a fusion protein with another gene in a cell, ALK is constantly activated to develop a tumor. There is no extracellular ligand-binding region in a fusion protein, and a fusion protein is not included in the target in the present invention, Accordingly, the term "ALK-expressing cell" used herein refers to, in particular, a cell that expresses ALK having an extracellular ligand-binding region.

The amino acid sequence and the nucleotide sequence of ALK protein are described in, for example, the NCBI database under Accession NOs. NM_004304.4 and NM_004304.5.

Leukocyte tyrosine kinase (LTK) is a receptor tyrosine kinase, a partial structure thereof was identified in 1988 and a full-length thereof was reported to be a 100 kDa glycosylated protein in 1991. LTK is known to be expressed in B-lymphocyte precursors, B lymphocytes, hematopoietic stem cells, the brain, the placenta, and various cancer cells. The amino acid sequence and the nucleotide sequence of LTK protein are described in, for example, the NCBI database under Accession NO. NM_002344.5.

The "chimeric antigen receptor (CAR)" used herein refers to a modified receptor that can impart its target specificity to cells such as T cells (e.g., naive T cells, stem cell memory T cells, central memory T cells, effector memory T cells, or a combination thereof). CAR is also known as an artificial T cell receptor, a chimeric T cell receptor, or a chimeric immunoreceptor.

The CAR for use in the method of the present invention has a target binding domain that binds to an extracellular ligand binding region of ALK, a transmembrane domain, and an intracellular signaling domain. The term "domain" used herein refers to a region within a polypeptide and folded into a particular structure independently of other regions.

The term "polynucleotide" used herein encompasses, but is not limited to, natural or synthetic DNA and RNA, for example, genomic DNA, cDNA (complementary DNA), mRNA (messenger RNA), rRNA (ribosomal RNA), shRNA (small hairpin RNA), snRNA (small nuclear RNA), snoRNA (small nucleolar RNA), miRNA (microRNA), and/or tRNA.

The term "encoding" used herein means that a predetermined nucleotide sequence has a code for information on the amino acid sequence of a predetermined protein or (poly)peptide, as usually used in the art. In the present description, both sense and antisense strands are used in the context of "encoding."

The CAR protein according to the present invention comprises a "target binding domain" that binds to an extracellular ligand binding region of ALK. The target binding domain is capable of binding to the extracellular ligand binding region of ALK, and it enables immune responses to the target cell expressing ALK on its cell surface.

As the target binding domain, accordingly, ligands for ALK; i.e., FAM150A, FAM150B, and fragments thereof binding to an extracellular ligand binding region of ALK, can be used. In a preferable embodiment of fragments, truncated fragments of FAM150A and/or FAM150B can be used.

The sequence information of FAM150A can be obtained as NCBI Accession Number: NM_207413.4, and FAM150A can be prepared on the basis of the nucleotide sequence represented by SEQ ID NO: 1, which encodes the translation region thereof. The sequence information of FAM150B can be obtained as NCBI Accession Number: NM_001002919.2, and FAM150B can be prepared on the basis of the nucleotide sequence represented by SEQ ID NO: 3, which encodes the translation region thereof.

In the present invention, full-length FAM150A and FAM150B existing in nature can be used as target domains. Specific examples of target binding domains that can be used include FAM150A represented by SEQ ID NO: 145 (Uniprot No: Q6UXT8-1(1-129)) or SEQ ID NO: 154 (Uniprot No: Q6UXT8-1(28-129)) and FAM150B represented by SEQ ID NO: 147 (Uniprot No: Q6UX46-1(1-152)) or SEQ ID NO: 155 (Uniprot No: Q6UX46-1(25-152)). Truncated fragments thereof can also be used as ALK-binding fragments. Specifically, in the amino acid sequence represented by SEQ ID NO: 147 (FAM150B), a polypeptide consisting of an amino acid sequence of, for example, amino acids 67 to 152, amino acids 69 to 152, amino acids 71 to 152, amino acids 73 to 152, amino acids 75 to 152, amino acids 77 to 152, amino acids 79 to 152, amino acids 81 to 152, amino acids 83 to 152, amino acids 85 to 152, amino acids 87 to 152, amino acids 89 to 152, amino acids 91 to 152, amino acids 93 to 152, amino acids 71 to 150, or amino acids 71 to 148, can be used as a target binding domain.

More preferably, a polypeptide consisting of an amino acid sequence selected from the group consisting of SEQ ID NO: 154 (FAM150A), SEQ ID NO: 146 (TrFAM150A), SEQ ID NO: 155 (FAM150B), and SEQ ID NO: 148 (TrFAM150B) can be used as a target binding domain.

A polypeptide consisting of an amino acid sequence having 90% or higher sequence identity to the amino acid sequence represented by SEQ ID NO: 146 (TrFAM150A) or SEQ ID NO: 148 (TrFAM150B) can also be used.

The ability of the target binding domain to bind to an extracellular ligand binding region of ALK is, for example, 100 nM or lower, preferably 10 nM or lower, and more preferably 5 nM or lower, in terms of a KD value. The binding ability may be lower than the antigen-antibody binding ability.

The CAR protein can optionally comprise an "extracellular spacer domain" between the target binding domain located extracellularly and the transmembrane domain. The extracellular spacer domain is desirably a sequence that promotes the binding of CAR to the antigen and facilitates signal transduction into a cell. For example, an Fc fragment of an antibody or a fragment or a derivative thereof, a hinge region of an antibody or a fragment or a derivative thereof, a CH2 region of an antibody, a CH3 region of an antibody, an artificial spacer sequence, or a combination thereof can be used.

In an aspect of the present invention, (i) hinge, CH2, and CH3 regions of IgG4, (ii) a hinge region of IgG4, (iii) hinge and CH2 regions of IgG4, (iv) a hinge region of CD8a, (v) hinge, CH2 and CH3 regions of IgG1, (vi) a hinge region of IgG1, or (vii) hinge and CH2 regions of IgG1, or a combination thereof can be used as the extracellular spacer domain. For example, a region having the following amino acid sequence (SEQ ID NO: 149) can be suitably used as the hinge region of IgG1, although the extracellular spacer domain is not limited thereto.

| | | |
|---|---|---|
| UniProt No.: | P01857 | (99-110) |
| EPKSCDKTHTCP | PCDPA | EPKSPDKTHTCP |
| Hinge | Spacer | Hinge |

A region having the amino acid sequence represented by SEQ ID NO: 150 and a region having the amino acid sequence represented by SEQ ID NO: 151 can be suitably used as the CH2 region and the CH3 region of IgG1, respectively.

In a preferred aspect, hinge, CH2, and CH3 regions of human IgG1 or a part thereof can be used as the extracellular spacer domain.

In a preferred aspect, (i) a hinge region of human IgG1 (SEQ ID NO: 149) by itself, (ii) a hinge region (SEQ ID NO: 149), a CH2 region (SEQ ID NO: 150), and a CH3 region (SEQ ID NO: 151) of human IgG1 in combination, (iii) a hinge region (SEQ ID NO: 149) and a CH3 region (SEQ ID NO: 151) of human IgG1 in combination, or (iv) a CH3 region (SEQ ID NO: 151) by itself can be used as the extracellular spacer domain.

In an aspect of the present invention, a spacer sequence represented by the formula (G4S)n can be used as the artificial spacer sequence for use in the extracellular spacer domain. In the formula, n is 1 to 10, and n is preferably 3. A spacer having such a spacer sequence may be referred to as a "peptide linker." Peptide linkers suitably used in the art can adequately be used in the present invention. In this case, the configuration and the chain length of the peptide linker can be adequately selected without impairing the function of the resulting CAR protein.

The extracellular spacer domain is not particularly limited. The extracellular spacer domain can be adequately selected from those listed above or further modified in accordance with the general technical knowledge in the art and used in the present invention.

Nucleotide sequences encoding the respective amino acid sequences of domains can be ligated, inserted into a vector, and expressed in host cells, so that the extracellular spacer domain can be located between the target binding domain and the transmembrane domain. Alternatively, the extracellular spacer domain may be modified using a polynucleotide encoding a plasmid CAR protein produced in advance as a template.

The modification of the extracellular spacer domain is useful when, for example, improvement in the CAR gene expression rate in host cells harboring an introduced polynucleotide encoding CAR, signal transduction, aging of cells, distribution in tumor, antigen recognition, or influence on *in vivo* activity, is taken into consideration.

The CAR protein comprises an extracellular domain comprising a target binding domain and, optionally, an extracellular spacer domain, a transmembrane domain, and an intracellular domain comprising an intracellular signaling domain and, optionally, a costimulatory domain. As is well known in the art, the "transmembrane domain" is a domain having affinity to a lipid bilayer constituting cell membrane, whereas both the extracellular domain and the intracellular domain are hydrophilic domains.

The transmembrane domain is not particularly limited, as long as the CAR protein can be present on the cell membrane without impairing the functions of the target binding domain and the intracellular signaling domain. A polypeptide derived from the same protein as that of the co-stimulatory domain described below may function as the transmembrane domain. For example, a transmembrane domain such as CD28, CD3ε, CD8α, CD3, CD4, or 4-1BB can be used. For example, human CD28 (Uniprot No.: P10747 (153-179)) can be used as the transmembrane domain. Specifically, a domain having an amino acid sequence encoded by the nucleotide sequence: NCBI Accession Number: NM_006139.3 (679-759) can be suitably used as the transmembrane domain.

The CAR protein can optionally comprise a "co-stimulatory domain." The costimulatory domain specifically binds to a costimulatory ligand, thereby mediating cellular costimulatory responses, such as CAR-T cell proliferation, cytokine production, functional differentiation, and target cell death, although the cellular costimulatory responses are not limited thereto. For example, CD27, CD28, 4-1BB (CD137), CD134 (OX40), Dap10, CD27, CD2, CD5, CD30, CD40, PD-1, ICAM-1, LFA-1 (CD11a/CD18), TNFR-1, TNFR-II, Fas, or Lck can be used as the co-stimulatory domain. For example, human CD28 (Uniprot No.: P10747 (180-220)) or 4-1BB (GenBank: U03397.1) can be used as the co-stimulatory domain. Specifically, a domain having an amino acid sequence encoded by the nucleotide sequence represented by NCBI Accession Number: NM_006139.3 (760-882) can be suitably used as the co-stimulatory domain.

When the transmembrane domain and the co-stimulatory domain both derived from human CD28 are used, for example, a domain having the amino acid sequence represented by SEQ ID NO: 152 can be used.

The CAR protein comprises an "intracellular signaling domain." The intracellular signaling domain transmits signals required for exerting effector functions of immune cells. For example, a human CD3ζ chain, FcyRIII, FcεRI, a cytoplasmic end of an Fc receptor, a cytoplasmic receptor having an immunoreceptor tyrosine activation motif (ITAM), or a combination thereof can be used as the intracellular signaling domain. For example, a human CD3ζ chain (e.g., nucleotides 299-637 of NCBI Accession Number: NM_000734.3) can be used as the intracellular signaling domain. Specifically, a domain having the amino acid sequence represented by SEQ ID NO: 153 can be suitably used as the intracellular signaling domain.

In order to accelerate CAR protein secretion, a signal (or leader) sequence that induces protein transfer during or after translation is adequately added to the N terminus of the protein. Examples of useful signal sequences that can be suitably used in the present invention include, but are not limited to, human immunoglobulin (Ig) heavy chain signal peptide, CD8α signal peptide, and human GM-CSF receptor α signal peptide. Ig heavy chain signal peptides derived from, for example, IgG1, IgG2, IgG3, IgA1, and IgM can be suitably used.

The polynucleotide of interest can be easily produced according to a conventional technique. Nucleotide sequences encoding the respective amino acid sequences of domains can be obtained from NCBI RefSeq IDs or GenBank Accession numbers indicating the amino acid sequences, and the polynucleotide of the present invention can be produced in accordance with standard molecular biological and/or chemical procedures. For example, nucleic acids can be synthesized on the basis of these nucleotide sequences. Also, DNA fragments obtained by polymerase chain reaction (PCR) from a cDNA library can be combined to produce the polynucleotide of the present invention.

Thus, the polynucleotide encoding the CAR protein can be produced by ligating the respective polynucleotides encoding the domains described above. Genetically modified cells can be produced by introducing this polynucleotide to adequate cells. Alternatively, the CAR protein may be produced by using, as a template, a polynucleotide encoding an existing CAR protein having the same constituents except for the target binding domain, and recombining the target binding domains in accordance with a conventional technique.

According to need, one or more domains, such as the extracellular spacer domain, can be modified by inverse-PCR (iPCR) or other means, using, as a template, a polynucleotide encoding an existing CAR protein. The technique of modifying the extracellular spacer domain is described in, for example, Oncoimmunology, 2016, Vol. 5, No. 12, e1253656.

Any general method of polynucleotide introduction may be employed to prepare genetically modified cells without particular limitation. When a polynucleotide is introduced using a vector, for example, a lentivirus vector, a retrovirus vector, a foamy virus vector, or an adeno-associated virus vector can be used, although a vector is not limited thereto. Alternatively, polynucleotide introduction can be carried out by a non-viral method based on a transposon method. The transposon method can be performed with the use of a plasmid transposon, and a sleeping beauty transposon system (described in, for example, Huang X, Guo H, et al., Mol. Ther., 2008; 16: 580-9; Singh H, Manuri PR, et al., Cancer Res., 2008; 68: 2961-71; Deniger DC, Yu J, et al., PLoS One, 2015; 10: e0128151; Singh H, Moyes JS, et al., Cancer Gene Ther., 2015; 22: 95-100; Hou X, Du Y, et al., Cancer Biol. Ther., 2015; 16: 8-16; Singh H, Huls H, et al., Immunol. Rev., 2014; 257: 181-90; and Maiti SN, Huls H, et al., J. Immunother., 2013; 36: 112-23) or a piggyBac transposon system (described in, for example, Nakazawa Y, Huye LE, et al., J. Immunother., 2009; 32: 826-36; Galvan DL, Nakazawa Y, et al., J Immunother., 2009; 32: 837-44; Nakazawa Y, Huye LE, et al., Mol. Ther., 2011; 19: 2133-43;Huye LE, Nakazawa Y, et al., Mol. Ther., 2011; 19: 2239-48; Saha S, Nakazawa Y, et al., J. Vis. Exp., 2012; (69): e4235; Nakazawa Y, Saha S, et al., J. Immunother., 2013; 36: 3-10; Saito S, Nakazawa Y, et al., Cytotherapy, 2014; 16: 1257-69; and Nakazawa et al., Journal of Hematology &Oncology, 2016, 9:27) can be preferably used.

In the case where using the piggyBac transposon system, typically, a plasmid carrying a gene encoding piggyBac transposase (referred to as a piggyBac plasmid herein) and a plasmid having a structure where the polynucleotide encoding a CAR protein is flanked by piggyBac inverted repeat sequences are introduced (transfection). Transfection can be carried out by various approaches, such as electroporation, nucleofection, lipofection, or the calcium phosphate method. Both the plasmids can contain a poly A addition signal sequence, a reporter gene, a selection marker gene, an enhancer sequence, and the like.

Examples of apparatuses that can be used for electroporation include, but are not limited to, 4D-Nucleofector (Lonza Japan Ltd.), NEPA21 (Nepa Gene Co., Ltd.), and Maxcyte GT (Maxcyte, Inc.), and such apparatuses can be operated according to their respective instruction manuals.

According to the method described above, gene introduction into 1 × 10⁶ to 2 × 10⁷ cells can be performed.

As cells into which the polynucleotides are to be introduced, cells derived from mammals, such as humans, T cells or a cell population containing T cells derived from nonhuman mammals, such as monkeys, mice, rats, pigs, cattle, and dogs can be used. Cells releasing a cytotoxic protein (perforin, granzyme, etc.) are preferably used. Specifically, for example, a cell population containing T cells, precursor cells of T cells (hematopoietic stem cells, lymphocyte precursor cells, etc.), and/or NK-T cells can be used. Further examples are cells capable of differentiating into these cells, including various stem cells such as ES cells and iPS cells. The T cells encompass CD8-positive T cells, CD4-positive T cells, regulatory T cells, cytotoxic T cells, and tumor-infiltrating lymphocytes. The cell population containing T cells and precursor cells of T cells includes PBMCs. The cells described above may be collected from a living organism, the expansion culture products thereof, or the established cell lines thereof. When transplanting the CAR-expressing cells produced or cells differentiated therefrom into a living organism, it is desirable to introduce a nucleic acid into cells collected from the living organism itself or a living organism of the same species thereas.

As T cells for use in adoptive immunotherapy into which the polynucleotide is to be introduced to produce the genetically modified cells of the present invention, T cells expected to have sustainable antitumor effects, such as stem cell memory T cells, can be used. The stem cell memory T cells can be analyzed in accordance with a conventional technique and easily confirmed as described in, for example, Yang Xu, et al., Blood, 2014; 123: 3750-3759.

In one embodiment, for example, CD45R0-, CD62L+, CD45RA+, and CCR7+ T cells can be used as stem cell memory T cells.

The present invention also provides a vector comprising the polynucleotide of the present invention.

The present invention also provides a genetically modified cell comprising the polynucleotide of the present invention or the vector of the present invention introduced thereinto. The genetically modified cell of the present invention can express a CAR protein binding to an ALK-expressing cell on a cell membrane.

The present invention further provides a method for preparing a CAR protein-expressing cell comprising introducing the polynucleotide of the present invention or the vector of the present invention into a cell.

CAR protein-expressing cells can be cultured and proliferated by any means without particular limitation. For example, a polynucleotide encoding a CAR protein is introduced into a cell in the manner described above, and non-specific or CAR-specific stimuli can then be applied to the cell, so as to activate the CAR protein-expressing cell. A method of cell stimulation is not limited. As a method of non-specific cell stimulation, for example, stimuli can be applied using anti-CD3 antibody and/or anti-CD28 antibody. As a method of CAR-specific stimulation, for example, stimuli can be applied using artificial antigen presenting cells (aAPCs) comprising CAR-binding antigen molecules or costimulatory factors expressed in K562 or other tumor cell lines. While culture conditions are not particularly limited, for example, culture is suitably carried out at 37°C for 1 to 21 days.

In the method according to the present invention described above, a method of introducing a polynucleotide into a cell is not limited, and the transposon method is suitably employed. As the transposon method, the transposon system described above or other systems suitable in the present invention may be employed. The method according to the present invention can be adequately performed by the piggyBac method, although the method is not limited thereto.

In another aspect of non-specific stimulation, after a cell population comprising T cells is stimulated by one or more types of virus peptide antigens, cells in which the virus growth ability is inactivated by a conventional technique are used as feeder cells to promote activation of cells into which CAR has been introduced. As virus peptide antigens, for example, an AdV antigen peptide mixture, a CMV antigen peptide mixture, an EBV antigen peptide mixture, or a combination thereof can be used. Specific examples are shown in the examples below.

In order to enhance cell viability and proliferation rate, culture can be carried out in the presence of one or more types of cytokines. For example, culture can be preferably carried out in the presence of cytokines, such as IL-7 and IL-15.

The present invention further provides a kit used for preparing a CAR protein-expressing cell that targets an ALK-expressing cell comprising the vector according to the present invention. The kit according to the present invention can adequately contain, for example, a reagent, a buffer, a reaction vessel, and instructions that are necessary for preparing a CAR protein-expressing cell. The kit according to the present invention can be suitably used for preparing the genetically modified cell according to the present invention.

The cell according to the present invention induces receptor-specific immune responses to a target cell expressing ALK on its surface. Thus, signal transduction takes place in the cell and the cell is then activated. Activation of a CAR-expressing cell can be confirmed using, as an indicator, release of cytokines, an enhanced cell proliferation rate, and changes in cell surface molecules, for example, although the indicator varies depending on the type of host cells or the intracellular domain of CARs. Release of cytotoxic proteins, such as perforin and granzyme, would damage cells expressing receptors.

The genetically modified cell according to the present invention can be used as a therapeutic agent for a disease associated with an ALK-expressing cell. Diseases expected to be cured by the therapeutic agent of the present invention are not limited, provided that they have sensitivity to the cell. For example, diseases are associated with a cell expressing ALK on its cell membrane, and include solid tumors, such as neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer.

The therapeutic agent according to an aspect of the present invention is an anti-cancer agent against an ALK-expressing tumor cell, such as the solid tumor described above. While the therapeutic agent or anti-cancer agent according to the present invention can be used alone, it can also be used in combination with an agent and/or treatment of different mechanisms.

Thus, the therapeutic agent according to the present invention can be in the form of a pharmaceutical composition comprising the therateutic agent alone or in combination with other active ingredients. The therapeutic agent or the pharmaceutical composition according to the present invention can be applied by topical or systemic administration. While the route of administration is not limited, for example, intravenous administration is preferable in the case of treatment of neuroblastoma. The pharmaceutical composition may comprise, in addition to the therapeutic agent according to the present invention and other active ingredients, a carrier, an excipient, a buffer, a stabilizer, and other substances that are commonly used in the art, depending on the route of administration. A dose of the therapeutic agent according to the present invention varies depending on, for example, body weight, age, and severity of disease of the patient. While a dose is not particularly limited, for example, 10⁴ to 10¹⁰ CAR-positive cells can be administered per kg body weight 1 to several times a day, every 2 days, every 3 days, every week, every 2 weeks, every month, every 2 months, or every 3 months.

The present invention further provides a method of treatment of a solid tumor selected from among neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer, comprising administering a therapeutically effective amount of the therapeutic agent or pharmaceutical composition according to the present invention to a patient. The therapeutically effective amount and the administration regimen can be adequately determined in consideration of various factors as described above.

### Examples

Hereafter, the present invention is described in greater detail with reference to the examples, although the present invention is not limited to the following examples.

### Example 1: Preparation of FAM150A (CAR 002; FAM150A-28z), FAM150B (CAR 003; FAM150B-28z), humanized ALK48 scFv (CAR 004; hALK48-28z), or mouse ALK48 scFv-type (CAR 005; ALK48-28z) CAR-expressing plasmids

### Artificial synthesis of FAM150A, FAM150B, humanized ALK48 scFv, or mouse ALK48 scFv gene

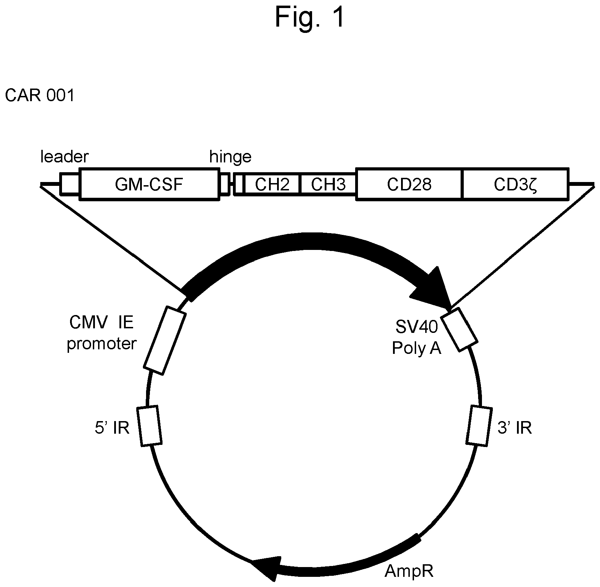

In order to prepare a CAR-expressing plasmid of the structure similar to that of the GMR CAR-expressing plasmid described in WO 2018/052142 (CAR 001, a vector map thereof is shown in Fig. 1), FAM150A, FAM150B, humanized ALK48 scFv, and mouse ALK48 scFv genes to be incorporated into the plasmid were synthesized.

A DNA sequence (*Xho*I-leader-FAM150A-Hinge-*Dra*III; SEQ ID NO: 2) comprising a restriction enzyme *Xho*I cleavage sequence and a leader sequence added to upstream a translation region (206 to 592 bp; SEQ ID NO: 1) of FAM150A (NCBI Accession Number: NM_207413.3) and a hinge region and a restriction enzyme *Dra*III cleavage sequence added to downstream thereof was designed. The leader sequence was a DNA sequence to be translated into the amino acid sequence of a human immunoglobulin (Ig) heavy chain signal peptide(SEQ ID NO: 143).

A DNA sequence (*Xho*I-leader-FAM150B-Hinge-*Dra*III; SEQ ID NO: 4) comprising a restriction enzyme cleavage sequence, a leader sequence, and a hinge region added to a translation region (354 to 809 bp; SEQ ID NO: 3) of FAM150B (NCBI Accession Number: NM_001002919.2) was also designed. The leader sequence was a DNA sequence to be translated into the amino acid sequence of a human immunoglobulin (Ig) heavy chain signal peptide (SEQ ID NO: 143).

The designed sequences were artificially synthesized as DNA sequences codon-optimized for human (synthesis was commissioned to Eurofins Genomics K.K.).

Separately, on the basis of the amino acid sequences of humanized ALK48 scFv and mouse ALK48 scFv (SEQ ID NO: 5 and SEQ ID NO: 6) described in WO 2015/069922, DNA sequences (*Xho*I-leader-humanized ALK48 scFv-Hinge-*Dra*III (SEQ ID NO: 7) and *Xho*I-leader-mouse ALK48 scFv-Hinge-*Dra*III (SEQ ID NO: 8)) comprising the restriction enzyme cleavage sequence, the leader sequence, and the hinge region added to the DNA sequence codon-optimized for human were designed as with the case of FAM150A and FAM150B, artificially synthesized, and then incorporated into the pEX-K4J1 vector (synthesis was commissioned to Eurofins Genomics K.K.).

### Preparation of FAM150A-type CAR-expressing plasmid (CAR 002), FAM150B-type CAR-expressing plasmid (CAR 003), humanized ALK48 scFv-type CAR-expressing plasmid (CAR 004), and mouse ALK48 scFv-type CAR-expressing plasmid (CAR 005)

CAR 001 (about 1 µg equivalent; prepared by the method described in WO 2015/069922) was digested with restriction enzymes *Xho*I and *Dra*III (New England Biolabs) at 37°C for about 2 hours. The 4 types of genes artificially synthesized above (about 1 µg equivalent each) were also digested with restriction enzymes *Xho*I and *Dra*III at 37°C for about 2 hours.

After the enzyme treatment, the reaction solution was separated via 1% or 2% agarose gel electrophoresis, the enzyme-treated CAR 001 fragment (on the vector side) and the artificially synthesized gene-inserted fragment (SEQ ID NO: 2, 4, 7 or 8) cleaved from the pEX-K4J1 vectors were removed from the gel, and the fragments were purified using NucleoSpin Gel and PCR Clean-up^{®} (MACHEREY-NAGEL, Takara Bio Inc.). The purified vector fragment was ligated to the purified insert fragment using the DNA ligation kit (Mighty Mix, Takara Bio Inc.). *E. coli* DH5α (Toyobo Co. Ltd.) cells were transformed using the ligated cyclic plasmid and then cultured on an LB agar medium containing 100 µg/ml carbenicillin for about 16 hours.

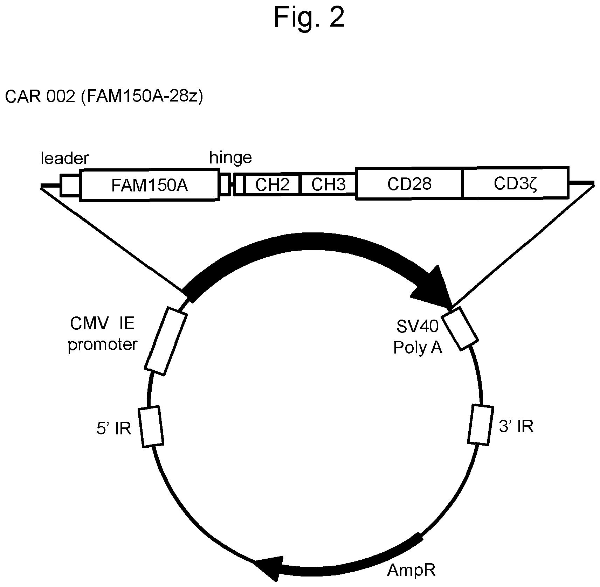

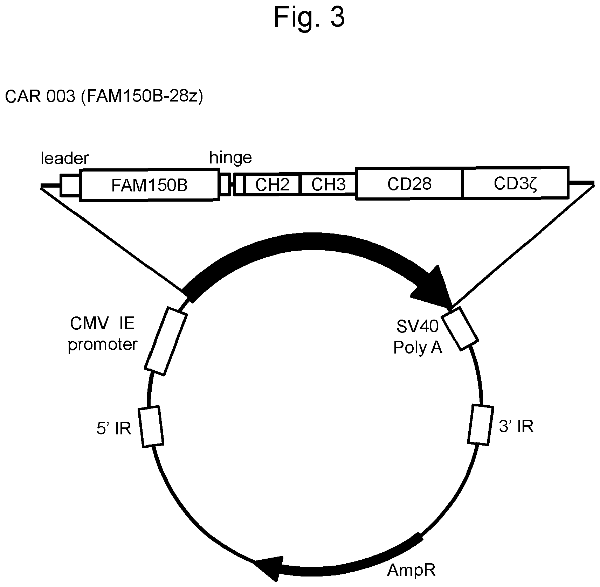

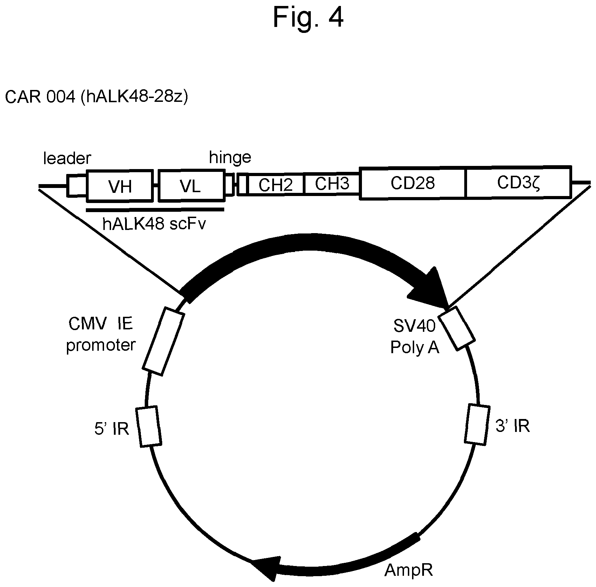

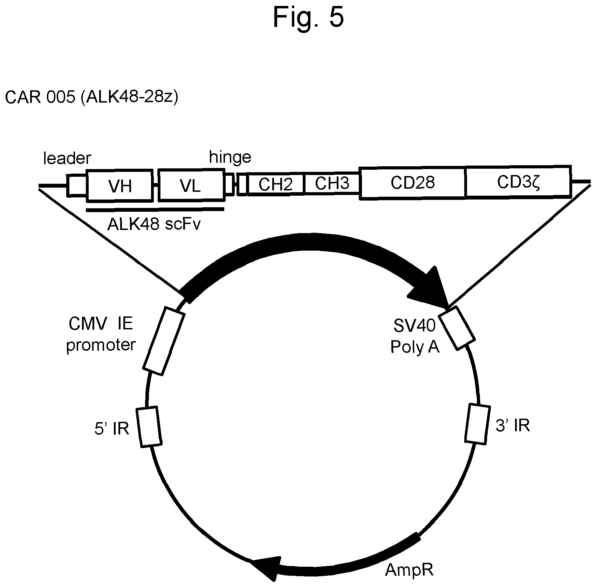

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence insertion was observed were obtained as FAM150A-type CAR-expressing plasmid (CAR 002; FAM150A-28z), FAM150B-type CAR-expressing plasmid (CAR 003; FAM150B-28z), humanized ALK48 scFv-type CAR-expressing plasmid (CAR 004; hALK48-28z), or mouse ALK48 scFv-type CAR-expressing plasmid (CAR 005; ALK48-28z). Nucleotide sequence analysis was commissioned to Eurofins Genomics K.K. Vector maps of the prepared plasmids are shown in Figs. 2 to 5.

### Example 2: Culture and proliferation of CAR-T cells

Peripheral blood mononuclear cells (PBMCs) were separated by any of the methods described below.

### Day 0: Separation of peripheral blood mononuclear cells (PBMCs) (density gradient centrifugation)

Peripheral blood samples were obtained from healthy adult donors and diluted to 2-fold with D-PBS (FUJIFILM Wako Pure Chemical Corporation). The diluted peripheral blood was superposed on Ficoll-Paque PLUS (GE Healthcare), and centrifugation was carried out at 400× g for 30 minutes to fractionate the PBMC layer. The fractionated PBMCs were washed 2 times with D-PBS and isolated via centrifugation.

### Day 0: Separation of PBMCs (Separation using SepMate-50)

Peripheral blood samples were obtained from healthy adult donors and diluted to 2-fold with D-PBS (FUJIFILM Wako Pure Chemical Corporation). SepMate-50 (STEMCELL Technologies) was filled with 15 ml of Ficoll-Paque PLUS in advance, the diluted peripheral blood samples were superposed thereon, centrifugation was carried out at 1,200× g for 10 minutes, and the supernatant containing PBMCs and plasma was transferred to another 50-ml centrifuge tube via decantation. After D-PBS was added to the supernatant to adjust the amount to 50 ml, centrifugation was carried out at 300× g for 8 minutes, the supernatant was removed, pellets were washed with D-PBS, centrifugation was carried out again at 300× g for 8 minutes, and the supernatant was removed to isolate PBMCs.

### Day 0: Virus peptide pulsing of PBMCs

The isolated PBMCs were stimulated with virus peptides using PepTivator Peptide Pools^{®} (Miltenyi Biotec). Specifically, PBMCs were suspended in Peptide Pools each comprising AdV5 Hexon, CMV pp65, EBV BZLF1, and EBV EBNA-1 added at 0.05 µg/µl to 50 µl of D-PBS, and the resulting suspensions were stimulated with virus peptides at 37°C for 30 minutes. Thereafter, 5 ml of D-PBS was added to PBMCs to prepare a suspension, and the suspension was then irradiated with UV for 4 minutes. PBMCs irradiated with UV were collected, the number of cells was counted, the cells were suspended at 0.5 to 4 × 10⁶ cells/2 ml/well in the TexMACS medium containing 10 ng/ml IL-7 and 5 ng/ml IL-15, and the cell suspension was transferred as feeder cells to a 24-well treated culture plate.

### Day 0: Gene introduction

CAR-expressing plasmids were introduced into 15 × 10⁶ PBMCs. Specifically, 5 µg of any of CAR 002 to CAR 005 obtained in Example 1, 5 µg of pCMV-piggyBac plasmid, and 100 µl of the P3 Primary Cell solution included in P3 Primary Cell 4D-Nucleofector^{™}X Kit (Lonza Japan Ltd.) were mixed with each other, and 15 × 10⁶ PBMCs were suspended in the mixture. All the suspended cells were transferred to Nucleocuvette, and the genes were introduced into cells through electrical pulses by the 4D-Nucleofection system (Program No: FI-115). The cells into which the genes had been introduced through electrical pulses were allowed to stand at room temperature for 10 minutes, all the cells were added to a 24-well treated culture plate containing feeder cells, and culture was initiated.

As the mock-T cells without gene introduction, 15 × 10⁶ PBMCs were cultured in the same manner. As CAR-T cells not damaging solid tumors, cells subjected to the same gene introduction procedure using CD19 scFv-type CAR-expressing plasmid (CAR 006; CD19-28z, prepared in the same manner as described in WO 2018/052142) were prepared. A half of the medium was discarded during the culture and a half of TexMACS medium containing 20 ng/ml IL-7 and 10 ng/ml IL-15 was added to exchange media, approximately every other day.

### Day 7: Stimulation of CAR-T cells with antibody and proliferation thereof

A 24-well non-treated culture plate was coated with D-PBS containing anti-CD3 antibody and anti-CD28 antibody at 37°C for 2 hours to prepare an antibody-coated plate, the total amount of the cell suspension was seeded on the plate, and the CAR-T cells cultured above were stimulated with antibodies for 2 days. On Day 9, the total amount of the cell suspension was transferred to a G-Rex 6-well plate (Wilson Wolf) filled with 30 ml of the TexMACS medium containing 10 ng/ml IL-7 and 5 ng/ml IL-15, and the cells were further cultured for 7 days and proliferated up to Day 16. The CAR expression rates in the ALK CAR-T cells proliferated up to Day 16 into which either of CAR 002 to CAR 005 had been introduced and the CD19 CAR-T cells into which CAR 006 had been introduced were determined in the manner described below. The experiment may be performed one day before or after the designated day.

PBMCs obtained from 2 healthy adult donors were subjected to the procedure described above.

### Day 16: Evaluation of CAR expression rate

The number of CAR-T cells into which the CAR-expressing plasmids had been introduced were counted, and 1 to 2 × 10⁵ cells were subjected to flow cytometric analysis to evaluate the CAR expression rates in the ALK CAR-T cells and in the CD19 CAR-T cells.

1 to 2 × 10⁵ cells were sampled, suspended with 2 µl of FITC Goat Anti-Human IgG (H+L) antibody (Jackson ImmunoResearch Inc) and 5 µl of APC Anti-Human CD3 antibody (Miltenyi Biotec), and the resulting suspension was subjected to antibody labeling at 4°C under shading conditions for 20 minutes. Thereafter, the cells were washed with 500 µl of D-PBS, and precipitated via centrifugation. After the supernatant was removed, the cells were resuspended in 500 µl of D-PBS. The resulting sample was analyzed using FACSCalibur (BD Biosciences), and the expression rates of IgG1/CD3-positive ALK CAR (CAR 002 to CAR 005) or CD19 CAR (CAR 006) were determined.

The CAR-T cells obtained by gene introduction through electrical pulses into PBMCs using expression plasmids (CAR 002 to CAR 006) and T cell culture and proliferation are described herein as follows.
CAR-T 002: FAM150A CD28-type CAR-T (FAM150A-28z CAR-T)
CAR-T 003: FAM150B CD28-type CAR-T (FAM150B-28z CAR-T)
CAR-T 004: Humanized ALK48 scFv CD28-type CAR-T (hALK48-28z CAR-T)
CAR-T 005: Mouse ALK48 scFv CD28-type CAR-T (ALK48-28z CAR-T)
CAR-T 006: CD19 scFv CD28-type CAR-T (CD19-28z CAR-T)

The amino acid sequence of CAR 002 and the nucleotide sequence encoding the same are represented by SEQ ID NO: 13 and SEQ ID NO: 9, respectively.

The amino acid sequence of CAR 003 and the nucleotide sequence encoding the same are represented by SEQ ID NO: 14 and SEQ ID NO: 10, respectively.

The amino acid sequence of CAR 004 and the nucleotide sequence encoding the same are represented by SEQ ID NO: 15 and SEQ ID NO: 11, respectively.

The amino acid sequence of CAR 005 and the nucleotide sequence encoding the same are represented by SEQ ID NO: 16 and SEQ ID NO: 12, respectively.

Table 1 shows CAR expression rates in CAR-T cells obtained from PBMCs derived from 2 donors.

**[Table 1]**

| Donor | Plasmid | CAR-T | Expression rate |
|---|---|---|---|
| Donor-1 | CAR 002 | CAR-T 002 | 22.3 |
| | CAR 003 | CAR-T 003 | 15.7 |
| | CAR 004 | CAR-T 004 | 22.1 |
| | CAR 005 | CAR-T 005 | 23.3 |
| Donor-2 | CAR 002 | CAR-T 002 | 33.2 |
| | CAR 003 | CAR-T 003 | 41.2 |
| | CAR 004 | CAR-T 004 | 36.9 |
| | CAR 005 | CAR-T 005 | 56.2 |
| | CAR 006 | CAR-T 006 | 60.7 |

### Example 3: Comparison of antitumor activity of FAM150A CD28-type CAR-T, FAM150B CD28-type CAR-T, humanized ALK48 scFv CD28-type CAR-T, and mouse ALK48 scFv CD28-type CAR-T

### Measurement of antitumor activity of CAR-T

In order to measure antitumor activity of CAR-T 002 to CAR-T 005 obtained in Example 2, co-culture with solid tumor cells was conducted. In this example, neuroblastoma cell line SH-SY5Y cells (DS Pharma Biomedical Co., Ltd.), NB-1 cells (JCRB Cell Bank), or IMR-32 cells (DS Pharma Biomedical Co., Ltd.) were used as target tumor cells.

SH-SY5Y cells, NB-1 cells, and IMR32 cells were subjected to passage culture and used for co-culture test. In passage culture, D-MEM/Ham's F-12 medium containing 15% FBS, 1% penicillin/streptomycin, and 1% non-essential amino acid solution is used for SH-SY5Y cells, RPMI 1640 medium containing 10% FBS and 1% penicillin/streptomycin is used for NB-1 cells, and E-MEM medium containing 10% FBS, 1% penicillin/streptomycin, and 1% non-essential amino acid solution is used for IMR-32 cells. Co-culture test was also performed in similar media.

The tumor cells (target (T)) was adjusted to 2 × 10⁵ cells/ml, and seeded on a 48-well treated culture plate at 500 µl/well (1 × 10⁵ cells/well). CAR-T (effector (E)) was diluted in media to adjust the E:T ratio to 4:1, 2:1, or 1:1.

For the E:T ratio of 4:1, CAR-T was adjusted to 8 × 10⁵ cells/ml and seeded on a 48-well treated culture plate at 500 µl/well (4 × 10⁵ cells/well) on which the tumor cells had been seeded.

For the E:T ratio of 2:1, also, CAR-T was adjusted to 4 × 10⁵ cells/ml and seeded on a 48-well treated culture plate at 500 µl/well (2 × 10⁵ cells/well) on which the tumor cells had been seeded.

For the E:T ratio of 1:1, CAR-T was adjusted to 2 × 10⁵ cells/ml and seeded on a 48-well treated culture plate at 500 µl/well (1 × 10⁵ cells/well) on which the tumor cells had been seeded.

Co-culture was performed for 4 days. CAR-T 006 and mock-T cells were subjected to co-culture in the same manner, and a control group consisting of the same number of tumor cells only to be cultured was prepared (CAR-T non-administered group). In addition, a group consisting of CAR-T cells only to be cultured for 4 days was prepared.

4 days after the initiation of co-culture, the cells were peeled from the wells using Accutase (Innovative Cell Technologies, Inc.) and the collected cells were centrifuged at 1,500 × g for 5 minutes. To the centrifuged cells, 5 µl of APC Anti-Human CD3 antibody (Miltenyi Biotec) and 5 µl of PE anti-human Ganglioside GD2 antibody were added to prepare a suspension, and the suspension was subjected to antibody labeling reaction at 4°C under shading conditions for 20 minutes. Thereafter, the cells were washed with 500 µl of D-PBS, and precipitated via centrifugation. After the supernatant was removed, the cells were resuspended in 400 µl of D-PBS. The sample further supplemented with 50 µl of CountBright absolute counting beads (Invitrogen) was assayed using FACSCalibur (BD Biosciences), the obtained data were analyzed using FlowJo (BD Biosciences), and the number of GD2-positive tumor cells was calculated based on the number of counting beads.

When the cell population consisting of the CAR-T cells subjected to culture partially overlaps with the tumor cell population, in the flow cytometric analysis, the number of cells in the group consisting of the CAR-T cells was subtracted to correct the number of tumor cells.

In accordance with the formula below, the tumor cell proliferation rate (%) of CAR-T or mock-T cells was calculated to provide, an index of antitumor activity of CAR-T cells.

Tumor cell proliferation rate (%) = the number of tumor cells in the CAR-T administered group/the number of tumor cells in the CAR-T non-administered group × 100

Lower tumor cell proliferation rate (%) indicates higher antitumor activity of CAR-T cells.

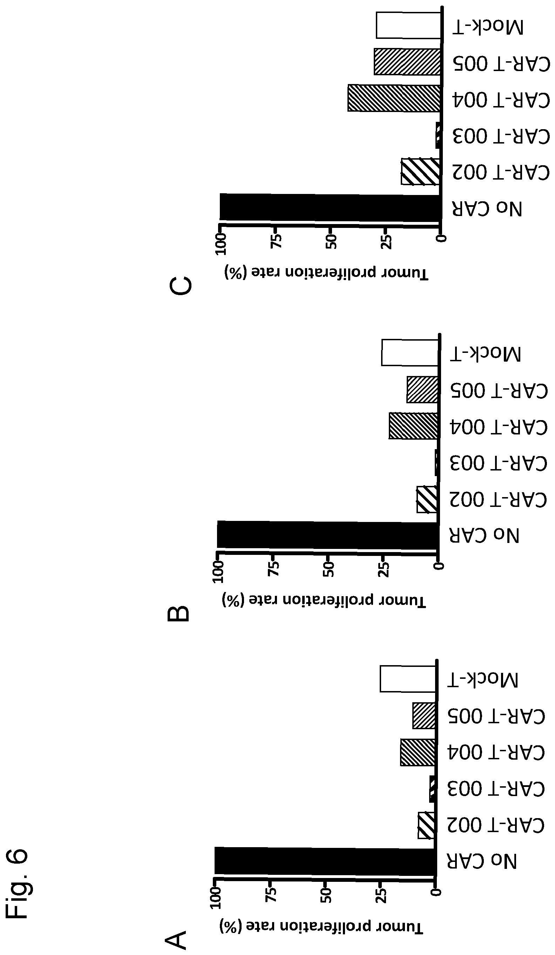

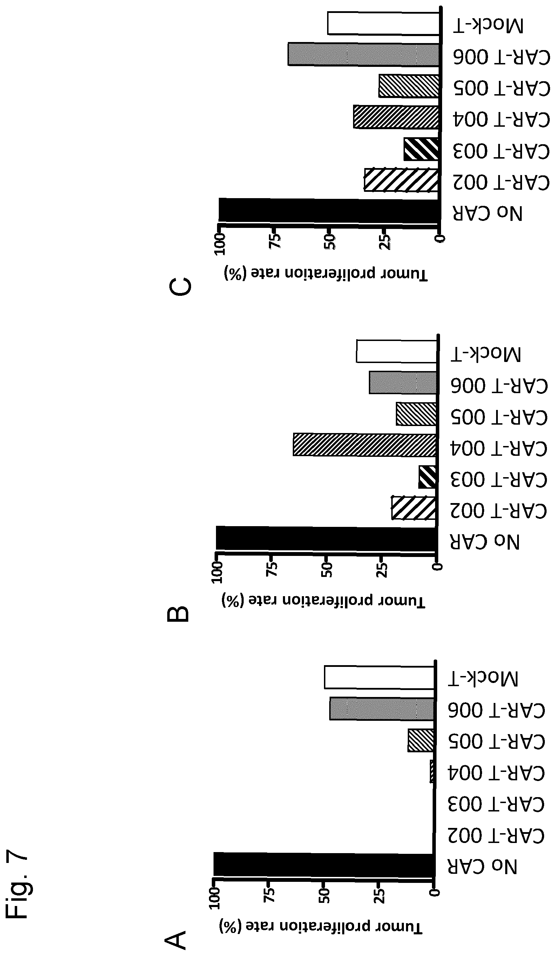

Fig. 6 shows antitumor activity of CAR-T 002 to CAR-T 005 or mock-T cells derived from Donor-1 against neuroblastoma cell line SH-SY5Y and Fig. 7 shows antitumor activity of CAR-T 002 to CAR-T 006 derived from Donor-2 against neuroblastoma cell line SH-SY5Y. Antitumor activity is shown in the charts in terms of the tumor cell proliferation rates (%) relative to the tumor cell proliferation rate exhibited by the CAR-T non-administered group (No CAR, 100%). As a result, the effects of CAR-T 002 to CAR-T 0005 to kill SH-SY5Y were confirmed, and the effects became lower in the order of CAR-T 003, CAR-T 002, CAR-T 005, and CAR-T 004.

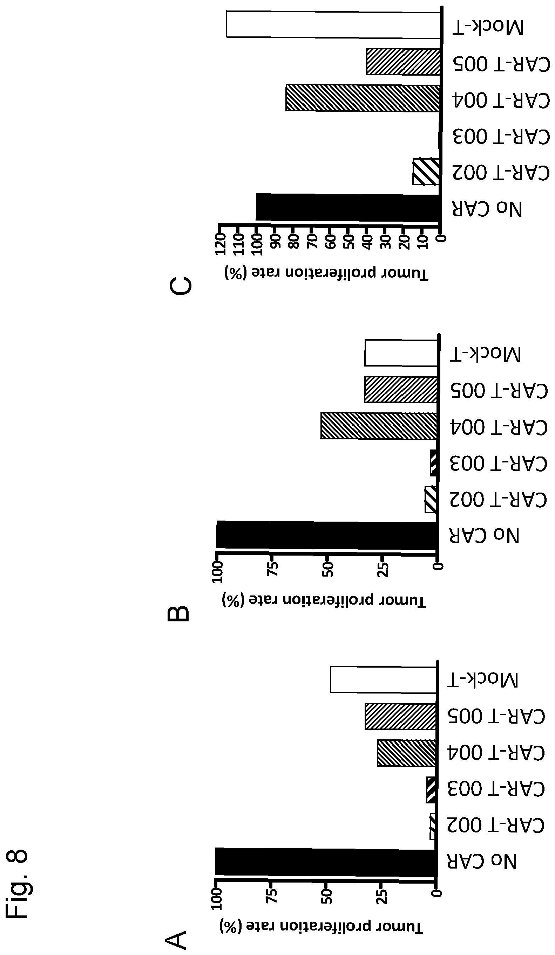

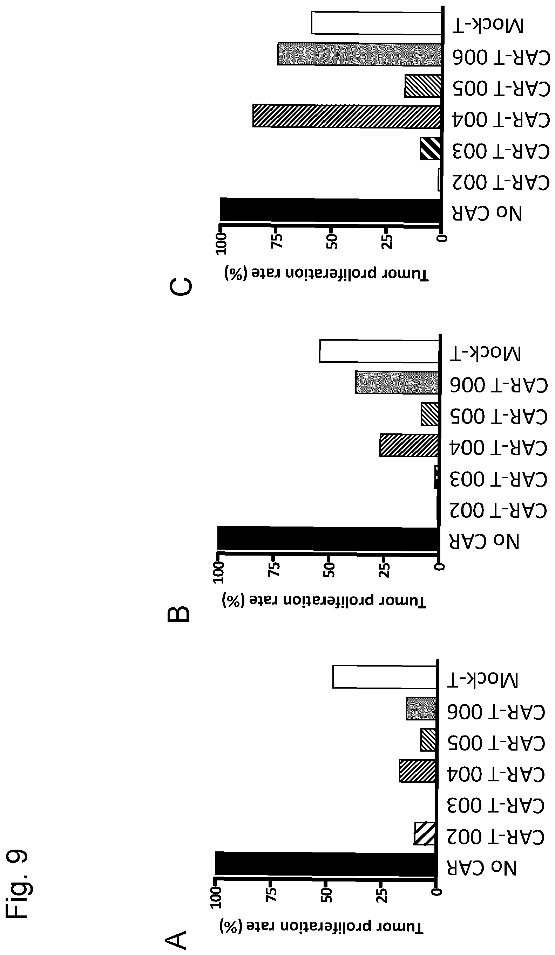

Fig. 8 shows antitumor activity of CAR-T 002 to CAR-T 005 or mock-T cells derived from Donor-1 against neuroblastoma cell line NB-1 and Fig. 9 shows antitumor activity of CAR-T 002 to CAR-T 006 or mock-T cells derived from Donor-2 against the neuroblastoma cell line NB-1. As a result, the effects of CAR-T 002 to CAR-T 005 to kill NB-1 were confirmed, the effects became lower in the order of CAR-T 003, CAR-T 002, CAR-T 005, and CAR-T 004, and the effects of CAR-T 002 and CAR-T 003 were higher than those of CAR-T 006 and mock-T cells.

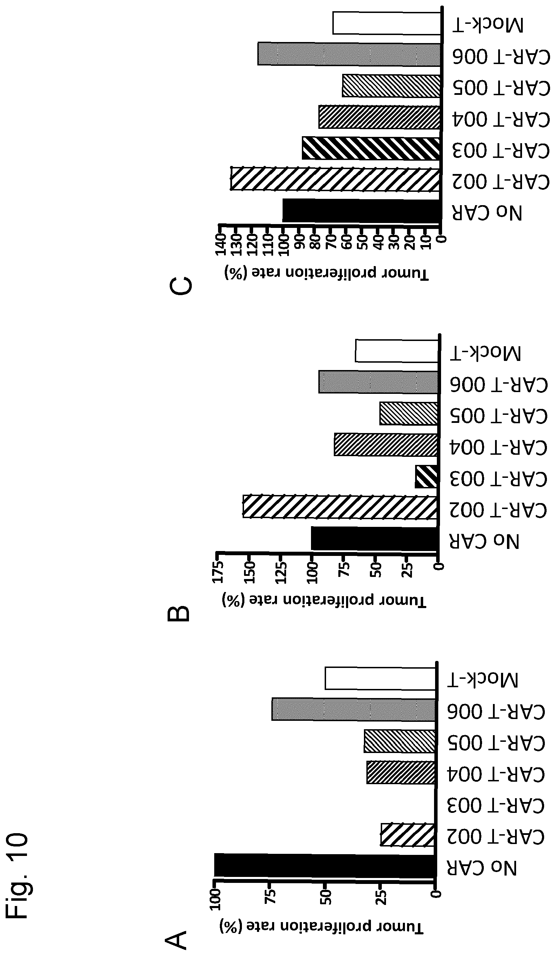

Fig. 10 shows antitumor activity of CAR-T 002 to CAR-T 006 or mock-T cells derived from Donor-2 against neuroblastoma cell line IMR-32. As a result, the effects of CAR-T 002 to CAR-T 005 to kill IMR-32 were confirmed. The effects of CAR-T 003 were particularly high.

In this example, antitumor activity of ligand-type CAR-T 002 and CAR-T 003 against neuroblastoma cells was found to be superior to that of scFv-type CAR-T 004 and CAR-T 005. As a result of comparison between CAR-T002 and CAR-T 003, antitumor activity of CAR-T 003 was considered to be slightly higher than that of CAR-T 002.

### Example 4: Preparation of plasmids for ALK (ALK 001), LTK (LTK 001), FAM150A (Ligand 001), FAM150B (Ligand 002 to Ligand 024) mammalian cell expression Artificial synthesis of ALK gene

A sequence comprising: a 953- to 1009-bp sequence (SEQ ID NO: 17), which is a part of the translation region of ALK (NCBI Accession Number: NM_004304.4) encoding a region comprising a secretory signal; a 2894- to 4042-bp sequence (SEQ ID NO: 18), which is also a part of the translation region; a sequence encoding an octahistidine tag (SEQ ID NO: 19); and a sequence encoding a PA tag (SEQ ID NO: 20) ligated tandemly from the 5' terminus was designed and codon-optimized for human. A DNA molecule comprising a Kozak sequence (SEQ ID NO: 21) at the 5' terminus and 2 stop codons ligated to the 3' terminus were then artificially synthesized (Kozak-ALK-His-PA) (synthesis was commissioned to Eurofins Genomics K.K., SEQ ID NO: 22).

### Artificial synthesis of LTK gene

A sequence comprising: a 179- to 1450-bp sequence (SEQ ID NO: 23), which is a part of the translation region of LTK (NCBI Accession Number: NM_002344.5); a sequence encoding an octahistidine tag (SEQ ID NO: 19); and a sequence encoding a PA tag (SEQ ID NO: 20) ligated tandemly from the 5' terminus was designed and codon-optimized for human, and then a DNA molecule comprising a Kozak sequence (SEQ ID NO: 21) at the 5' terminus and 2 stop codons ligated to the 3' terminus was artificially synthesized (Kozak-LTK-His-PA) (synthesis was commissioned to Eurofins Genomics K.K., SEQ ID NO: 24).

### Artificial synthesis of IgG1 Fc gene

A sequence comprising: a sequence encoding an HRV3C protease cleavage sequence (SEQ ID NO: 25); a sequence encoding an Fc sequence (SEQ ID NO: 26) containing an IgG1-derived hinge region; a sequence encoding a G4S linker (SEQ ID NO: 27); a sequence encoding an octahistidine tag (SEQ ID NO: 19); and a sequence encoding a PA tag (SEQ ID NO: 20) ligated tandemly from the 5' terminus was designed and codon-optimized for human, and then a DNA molecule comprising a Kozak sequence (SEQ ID NO: 21) at the 5' terminus and 2 stop codons ligated to the 3' terminus was artificially synthesized (Kozak-HRV3C-Fc-His-PA) (synthesis was commissioned to Eurofins Genomics K.K., SEQ ID NO: 28).

### Artificial synthesis of FAM150A gene

A sequence comprising: a sequence of a translation region (206 to 592 bp; SEQ ID NO: 1) of FAM150A (NCBI Accession Number: NM_207413.3); a sequence encoding an HRV3C protease cleavage sequence (SEQ ID NO: 25); and a sequence encoding an octahistidine tag (SEQ ID NO: 19) ligated tandemly from the 5' terminus was designed and codon-optimized for human, and then a DNA molecule comprising a Kozak sequence (SEQ ID NO: 21) at the 5' terminus and 2 stop codons ligated to the 3' terminus was artificially synthesized (Kozak-FAM150A-HRV3C-His) (synthesis was commissioned to Eurofins Genomics K.K., SEQ ID NO: 29).

### Artificial synthesis of FAM150B gene

A sequence comprising: a sequence of a translation region (354 to 809 bp; SEQ ID NO: 3) of FAM150B (NCBI Accession Number: NM_001002919.2); a sequence encoding an HRV3C protease cleavage sequence (SEQ ID NO: 25); and a sequence encoding an octahistidine tag (SEQ ID NO: 19) ligated tandemly from the 5' terminus was designed and codon-optimized for human, and then a DNA molecule comprising a Kozak sequence (SEQ ID NO: 21) at the 5' terminus and 2 stop codons ligated to the 3' terminus was artificially synthesized (Kozak-FAM150B-HRV3C-His) (synthesis was commissioned to Eurofins Genomics K.K., SEQ ID NO: 30).

### Preparation of ALK 001 and LTK 001 plasmids

With the use of the artificial gene encoding ALK (Kozak-ALK-His-PA; SEQ ID NO: 22) as a template and primers represented by SEQ ID NO: 31 and SEQ ID NO: 32 (synthesis thereof was commissioned to Eurofins Genomics K.K.), a DNA sequence of an ALK-encoding region from the Kozak sequence was amplified by PCR (Kozak-ALK).

Further, with the use of the artificial gene encoding LTK (Kozak-LTK-His-PA; SEQ ID NO: 24) as a template and primers represented by SEQ ID NO: 33 and SEQ ID NO: 34 (synthesis thereof was commissioned to Eurofins Genomics K.K.), a DNA sequence of an LTK-encoding region from the Kozak sequence was amplified by PCR (Kozak-LTK).

In addition, with the use of the artificial gene encoding IgG1 Fc (Kozak-HRV3C-Fc-His-PA; SEQ ID NO: 28) as a template and primers represented by SEQ ID NO: 35 and SEQ ID NO: 36 (synthesis thereof was commissioned to Eurofins Genomics K.K.), a region from the sequence encoding the HRV3C protease cleavage sequence to the stop codon (HRV3C-Fc-His-PA) was amplified by PCR.

As a result of PCRs above, an overlap sequence to be ligated to pcDNA3.4 (Thermo Fisher Scientific) was added to the 5' side of Kozak-ALK and Kozak-LTK, and an overlap sequence to be ligated to HRV3C-Fc-His-PA was added to the 3' side thereof. Also, an overlap sequence to be ligated to pcDNA3.4 was added to the 3' side of HRV3C-Fc-His-PA. PCR was carried out using KAPA HiFi HotStart ReadyMix (2X) (KAPA BIOSYSTEMS) with a cycle consisting of (i) 95°C for 2 minutes, (ii) 98°C for 20 seconds, (iii) 65°C for 15 seconds, and (iv) 72°C for 30 seconds, and a cycle of steps (ii), (iii), and (iv) was repeated 25 times.

With the use of NEBuilder HiFi DNA Assembly Master Mix (New England Biolabs), amplified Kozak-ALK and HRV3C-Fc-His-PA and Kozak-LTK and HRV3C-Fc-His-PA were ligated to pcDNA3.4 (Thermo Fisher Scientific) cleaved with *Xba*I (New England Biolabs) and *Age*I (New England Biolabs) in accordance with the instructions of the kit. *E. coli* DH5α (Toyobo Co. Ltd.) cells were transformed using the ligated cyclic plasmid and cultured on an LB agar medium containing 100 µg/ml carbenicillin at 37°C overnight.

The appeared colonies were further cultured in an LB liquid medium containing 100 µg/ml carbenicillin at 37°C overnight. Plasmids were purified from the cultured *E. coli* cells using Wizard Plus SV Minipreps DNA Purification System (Promega), nucleotide sequences were determined, and plasmids in which target nucleotide sequence insertion was observed were obtained as ALK 001 and LTK 001. Nucleotide sequence analysis was commissioned to Macrogen Japan.

The amino acid sequence of the ALK 001P protein comprising an ALK translation region encoded by ALK 001 and the DNA sequence encoding the same are represented by SEQ ID NO: 37 and SEQ ID NO: 38, respectively.

The amino acid sequence of the LTK 001P protein comprising an LTK translation region encoded by LTK 001 and the DNA sequence encoding the same are represented by SEQ ID NO: 39 and SEQ ID NO: 40, respectively.

### Preparation of Ligand 001 plasmid

First-phase PCR was performed using the FAM150A artificial gene (Kozak-FAM150A-HRV3C-His; SEQ ID NO: 29) as a template and primers represented by SEQ ID NO: 41 and SEQ ID NO: 42 (synthesis thereof was commissioned to Eurofins Genomics K.K.). Second-phase PCR was then performed using the first-phase PCR product as a template and primers represented by SEQ ID NO: 43 and SEQ ID NO: 42 (synthesis thereof was commissioned to Eurofins Genomics K.K.). As a result of the two-phase PCRs, a DNA molecule comprising an overlap sequence to be ligated to pM-secSUMOstar (LifeSensors) and a DNA sequence encoding an HRV3C protease cleavage sequence added to the 5' side and a stop codon and an overlap sequence to be ligated to pM-secSUMOstar (LifeSensors) added to the 3' side of the sequence encoding a region of 50 to 129 residues of FAM150A were amplified.

PCR was carried out using KAPA HiFi HotStart ReadyMix (2X) (KAPA BIOSYSTEMS) with a cycle consisting of (i) 95°C for 2 minutes, (ii) 98°C for 20 seconds, (iii) 65°C for 15 seconds, and (iv) 72°C for 15 seconds. A cycle of steps (ii), (iii), and (iv) was repeated 25 times in the first-phase PCR and 13 times in the second-phase PCR. With the use of NEBuilder HiFi DNA Assembly Master Mix (New England Biolabs), the second-phase PCR product was ligated to pM-secSUMOstar (LifeSensors) cleaved with *Bsm*BI (New England Biolabs) in accordance with the instructions of the kit. Thus, a DNA sequence (DNA sequence: SEQ ID NO: 44; amino acid sequence: SEQ ID NO: 45) encoding a fusion protein comprising an Ig κ light chain secretory signal, a hexahistidine tag, and SUMOstar ligated tandemly to the 5' side of the inserted DNA sequence was cloned in-frame. *E. coli* DH5α (Toyobo Co. Ltd.) cells were transformed using the ligated cyclic plasmid and cultured on an LB agar medium containing 100 µg/ml carbenicillin at 37°C overnight.

The appeared colonies were further cultured in an LB liquid medium containing 100 µg/ml carbenicillin at 37°C overnight. Plasmids were purified from the cultured *E. coli* cells using Wizard Plus SV Minipreps DNA Purification System (Promega), nucleotide sequences were determined, and a plasmid in which target nucleotide sequence insertion had been observed was obtained as Ligand 001. Nucleotide sequence analysis was commissioned to Macrogen Japan.

The amino acid sequence of the Ligand 001P protein comprising a region of 50 to 129 residues of FAM150A encoded by Ligand 001 and the DNA sequence encoding the same are represented by SEQ ID NO: 46 and SEQ ID NO: 47, respectively.

### Preparation of Ligand 002 plasmid

First-phase PCR was performed using the FAM150B artificial gene (Kozak-FAM150B-HRV3C-His; SEQ ID NO: 30) as a template and primers represented by SEQ ID NO: 48 and SEQ ID NO: 49 (synthesis thereof was commissioned to Eurofins Genomics K.K.). Second-phase PCR was performed using the first-phase PCR product as a template and primers represented by SEQ ID NO: 43 and SEQ ID NO: 49 (synthesis thereof was commissioned to Eurofins Genomics K.K.). Ligand 002 was prepared in the same manner as with the case of Ligand 001.

The amino acid sequence of the Ligand 002P protein comprising a region of 71 to 152 residues of FAM150B encoded by Ligand 002 and the DNA sequence encoding the same are represented by SEQ ID NO: 50 and SEQ ID NO: 51, respectively.

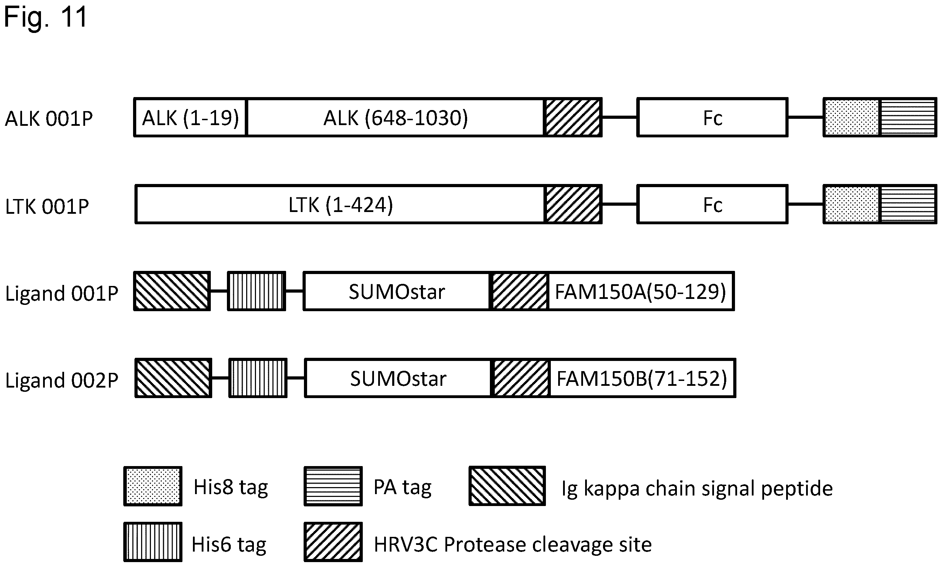

Constitutions of the proteins prepared in this example (ALK 001P, LTK 001P, Ligand 001P, and Ligand 002P) are schematically shown below and in Fig. 11. Numerical values in parentheses indicate amino acid positions.

### ALK 001P:

ALK (1-19) - ALK (648-1030)-HRV3C protease cleavage sequence-Fc sequence containing hinge region-G4S linker-octahistidine tag-PA tag

### LTK 001P:

LTK (1-424)-HRV3C protease cleavage sequence-Fc sequence containing hinge region-G4S linker-octahistidine tag-PA tag

### Ligand 001P:

Ig κ light chain secretory signal-hexahistidine tag-SUMOstar-HRV3C protease cleavage sequence-FAM150A (50-129)

### Ligand 002P:

Ig κ light chain secretory signal-hexahistidine tag-SUMOstar-HRV3C protease cleavage sequence-FAM150B (71-152)

### Example 5: Preparation of ALK 001P, LTK 001P, Ligand 001P, and Ligand 002P

### 5-1. Preparation of ALK 001P

### ALK 001P expression

ALK 001P was expressed using the plasmid ALK 001 prepared in Example 4 and Expi293 Expression System (Thermo Fisher Scientific, mammalian cell expression system).

Expi293 cells cultured in Expi293 Expression Medium were diluted to 2.9 × 10⁶ cells/ml with Expression Medium, the resulting culture solution (2.55 ml) was prepared on a 6-well plate, and 3 µg of ALK 001 was transfected into the Expi293 cells in accordance with the instructions. Culture was carried out in the presence of 8% CO₂ at 125 rpm and 37°C. 23 hours after transfection, Enhancers 1 and 2 included in the kit were added in amounts of 15 µl and 150 µl, respectively, and culture was continued. The culture supernatant was collected via centrifugation 4 days after transfection. The collected supernatant was cryopreserved at - 30°C to -80°C before purification.

### ALK 001P Purification

After the culture supernatant was thawed, the buffer was exchanged with 50 mM Tris-HCl (pH 8.0) and 500 mM NaCl using PD-10 (GE Healthcare Japan). The resultant was mixed with 0.5 ml of Ni Sepharose 6 Fast Flow (GE Healthcare Japan) in a chromatography column, the mixture was stirred at 4°C for 2.5 hours, and ALK 001P was allowed to adsorb to resin. After the resin was washed with 50 mM Tris-HCl (pH 8.0), 500 mM NaCl, and 20 mM imidazole, ALK 001P was eluted with the aid of 50 mM Tris-HCl (pH 8.0), 500 mM NaCl, and 500 mM imidazole.

The resulting eluate was subjected to separation using Superdex 200 increase 10/300 GL (GE Healthcare Japan) connected to AKTA explorer (GE Healthcare Japan) at a flow rate of 0.8 ml/min, and the main peak fraction was collected and used as a sample for SPR-based interaction analysis.

### 5-2. Preparation of LTK 001P

### LTK 001P expression

LTK 001P was expressed using the plasmid LTK 001 prepared in Example 4 and Expi293 Expression System (Thermo Fisher Scientific).

Expi293 cells cultured in Expi293 Expression Medium were diluted to 2.9 × 10⁶ cells/ml with Expression Medium, the resulting culture solution (25.5 ml) was prepared in a 125-ml flask, and 30 µg of LTK 001 was transfected into the Expi293 cells in accordance with the instructions. 21 hours after transfection, Enhancers 1 and 2 included in the kit were added in amounts of 0.15 ml and 1.5 ml, respectively, and culture was continued. Culture was carried out in the presence of 8% CO₂ at 125 rpm and 37°C. The culture supernatant was collected via centrifugation 8 days after transfection. The collected supernatant was cryopreserved at -30°C to -80°C before purification.

### LTK 001P purification

After the culture supernatant was thawed, the supernatant was diluted to 2-fold with 50 mM Tris-HCl (pH 8.0) and 500 mM NaCl, the resultant was mixed with 3 ml of Ni Sepharose 6 Fast Flow (GE Healthcare Japan), the mixture was stirred at 4°C for 1.5 hours, and LTK 001P was allowed to adsorb to resin. Thereafter, the suspension was transferred to an empty chromatography column, the resin was washed with 50 mM Tris-HCl (pH 8.0), 500 mM NaCl, and 20 mM imidazole, and LTK 001P was eluted with the aid of 50 mM Tris-HCl (pH 8.0), 500 mM NaCl, and 500 mM imidazole.

The resulting eluate was subjected to separation using Superdex 200 increase 10/300 GL (GE Healthcare Japan) connected to AKTA explorer (GE Healthcare Japan) at a flow rate of 0.8 ml/min, and the main peak fraction was collected and used as a sample for SPR-based interaction analysis.

### 5-3. Preparation of Ligand 001P and Ligand 002P

### Ligand 001P and Ligand 002P expression

Ligand 001P and Ligand 002P were expressed using Ligand 001 and Ligand 002 prepared in Example 4 and the Expi293 Expression System (Thermo Fisher Scientific).

Expi293 cells cultured in Expi293 Expression Medium were diluted to 2.9 × 10⁶ cells/ml with Expression Medium, the resulting culture solution (25.5 ml) was prepared in a 125-ml flask, and 30 µg of Ligand 001 or Ligand 002 were transfected into the Expi293 cells in accordance with the instructions. 19 hours after transfection, Enhancers 1 and 2 included in the kit were added in amounts of 0.15 ml and 1.5 ml, respectively, and culture was continued. Culture was carried out under 8% CO₂ at 125 rpm and at 37°C. The culture supernatant was collected via centrifugation 4 days after transfection.

### Ligand 001P and Ligand 002P purification

The culture supernatant was diluted to 2-fold with 50 mM Tris-HCl (pH 8.0) and 500 mM NaCl, the resultant was mixed with 1 ml of cOmplete^{™} His-Tag Purification Resin (Roche Diagnostics), the mixture was stirred at 4°C for 1.5 hours, and Ligand 001P or Ligand 002P was allowed to adsorb to resin. Thereafter, the suspension was transferred to an empty chromatography column, the resin was washed with 50 mM Tris-HCl (pH 8.0), 500 mM NaCl, and 20 mM imidazole, and Ligand 001P or Ligand 002P was eluted with the aid of 50 mM Tris-HCl (pH 8.0), 500 mM NaCl, and 500 mM imidazole.

The resulting eluate was subjected to separation using Superdex 75 10/300 GL (GE Healthcare Japan) connected to AKTA explorer (GE Healthcare Japan) at a flow rate of 0.7 ml/min, and the main peak fraction was collected and used as a sample for SPR-based interaction analysis.

### Example 6: Verification of binding of ALK 001P and LTK 001P to Ligand 001P and Ligand 002P via surface plasmon resonance analysis

Whether or not ALK 001P and LTK 001P obtained in Example 5 had bound to Ligand 001P and Ligand 002P was examined using Biacore 8K instrument (GE Healthcare Japan) by the surface plasmon resonance (SPR) technique.

### Immobilization of ALK 001P and LTK 001P on sensor chip

ALK 001P contained at 4.8 µg/ml (calculated based on the molecular weight without sugar chain) and LTK 001P contained at 10 µg/ml (calculated based on the molecular weight without sugar chain) in HBS-P+ (10 mM HEPES (pH 7.4), 150 mM NaCl, 0.05% (v/v) Surfactant P20) were introduced at a flow rate of 10 µl/min for 90 seconds and immobilized on the surface coated with Protein A of Series S Sensor Chip Protein A (GE Healthcare Japan). The immobilized amount of ALK001P was 830 to 990 RU, and the immobilized amount of LTK 001P was 670 to 790 RU.

### Acquisition of bond dissociation curve

Six concentrations of Ligand 001P at 2-fold dilution with HBS-P+ from 19.8 nM and six concentrations thereof at 2-fold dilution with HBS-P+ from 297 nM were prepared separately. Six concentrations of Ligand 002P at 2-fold dilution with HBS-P+ from 19.9 nM was also prepared.

Ligand 001P and Ligand 002P at various concentrations and HBS-P+ (concentration 0) were introduced on the surface of the immobilized ALK 001P or LTK 001P and on the untreated surface coated with Protein A (baseline) at a flow rate of 30 µl/min for 120 seconds, in the combinations as shown in Table 2, and the binding curves were recorded. Subsequently, HBS-P+ was introduced on the surface of the immobilized ALK 001P or LTK 001P and on the untreated surface coated with Protein A (baseline) at a flow rate of 30 µl/min for 600 seconds, and the dissociation curves were recorded.

ALK 001P and LTK 001P were dissociated (i.e., regenerated) from Protein A coating the sensor chip surface (i.e., regeneration) by injecting 10 mM Glycine-HCl (pH 1.6) at a flow rate of 30 µl/min for 30 seconds. The bond dissociation curve modified by subtracting the baseline value was prepared using Biacore8K Evaluation Software (GE Healthcare Japan).

**[Table 2]**

| | ALK 001P | LTK 001P |
|---|---|---|
| Ligand 001P | 6 concentration levels from 297 nM | 6 concentration levels from 19.8 nM |
| Ligand 002P | 6 concentration levels from 19.9 nM | 6 concentration levels from 19.9 nM |

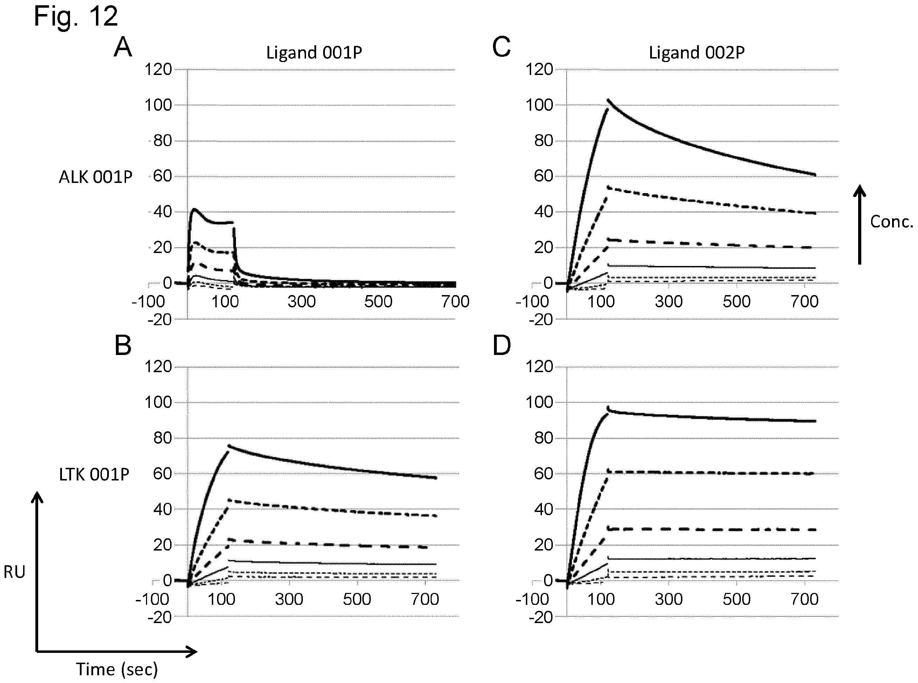

Fig. 12 shows the results of verification of binding of ALK 001P and LTK 001P to Ligand 001P and Ligand 002P via surface plasmon resonance analysis. Ligand 001P shows a higher binding ability to LTK 001P than to ALK 001P. Ligand 002P is slowly detached from ALK 001P and LTK 001P, indicating a high binding ability thereto.

### Example 7: Preparation of FAM150A truncated CAR-expressing plasmid (CAR 007; FAM150ATr-28z) and FAM150B truncated CAR-expressing plasmid (CAR 008; FAM150BTr-28z)

With the use of CAR 002 or CAR 003 prepared in Example 1 as a template, FAM150A truncated (CAR 007) and FAM150B truncated (CAR 008) CAR-expressing plasmids were prepared via inverse-PCR.

As PCR primers used for inverse-PCR, primers represented by SEQ ID NO: 52 and SEQ ID NO: 53 (for CAR 002 linearization) and primers represented by SEQ ID NO: 54 and SEQ ID NO: 55 (for CAR 003 linearization) were designed and synthesized (synthesis was commissioned to Eurofins Genomics K.K.).

CAR 002 or CAR 003 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times.

After the PCR reaction, an aliquot of the sample was separated via 1% agarose gel electrophoresis, amplification of a linear plasmid of the size of interest was confirmed, the remaining sample after PCR was treated with *Dpn*I in accordance with the instructions of the kit, and methylated template plasmid CAR 002 or CAR 003 was cleaved and removed. Thereafter, the linear plasmid was phosphorylated with T4 Polynucleotide Kinase included in the kit and self-ligated to form a cyclic plasmid. Thereafter, *E. coli* DH5α (Toyobo Co. Ltd.) cells were transformed using the cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence deletion was observed were obtained as a FAM150A truncated CAR-expressing plasmid (CAR 007; FAM150ATr-28z) and a FAM150B truncated CAR-expressing plasmid (CAR 008; FAM150BTr-28z).

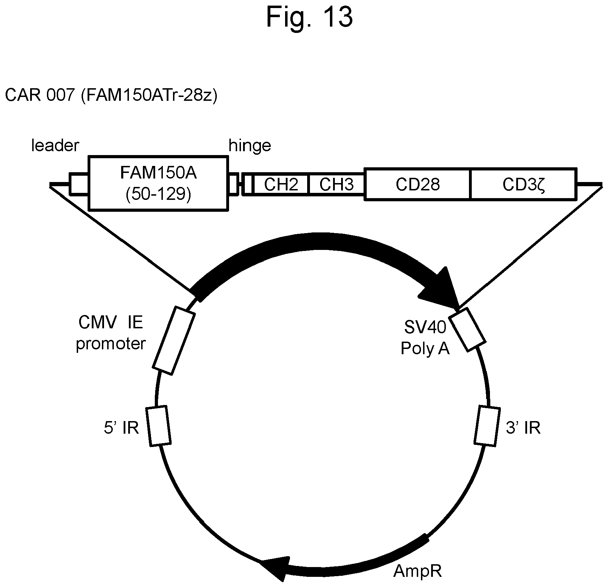

Fig. 13 shows a vector map of CAR 007 (FAM150ATr-28z) and the DNA sequence of a translation region of CAR 007 is represented by SEQ ID NO: 56.

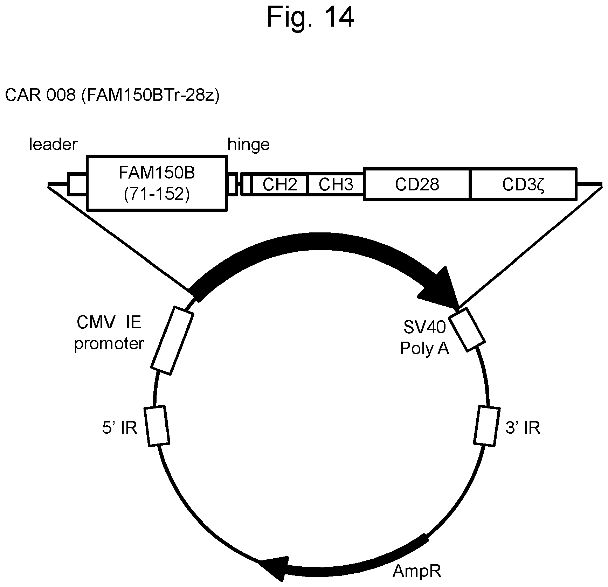

Fig. 14 shows a vector map of CAR 008 (FAM150BTr-28z) and the DNA sequence of a translation region of CAR 008 is represented by SEQ ID NO: 57.

### Example 8: Antitumor activity of FAM150B truncated CD28-type CAR-T

In accordance with the method of CAR-T cell culture and proliferation in Example 2, CAR-T cells were prepared by introducing genes into PBMCs derived from 2 healthy adult donors using the CAR-expressing plasmids CAR 003, CAR 008, or CAR 006.
CAR-T cells obtained by electrical introduction into PBMCs using CAR 003, CAR 008, or CAR 006 and T cell culture and proliferation are described herein as follows.
CAR-T 003: FAM150B CD28-type CAR-T (FAM150B-28z CAR-T)
CAR-T 006: CD19 scFv CD28-type CAR-T(CD19-28z CAR-T)
CAR-T 008: FAM150B truncated CD28-type CAR-T (FAM150BTr-28z CAR-T)

Table 3 shows CAR expression rates in the CAR-T cells obtained by introduction of CAR-expressing plasmids into PBMCs derived from 2 healthy adult donors and culture.

The amino acid sequence of CAR 003 and the nucleotide sequence encoding the same are represented by SEQ ID NO: 14 and SEQ ID NO: 10, respectively.

The amino acid sequence of CAR 008 and the nucleotide sequence encoding the same are represented by SEQ ID NO: 58 and SEQ ID NO: 57, respectively.

**[Table 3]**

| Donor | Plasmid | CAR-T | Expression rate |
|---|---|---|---|
| Donor-1 | CAR 003 | CAR-T 003 | 23.6 |
| | CAR 008 | CAR-T 008 | 39.0 |
| | CAR 006 | CAR-T 006 | 48.9 |
| Donor-2 | CAR 003 | CAR-T 003 | 20.8 |
| | CAR 008 | CAR-T 008 | 27.8 |
| | CAR 006 | CAR-T 006 | 27.5 |

### Measurement of antitumor activity of CAR-T

In order to measure antitumor activity of CAR-T 003, CAR-T 008, and CAR-T 006 obtained above, co-culture with solid tumor cells was conducted. In this example, SH-SY5Y and IMR-32 were used as target tumor cells, and activity evaluation was performed in accordance with the method of Example 3. In this example, in addition, the number of tumor cells measured using a flow cytometer when co-culture was initiated was designated to be 1, and the relative ratio of the number of GD2-positive tumor cells when co-culture was terminated is plotted to prepare the tumor cell proliferation curves.

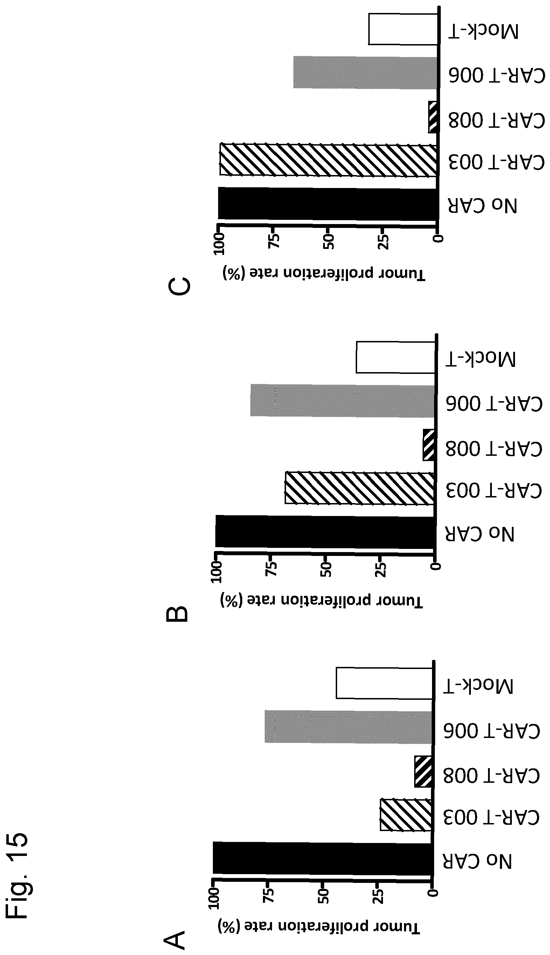

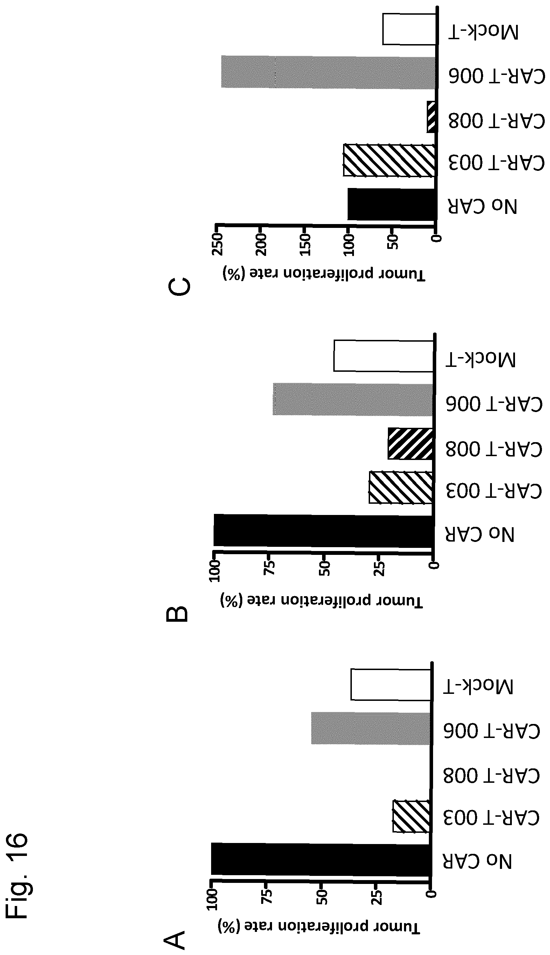

Fig. 15 and Fig. 16 each show antitumor activity of CAR-T 003, CAR-T 008, and CAR-T 006 or mock-T cells derived from Donor-1 against SH-SY5Y or IMR-32. As a result, the effects of CAR-T 003 and CAR-T 008 to kill SH-SY5Y and IMR-32 were verified. In particular, the effects of CAR-T 008 were higher than those of CAR-T 003 and also high at E:T of 1:1.

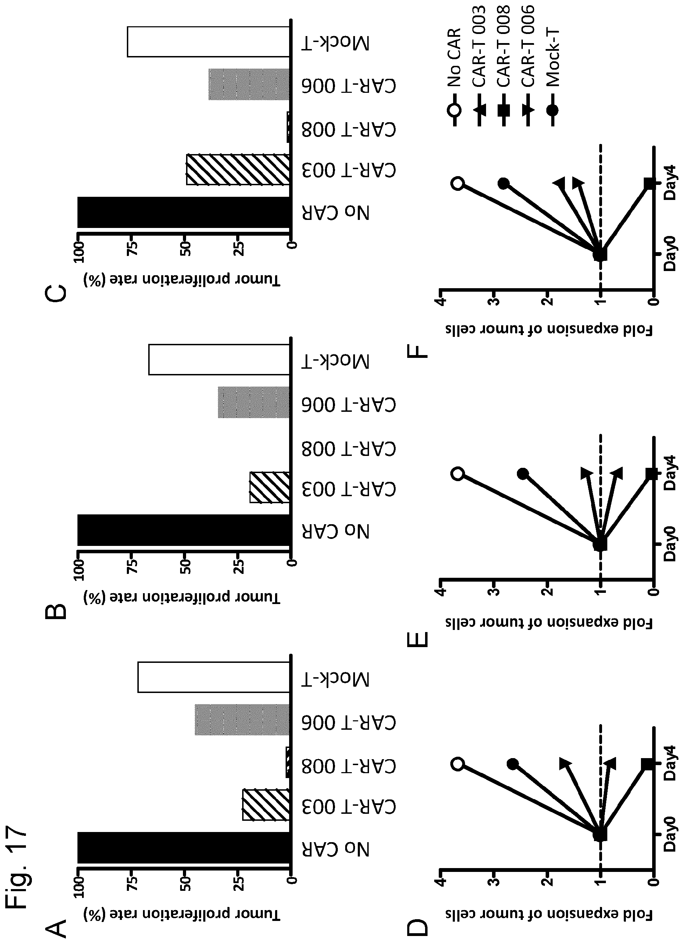

Fig. 17 shows antitumor activity of CAR-T 003, CAR-T 008, and CAR-T 006 or mock-T cells derived from Donor-2 against SH-SY5Y and the tumor cell proliferation curves. As a result, the effects of CAR-T 003 and CAR-T 008 to kill SH-SY5Y were verified. In particular, the effects of CAR-T 008 were higher than those of CAR-T 003 and also high at E:T of 1:1.

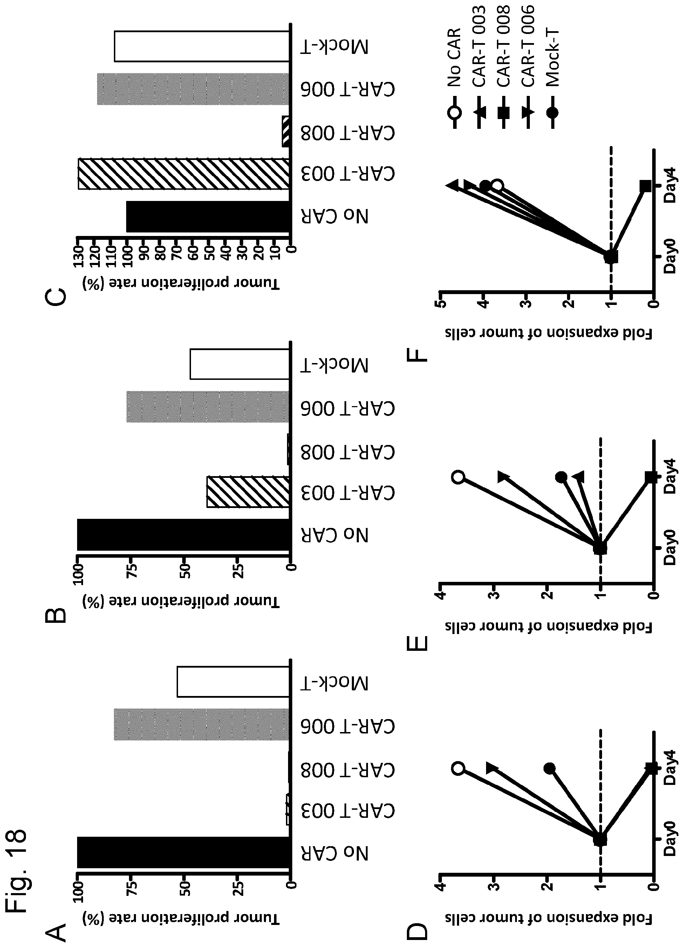

Fig. 18 shows antitumor activity of CAR-T 003, CAR-T 008, and CAR-T 006 or mock-T cells derived from Donor-2 against IMR-32 and the tumor cell proliferation curves. As a result, the effects of CAR-T 003 and CAR-T 008 to kill IMR-32 were verified. In particular, the effects of CAR-T 008 were higher than those of CAR-T 003 and also high at E:T of 1:1.

This example indicates that antitumor activity of FAM150B truncated CAR-T (CAR-T 008) is higher than that of FAM150B full-length CAR-T (CAR-T 003).

### Example 9: Preparation of Ligand 003 plasmid

In order to increase the expression level, a DNA sequence encoding Ligand 002P was introduced into pcDNA3.4.

With the use of Ligand 002 as a template and primers represented by SEQ ID NO: 59 and SEQ ID NO: 60 (synthesis thereof was commissioned to Eurofins Genomics K.K.), a region from the sequence encoding the Ig κ light chain secretory signal sequence to a sequence encoding a region of 71 to 152 residues of FAM150B (Ig kappa-SUMOstar-FAM150B (71-152)) was amplified by PCR. As a result of PCR, a DNA sequence comprising an overlap sequence to be ligated to pcDNA3.4 (Thermo Fisher Scientific) added to the 5' side and a stop codon and an overlap sequence to be ligated to pcDNA3.4 (Thermo Fisher Scientific) added to the 3' side of Ig kappa-SUMOstar-FAM150B (71-152) was amplified.

PCR was carried out using KAPA HiFi HotStart ReadyMix (2X) (KAPA BIOSYSTEMS) with a cycle consisting of (i) 95°C for 2 minutes, (ii) 98°C for 20 seconds, (iii) 65°C for 15 seconds, and (iv) 72°C for 15 seconds, and a cycle of steps (ii), (iii), and (iv) was repeated 25 times. With the use of NEBuilder HiFi DNA Assembly Master Mix (New England Biolabs), the amplified sequence was ligated to pcDNA3.4 (Thermo Fisher Scientific) cleaved with *Xba*I (New England Biolabs) and *Age*I (New England Biolabs) in accordance with the instructions of the kit. *E. coli* DH5α (Toyobo Co. Ltd.) cells were transformed using the ligated cyclic plasmid and cultured on an LB agar medium containing 100 µg/ml carbenicillin at 37°C overnight.

The appeared colonies were further cultured in a MMI liquid medium (20 mM Tris-HCl (pH 7.2), 1.25% (w/v) tryptone, 2.5% (w/v) yeast extract, 0.85% (w/v) NaCl, 0.4% (v/v) glycerol) containing 100 µg/ml carbenicillin at 37°C for about 9.5 hours. Plasmids were purified from the cultured *E. coli* cells using Wizard Plus SV Minipreps DNA Purification System (Promega), nucleotide sequences were determined, and a plasmid in which target nucleotide sequence insertion was observed was obtained as Ligand 003. Nucleotide sequence analysis was commissioned to Macrogen Japan.

The amino acid sequence of the Ligand 003P protein containing 71 to 152 residues of FAM150B encoded by Ligand 003 and the DNA sequence encoding the same are the same as those of Ligand 002P (amino acid sequence: SEQ ID NO: 50, DNA sequence: SEQ ID NO: 51).

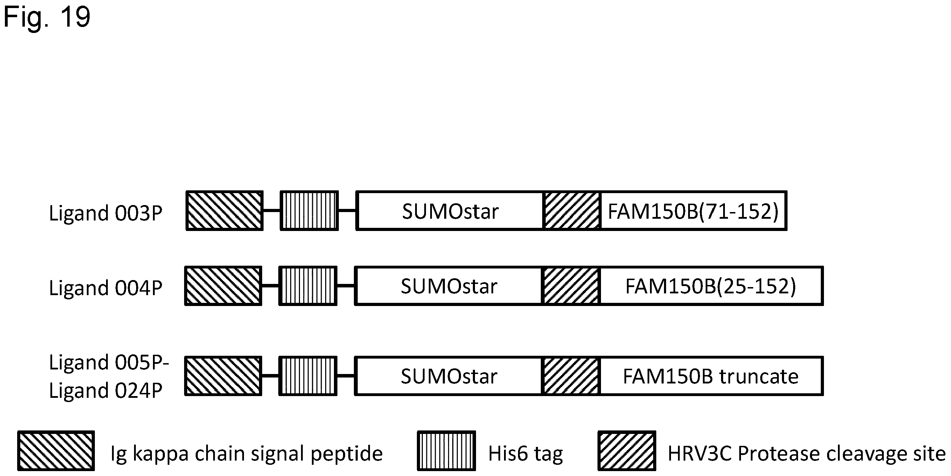

The constitution of Ligand 003P is schematically shown below and in Fig. 19. Numerical values in parentheses indicate amino acid positions.

### Ligand 003P:

Ig Kappa light chain secretory signal-hexahistidine tag-SUMOstar-HRV3C protease cleavage sequence -FAM150B (71-152)

### Example 10: Preparation of expression plasmids for various FAM150B truncated forms Preparation of Ligand 004 plasmid

In order to prepare expression plasmids for various FAM150B truncated forms, Ligand 004 to be used as a template was prepared. First-phase PCR was carried out using the FAM150B artificial gene obtained in Example 4 (Kozak-FAM150B-HRV3C-His; SEQ ID NO: 30) as a template and primers represented by SEQ ID NO: 61 and SEQ ID NO: 49 (synthesis thereof was commissioned to Eurofins Genomics K.K.). Second-phase PCR was carried out using the first-phase PCR product as a template and primers represented by SEQ ID NO: 43 and SEQ ID NO: 49 (synthesis thereof was commissioned to Eurofins Genomics K.K.). Thereafter, in the same manner as in the case of Ligand 001, Ligand 004 was prepared.

The amino acid sequence of the Ligand 004P protein containing 25 to 152 residues of FAM150B encoded by Ligand 004 and the DNA sequence encoding the same are represented by SEQ ID NO: 62 and SEQ ID NO: 63, respectively.

### Preparation of Ligand 005 to Ligand 022 plasmids

First-phase PCR was carried out using the combination of the template with the primers shown in Table 4 below (synthesis thereof was commissioned to Eurofins Genomics K.K.) to amplify DNA sequences encoding various FAM150B truncated forms. As a result of PCR, DNA sequences comprising an overlap sequence to be ligated to Ig kappa-SUMOstar-HRV3C added to the 5' side and a stop codon and an overlap sequence to be ligated to pcDNA3.4 (Thermo Fisher Scientific) added to the 3' side of DNA sequences encoding various FAM150B truncated forms were amplified.

At the same time, first-phase PCR was carried out using Ligand 004 as a template and primers represented by SEQ ID NO: 59 and SEQ ID NO: 84 (synthesis thereof was commissioned to Eurofins Genomics K.K.) to amplify a region from the DNA sequence encoding the Ig kappa light chain secretory signal sequence to the DNA sequence encoding the HRV3C protease cleavage sequence (Ig kappa-SUMOstar-HRV3C) by PCR. As a result of PCR, a DNA sequence comprising an overlap sequence to be ligated to pcDNA3.4 (Thermo Fisher Scientific) and a Kozak sequence added to the 5' side was amplified.

Subsequently, the first-phase PCR products comprising DNA sequences encoding various FAM150B truncated forms were mixed with the first-phase PCR product comprising Ig kappa-SUMOstar-HRV3C. Second-phase PCR was then carried out using the primers represented by SEQ ID NO: 59 and SEQ ID NO: 60 (synthesis thereof was commissioned to Eurofins Genomics K.K.) to amplify a DNA sequence comprising both the PCR products ligated to each other.

PCR was carried out using KAPA HiFi HotStart ReadyMix(2X) (KAPA BIOSYSTEMS) with a cycle consisting of (i) 95°C for 2 minutes, (ii) 98°C for 20 seconds, (iii) 65°C for 15 seconds, and (iv) 72°C for 15 seconds. In the first-phase PCR, a cycle of steps (ii), (iii), and (iv) was repeated 25 times, and in the second-phase PCR it was repeated 13 times. However, the first-phase PCR for amplifying a DNA fragment encoding a FAM150B truncated form when preparing Ligand 015 and Ligand 016 was carried out using KAPA HiFi HotStart ReadyMix(2X) (KAPA BIOSYSTEMS) with a cycle consisting of (i) 95°C for 2 minutes, (ii) 98°C for 20 seconds, (iii) 70.6°C for 15 seconds, and (iv) 72°C for 15 seconds, and a cycle of steps (ii), (iii), and (iv) was repeated 25 times.

With the use of NEBuilder HiFi DNA Assembly Master Mix (New England Biolabs), the second-phase PCR product was ligated to pcDNA3.4 (Thermo Fisher Scientific) cleaved with *Xba*I (New England Biolabs) and *Age*I (New England Biolabs) in accordance with the instructions of the kit. *E. coli* DH5α (Toyobo Co. Ltd.) cells were transformed using the ligated cyclic plasmid and cultured on an LB agar medium containing 100 µg/ml carbenicillin at 37°C overnight.

The appeared colonies were further cultured in an LB liquid medium containing 100 µg/ml carbenicillin at 37°C overnight. Plasmids were purified from the cultured *E. coli* cells using FastGene Plasmid Mini Kit (Nippon Genetics Co., Ltd.), nucleotide sequences were determined, and plasmids in which target nucleotide sequence insertion was observed were obtained as Ligand 005 to Ligand 022. Nucleotide sequence analysis was commissioned to Macrogen Japan.

### Preparation of Ligand 023 and Ligand 024 plasmids

A DNA sequence encoding a FAM150B truncated form was amplified via PCR using the template in combination with the primers shown in Table 4 below (synthesis thereof was commissioned to Eurofins Genomics K.K.). As a result of PCR, a DNA sequence, comprising an overlap sequence to be ligated to pcDNA3.4 (Thermo Fisher Scientific) and a Kozak sequence added to the 5' side and a stop codon and an overlap sequence to be ligated to pcDNA3.4 (Thermo Fisher Scientific) added to the 3' side of the DNA sequence encoding the FAM150B truncated form, was amplified.

PCR was carried out using KAPA HiFi HotStart ReadyMix(2X) (KAPA BIOSYSTEMS) with a cycle consisting of (i) 95°C for 2 minutes, (ii) 98°C for 20 seconds, (iii) 65°C for 15 seconds, and (iv) 72°C for 15 seconds, and a cycle of steps (ii), (iii), and (iv) was repeated 25 times. With the use of NEBuilder HiFi DNA Assembly Master Mix (New England Biolabs), the amplified DNA fragment was ligated to pcDNA3.4 (Thermo Fisher Scientific) cleaved with *Xba*I (New England Biolabs) and *Age*I (New England Biolabs) in accordance with the instructions of the kit. *E. coli* DH5α (Toyobo Co. Ltd.) cells were transformed using the ligated cyclic plasmid and cultured on an LB agar medium containing 100 µg/ml carbenicillin at 37°C overnight.

The appeared colonies were further cultured in an LB liquid medium containing 100 µg/ml carbenicillin at 37°C overnight. Plasmids were purified from the cultured *E. coli* cells using FastGene Plasmid Mini Kit (Nippon Genetics Co., Ltd.), nucleotide sequences were determined, and plasmids in which target nucleotide sequence insertion was observed were obtained as Ligand 023 and Ligand 024. Nucleotide sequence analysis was commissioned to Macrogen Japan.

Table 4 shows the names of plasmids to express various FAM150B truncated forms, terminal amino acids of FAM150B truncated forms, primers (Fw and Rev) used for first-phase PCR when preparing plasmids to express FAM150B truncated forms, and templates.

**[Table 4]**

| Ligand | Terminal amino acid | | First-phase PCR | | |
|---|---|---|---|---|---|
| | N | C | Fw primer | Rev primer | Template |
| Ligand 005 | A67 | Q152 | SEQ ID NO: 64 | SEQ ID NO: 60 | Ligand 004 |
| Ligand 006 | G69 | Q152 | SEQ ID NO: 65 | SEQ ID NO: 60 | Ligand 004 |
| Ligand 007 | A73 | Q152 | SEQ ID NO: 66 | SEQ ID NO: 60 | Ligand 004 |
| Ligand 008 | G75 | Q152 | SEQ ID NO: 67 | SEQ ID NO: 60 | Ligand 004 |
| Ligand 009 | G77 | Q152 | SEQ ID NO: 68 | SEQ ID NO: 60 | Ligand 004 |
| Ligand 010 | S79 | Q152 | SEQ ID NO: 69 | SEQ ID NO: 60 | Ligand 004 |
| Ligand 011 | E81 | Q152 | SEQ ID NO: 70 | SEQ ID NO: 60 | Ligand 004 |
| Ligand 012 | R83 | Q152 | SEQ ID NO: 71 | SEQ ID NO: 60 | Ligand 004 |
| Ligand 013 | E85 | Q152 | SEQ ID NO: 72 | SEQ ID NO: 60 | Ligand 004 |
| Ligand 014 | V87 | Q152 | SEQ ID NO: 73 | SEQ ID NO: 60 | Ligand 004 |
| Ligand 015 | R89 | Q152 | SEQ ID NO: 74 | SEQ ID NO: 60 | Ligand 004 |
| Ligand 016 | L91 | Q152 | SEQ ID NO: 75 | SEQ ID NO: 60 | Ligand 004 |
| Ligand 017 | M93 | Q152 | SEQ ID NO: 76 | SEQ ID NO: 60 | Ligand 004 |
| Ligand 018 | D95 | Q152 | SEQ ID NO: 77 | SEQ ID NO: 60 | Ligand 004 |
| Ligand 019 | F97 | Q152 | SEQ ID NO: 78 | SEQ ID NO: 60 | Ligand 004 |
| Ligand 020 | K99 | Q152 | SEQ ID NO: 79 | SEQ ID NO: 60 | Ligand 004 |
| Ligand 021 | L101 | Q152 | SEQ ID NO: 80 | SEQ ID NO: 60 | Ligand 004 |
| Ligand 022 | G103 | Q152 | SEQ ID NO: 81 | SEQ ID NO: 60 | Ligand 004 |
| Ligand 023 | A71 | D150 | SEQ ID NO: 59 | SEQ ID NO: 82 | Ligand 002 |
| Ligand 024 | A71 | M148 | SEQ ID NO: 59 | SEQ ID NO: 83 | Ligand 002 |

SEQ ID NO: 85 shows the amino acid sequence of Ligand 005P protein containing amino acids 67 to 152 of FAM150B encoded by Ligand 005, and SEQ ID NO: 86 shows the DNA sequence encoding the same.

SEQ ID NO: 87 shows the amino acid sequence of Ligand 006P protein containing amino acids 69 to 152 of FAM150B encoded by Ligand 006, and SEQ ID NO: 88 shows the DNA sequence encoding the same.

SEQ ID NO: 89 shows the amino acid sequence of Ligand 007P protein containing amino acids 73 to 152 of FAM150B encoded by Ligand 007, and SEQ ID NO: 90 shows the DNA sequence encoding the same.

SEQ ID NO: 91 shows the amino acid sequence of Ligand 008P protein containing amino acids 75 to 152 of FAM150B encoded by Ligand 008, and SEQ ID NO: 92 shows the DNA sequence encoding the same.

SEQ ID NO: 93 shows the amino acid sequence of Ligand 009P protein containing amino acids 77 to 152 of FAM150B encoded by Ligand 009, and SEQ ID NO: 94 shows the DNA sequence encoding the same.

SEQ ID NO: 95 shows the amino acid sequence of Ligand 010P protein containing amino acids 79 to 152 of FAM150B encoded by Ligand 010, and SEQ ID NO: 96 shows the DNA sequence encoding the same.

SEQ ID NO: 97 shows the amino acid sequence of Ligand 011P protein containing amino acids 81 to 152 of FAM150B encoded by Ligand 011, and SEQ ID NO: 98 shows the DNA sequence encoding the same.

SEQ ID NO: 99 shows the amino acid sequence of Ligand 012P protein containing amino acids 83 to 152 of FAM150B encoded by Ligand 012, and SEQ ID NO: 100 shows the DNA sequence encoding the same.

SEQ ID NO: 101 shows the amino acid sequence of Ligand 013P protein containing amino acids 85 to 152 of FAM150B encoded by Ligand 013, and SEQ ID NO: 102 shows the DNA sequence encoding the same.

SEQ ID NO: 103 shows the amino acid sequence of Ligand 014P protein containing amino acids 87 to 152 of FAM150B encoded by Ligand 014, and SEQ ID NO: 104 shows the DNA sequence encoding the same.

SEQ ID NO: 105 shows the amino acid sequence of Ligand 015P protein containing amino acids 89 to 152 of FAM150B encoded by Ligand 015, and SEQ ID NO: 106 shows the DNA sequence encoding the same.

SEQ ID NO: 107 shows the amino acid sequence of Ligand 016P protein containing amino acids 91 to 152 of FAM150B encoded by Ligand 016 and SEQ ID NO: 108 shows the DNA sequence encoding the same.

SEQ ID NO: 109 shows the amino acid sequence of Ligand 017P protein containing amino acids 93 to 152 of FAM150B encoded by Ligand 017, and SEQ ID NO: 110 shows the DNA sequence encoding the same.

SEQ ID NO: 111 shows the amino acid sequence of Ligand 018P protein containing amino acids 95 to 152 of FAM150B encoded by Ligand 018, and SEQ ID NO: 112 shows the DNA sequence encoding the same.

SEQ ID NO: 113 shows the amino acid sequence of Ligand 019P protein containing amino acids 97 to 152 of FAM150B encoded by Ligand 019, and SEQ ID NO: 114 shows the DNA sequence encoding the same.

SEQ ID NO: 115 shows the amino acid sequence of Ligand 020P protein containing amino acids 99 to 152 of FAM150B encoded by Ligand 020, and SEQ ID NO: 116 shows the DNA sequence encoding the same.

SEQ ID NO: 117 shows the amino acid sequence of Ligand 021P protein containing amino acids 101 to 152 of FAM150B encoded by Ligand 021, and SEQ ID NO: 118 shows the DNA sequence encoding the same.

SEQ ID NO: 119 shows the amino acid sequence of Ligand 022P protein containing amino acids 103 to 152 of FAM150B encoded by Ligand 022, and SEQ ID NO: 120 shows the DNA sequence encoding the same.

SEQ ID NO: 121 shows the amino acid sequence of Ligand 023P protein containing amino acids 71 to 150 of FAM150B encoded by Ligand 023, and SEQ ID NO: 122 shows the DNA sequence encoding the same.

SEQ ID NO: 123 shows the amino acid sequence of Ligand 024P protein containing amino acids 71 to 148 of FAM150B encoded by Ligand 024, and SEQ ID NO: 124 shows the DNA sequence encoding the same.

Constitutions of proteins prepared in this example are schematically shown below and in Fig. 19. Numerical values in parentheses indicate amino acid positions.

### Ligand 004P:

Ig Kappa light chain secretory signal-hexahistidine tag-SUMOstar-HRV3C protease cleavage sequence-FAM150B (25-152)

### Ligand 005P:

Ig Kappa light chain secretory signal-hexahistidine tag-SUMOstar-HRV3C protease cleavage sequence-FAM150B (67-152)

### Ligand 006P:

Ig Kappa light chain secretory signal-hexahistidine tag-SUMOstar-HRV3C protease cleavage sequence-FAM150B (69-152)

### Ligand 007P

Ig Kappa light chain secretory signal-hexahistidine tag-SUMOstar-HRV3C protease cleavage sequence-FAM150B (73-152)

### Ligand 008P:

Ig Kappa light chain secretory signal-hexahistidine tag-SUMOstar-HRV3C protease cleavage sequence-FAM150B (75-152)

### Ligand 009P:

Ig Kappa light chain secretory signal-hexahistidine tag-SUMOstar-HRV3C protease cleavage sequence-FAM150B (77-152)

### Ligand 010P:

Ig Kappa light chain secretory signal-hexahistidine tag-SUMOstar-HRV3C protease cleavage sequence-FAM150B (79-152)

### Ligand 011P:

Ig Kappa light chain secretory signal-hexahistidine tag-SUMOstar-HRV3C protease cleavage sequence-FAM150B (81-152)

### Ligand 012P:

Ig Kappa light chain secretory signal-hexahistidine tag-SUMOstar-HRV3C protease cleavage sequence-FAM150B (83-152)

### Ligand 013P:

Ig Kappa light chain secretory signal-hexahistidine tag-SUMOstar-HRV3C protease cleavage sequence-FAM150B (85-152)

### Ligand 014P:

Ig Kappa light chain secretory signal-hexahistidine tag-SUMOstar-HRV3C protease cleavage sequence-FAM150B (87-152)

### Ligand 015P:

Ig Kappa light chain secretory signal-hexahistidine tag-SUMOstar-HRV3C protease cleavage sequence-FAM150B (89-152)

### Ligand 016P:

Ig Kappa light chain secretory signal-hexahistidine tag-SUMOstar-HRV3C protease cleavage sequence-FAM150B (91-152)

### Ligand 017P:

Ig Kappa light chain secretory signal-hexahistidine tag-SUMOstar-HRV3C protease cleavage sequence-FAM150B (93-152)

### Ligand 018P:

Ig Kappa light chain secretory signal-hexahistidine tag-SUMOstar-HRV3C protease cleavage sequence-FAM150B (95-152)

### Ligand 019P:

Ig Kappa light chain secretory signal-hexahistidine tag-SUMOstar-HRV3C protease cleavage sequence-FAM150B (97-152)

### Ligand 020P:

Ig Kappa light chain secretory signal-hexahistidine tag-SUMOstar-HRV3C protease cleavage sequence-FAM150B (99-152)

### Ligand 021P:

Ig Kappa light chain secretory signal-hexahistidine tag-SUMOstar-HRV3C protease cleavage sequence-FAM150B (101-152)

### Ligand 022P:

Ig Kappa light chain secretory signal-hexahistidine tag-SUMOstar-HRV3C protease cleavage sequence-FAM150B (103-152)

### Ligand 023P:

Ig Kappa light chain secretory signal-hexahistidine tag-SUMOstar-HRV3C protease cleavage sequence-FAM150B (71-150)

### Ligand 024P:

Ig Kappa light chain secretory signal-hexahistidine tag-SUMOstar-HRV3C protease cleavage sequence-FAM150B (71-148)

### Example 11: Measurement of binding activity to ALK 001P and LTK 001P by surface plasmon resonance analysis

### Expression of FAM150B truncated form

Various FAM150B truncated forms were expressed using Ligand 003 and Ligand 005 to Ligand 024 plasmids and Expi293 Expression System (Thermo Fisher Scientific). Expi293 cells cultured in Expi293 Expression Medium were diluted to 2.9 × 10⁶ cells/ml with Expression Medium, the resulting culture solution (25.5 ml) was introduced into a 125-ml flask, and 30 µg each of Ligand 003 and Ligand 005 to Ligand 024 were transfected into the Expi293 cells in accordance with the instructions.

18 to 20 hours after transfection, Enhancers 1 and 2 included in the kit were added in amounts of 0.15 ml and 1.5 ml, respectively, and culture was continued. Culture was carried out in the presence of 8% CO₂ at 125 rpm and 37°C. The culture supernatant was collected via centrifugation 4 days after transfection. The collected supernatant was cryopreserved at - 30°C to -80°C before purification.

### Purification of FAM150B truncated form

After the culture supernatant was thawed, 1.5 ml of 1 M Tris-HCl (pH 8.0) and 2 ml of a 50% suspension of Ni Sepharose 6 FastFlow (GE Healthcare Japan) in DW were added, the mixture was stirred at 4°C for 10 minutes, and Ligand 003P and Ligand 005P to Ligand 024P were allowed to adsorb to resin. Thereafter, the suspension was transferred to an empty chromatography column, the resin was washed with 50 mM Tris-HCl (pH 8.0), 500 mM NaCl, and 20 mM imidazole, and Ligand 003P and Ligand 005P to Ligand 024P were eluted with the aid of 50 mM Tris-HCl (pH 8.0), 500 mM NaCl, and 500 mM imidazole.

The resulting eluate was subjected to separation using Superdex 75 10/300 (GE Healthcare Japan) connected to AKTA explorer (GE Healthcare Japan) at a flow rate of 0.7 ml/min, and the main peak fraction was collected and used as a sample for SPR-based interaction analysis.

### Verification of binding of ALK 001P or LTK 001P to FAM150B truncated form

A region of amino acids 71 to 152 of FAM150B was found to bind to ALK 001P and LTK 001P. Thus, various FAM150B truncated forms were examined using Biacore 8K instrument (GE Healthcare Japan) by the surface plasmon resonance (SPR) technique to identify border amino acid residue that would exhibit changes in intensity to bind to ALK 001P and LTK 001P.

### Immobilization of ALK 001P and LTK 001P on sensor chip

ALK 001P contained at 7.5 µg/ml (calculated based on the molecular weight without sugar chain) and LTK 001P contained at 10 µg/ml (calculated based on the molecular weight without sugar chain) in HBS-P+ were introduced at a flow rate of 10 µl/min for 60 seconds and immobilized on the surface coated with Protein A of Series S Sensor Chip Protein A (GE Healthcare Japan). The amount of ALK001P immobilized was 590 to 670 RU, and the amount of LTK 001P immobilized was 520 to 640 RU.

### Acquisition of bond dissociation curve

Six concentrations of Ligand 003P and Ligand 005P to Ligand 024P at 2-fold dilution with HBS-P+ each from 20 nM were prepared. Ligand 003P and Ligand 005P to Ligand 024P at various concentrations and HBS-P+ (concentration 0) were introduced on the surface of the immobilized ALK 001P or LTK 001P and on the untreated surface coated with Protein A (baseline) at a flow rate of 30 µl/min for 360 seconds, and the binding curves were recorded.

Subsequently, HBS-P+ was introduced on the surface of the immobilized ALK 001P or LTK 001P and on the untreated surface coated with Protein A (baseline) at a flow rate of 30 µl/min for 600 seconds, and the dissociation curves were recorded.

ALK 001P and LTK 001P were dissociated from Protein A coating the sensor chip surface (i.e., regeneration) by introduction of 10 mM Glycine-HCl (pH 1.6) at a flow rate of 30 µl/min for 30 seconds. The bond dissociation curve modified by subtracting the baseline was prepared using Biacore8K Evaluation Software (GE Healthcare Japan).

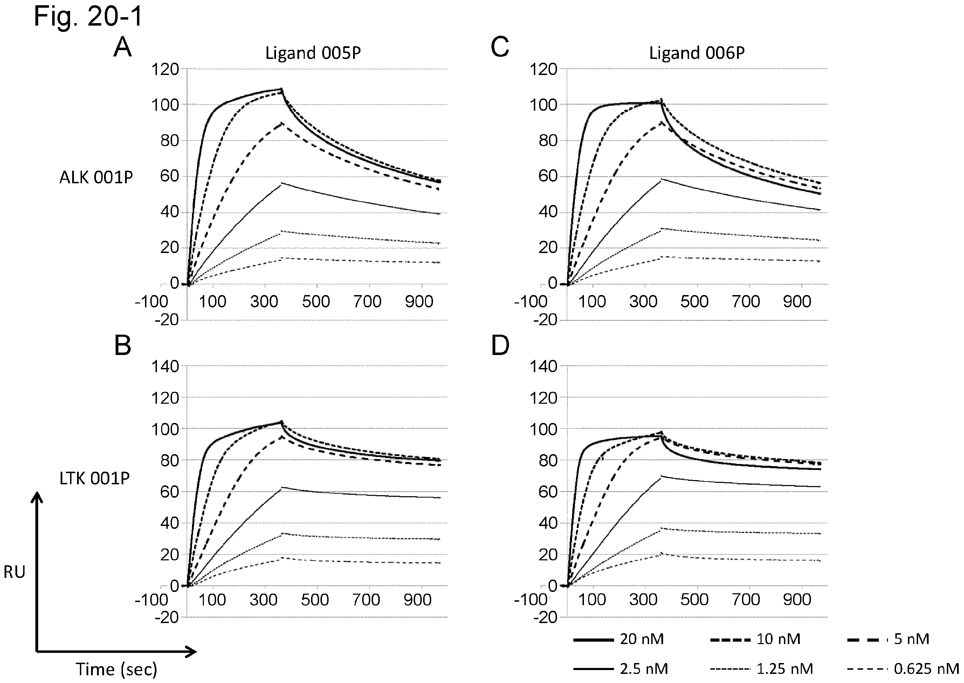

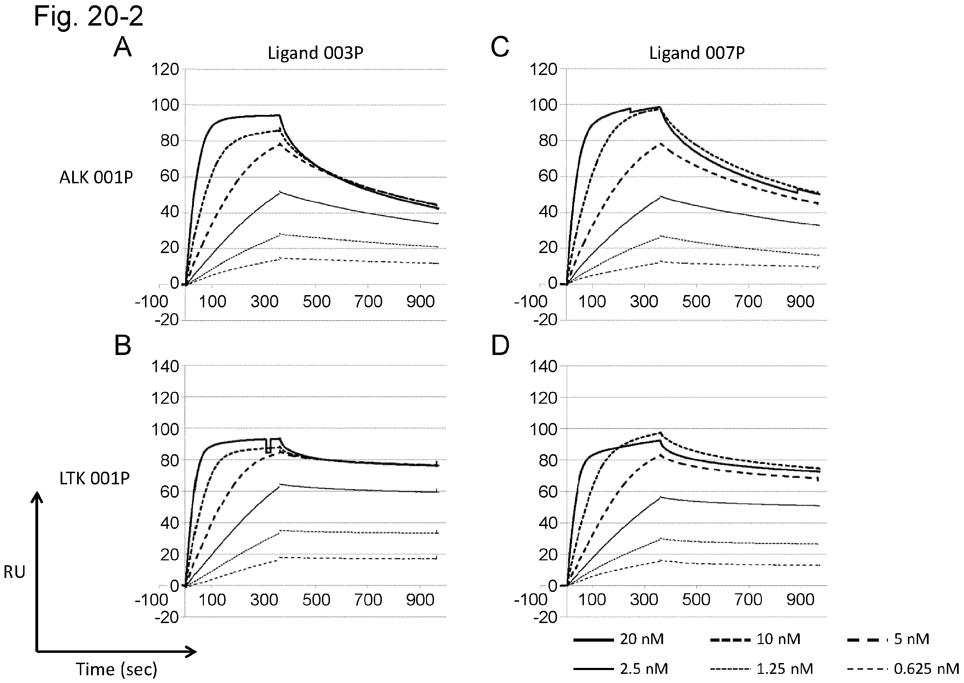

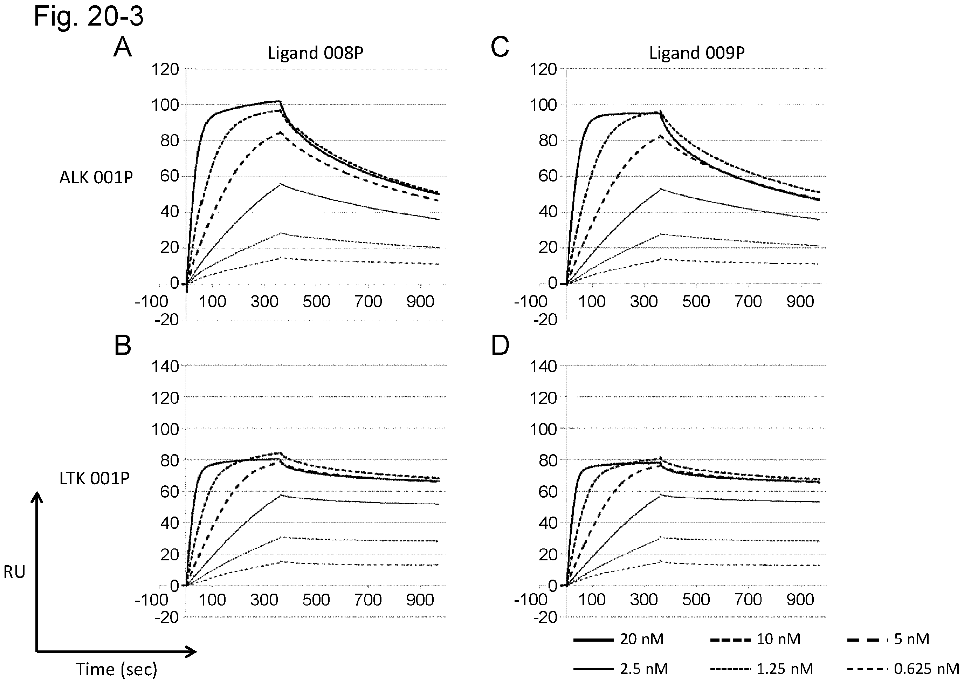

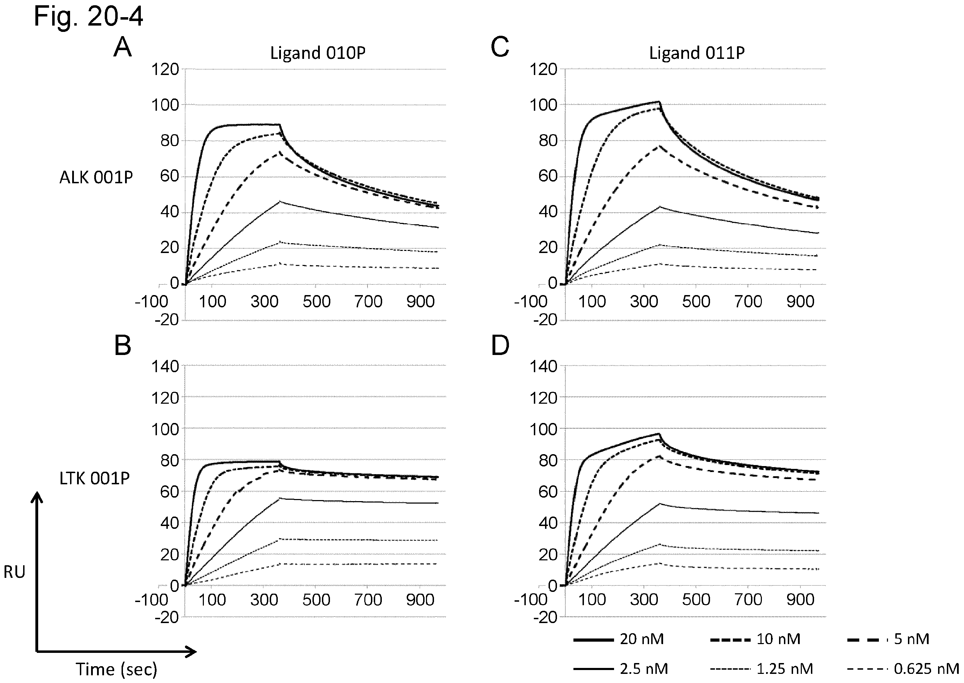

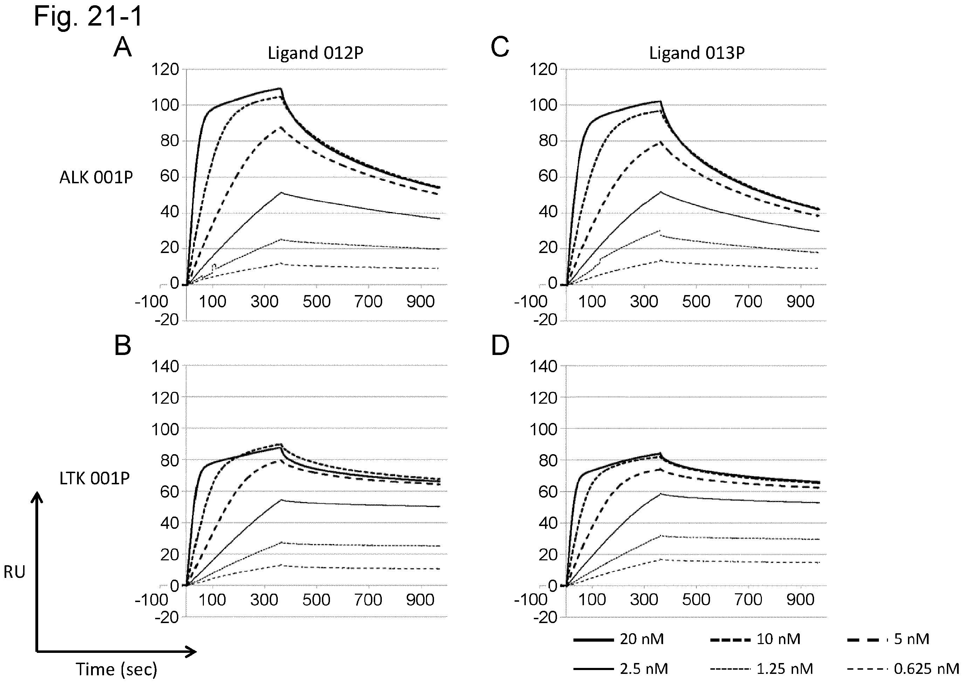

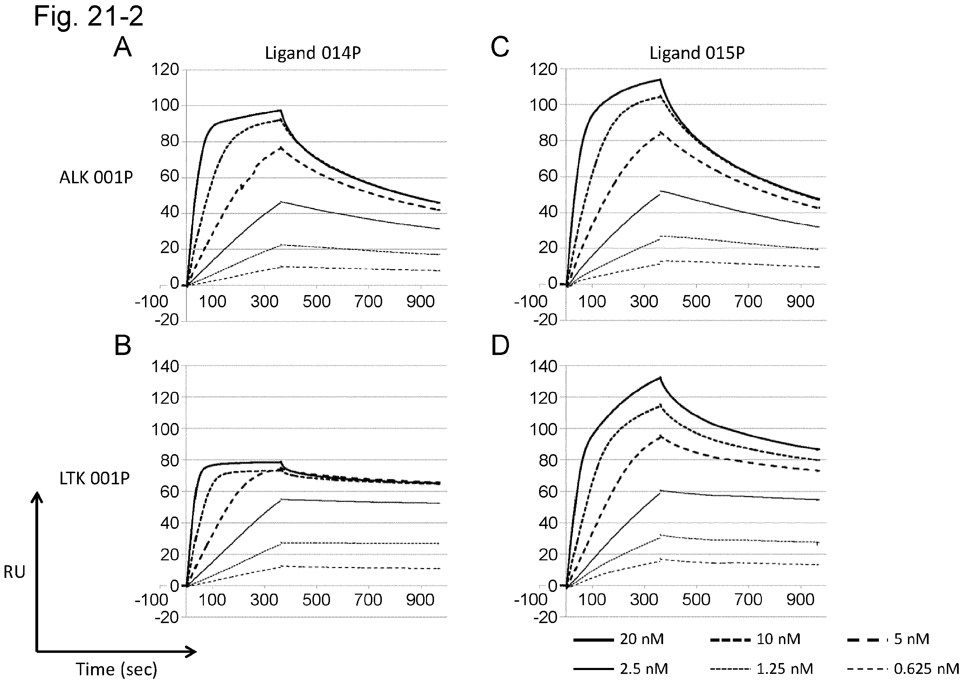

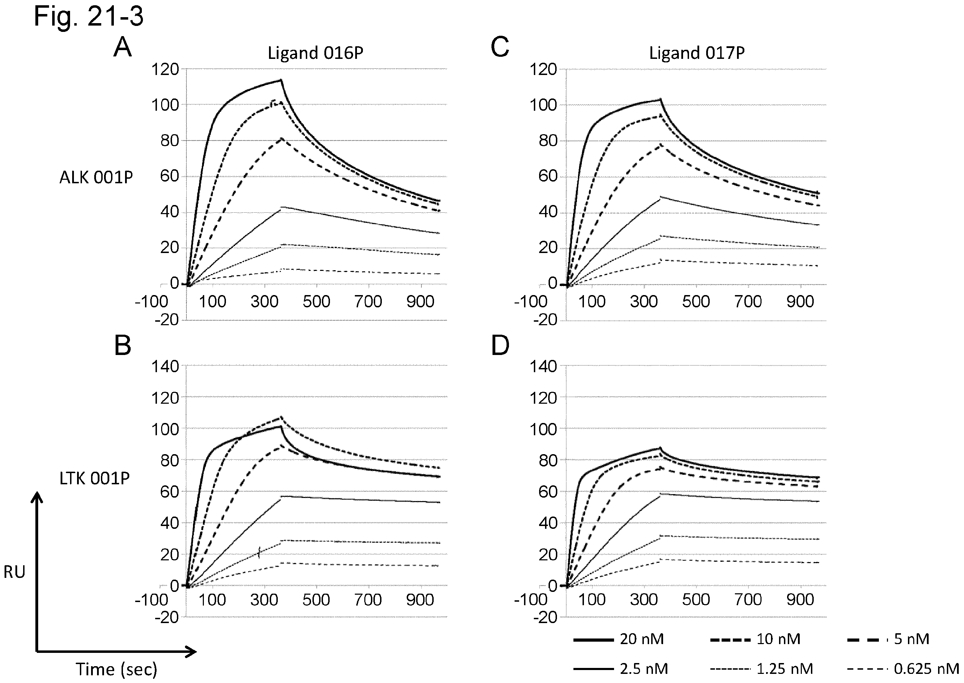

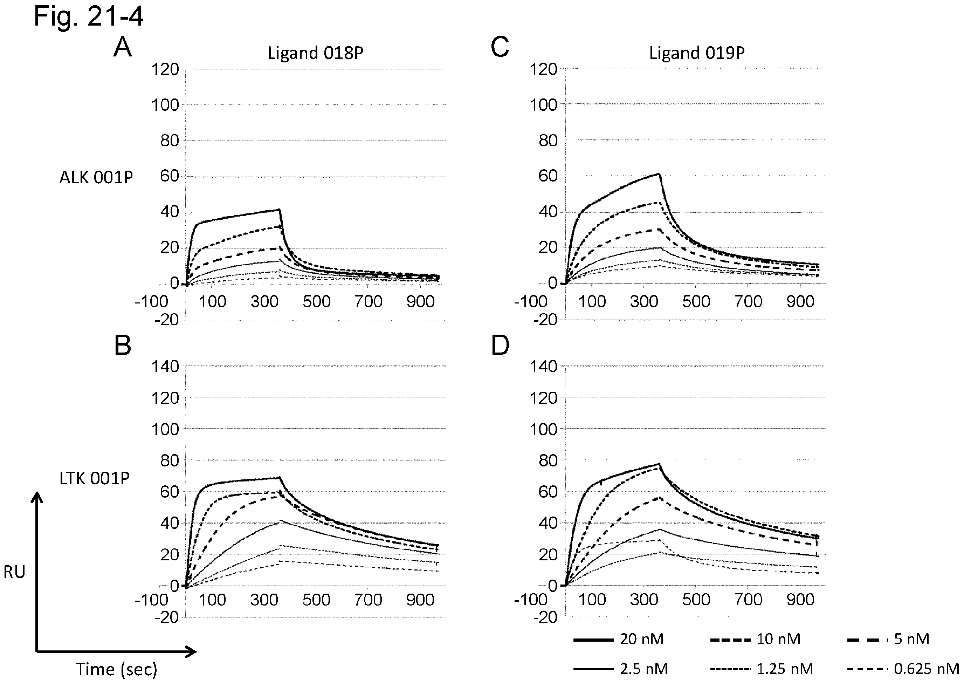

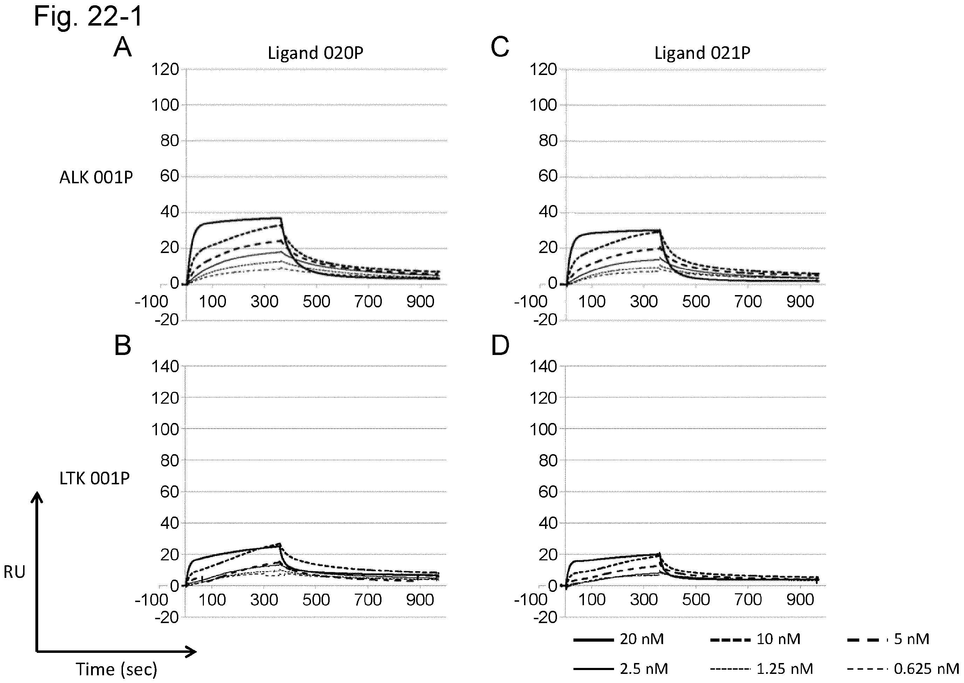

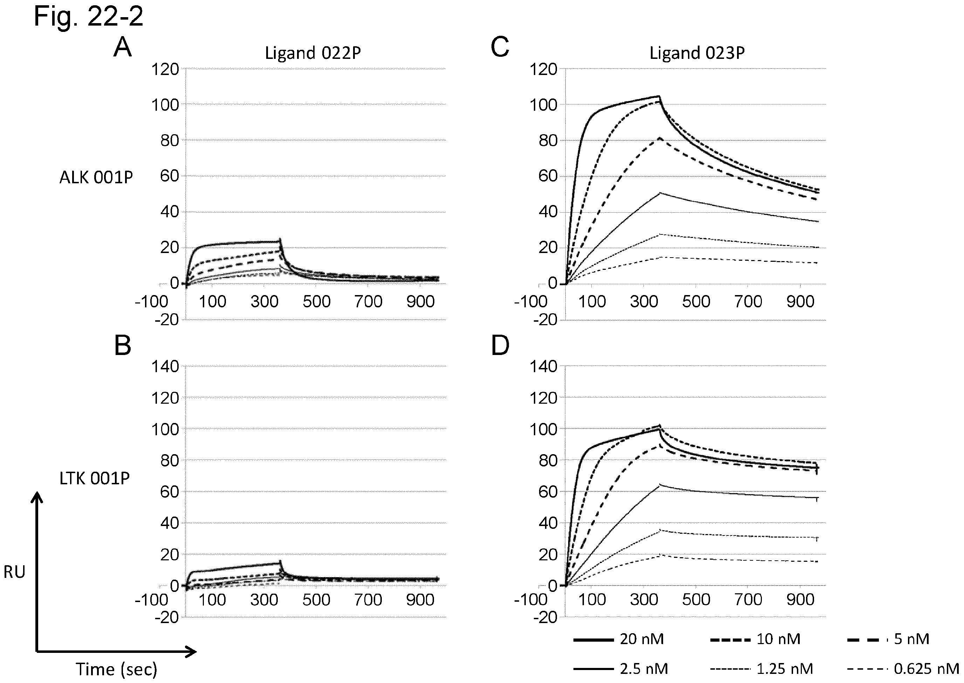

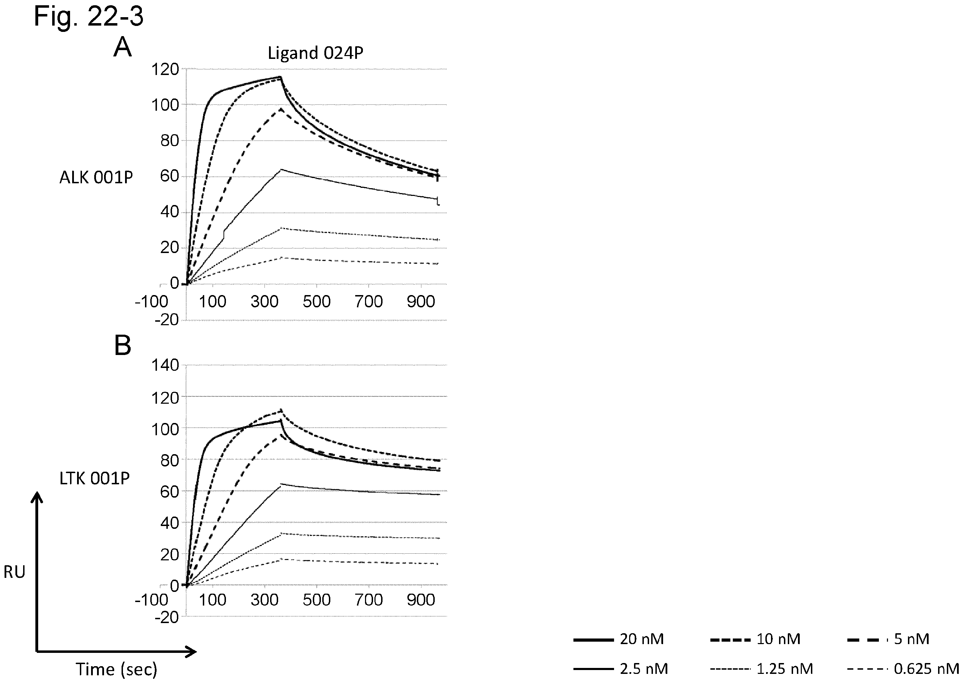

Figs. 20-1 to 20-4, Figs. 21-1 to 21-4, and Figs. 22-1 to 22-3 each show the results of verification of binding of ALK 001P and LTK 001P to Ligand 003P and Ligand 005P to Ligand 024P via surface plasmon resonance analysis. The results demonstrate that Ligand 003P, Ligand 005P to Ligand 017P, Ligand 023P, and Ligand 024P sufficiently bind to both ALK 001P and LTK 001P and that they are suitable as CAR target binding domains targeting ALK and LTK. In contrast, Ligand 018P to Ligand 022P were dissociated from ALK and LTK significantly faster than other ligands and exhibited lowered binding intensity.

### Example 12: Preparation of 4-1BB-type CAR-expressing plasmid

In this example, CAR 009 and CAR 010 plasmids were prepared by replacing the CD28 domain of CAR 002 and 003 with 4-1BB.

### Artificial synthesis of CD8α + 4-1BB gene

On the basis of the amino acid sequence information of human 4-1BB (SEQ ID NO: 125) described in WO 2015/069922, a DNA sequence (SEQ ID NO: 126) comprising an upstream 15-base region and a downstream 15-base region of the CD28 region of CAR 002 and CAR 003 added to the DNA sequence codon-optimized for human was designed, artificially synthesized, and then inserted into a pEX-K4J1 vector (synthesis was commissioned to Eurofins Genomics K.K.).

### Amplification of CD8α + 4-1BB by PCR

PCR amplification was conducted using the artificially synthesized gene (SEQ ID NO: 126) as a template. Primers used for PCR are represented by SEQ ID NO: 127 and SEQ ID NO: 128. PCR was performed using PrimeSTAR Max DNA polymerase (Takara Bio Inc.) with a cycle consisting of 98°C for 10 seconds and 68°C for 30 seconds, which was repeated 35 times. After the PCR reaction, an aliquot of the sample was separated via 2% agarose gel electrophoresis, the amplification product of the size of interest was confirmed, and the remaining sample after PCR was treated with *Dpn*I in accordance with the instructions of the KOD-Plus-Mutagenesis Kit (Toyobo Co. Ltd.) to remove the template plasmid.

### Amplification of linear fragment of CAR 002 or CAR 003 by inverse-PCR

In order to delete the CD28 region from CAR 002 or CAR 003, a linear fragment was prepared by inverse-PCR. As primers used for inverse-PCR, primers represented by SEQ ID NO: 129 and SEQ ID NO: 130 were designed and synthesized (synthesis was commissioned to Eurofins Genomics K.K.). CAR 002 or CAR 003 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times. After the PCR reaction, an aliquot of the sample was separated via 1% agarose gel electrophoresis, amplification of a linear fragment of the size of interest was confirmed, the remaining sample after PCR was treated with *Dpn*I in accordance with the instructions of the KOD-Plus-Mutagenesis Kit (Toyobo Co. Ltd.), and methylated template plasmid CAR 002 or CAR 003 was cleaved and removed.

### Ligation of CAR 002 or CAR 003 linear fragment to CD8α + 4-1BB amplified fragment

The CAR 002 or CAR 003 linear fragment was ligated to the CD8α+4-1BB amplified fragment using NEBuilder HiFi DNA Assembly Master Mix (New England Biolabs). After the reaction at 50°C for 15 minutes, *E. coli* DH5α (Toyobo Co. Ltd.) cells were transformed using the ligated cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence insertion was observed were obtained as a FAM150A 4-1BB-type plasmid (CAR 009; FAM150A-8αBBz) and a FAM150B 4-1BB-type plasmid (CAR 010; FAM150B-8αBBz). Nucleotide sequence analysis was commissioned to Eurofins Genomics K.K.

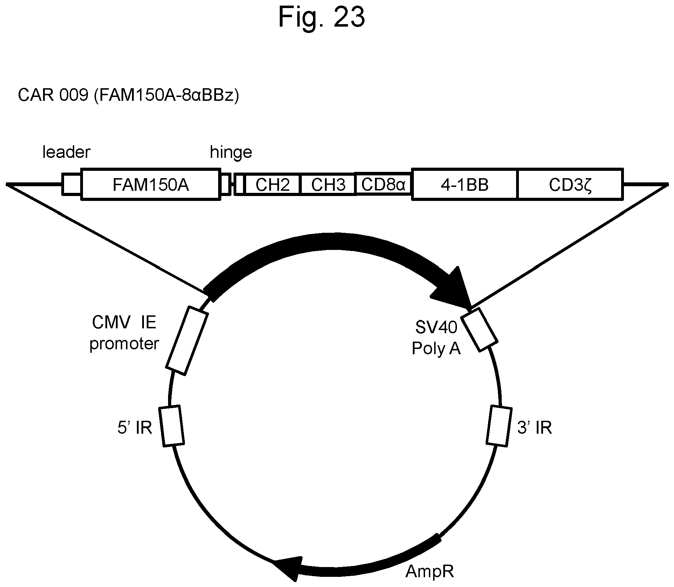

Fig. 23 shows a vector map of CAR 009 (FAM150A-8α28z), and the DNA sequence of a translation region of CAR 009 is represented by SEQ ID NO: 131.

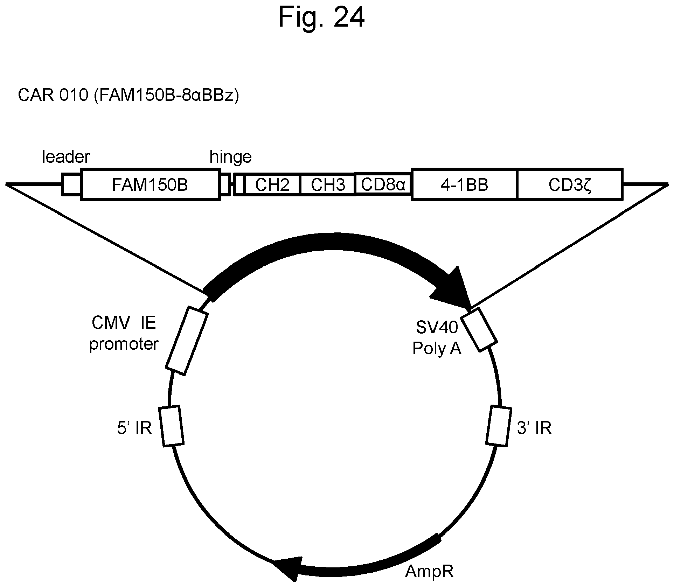

Fig. 24 shows a vector map of CAR 010 (FAM150B-8α28z), and the DNA sequence of a translation region of CAR 010 is represented by SEQ ID NO: 132.

### Preparation of FAM150A truncated 4-1BB-type CAR-expressing plasmid (CAR 011; FAM150ATr-8αBBz) and FAM150B truncated 4-1BB-type CAR-expressing plasmid (CAR 012; FAM150BTr-8αBBz)

With the use of CAR 009 or CAR 010 prepared above as a template, a FAM150A truncated 4-1BB-type CAR-expressing plasmid (CAR 011; FAM150ATr-8αBBz) and a FAM150B truncated 4-1BB-type CAR-expressing plasmid (CAR 012; FAM150BTr-8αBBz) were prepared by inverse-PCR. The primers for CAR 002 linearization (SEQ ID NO: 52 and SEQ ID NO: 53) and the primers for CAR 003 linearization (SEQ ID NO: 54 and SEQ ID NO: 55) described above were used for inverse-PCR.

CAR 009 or CAR 010 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times.

After the PCR reaction, an aliquot of the sample was separated via 1% agarose gel electrophoresis, amplification of the linear plasmid of the size of interest was confirmed, the remaining sample after PCR was treated with *Dpn*I in accordance with the instructions of the KOD-Plus-Mutagenesis Kit (Toyobo Co. Ltd.), and methylated template plasmid CAR 009 or CAR 010 was cleaved and removed. Thereafter, the linear plasmid was phosphorylated with T4 Polynucleotide Kinase included in the kit and self-ligated to form a cyclic plasmid. Thereafter, *E. coli* DH5α (Toyobo Co. Ltd.) cells were transformed using the cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence deletion was observed were obtained as a FAM150A truncated 4-1BB-type CAR-expressing plasmid (CAR 011; FAM150ATr-8αBBz) and a FAM150B truncated 4-1BB-type CAR-expressing plasmid (CAR 012; FAM150BTr-8αBBz).

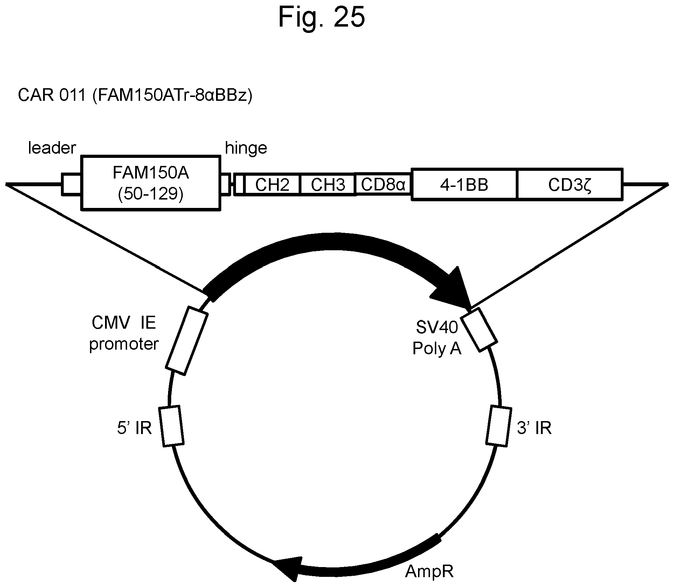

Fig. 25 shows a vector map of CAR 011 (FAM150ATr-8αBBz), and the DNA sequence of a translation region of CAR 011 is represented by SEQ ID NO: 133.

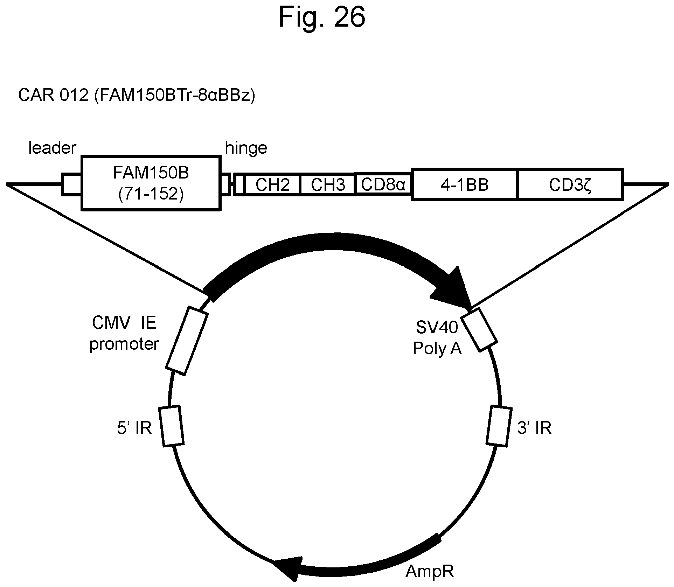

Fig. 26 shows a vector map of CAR 012 (FAM150BTr-8αBBz), and the DNA sequence of a translation region of CAR 012 is represented by SEQ ID NO: 134.

### Preparation of humanized ALK48 scFv 4-1BB-type (CAR 013; hALK48-8αBBz) and mouse ALK48 scFv 4-1BB-type CAR-expressing plasmids (CAR 014 ALK48-8αBBz)

CAR 004, CAR 005, CAR 009, or CAR 010 (about 1 µg equivalent each) was digested with restriction enzymes *Xho*I and *Dra*III (New England Biolabs) at 37°C for about 2 hours. After the enzyme treatment, the reaction solution was separated via 1% agarose gel electrophoresis, the enzyme-treated CAR 009 or CAR 010 fragment (on the vector side) and the insert fragment cleaved from CAR 004 or CAR 005 (SEQ ID NO: 5 or 6) were removed from the gel, and the fragments were purified using NucleoSpin Gel and PCR Clean-up^{®} (MACHEREY-NAGEL, Takara Bio Inc.). The purified vector fragment was ligated to the insert fragment using DNA ligation kit (Mighty Mix, Takara Bio Inc.). *E. coli* DH5α (Toyobo Co. Ltd.) cells were transformed using the ligated cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence insertion was observed were obtained as a humanized ALK48 scFv 4-1BB-type (CAR 013; hALK48-8αBBz) and a mouse ALK48 scFv 4-1BB-type CAR-expressing plasmids (CAR 014; ALK48-8αBBz). Nucleotide sequence analysis was commissioned to Eurofins Genomics K.K.

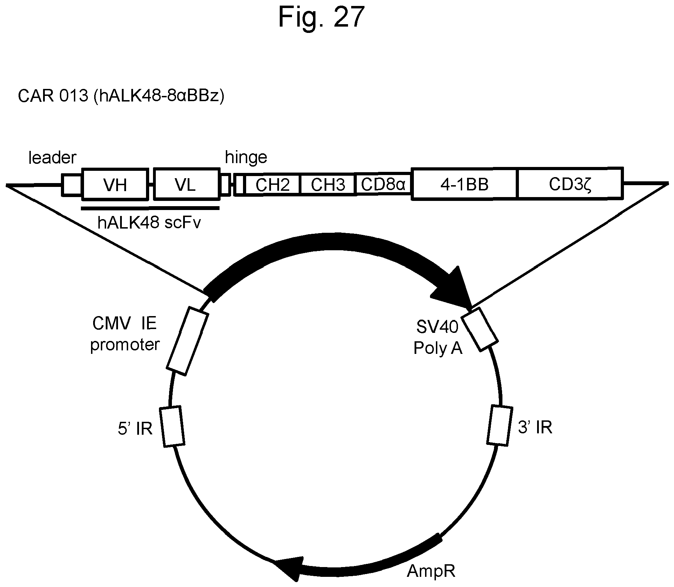

Fig. 27 shows a vector map of CAR 013 (hALK48-8αBBz), and the DNA sequence of a translation region of CAR 013 is represented by SEQ ID NO: 135.

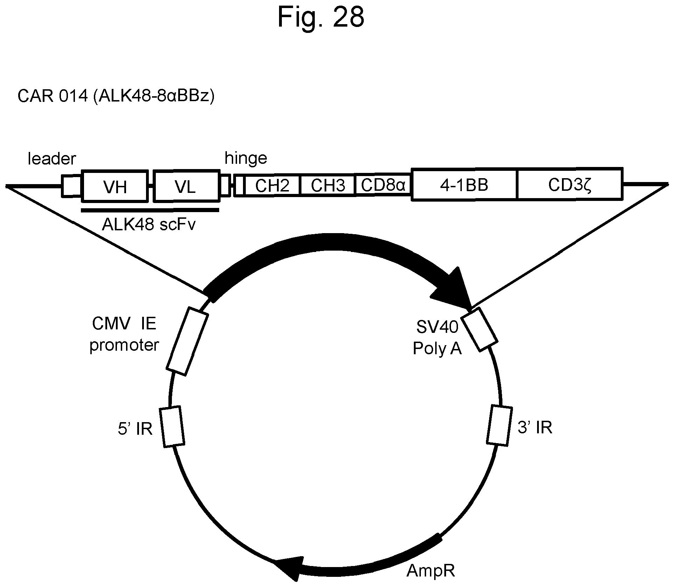

Fig. 28 shows a vector map of CAR 014 (ALK48-8αBBz), and the DNA sequence of a translation region of CAR 014 is represented by SEQ ID NO: 136.

### Example 13: Comparison of antitumor activity of FAM150A 4-1BB-type CAR-T, FAM150B 4-1BB-type CAR-T, FAM150A truncated 4-1BB-type CAR-T, FAM150B truncated 4-1BB-type CAR-T, humanized ALK48 scFv 4-1BB-type CAR-T, and mouse ALK48 scFv 4-1BB-type CAR-T

In accordance with the method of CAR-T cell culture and proliferation in Example 2, CAR-T cells were prepared using the CAR 009- to CAR 014-expressing plasmids prepared in the examples.

CAR-T cells obtained by electrical introduction into PBMCs using CAR 009 to CAR 014 and T cell culture and proliferation are described herein as follows.
CAR-T 009: FAM150A 4-1BB-type CAR-T (FAM150A-8αBBz CAR-T)
CAR-T 010: FAM150B 4-1BB-type CAR-T (FAM150B-8αBBz CAR-T)
CAR-T 011: FAM150A truncated 4-1BB-type CAR-T (FAM150ATr-8αBBz CAR-T)
CAR-T 012: FAM150B truncated 4-1BB-type CAR-T (FAM150BTr-8αBBz CAR-T)
CAR-T 013: Humanized ALK48 scFv 4-1BB-type CAR-T (hALK48-8αBBz CAR-T)
CAR-T 014: Mouse ALK48 scFv 4-1BB-type CAR-T (ALK48-8αBBz CAR-T)

The amino acid sequence of CAR 009 and the nucleotide sequence encoding the same are represented by SEQ ID NO: 137 and SEQ ID NO: 131, respectively.

The amino acid sequence of CAR 010 and the nucleotide sequence encoding the same are represented by SEQ ID NO: 138 and SEQ ID NO: 132, respectively.

The amino acid sequence of CAR 011 and the nucleotide sequence encoding the same are represented by SEQ ID NO: 139 and SEQ ID NO: 133, respectively.

The amino acid sequence of CAR 012 and the nucleotide sequence encoding the same are represented by SEQ ID NO: 140 and SEQ ID NO: 134, respectively.

The amino acid sequence of CAR 013 and the nucleotide sequence encoding the same are represented by SEQ ID NO: 141 and SEQ ID NO: 135, respectively.

The amino acid sequence of CAR 014 and the nucleotide sequence encoding the same are represented by SEQ ID NO: 142 and SEQ ID NO: 136, respectively.

Table 5 shows CAR expression rates in the CAR-T cells obtained by introduction of CAR-expressing plasmids into PBMCs derived from a healthy adult donor (Donor-1) and culture.

**[Table 5]**

| Donor | Plasmid | CAR-T | Expression rate |
|---|---|---|---|
| Donor-1 | CAR 009 | CAR-T 009 | 29.3 |
| | CAR 010 | CAR-T 010 | 12.3 |
| | CAR 011 | CAR-T 011 | 29.0 |
| | CAR 012 | CAR-T 012 | 6.04 |
| | CAR 013 | CAR-T 013 | 19.7 |
| | CAR 014 | CAR-T 014 | 14.7 |

### Measurement of antitumor activity of CAR-T

In order to measure antitumor activity of CAR-T 009 to CAR-T 014 obtained above, co-culture with solid tumor cells was conducted. In this example, passaged cell lines of SH-SY5Y and a breast cancer cell line (MDA-MB231 ffLuc, JCRB Cell Bank) were used as target tumor cells. SH-SY5Y cells and MDA-MB231 ffLuc cells were subjected to passage culture in D-MEM/Ham's F-12 medium containing 15% FBS, 1% penicillin/streptomycin, and 1% non-essential amino acid solution, and co-culture was also performed in a similar medium.

Activity evaluation was performed in accordance with the method of Example 3 using BD Accuri^{™}C6 Plus flow cytometer (BD Biosciences). MDA-MB231 ffLuc cells were analyzed by flow cytometry using 2 µl of PE Anti-Human EGFR antibody (Miltenyi Biotec) instead of PE anti-human Ganglioside GD2 antibody, and the number of EGFR-positive tumor cells was determined based on the number of counting beads.

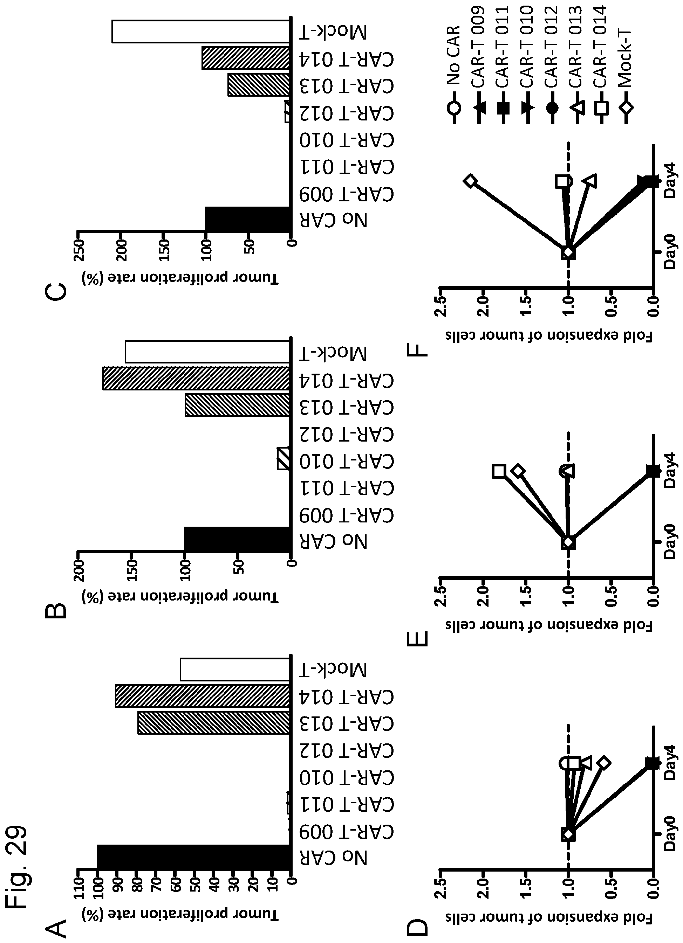

Fig. 29 shows antitumor activity of CAR-T 009 to CAR-T 014 or mock-T cells derived from Donor-1 against SH-SY5Y and the tumor cell proliferation curves. As a result, the effects of CAR-T 009, CAR-T 010, CAR-T 011, and CAR-T 012 to kill SH-SY5Y were verified. In contrast, the effects of CAR-T 013 and CAR-T 014 were not significantly different from those of mock-T cells, and substantially no effects were observed.

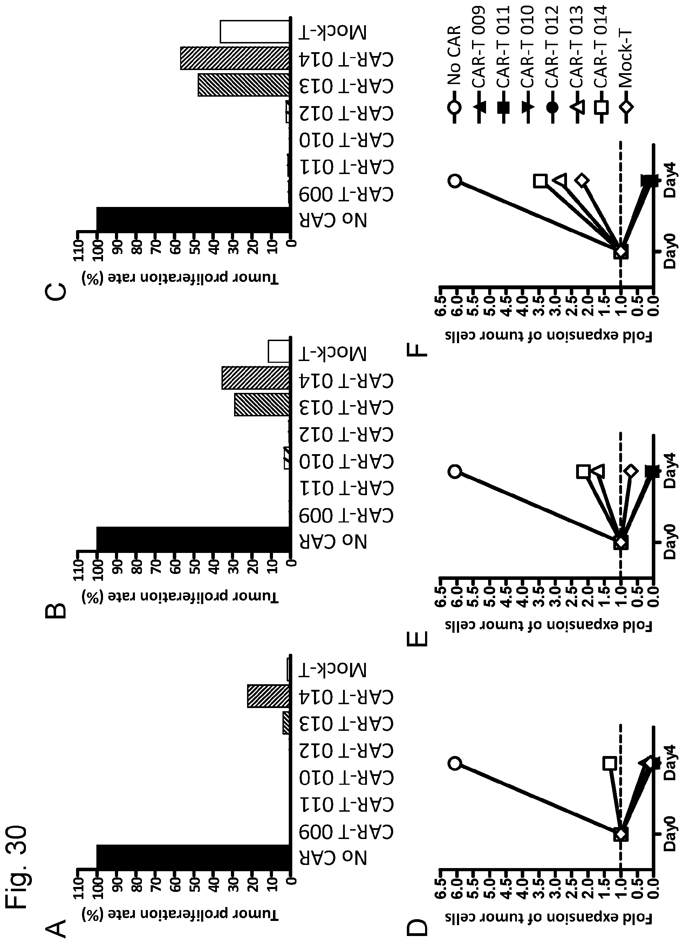

Fig. 30 shows antitumor activity of CAR-T 009 to CAR-T 014 or mock-T cells derived from Donor-1 against MDA-MB231 ffLuc and the tumor cell proliferation curves. As a result, the effects of CAR-T 009, CAR-T 010, CAR-T 011, and CAR-T 012 to kill MDA-MB231 ffLuc were verified. In contrast, the effects of CAR-T 013 and CAR-T 014 were lower than those of mock-T cells, and substantially no effects were observed.

This example indicates that antitumor activity of FAM150A (full-length and truncated) CAR-T and FAM150B (full-length and truncated) CAR-T against neuroblastoma cell line and breast cancer cell line is higher than that of ALK48 scFv-type (humanized and mouse) CAR-T.

### Example 14: Preparation of FAM150B T14, FAM150B T15, and FAM150B T17 to FAM150B T19 truncated CAR-expressing plasmids

With the use of CAR 008 (FAM150BTr-28z) prepared in Example 7 as a template, plasmids expressing truncated forms CAR 015 to CAR 019 were further prepared. Forward primers for inverse-PCR were designed for CAR 015 amplification (SEQ ID NO: 156), CAR 016 amplification (SEQ ID NO: 157), CAR 017 amplification (SEQ ID NO: 158), CAR 018 amplification (SEQ ID NO: 159), and CAR 019 amplification (SEQ ID NO: 160). The sequence represented by SEQ ID NO: 55 was used as a reverse primer.

CAR 008 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times.

After the PCR reaction, an aliquot of the sample was separated via 1% agarose gel electrophoresis, amplification of a linear plasmid of the size of interest was confirmed, the remaining sample after PCR was treated with *Dpn*I in accordance with the instructions of the KOD-Plus-Mutagenesis Kit (Toyobo Co. Ltd.), and methylated template plasmid CAR 008 was cleaved and removed. Thereafter, the linear plasmid was phosphorylated with T4 Polynucleotide Kinase included in the kit and self-ligated to form a cyclic plasmid. Thereafter, *E. coli* DH5α (Toyobo Co. Ltd.) cells were transformed using the cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and plasmids in which target nucleotide sequence deletion was observed were obtained as FAM150B truncated CD28-type CAR-expressing plasmids (CAR 015: FAM150BT14-28z, CAR 016: FAM150BT15-28z, CAR 017: FAM150BT17-28z, CAR 018: FAM150BT18-28z, and CAR 019: FAM150BT19-28z).

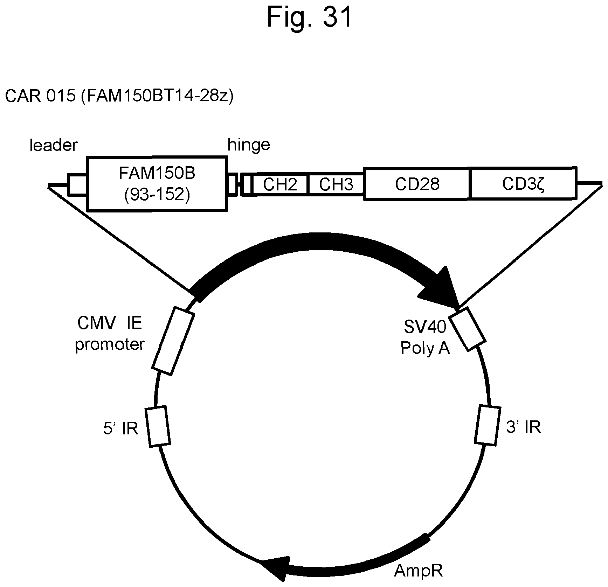

Fig. 31 shows a vector map of CAR 015 (FAM150BT14-28z), and the DNA sequence of a translation region of CAR 015 is represented by SEQ ID NO: 161.

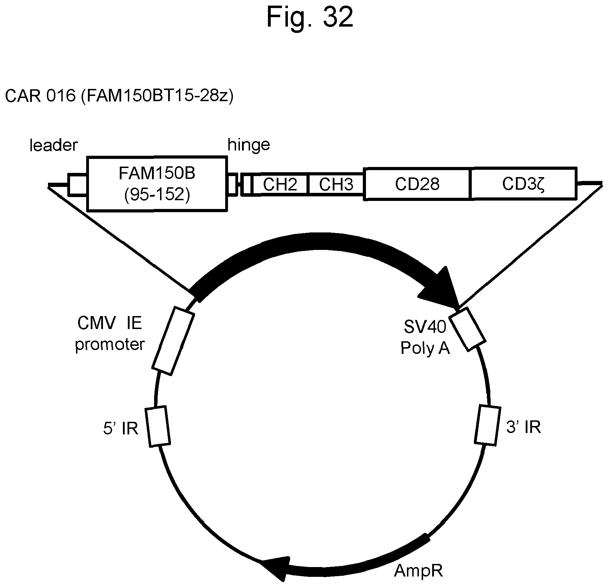

Fig. 32 shows a vector map of CAR 016 (FAM150BT15-28z), and the DNA sequence of a translation region of CAR 016 is represented by SEQ ID NO: 162.

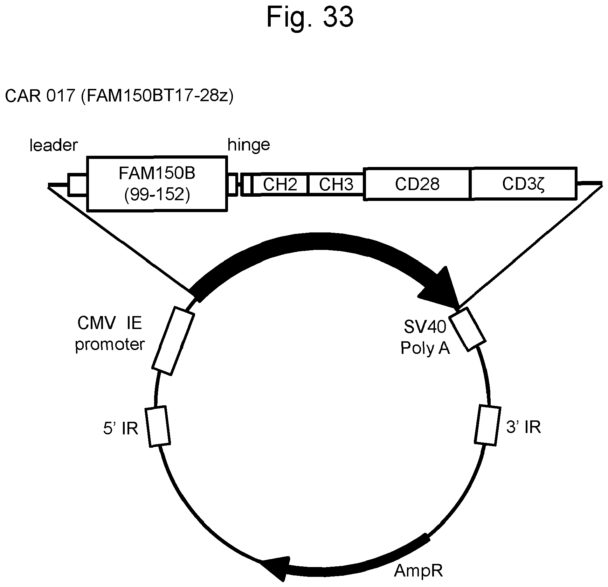

Fig. 33 shows a vector map of CAR 017 (FAM150BT17-28z), and the DNA sequence of a translation region of CAR 017 is represented by SEQ ID NO: 163.

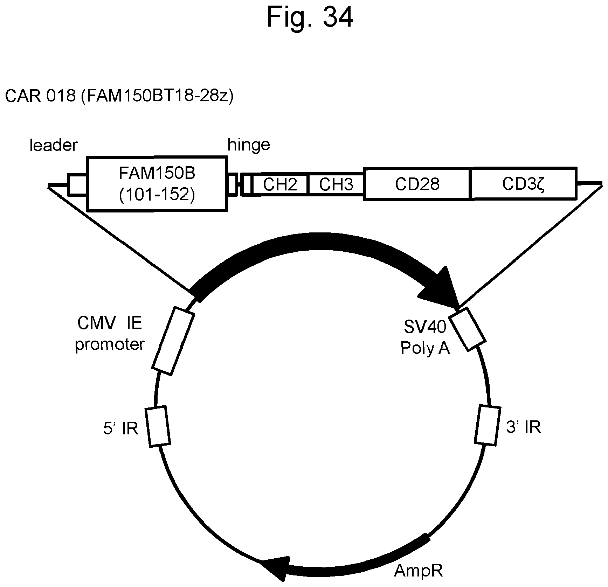

Fig. 34 shows a vector map of CAR 018 (FAM150BT18-28z), and the DNA sequence of a translation region of CAR 018 is represented by SEQ ID NO: 164.

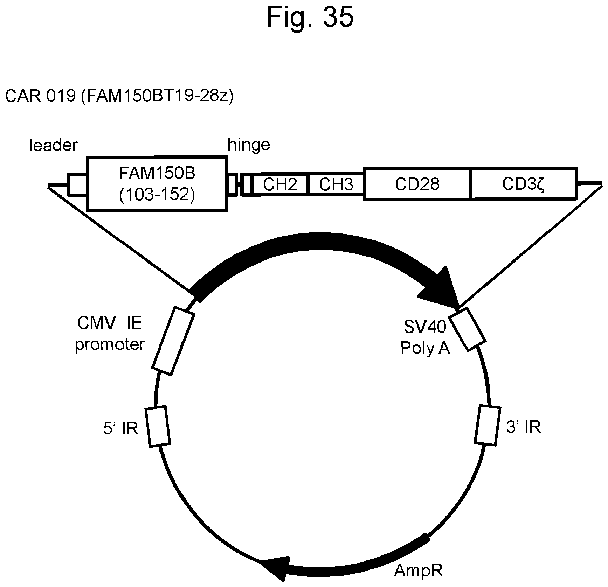

Fig. 35 shows a vector map of CAR 019 (FAM150BT19-28z) and the DNA sequence of a translation region of CAR 019 is represented by SEQ ID NO: 165.

### Example 15: Comparison of antitumor activity of FAM150BT14, FAM150BT15, and FAM150BT17 to FAM150BT19 truncated CAR-expressing T cells

In accordance with the method of CAR-T cell culture and proliferation in Example 2, CAR-T cells were prepared using the CAR 015 to CAR 019-expressing plasmids prepared in Example 14.

CAR-T cells obtained by electrical introduction into PBMCs with CAR 015 to CAR 019 and T cell culture and proliferation are described as follows.
CAR-T 015: FAM150B truncated T14 CD28-type CAR-T (FAM150BT14-CD28z CAR-T)
CAR-T 016: FAM150B truncated T15 CD28-type CAR-T (FAM150BT15-CD28z CAR-T)
CAR-T 017: FAM150B truncated T17 CD28-type CAR-T (FAM150BT17-CD28z CAR-T)
CAR-T 018: FAM150B truncated T18 CD28-type CAR-T (FAM150BT18-CD28z CAR-T)
CAR-T 019: FAM150B truncated T19 CD28-type CAR-T (FAM150BT19-CD28z CAR-T)

The amino acid sequence of CAR 015 and the nucleotide sequence encoding the same are represented by SEQ ID NO: 166 and SEQ ID NO: 161, respectively.

The amino acid sequence of CAR 016 and the nucleotide sequence encoding the same are represented by SEQ ID NO: 167 and SEQ ID NO: 162, respectively.

The amino acid sequence of CAR 017 and the nucleotide sequence encoding the same are represented by SEQ ID NO: 168 and SEQ ID NO: 163, respectively.

The amino acid sequence of CAR 018 and the nucleotide sequence encoding the same are represented by SEQ ID NO: 169 and SEQ ID NO: 164, respectively.

The amino acid sequence of CAR 019 and the nucleotide sequence encoding the same are represented by SEQ ID NO: 170 and SEQ ID NO: 165, respectively.

Table 6 shows CAR expression rates in the CAR-T cells obtained by introduction of CAR-expressing plasmids into PBMCs derived from a healthy adult donor (Donor-1) and culture.

**[Table 6]**

| Donor | Plasmid | CAR-T | Expression rate |
|---|---|---|---|
| Donor-1 | CAR 015 | CAR-T 015 | 17.6 |
| | CAR 016 | CAR-T 016 | 23.7 |
| | CAR 017 | CAR-T 017 | 31.8 |
| | CAR 018 | CAR-T 018 | 32.3 |
| | CAR 019 | CAR-T 019 | 27.0 |
| | CAR 008 | CAR-T 008 | 26.7 |
| | CAR 006 | CAR-T 006 | 21.4 |

### Measurement of antitumor activity of CAR-T

In order to measure antitumor activity of CAR-T 015 to CAR-T 019 obtained above, co-culture with solid tumor cells was conducted. In this example, passaged cell lines of SH-SY5Y, NB-1, IMR32, and MDA-MB231 ffLuc were used as target tumor cells.

Activity evaluation was performed in accordance with the method of Example 3 using BD Accuri^{™}C6 Plus flow cytometer (BD Biosciences). MDA-MB231 ffLuc cells were analyzed by flow cytometry using 2 µl of PE Anti-Human EGFR antibody (Miltenyi Biotec) instead of PE anti-human Ganglioside GD2 antibody, and the number of EGFR-positive tumor cells was determined based on the number of counting beads.

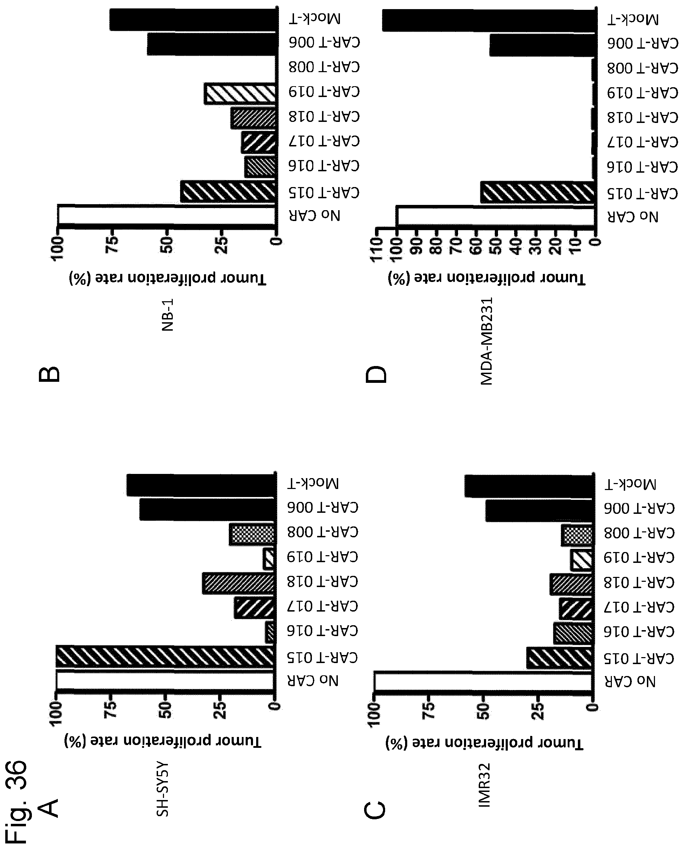

Fig. 36 shows antitumor activity of CAR-T 015 to CAR-T 019, CAR-T 008, and CAR-T 006 or mock-T cells derived from Donor-1 against SH-SY5Y, NB-1, IMR32, and MDA-MB231 ffLuc. As a result, the effects of CAR-T 016 to CAR-T 019 to kill SH-SY5Y, NB-1, IMR32, and MDA-MB231 ffLuc were verified. Activity thereof was not significantly different from high-level antitumor activity of CAR-T 008 observed in Example 8.

This example indicates that antitumor activity of CAR-T 016 to CAR-T 019 against neuroblastoma cell lines and breast cancer cell lines is substantially equivalent to that of CAR-T 008.

### Example 16: Preparation of FAM150B truncated CH2CH3-deletion CAR-expressing plasmid

With the use of CAR 012 (FAM150BTr-8αBBz) prepared in Example 12 as a template, a CH2CH3-deletion expression plasmid CAR 020 was prepared. A forward primer having the sequence represented by SEQ ID NO: 171 and a reverse primer having the sequence represented by SEQ ID NO: 172 were used for inverse-PCR.

CAR 012 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times.

After the PCR reaction, an aliquot of the sample was separated via 1% agarose gel electrophoresis, amplification of a linear plasmid of the size of interest was confirmed, the remaining sample after PCR was treated with *Dpn*I in accordance with the instructions of the KOD-Plus-Mutagenesis Kit (Toyobo Co. Ltd.), and methylated template plasmid CAR 012 was cleaved and removed. Thereafter, the linear plasmid was phosphorylated with T4 Polynucleotide Kinase included in the kit and self-ligated to form a cyclic plasmid. Thereafter, *E. coli* DH5α (Toyobo Co. Ltd.) cells were transformed using the cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and a plasmid in which target nucleotide sequence deletion was observed was obtained as FAM150B truncated CH2CH3-deletion CAR-expressing plasmid (CAR 020; FAM150BTr-BBz dCH2CH3).

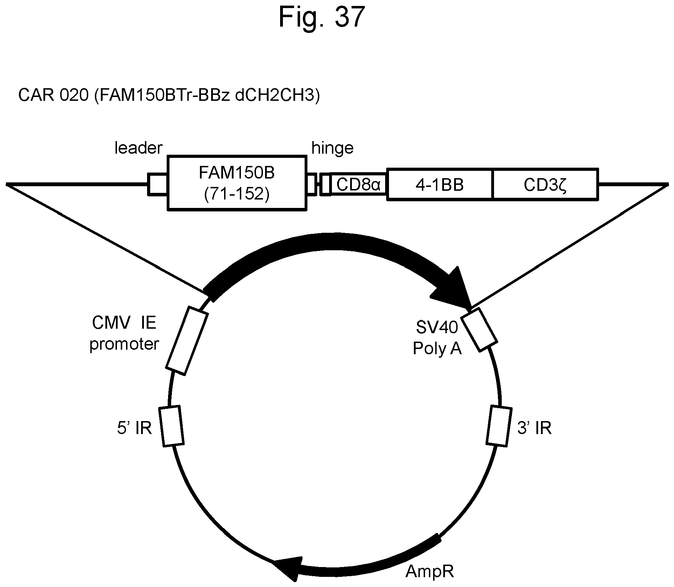

Fig. 37 shows a vector map of CAR 020 (FAM150BTr-BBz dCH2Ch3), and the DNA sequence of a translation region of CAR 020, and the amino acid sequence of CAR 020 are represented by SEQ ID NO: 173 and SEQ ID NO: 174, respectively.

### Culture method

In accordance with the method of CAR-T cell culture and proliferation in Example 2, CAR-T cells were prepared using the CD19 scFv-type CAR-expressing plasmid (CAR 006; CD19-28z), CAR 012 (FAM150BTr-8αBBz) prepared in Example 12, and CAR 020 expression plasmid described above. The CAR-T cells prepared using CAR 020 were subjected to flow cytometric analysis with the use of 5 µl of Mouse Anti-Human IgG1 Hinge-FITC Secondary Antibody (ABGENT Inc) instead of FITC Goat Anti-Human IgG (H+L) antibody (Jackson ImmunoResearch Inc).

Table 7 shows CAR expression rates in the CAR-T cells obtained by introduction of CAR-expressing plasmids into PBMCs derived from a healthy adult donor (Donor-1) and culture.

**[Table 7]**

| Donor | Plasmid | CAR-T | Expression rate |
|---|---|---|---|
| Donor-1 | CAR 012 | CAR-T 012 | 17.5 |
| | CAR 020 | CAR-T 020 | 29.3 |
| | CAR 006 | CAR-T 006 | 31.8 |

### Measurement of antitumor activity of CAR-T

In order to measure antitumor activity of CAR-T 006, CAR-T 012, and CAR-T 020 obtained above, co-culture with solid tumor cells was conducted. In this example, passaged cell lines of SH-SY5Y, NB-1, IMR32, and MDA-MB231 ffLuc were used as target tumor cells.

Activity evaluation was performed in accordance with the method of Example 3 using BD Accuri^{™}C6 Plus flow cytometer (BD Biosciences). MDA-MB231 ffLuc cells were analyzed by flow cytometry using 2 µl of PE Anti-Human EGFR antibody (Miltenyi Biotec) instead of PE anti-human Ganglioside GD2 antibody, and the number of EGFR-positive tumor cells was determined based on the number of counting beads.

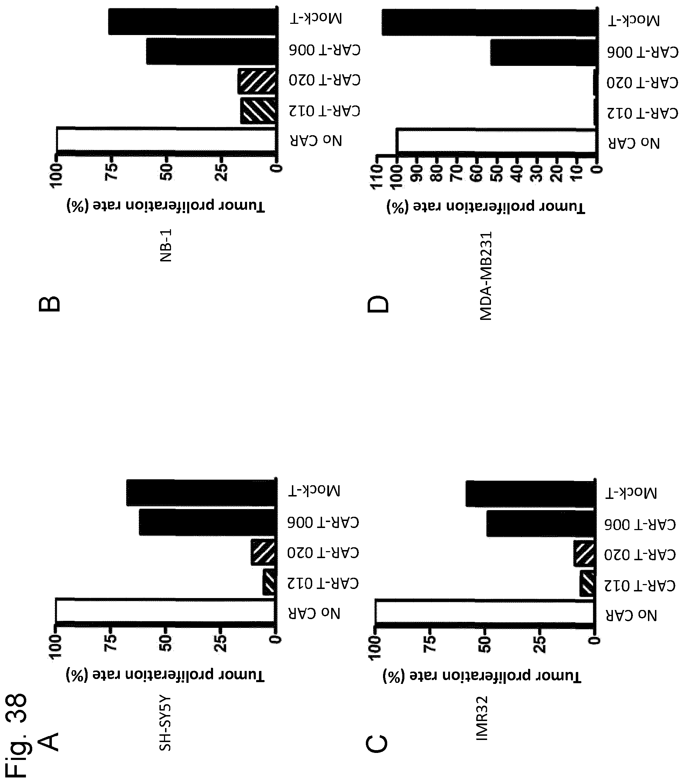

Fig. 38 shows antitumor activity of CAR-T 006, CAR-T 012, and CAR-T 020 or mock-T cells derived from Donor-1 against SH-SY5Y, NB-1, IMR32, and MDA-MB231 ffLuc. As a result, the effects of CAR-T 020 to kill SH-SY5Y, NB-1, IMR32, and MDA-MB231 ffLuc were verified, and activity thereof was equivalent to that of CAR-T 012.

### Example 17: Preparation of ALK- or LTK-expressing plasmid

### Preparation of ALK-expressing plasmid (pEHX-ALK)

A DNA sequence (*Hind*III-Kozak-ALK-*Dra*III; SEQ ID NO: 176) comprising a restriction enzyme *Hind*III cleavage sequence and a Kozak sequence (SEQ ID NO: 21) added to a region upstream of a sequence from the start codon to a restriction enzyme *Dra*III cleavage sequence of a translation region of ALK (NCBI Accession Number: NM_004304.5) (928 to 3,198 bp; SEQ ID NO: 175) was designed. Separately, a DNA sequence (*Dra*III-ALK-TGA-*Not*I; SEQ ID NO: 178) comprising a stop codon TGA and a restriction enzyme *Not*I cleavage sequence added to a region downstream of a sequence from the restriction enzyme *Dra*III cleavage sequence in a translation region of ALK (NCBI Accession Number: NM_004304.5) (3,190 to 5,790 bp; SEQ ID NO: 177) was designed. The two designed DNA sequences were artificially synthesized and incorporated into a pEX-K4J2 vector (synthesis was commissioned to Eurofins Genomics K.K.).

The pEX-K4J2 vector (1 µg equivalent) comprising the sequence of SEQ ID NO: 176 incorporated thereinto was digested with restriction enzymes HindIII, *Dra*III, and *Sph*I at 37°C for about 3 hours. The pEX-K4J2 vector comprising the sequence of SEQ ID NO: 178 incorporated thereinto was digested with restriction enzymes *Dra*III, *Not*I, and *Sph*I at 37°C for about 3 hours. After the enzyme treatment, the reaction solution was separated via 1% agarose gel electrophoresis, and the enzyme-treated fragment for SEQ ID NO: 176 with the theoretical value of 2,600 bp and the enzyme-treated fragment for SEQ ID NO: 178 with the theoretical value of 2,279 bp were cleaved from the gel and purified using NucleoSpin Gel and PCR Clean-up^{®} (MACHEREY-NAGEL, Takara Bio Inc.).

Separately, the Mammalian PowerExpress System vector pEHX1.2^{®} (Toyobo Co. Ltd.) used as a host vector was digested with restriction enzymes *Hind*III and *Not*I at 37°C for about 2 hours, and a fragment on the vector side was purified in the same manner as described above.

Thereafter, enzyme-treated fragments of the sequences of SEQ ID NO: 176 and SEQ ID NO: 178, and pEHX1.2 were ligated using DNA Ligation Kit (Mighty Mix, Takara Bio Inc.). *E. coli* DH5α (Toyobo Co. Ltd.) cells were transformed using the ligated cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and a plasmid in which target nucleotide sequence insertion had been observed was obtained as an ALK-expressing plasmid (pEHX-ALK). Nucleotide sequence analysis was commissioned to Eurofins Genomics K.K.

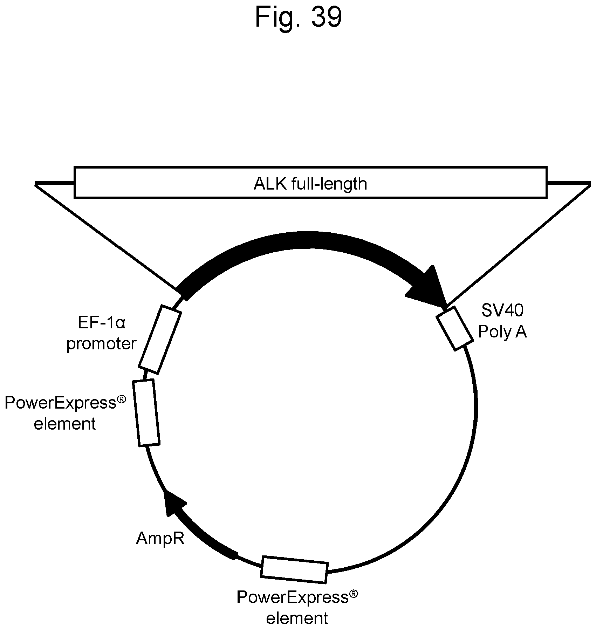

Fig. 39 shows a vector map of pEHX-ALK, and the DNA sequence of a translation region of pEHX-ALK is represented by SEQ ID NO: 179.

### Preparation of LTK-expressing plasmid (pEHX-LTK)

With the use of LTK 001 prepared in Example 4 as a template and the PCR primers represented by SEQ ID NO: 180 and SEQ ID NO: 181, a restriction enzyme *Not*I cleavage sequence was added to a region upstream of the Kozak sequence (SEQ ID NO: 21) of LTK 001, and a region up to the restriction enzyme *Nhe*I cleavage sequence in the LTK sequence (*Not*I-Kozak-LTK-NheI; SEQ ID NO: 182) was amplified by PCR. PCR was carried out with the use of PrimeSTAR Max DNA polymerase (Takara Bio Inc.) with a cycle consisting of 98°C for 10 seconds, 55°C for 5 seconds, and 72°C for 10 seconds, which was repeated 35 times.

After the PCR reaction, the sample was separated via 1% agarose gel electrophoresis, and the polynucleotide of SEQ ID NO: 182 was purified using NucleoSpin Gel and PCR Clean-up^{®} (MACHEREY-NAGEL, Takara Bio Inc.). Thereafter, the resultant was incorporated into a pCR-BluntII-TOPO vector via blunt-end cloning using Zero Blunt TOPO PCR Cloning Kit^{®} (Thermo Fisher Scientific). *E. coli* DH5α (Toyobo Co. Ltd.) cells were transformed using the cyclic plasmid comprising the sequence of SEQ ID NO: 182 incorporated thereinto and then cultured on an LB agar medium containing 50 µg/ml kanamycin for about 16 hours.

The appeared colonies were further cultured in an LB liquid medium containing 50 µg/ml carbenicillin for about 16 hours. Plasmids were purified from the cultured *E. coli* cells using QIAprep Spin Miniprep Kit (Qiagen) (pCR-BluntII-*Not*I-Kozak-LTK-*Nhe*I).

Separately, a DNA sequence (*Nhe*I-LTK-TAA-*Xba*I; SEQ ID NO: 183) comprising a stop codon TAA and a restriction enzyme *Xba*I cleavage sequence added to a region downstream of a region from a restriction enzyme *Nhe*I cleavage sequence to the stop codon TGA of LTK (NCBI Accession Number: NM_002344.5) was designed and codon-optimized for human. The designed DNA sequence was artificially synthesized and incorporated into a pEX-K4J2 vector (synthesis was commissioned to Eurofins Genomics K.K.).

With the use of the pEX-K4J2 vector comprising the sequence of SEQ ID NO: 183 incorporated therein as a template and PCR primers represented by SEQ ID NO: 184 and SEQ ID NO: 185, a sequence (*Nhe*I-LTK-TGATAA-*Not*I; SEQ ID NO: 186) comprising a restriction enzyme *Not*I cleavage sequence added to a region downstream of a region from a restriction enzyme *Nhe*I cleavage sequence to 2 stop codons (TGATAA) of LTK was amplified by PCR. PCR was carried out with the use of PrimeSTAR Max DNA polymerase (Takara Bio Inc.) with a cycle consisting of 98°C for 10 seconds, 55°C for 5 seconds, and 72°C for 10 seconds, which was repeated 35 times.

After the PCR reaction, the sample was separated via 1% agarose gel electrophoresis, and the polynucleotide of SEQ ID NO: 186 was purified using NucleoSpin Gel and PCR Clean-up^{®} (MACHEREY-NAGEL, Takara Bio Inc.). Thereafter, the resultant was incorporated into a pCR-BluntII-TOPO vector via blunt-end cloning using Zero Blunt TOPO PCR Cloning Kit^{®} (Thermo Fisher Scientific). *E. coli* DH5α (Toyobo Co. Ltd.) cells were transformed using the cyclic plasmid comprising the sequence of SEQ ID NO: 186 incorporated thereinto and then cultured on an LB agar medium containing 50 µg/ml kanamycin for about 16 hours.

The appeared colonies were further cultured in an LB liquid medium containing 50 µg/ml carbenicillin for about 16 hours. Plasmids were purified from the cultured *E. coli* cells using QIAprep Spin Miniprep Kit (Qiagen) (pCR-BluntII-*Nhe*I-LTK-*Not*I).

pCR-BluntII-*Not*I-Kozak-LTK-*Nhe*I (1 µg equivalent) was digested with restriction enzymes *Hind*III and *Nhe*I at 37°C for about 3 hours. Separately, pCR-BluntII-*Nhe*I-LTK-*Not*I (1 µg equivalent) was digested with restriction enzymes *Nhe*I and *Not*I at 37°C for 2 hours. After the enzyme treatment, the reaction solution was separated via 1% agarose gel electrophoresis, and a cleavage fragment of SEQ ID NO: 182 or SEQ ID NO: 186 was cleaved from the gel and purified using NucleoSpin Gel and PCR Clean-up^{®} (MACHEREY-NAGEL, Takara Bio Inc.).

Separately, the Mammalian PowerExpress System vector pEHX1.2^{®} (Toyobo Co. Ltd.) used as a host vector was digested with restriction enzymes *Hind*III and *Not*I at 37°C for about 2 hours, and a fragment on the vector side was purified in the same manner as described above.

Thereafter, enzyme-treated fragments of the sequences of SEQ ID NO: 182 and SEQ ID NO: 186, and pEHX1.2 were ligated using DNA Ligation Kit (Mighty Mix, Takara Bio Inc.). *E. coli* DH5α (Toyobo Co. Ltd.) cells were transformed using the ligated cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and a plasmid in which target nucleotide sequence insertion was observed was obtained as an LTK-expressing plasmid (pEHX-LTK). Nucleotide sequence analysis was commissioned to Eurofins Genomics K.K.

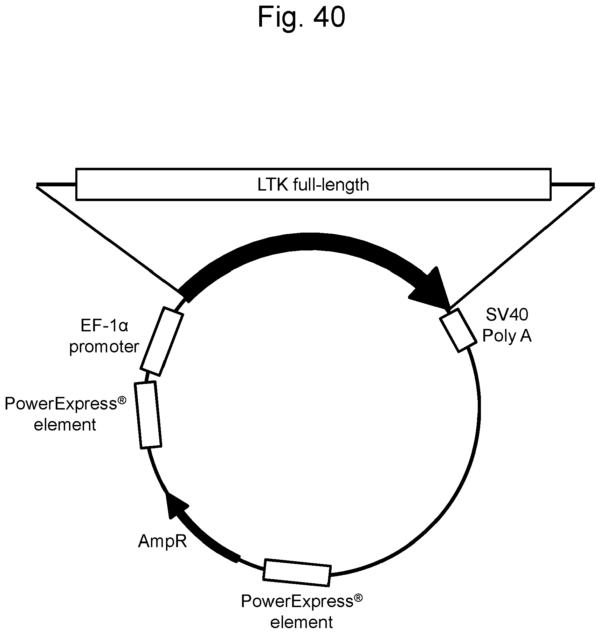

Fig. 40 shows a vector map of pEHX-LTK, and the DNA sequence of a translation region of pEHX-LTK is represented by SEQ ID NO: 187.

### Example 18: Isolation of ALK- or LTK-expressing stable CHO-K1 cells

### Transfection into CHO-K1 cells

The 2 types of plasmids prepared in Example 17 (pEHX-ALK and pEHX-LTK) were introduced into CHO-K1 cells using Lipofectamine 3000 Transfection Reagent^{®} (Thermo Fisher Scientific). Specifically, CHO-K1 cells were seeded into 4 wells of a 12-well treated culture plate at 2 × 10⁵ cells·2 ml/well on the day before gene transfection (Day -1). Ham's F-12 medium with L-glutamine and phenol red (FUJIFILM Wako Pure Chemical Corporation) containing penicillin-streptomycin (FUJIFILM Wako Pure Chemical Corporation) and 10% FBS (GE Healthcare Japan) was used. On the following day (Day 0), a mixture of the plasmid solution (1 µg equivalent), 2 µl of P3000 Reagent (Thermo Fisher Scientific), and 50 µl of Opti-MEM I Reduced-Serum Medium (Thermo Fisher Scientific) was added to a mixture of 3 µl of Lipofectamine 3000 Reagent and 50 µl of the Opti-MEM I Reduced-Serum Medium, and the mixture was subjected to the reaction at room temperature for 15 minutes. Thereafter, the reaction solution was added to each well of the 12-well treated culture plate for gene transfection.

### Drug selection and cloning of transfected CHO-K1 cells

The cells were collected using 0.25 w/v% trypsin-1 mmol/1 EDTA·4Na solution (FUJIFILM Wako Pure Chemical Corporation) 24 hours after gene transfection (Day 1), and the CHO-K1 cells collected from 2 wells were transferred to a 100-mm dish. In addition, puromycin dihydrochloride (Thermo Fisher Scientific) was added at 10 µg/ml, the medium was exchanged with a fresh medium every 3 or 4 days, and culture was continued up to Day 12. On Day 12, the resultant was seeded on a 96-well treated plate at 1 cell/well. The cells were transferred to a 24-well treated plate on Day 29 for expansion culture.

### Screening of cloned cells based on mRNA expression level

Some cells were collected on Day 33, and RNA was extracted using RNeasy Mini Kit (Qiagen). The extracted RNA was subjected to reverse transcription using PrimeScript RT Master Mix (Perfect Real Time, Takara Bio Inc.) to prepare cDNA. Using the resulting cDNA as a template, quantitative PCR was performed by 7500 Fast real-time PCR System (Thermo Fisher Scientific) with the use of TB Green Premix Ex Taq II (Takara Bio Inc.) in combination with primers for ALK amplification (SEQ ID NO: 188 and SEQ ID NO: 189) or primers for LTK amplification (SEQ ID NO: 190 and SEQ ID NO: 191). The mRNA expression level in quantitative PCR was determined by correcting the number of cycles (Ct value) using 18S ribosomal RNA as the internal reference gene, and clones having many copies of mRNA were selected based on the results of relative quantification of ALK and LTK.

### Confirmation of ALK and LTK expression on selected clone cell surfaces

There is no adequate fluorescence-labeled antibody that specifically detects the extracellular domain of ALK or LTK. Thus, flow cytometric analysis was performed using binding affinity of ALK or LTK to its ligand FAM150B. Specifically, a suspension of 0.6 to 1.8 × 10⁶ cells was centrifuged to remove the supernatant, the FAM150B-His protein prepared in Example 5 (about 0.2 to 0.3 µmol equivalent) was added, and the resultant was incubated at 37°C for 30 minutes. Thereafter, the product was washed with 1 ml of D-PBS containing 1% FBS, centrifuged to remove the supernatant, and then subjected to the same washing procedure 3 times. Thereafter, 5 µl of PE (phycoerythrin) anti-His tag antibody (BioLegend) was added, and the resultant was incubated at 4°C under shading conditions for 20 minutes. Thereafter, the resultant was washed with 1 ml of D-PBS containing 1% FBS and centrifuged to remove the supernatant. The sample re-suspended in 500 µl of D-PBS containing 1% FBS was assayed using BD Accuri C6 Plus (BD Biosciences), and the obtained data were analyzed using FlowJo (BD Biosciences).

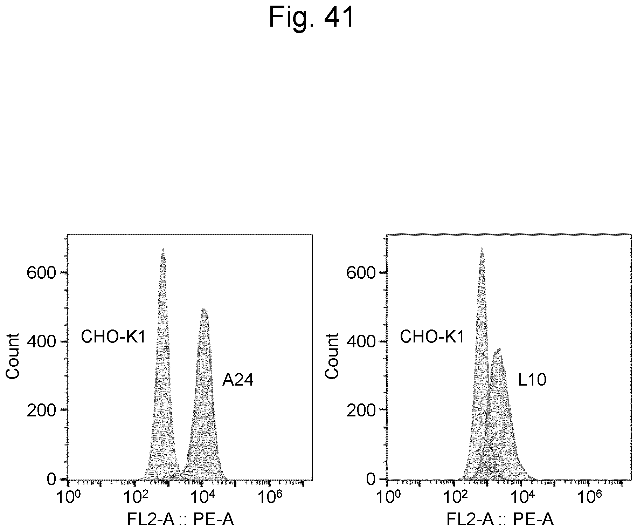

Fig. 41 shows the results of flow cytometric analysis using binding affinity of clones to the FAM150B protein. PE fluorescence intensity exhibited by the clone comprising ALK gene introduced thereinto (A24) and the clone comprising LTK gene introduced thereinto (L10) is shifted significantly to the right, compared with the histogram of the control group (CHO-K1). It was thus demonstrated that A24 expresses ALK and L10 expresses LTK constitutionally on their cell surfaces.

### Example 19: Preparation of FAM150B truncated CH2CH3-deletion CD28-type CAR-expressing plasmid

CAR 008 prepared in Example 7 was used as a template, a FAM150B truncated CH2CH3-deletion CD28-type CAR-expressing plasmid (CAR 021) was prepared by inverse-PCR. The forward primer (SEQ ID NO: 171) and the reverse primer (SEQ ID NO: 172) were used for inverse-PCR. CAR 008 was adjusted to 50 ng/µl and used as a template, and the concentration of each PCR primer was adjusted to 0.2 µM in the reaction solution. Inverse-PCR was performed using KOD-Plus-Mutagenesis Kit (Toyobo Co. Ltd.) with the reaction composition designated in the instructions of the kit and with a cycle consisting of (i) 94°C for 2 minutes, (ii) 98°C for 10 seconds, and (iii) 68°C for 7 minutes, and a cycle of steps (ii) and (iii) was repeated 10 times.

After the PCR reaction, an aliquot of the sample was separated via 1% agarose gel electrophoresis, amplification of the linear plasmid of the size of interest was confirmed, the remaining sample after PCR was treated with *Dpn*I in accordance with the instructions of the kit, and methylated template plasmid CAR 008 was cleaved and removed. Thereafter, the linear plasmid was phosphorylated with T4 Polynucleotide Kinase included in the kit and self-ligated to form a cyclic plasmid. Thereafter, *E. coli* DH5α (Toyobo Co. Ltd.) cells were transformed using the cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and a plasmid in which target nucleotide sequence deletion was observed was obtained as a FAM150B truncated CH2CH3-deletion CD28-type CAR-expressing plasmid (CAR 021; FAM150BTr-28z dCH2CH3).

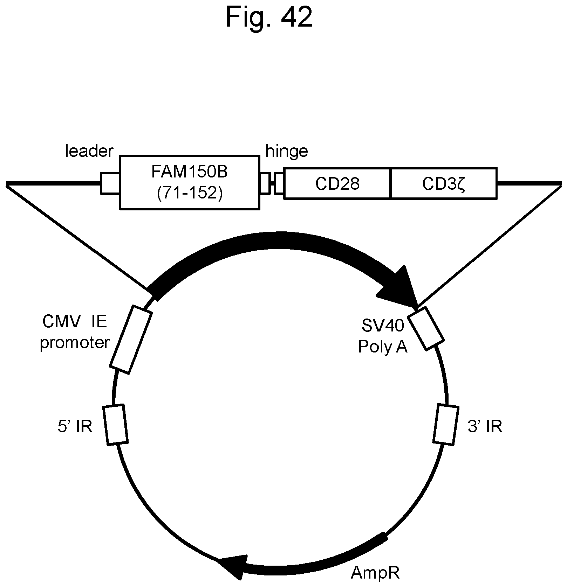

Fig. 42 shows a vector map of CAR 021 (FAM150BTr-28z dCH2CH3), and the DNA sequence of a translation region of CAR 021 is represented by SEQ ID NO: 192.

### Example 20: Preparation of mouse ALK48 scFv-type CD28-type CH2CH3-deletion CAR-expressing plasmid (CAR 022; ALK48-28z dCH2CH3)

CAR 021 prepared in Example 19 (about 1 µg equivalent) was digested with restriction enzymes *Xho*I and *Dra*III (New England Biolabs) at 37°C for about 2 hours. Also, the pEX-K4J1 vector (about 1 µg equivalent) comprising the sequence of SEQ ID NO: 8, which was designed and artificially synthesized in Example 1, incorporated thereinto was digested with restriction enzymes *Xho*I and *Dra*III at 37°C for about 2 hours.

After the enzyme treatment, the reaction solution was separated via 1% or 2% agarose gel electrophoresis, the enzyme-treated CAR 021 fragment (on the vector side) and the artificially synthesized gene-inserted fragment (SEQ ID NO: 8) cleaved from the pEX-K4J1 vector were removed from the gel, and the fragments were purified using NucleoSpin Gel and PCR Clean-up^{®} (MACHEREY-NAGEL, Takara Bio Inc.). The purified vector fragment was ligated to the purified insert fragment using the DNA ligation kit (Mighty Mix, Takara Bio Inc.). Thereafter, *E. coli* DH5α (Toyobo Co. Ltd.) cells were transformed using the ligated cyclic plasmid and then cultured on an LB agar medium containing 100 µg/ml carbenicillin for about 16 hours.

The appeared colonies were further cultured in an LB liquid medium containing 100 µg/ml carbenicillin for about 16 hours. Plasmids were purified from the cultured *E. coli* cells using QIAprep Spin Miniprep Kit (Qiagen), nucleotide sequences were determined, and a plasmid in which target nucleotide sequence insertion was observed was obtained as a mouse ALK48 scFv-type CD28-type CH2CH3-deletion CAR-expressing plasmid (CAR 022; ALK48-28z dCH2CH3). Nucleotide sequence analysis was commissioned to Eurofins Genomics K.K.

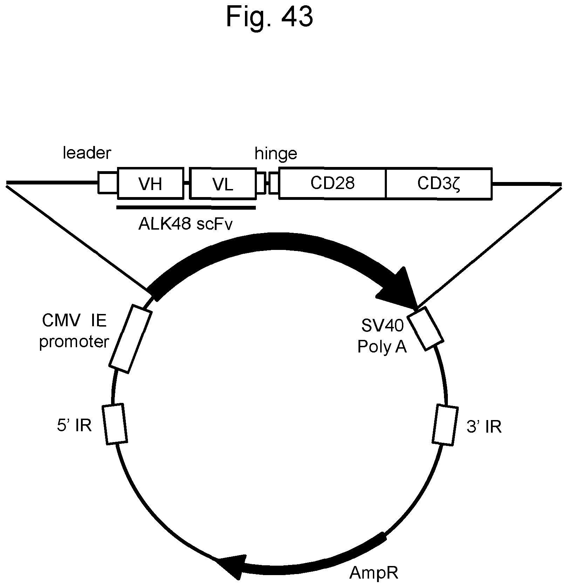

Fig. 43 shows a vector map of CAR 022 (ALK48 scFv-28z dCH2CH3), and the DNA sequence of a translation region of CAR 022 is represented by SEQ ID NO: 193.

### Example 21: Culture and proliferation of CAR-T cells

In accordance with the method of CAR-T cell culture and proliferation in Example 2, CAR-T cells were prepared using the CAR 021- or CAR 022-expressing plasmid prepared in Examples 19 and 20.

### Day 16: Evaluation of CAR expression rate

The number of CAR-T cells into which the CAR 021- or CAR 022-expressing plasmid had been introduced was counted, and 1 to 2 × 10⁵ cells were subjected to flow cytometric analysis to evaluate the CAR expression rates in the ALK CAR-T cells.

1 to 2 × 10⁵ cells were collected and washed with 1 ml of D-PBS containing 1% FBS, 100 µl of ALK 001P prepared in Example 5 was added, and the resultant was incubated at 37°C for 30 minutes. Thereafter, the cells were washed with 1 ml of D-PBS containing 1% FBS, centrifuged to remove the supernatant, and then subjected to the same washing procedure 3 times. Thereafter, 5 µl of PE anti-His tag antibody (BioLegend) and 5 µl of APC Anti-Human CD3 antibody (Miltenyi Biotec) were added to prepare a suspension, and the antibody labeling reaction was conducted at 4°C under shading conditions for 20 minutes. Thereafter, the cells were washed with 1 ml of D-PBS containing 1% FBS, and precipitated by centrifugation to remove the supernatant. Thereafter, the sample re-suspended in 500 µl of D-PBS containing 1% FBS was assayed using BD Accuri C6 Plus (BD Biosciences) to determine CAR 021 or CAR 022 positive rate.

The CAR-T cells obtained by gene introduction through electrical pulses into PBMCs using CAR 021 or CAR 022 and T cell culture and proliferation are described herein as follows.
CAR-T 021: FAM150B CD28-type CH2CH3-deletion CAR-T (FAM150B-28z dCH2CH3 CAR-T)
CAR-T 022: Mouse CD28-type CH2CH3-deletion CAR-T (ALK scFv-28z dCH2CH3 CAR-T)

The amino acid sequence of CAR 021 and the nucleotide sequence encoding the same are represented by SEQ ID NO: 194 and SEQ ID NO: 192, respectively.

The amino acid sequence of CAR 022 and the nucleotide sequence encoding the same are represented by SEQ ID NO: 195 and SEQ ID NO: 193, respectively.

Table 8 shows CAR expression rates (%) in CAR-T cells obtained from PBMCs derived from a healthy adult donor.

**[Table 8]**

| Donor | Plasmid | CAR-T | Expression rate |
|---|---|---|---|
| Donor-1 | CAR 021 | CAR-T 021 | 24.1 |
| | CAR 022 | CAR-T 022 | 24.4 |

### Example 22: Evaluation of cytotoxic activity of CAR-T 021 or CAR-T 022

The clones A24 and L10 selected in Example 18 and CHO-K1 cells were seeded as target (T) cells at 5,000 cells/well on E-Plate VIEW (ACEA Biosciences). The plate was mounted on xCELLigence DP (ACEA Biosciences), and about 24 hours later, CAR-T 021, CAR-T 022 and mock-T cells (T cells into which no genes had been introduced) were added as effector (E) cells at the E:T ratios of 40:1 (200,000 cells:5,000 cells), 20:1 (100,000 cells:5,000 cells), and 10:1 (50,000 cells:5,000 cells). About 48 hours after the addition of effector cells, the cell index; i.e., transition in the number of adhesive cells, was evaluated. Evaluation was performed using Ham's F-12 medium with L-glutamine and phenol red (FUJIFILM Wako Pure Chemical Corporation) containing penicillin-streptomycin (FUJIFILM Wako Pure Chemical Corporation) and 10% FBS (GE Healthcare Japan).

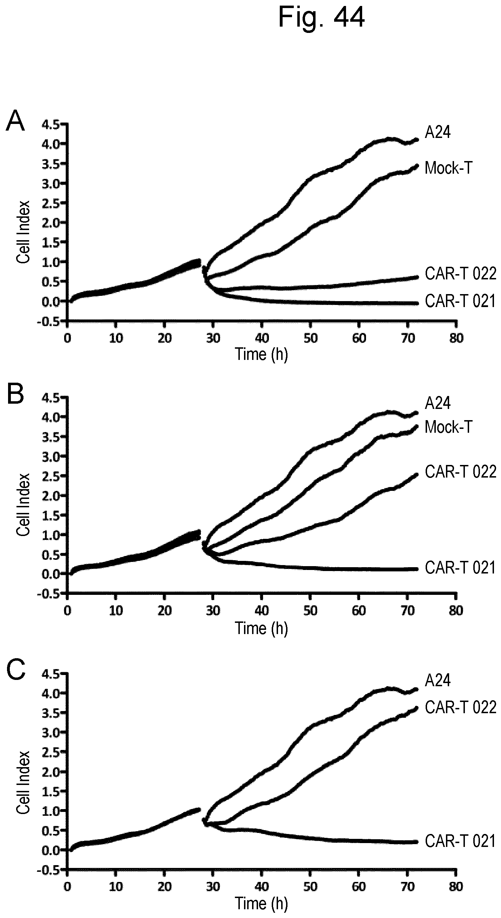

Fig. 44 shows transitions in the cell index over time when CAR-T 021, CAR-T 022, or mock-T cells are added to A24 cells expressing high levels of ALK. While CAR-T 021 and CAR-T 022 significantly lowered the cell index of A24 cells in an E:T ratio-dependent manner, mock-T cells did not affect the cell index of A24. The results demonstrate that CAR-T 021 and CAR-T 022 have specific cytotoxic activity against cells expressing high levels of ALK and that cytotoxic activity of CAR-T 021 is higher.

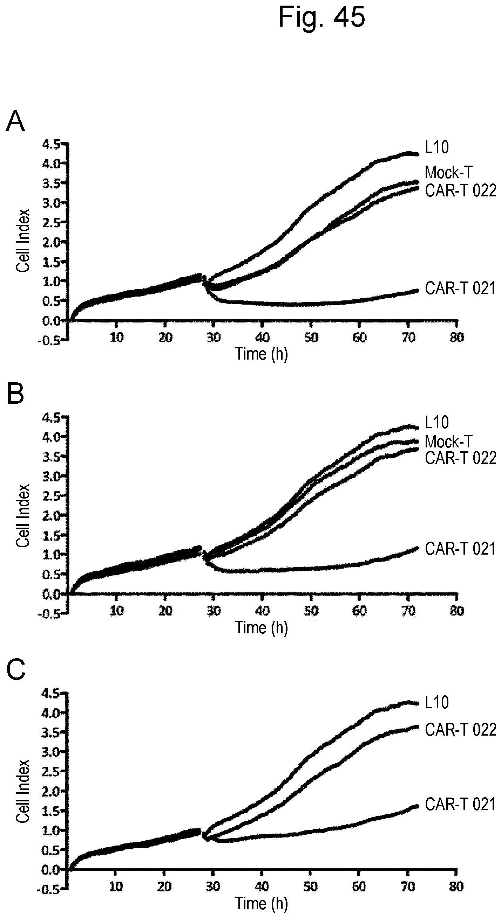

Fig. 45 shows transitions in the cell index over time when CAR-T 021, CAR-T 022, or mock-T cells are added to L10 cells expressing high levels of LTK. While CAR-T 021 significantly lowered the cell index of L10 cells in an E:T ratio-dependent manner, CAR-T 022 and mock-T cells did not affect the cell index of L10. The results demonstrate that CAR-T 021 has specific cytotoxic activity against cells expressing high levels of LTK.

In this example, FAM150B CAR-T was found to specifically recognize both the cells expressing high levels of ALK and the cells expressing high levels of LTK and exert cytotoxic activity thereon because of the ligand-type properties. In contrast, ALK scFv-type CAR-T was found to selectively act on the cells expressing high levels of ALK.

In this example, in addition, antitumor activity of FAM150B truncated CH2CH3-deletion CAR-T against neuroblastoma cell line and breast cancer cell line was found to be substantially equivalent to that of an FAM150B truncated form having CH2CH3.

All publications, patents, and patent applications cited herein are incorporated herein by reference in their entirety.

## Claims

1. A polynucleotide encoding a chimeric antigen receptor (CAR) protein comprising a target binding domain that binds to an extracellular ligand binding region of anaplastic lymphoma kinase (ALK), a transmembrane domain, and an intracellular signaling domain, wherein the target binding domain is selected from among FAM150A, FAM150B, and fragments thereof binding to the extracellular ligand binding region of ALK.

2. The polynucleotide according to Claim 1, wherein the target binding domain is a truncated fragment of FAM150A and/or FAM150B.

3. The polynucleotide according to Claim 1, wherein the target binding domain is a polypeptide consisting of an amino acid sequence selected from the group consisting of SEQ ID NO: 154 (FAM150A), SEQ ID NO: 146 (TrFAM150A), SEQ ID NO: 155 (FAM150B), and SEQ ID NO: 148 (TrFAM150B).

4. The polynucleotide according to Claim 1, wherein the target binding domain is a polypeptide consisting of an amino acid sequence having 90% or higher sequence identity to the amino acid sequence represented by SEQ ID NO: 146 (TrFAM150A) or SEQ ID NO: 148 (TrFAM150B).

5. A vector comprising the polynucleotide according to any one of Claims 1 to 4.

6. A genetically modified cell comprising the polynucleotide according to any one of Claims 1 to 4 or the vector according to Claim 5 introduced thereinto.

7. The cell according to Claim 6, which expresses a CAR protein binding to an ALK-expressing cell on a cell membrane.

8. A method for preparing a CAR protein-expressing cell comprising introducing the polynucleotide according to any one of Claims 1 to 4 or the vector according to Claim 5 into a cell.

9. The method according to Claim 8, wherein the polynucleotide or the vector is introduced into the cell by the transposon method.

10. The method according to Claim 9, wherein the transposon method is the piggyBac method.

11. A kit comprising the vector according to Claim 5 used for preparing a CAR protein-expressing cell targeting an ALK-expressing cell.

12. A therapeutic agent for a disease associated with an ALK-expressing cell comprising the cell according to Claim 6 or 7.

13. A pharmaceutical composition comprising the therapeutic agent according to Claim 12 and a pharmaceutically acceptable carrier.

14. The therapeutic agent according to Claim 12 or the composition according to Claim 13, wherein the disease associated with an ALK-expressing cell is a solid tumor selected from among neuroblastoma, breast cancer, uterine cancer, endometrial cancer, ovarian cancer, melanoma, astroglioma, Ewing's sarcoma, glioblastoma, retinoblastoma, rhabdomyoblastoma, non-small cell lung cancer, prostate cancer, and urothelial cancer.
